# EUROPEAN PATENT SPECIFICATION

(11) **EP 3 765 101 B1**
(45) Date of publication and mention of the grant of the patent: **15.10.2025**
(21) Application number: 19716479.1
(22) Date of filing: 14.03.2019
(51) Int. Cl.: A61L 26/00

(54) **BETA-SOLENOID PROTEINS AND THEIR APPLICATION IN TEXTILE PRODUCTION**
BETA-SOLENOID-PROTEINE UND IHRE ANWENDUNG IN DER TEXTILHERSTELLUNG
PROTÉINES BÉTA-SOLÉNOIDES ET LEUR APPLICATION DANS LA PRODUCTION DE TEXTILES

(30) Priority: 14.03.2018 GB 201804092
(43) Date of publication of application: 20.01.2021
(60) Divisional application: 25200880.0
(73) Proprietor: Imperial College Innovations Limited, London SW7 2AZ (GB)
(72) Inventor: SHAFFER, Milo, London SW7 2AX (GB); MACDONALD, James T., London SW7 2AX (GB); FREEMONT, Paul S., London SW7 2AX (GB); LEESE, Hannah, London SW7 2AX (GB)
(74) Representative: Potter Clarkson
(86) International application number: PCT/GB2019/050720
(87) International publication number: WO 2019/175591

(56) References cited:
- MARIA D.R. PERALTA ET AL: "Engineering Amyloid Fibrils from &bgr;-Solenoid Proteins for Biomaterials Applications", ACS NANO, vol. 9, no. 1, 27 January 2015 (2015-01-27), pages 449 - 463, XP055215096, ISSN: 1936-0851, DOI: 10.1021/nn5056089
- AMANDA S PARKER ET AL: "Molecular Dynamics-Based Strength Estimates of Beta-Solenoid Proteins", ARXIV.ORG, CORNELL UNIVERSITY LIBRARY, 201 OLIN LIBRARY CORNELL UNIVERSITY ITHACA, NY 14853, 17 June 2017 (2017-06-17), XP080770481
- JAMES T. MACDONALD ET AL: "Synthetic beta-solenoid proteins with the fragment-free computational design of a beta-hairpin extension", PNAS, vol. 113, no. 37, 29 August 2016 (2016-08-29), US, pages 10346 - 10351, XP055586100, ISSN: 0027-8424, DOI: 10.1073/pnas.1525308113
- JAMES T MACDONALD ET AL: "Supplementary Information: Synthetic beta-solenoid proteins with the fragment-free computational design of a beta-hairpin extension", 18 August 2016 (2016-08-18), XP055600817, Retrieved from the Internet <URL:www.pnas.org/content/113/37/10346> [retrieved on 20190628], DOI: 10.1073/pnas.1525308113
- PENG ZEYU ET AL: "High Tensile Strength of Engineered[beta]-Solenoid Fibrils via Sonication and Pulling", BIOPHYSICAL JOURNAL, vol. 113, no. 9, 7 November 2017 (2017-11-07), pages 1945 - 1955, XP085284483, ISSN: 0006-3495, DOI: 10.1016/J.BPJ.2017.09.003
- AMANDA PARKER ET AL: "Molecular dynamics-based strength estimates of beta solenoid proteins", SOFT MATTER, vol. 13, no. 36, 1 January 2017 (2017-01-01), pages 6218 - 6226, XP055601356, ISSN: 1744-683X, DOI: 10.1039/C7SM01070B
- LEONARD P. HEINZ ET AL: "In Silico Measurements of Twist and Bend Moduli for [beta]-Solenoid Protein Self-Assembly Units", NANO LETTERS, vol. 15, no. 5, 30 April 2015 (2015-04-30), US, pages 3035 - 3040, XP055601129, ISSN: 1530-6984, DOI: 10.1021/nl5049719
- "ADVANCES IN PROTEIN CHEMISTRY.", vol. 73, 1 January 2006, ACADEMIC PRESS, NEW YORK, NY., US, ISSN: 0065-3233, article ANDREY V. KAJAVA ET AL: "[beta]-Rolls, [beta]-Helices, and Other [beta]-Solenoid Proteins", pages: 55 - 96, XP055600705, DOI: 10.1016/S0065-3233(06)73003-0

## Description

### Field of the invention

The present invention relates to the field of synthetic fibres.

### Background

High performance synthetic fibres, such as aramids, typically have high tensile strength (2-6 GPa) and stiffness (50-300 GPa) but low ductility, typically extending less than 5% in length before failure. These synthetic fibres are widely used in a variety of applications, as unidirectional tows, weaves, felts, and knits, either as a fabric or as a composite. Their ability to absorb impact energy, including as flexible textiles, makes them attractive in protection applications. However, their low breathability and low thermal conductivity make them uncomfortable to wear, whilst their tendency to degrade in the presence of water, heat and light reduces service lifetime particularly after washing.

In contrast, cellulosic- and protein-based natural fibres are generally comfortable to wear next to the skin due to their high water absorbency and breathability but typically have lower tensile performance than synthetic fibres. Natural silk fibres can combine both high tensile strength and comfort but these protein-based materials are difficult to produce at high molecular weight in recombinant expression systems and it is difficult to recapitulate the natural spinning mechanism. Both the molecular weight of the polymers and the spinning process are critical to achieving high performance fibres.

High molecular weight rigid polymers generally have low solubility and high viscosity. However, liquid crystal polymers (e.g. aramids) in the nematic phase behave as non-Newtonian fluids and have decreased viscosity under shear strain, and an intrinsically greater tendency to align, aiding the spinning process. The ordering of polymers in a fibre is an important determining factor of their mechanical properties. For these reasons, high performance synthetic fibres, such aramids and other liquid crystal polymers, are spun from dope solutions in the nematic phase (although usually from challenging solvents such as superacids). Similarly, it is thought that natural silk is spun from a highly concentrated aqueous dope solution in the nematic phase, having been produced by the arthropod's silk glands.

Many materials in nature (including bone, nacre and silks) use the principles of hidden length and sacrificial bonds to simultaneously increase toughness and stiffness by providing a mechanism for energy dissipation (Fantner *et al* DOI: 10.1529/biophysj.105.069344). In this invention, we present a general approach to designing artificial proteins that simultaneously exhibit liquid crystalline behaviour and incorporate a high degree of hidden length. We then show that these proteins can then be spun from highly concentrate dope solutions into continuous fibres.

The fibres of the present invention can able be produced through recombinant protein expression technology, circumventing many of the problems associated with the production of such advantageous fibres.

Despite advances in synthetic textiles, there is still a need for the sustainable and commercially viable production of fibres that combine the desirable properties of natural fibres (breathable and water absorbent) with the high performance aspects of synthetic fibres. The present invention addresses this objective through the production and subsequent spinning of entirely synthetically designed proteins.

MacDonald et al 2016 PNAS 113: 10346-10351 describes the computational design og synthetic beta-solenoid proteins.

Peng et al 2017 Biophysical Journal 113: 1945-1955 discusses the high tensile strength of engineered beta solenoid fibrils via sonication and pulling.

Parker et al 2017 Soft Matter 13: 6218-6226 describes the molecular dynamics-based strength estimates of beta solenoid proteins.

Heinz et al 2015 Nano Letters 15: 3035-3040 describes in silico measurements of twist and end moduli for beta solenoid protein self-assembly units.

Kajava et al 2006 Advances In Protein Chemistry 73: 55-96 discusses beta rolls, beta helices and other beta solenoid proteins.

### Summary of the invention

The invention is set out in the appended set of claims. The claims relate to a concatemer of beta solenoid domains, beta solenoid domains, a composition comprising the concatemer of beta solenoid domains, nucleic acid encoding said concatemer, and fibres and textiles comprising the concatemer for a number of applications.

The invention provides:
A concatemer of beta solenoid domains, wherein the concatemer comprises at least two beta solenoid domains,
wherein the at least two beta solenoid domains are linked by a linker, and
wherein each of the at least two beta solenoid domains comprise an amino acid sequence that comprises at least 4 units of a consensus motif, and
wherein the concatemer has a molecular weight of at least 30 kDa,
wherein each unit within one of the beta solenoid domains conforms to the following consensus sequence:
   A X₁ L/F X₂ X₃ [SEQ ID NO: 147]
   wherein X₁ is A, C, D, E, I, K, L, M, N, R, S, T or V
   X₂ is A, C, D, E, F, G, H, I, K, N, Q, R, S, T, V, W, or Y
   X₃ is A, C, D, E, G, H, I, K, N, Q, R, S ,Y.

The invention also provides:
A beta solenoid domain that comprises an amino acid sequence that comprises at least 4 units of the following consensus motif:
   i) A X₁ L/F X₂ X₃ [SEQ ID NO: 147]
      wherein X₁ is A, C, D, E, I, K, L, M, N, R, S, T or V
      X₂ is A, C, D, E, F, G, H, I, K, N, Q, R, S, T, V, W, or Y
      X₃ is A, C, D, E, G, H, I, K, N, Q, R, S ,Y;
      wherein the beta solenoid domain does not consist of any of the sequences of SEQ ID NO: 1, 34, 36, 38, 40, 42, 44, 46, 48, 50, 52, 54, 56, 58, 60, 62; and/or SEQ ID NO: 66-80
And wherein the beta solenoid domain:
   a) has a longitudinal axis and a transverse axis and the ratio of the longitudinal axis to transverse axis is at least 2:1;
   b) comprises at least 29 units;
   c) has a molecular weight of at least 60KDa;
   d) is soluble at a concentration of at least 20 mg/ml.
The invention also provides a composition comprising a concatemer of the invention or a beta solenoid domain of the invention, optionally wherein the concatemer or beta solenoid domain is at a concentration of at least 20 mg/ml, optionally at least 30 mg/ml, optionally at least 40 mg/ml, optionally at least 50 mg/ml, optionally at least 60 mg/ml, optionally at least 70 mg/ml, optionally at least 80 mg/ml, optionally at least 90 mg/ml, optionally at least 100 mg/ml, optionally at least 120 mg/ml, optionally at least 130 mg/ml, optionally at least 150 mg/ml, optionally at least 170 mg/ml, optionally at least 190 mg/ml, optionally at least 200 mg/ml, optionally at least 250 mg/ml, optionally at least 300 mg/ml, optionally at least 350 mg/ml, optionally at least 400 mg/ml.
The invention further provides: a) a nucleic acid encoding the concatemer of the invention or the beta solenoid domain of the invention; or a vector comprising the nucleic acid encoding the concatemer of the invention or the beta solenoid domain of the invention;
   b) a host cell comprising the nucleic acid of the invention and/or the vector, optionally wherein the host cell is
   a) a prokaryotic cell,
      optionally a bacterial cell, optionally an *E. coli* cell, a *Bacillus subtilis* cell, a
      *Bacillus megaterium* cell, a *Vibrio natriegens* cell, or a *Pseudomonas fluorescens* cell; and provides
   b) is a eukaryotic cell, optionally
      a yeast cell, optionally *Pichia pastoris* or Saccharomyces cerevisiae;
      an insect cell, optionally a baculovirus infected insect cell; or
      a mammalian cell, optionally a baculovirus infected mammalian cell, a HEK293 cell, a HeLa cell, or CHO cells.

The invention also provides a fibre comprising the concatemer of the invention or the beta solenoid domain of the invention optionally wherein the fibre has:
a) been produced via a wet-spinning method; and/or
b) an average diameter of between 100 nm and 100um, optionally between 500 nm and 50um, optionally between 1000nm and 40um, optionally between 2.5um and 35 um, optionally between 5 um and 30 um, optionally between 7.5 um and 25 um, optionally between 10 um and 20 um; and/or
c) a Young's modulus of between 0.5 GPa and 200 Gpa, optionally between 1.0 Gpa and 175 Gpa, optionally between 2 Gpa and 150 Gpa, optionally between 5.0 Gpa and 125 Gpa, optionally between 10.0 Gpa and 100.0 Gpa, optionally between 15.0 Gpa and 90.0 Gpa, optionally between 20.0 Gpa and 80.0 Gpa, optionally between 25.0 Gpa and 75.0 Gpa, optionally between 30.0 Gpa and 70.0 Gpa, optionally between 35.0 Gpa and 60.0 Gpa, optionally between 40.0 GPa and 50.0 GPa; and/or
   a Young's modulus of at least 0.5 GPa, 1.0 GPa, 2 GPa, 5.0 GPa, 10.0 GPa, 15.0 GPa, 20.0 GPa, 25.0 GPa, 30.0GPa, 35.0 GPa, 40.0 GPa, 50.0 GPa, 60.0 GPa, 70.0 GPa, 75.0 GPa, 80.0 GPa, 90.0 GPa, 100.0 GPa, 125 GPa, 150 GPa, 175 GPa or at least 200 GPa; and/or
d) an engineered strength of:
   between 0.1 GPa and 3 GPa, optionally between 0.2 GPa and 2.8 GPa, optionally between 0.3 GPa and 2.6 GPa, optionally between 0.4 GPa and 2.4 GPa,
   optionally between 0.5 GPa and 2.2 GPa, optionally between 0.6 GPa and 2.0 GPa, optionally between 0.7 GPa and 1.8 GPa, optionally between 0.8 GPa and 1.6 GPa, optionally between 0.9 GPa and 1.4 GPa, optionally between 1.0 GPa and 1.2 GPa; and/or
   at least 0.1 GPa, optionally at least 0.2 GPa, optionally at least 0.3 GPa, optionally at least 0.4 GPa, optionally at least 0.5 GPa, optionally at least 0.6 GPa, optionally at least 0.7 GPa, optionally at least 0.8 GPa, optionally at least 0.9 GPa, optionally at least 1.0 GPa, optionally at least 1.1 GPa, optionally at least 1.2 GPa, optionally at least 1.3 GPa, optionally at least 1.4 GPa, optionally at least 1.5 GPa, optionally at least 1.6 GPa, optionally at least 1.7 GPa, optionally at least 1.8 GPa, optionally at least 1.9 GPa, optionally at least 2.0 GPa, optionally at least 2.1 GPa, optionally at least 2.2 GPa, optionally at least 2.3 GPa, optionally at least 2.4 GPa, optionally at least 2.5 GPa, optionally at least 2.6 GPa, optionally at least 2.7 GPa, optionally at least 2.8 GPa, optionally at least 2.9 GPa, optionally at least 3.0 GPa; and/or
e) a strain to failure of between 2% and 300%, optionally between 5% and 275%, optionally between 10% and 250%, optionally between 15% and 225%, optionally between 20% and 200%, optionally between 25% and 175%, optionally between 30% and 150%, optionally between 35% and 125%, optionally between 40% and 100%, optionally between 45% and 80%, optionally between 50% and 75%, optionally between 55% and 70%; and/or
   a strain to failure of at least 2% , 5%, 10%, 15%, 20%, 25%, 30%, 35%, 40%, 45%, 50%, 55%, 60%, 65%, 70%, 75%, 80%, 90%, 100%, 125%, 150%, 175%, 200%, 225%, 250%, 275% or at least 300%; and/or
f) a toughness of at least 10 J/g, optionally at least 20 J/g, optionally at least 40 J/g, optionally at least 60 J/g, optionally at least 80 J/g, optionally at least 100 J/g, optionally at least 120 J/g, optionally at least 140 J/g, optionally at least 160 J/g, optionally at least 180 J/g, optionally at least 200 J/g, optionally at least 250 J/g, optionally at least 300 J/g, optionally at least 350 J/g, optionally at least 400 J/g, optionally at least 450 J/g, optionally at least 500 J/g, optionally at least 550 J/g, optionally at least 600 J/g, optionally at least 650 J/g, optionally at least 700 J/g, optionally at least 750 J/g, optionally at least 800 J/g, optionally at least 850 J/g, optionally at least 900 J/g, optionally at least 950 J/g, optionally at least 1000 J/g.

The invention also provides a textile comprising the concatemer of the invention, the beta solenoid domain of the invention, or the fibre according the invention.

The invention also provides sportswear (e.g. for protective uses such as rash guards); Protective wear for motorcyclists and/or other motorsports; Personal protective equipment (e.g. body armour, protective underwear for soldiers); Blast containment; Blade containment (e.g. for aircraft engines); Textiles for parachutes; Ropes; Textiles for clothing; Textiles for shoes; general medical textiles; Wound dressings; Resorbable and non-resorbable surgical sutures; Resorbable and non-resorbable stents; Guide-wires for surgery (including non-metallic guide wires suitable for MRI); Biocompatible coatings for implanted medical devices; Surgical meshes; or Scaffolds for 3D tissue engineering comprising a concatemer of the invention, a beta solenoid domain of the invention, a fibre according to the invention or a textile of the invention.

The inventors have devised synthetic rod-shaped protein domains with a high degree of hidden length that form liquid crystalline states at sufficient concentrations. The inventors have subsequently developed entirely synthetic high molecular weight proteins that can be spun from highly concentrated nematic phase aqueous dopes solutions into tough continuous fibres but that can be manufactured in a sustainable way using microbial recombinant expression systems, for example. This approach provides improved performance, secure supply, and opportunities to develop new functionalities (such as self-reporting, biosensing, catalytic or biological activity, and signature management). Since these fibres are spun from proteins, they are expected to retain the desirable characteristics of natural silks, including breathability, dyability, washability, high crimp, and a degree of fire retardancy (no dripping/melting and high nitrogen content). Related requirements have stimulated numerous commercial activities synthesising artificial spiders silk (e.g. Bolt Threads https://boltthreads.com/ and Spiber https://www.spiber.jp/en/). We consider, however, that by stepping away from the direct biomimetic approach, the synthetic approach disclosed herein allows for improved processability, the preparation of superior fibres designed and customised for specific applications, rather than being limited to natural biological functionality.

The present inventors have developed a radically different approach to the creation of a new class of artificial peptide-based fibres from recombinant proteins. Instead of reusing natural sequences taken from spiders, or other species, completely artificial peptide sequences have been designed with entirely new structural motifs that maximise the hidden length and optimise the number of sacrificial bonds in the resulting polymer. This general molecular mechanism has been frequently exploited by nature, in other contexts, to produce materials of extraordinary toughness. Here, the hidden length concept has been applied to focus on the production of fibres of maximum toughness, without needing to address the multiple requirements of spiders or other arthropod species.

### Detailed description

The invention is as set out in the claims.

The present inventors have surprisingly found that concatemers of synthetic beta solenoid domains can be spun into novel fibres with useful properties as described above. In one embodiment the concatemers can be produced by microbial organisms, for example by *E. coli,* addressing many of the issues with the prior art fibres, for example those that rely on arthropods for their production.

Accordingly, one disclosure provides a concatemer of beta solenoid domains, wherein the concatemer comprises at least two beta solenoid domains,
wherein the at least two beta solenoid domains are linked by a linker, and
wherein each of the at least two beta solenoid domains comprises an amino acid sequence that comprises at least 4 units of a consensus motif. In the invention, the concatemer has a molecular weight of at least 30 kDa.

The beta solenoid domains that are concatenated in the concatemer are also the subject of the present invention, and preferences for both the concatemer of the invention and the beta solenoid domain of the invention are described below.

The skilled person will understand what is meant by the term "beta solenoid domain", see for example Kajava and Steven 2006 Adv Protein Chem.73:55-96 *Beta-rolls, beta-helices, and other beta-solenoid proteins.* The beta solenoid domains described herein are defined by the presence of at least 4 units of an appropriate consensus motif.

Solenoid protein domains are a highly modular. They consist of a chain of nearly identical folds, often simply called "repeats" and herein termed "units of a consensus motif" for clarity, since strictly the "repeats" do not have to be of identical sequence. The "repeats" or "units of a consensus motif" can be identical or near-identical.

Examples of types of solenoid domain include the linear (open) solenoids and the circular or closed solenoids. It is considered that in a preferred option the solenoid domain is a linear solenoid which forms a rod-shaped structure.

The beta solenoid domains according to the present invention are defined by the presence of at least 4 units of a consensus motif. By this we include the meaning that each unit conforms to a particular consensus motif, but each of the at least four units may have a different sequence. The skilled person will understand what is meant by the term "consensus motif" and how each of the units can conform to the consensus sequence yet still be different from one another. The skilled person will also understand which consensus motifs are suitable for use in the present invention and can be used to generate beta solenoid domains.

In one option, at least one of the beta solenoid domains, or both of the at least two beta solenoid domains, or all of the beta solenoid domains comprises any one or more of the following consensus sequences. In a preferred option, each unit within a single beta solenoid domain conforms to the same consensus sequence (without necessarily comprising the same actual sequence as discussed above). Preferably, each unit within each beta solenoid domains in the concatemer of the disclosure conforms to the same consensus sequence. However, concatemers of different beta solenoid domains are contemplated and included in the disclosure, for example a concatemer may comprise a beta solenoid that conforms to SEQ ID NO: 82 below and a beta solenoid domain that conforms to SEQ ID NO: 87 below. Examples of appropriate consensus sequences are provided below:
i) A X₁ L/F X₂ X₃ [SEQ ID NO: 147]
   wherein X₁ is any residue
   X₂ is any residue
   X₃ is any residue;
   optionally
   wherein X₁ is A, C, D, E, I, K, L, M, N, R, S, T or V
   X₂ is A, C, D, E, F, G, H, I, K, N, Q, R, S, T, V, W, or Y
   X₃ is A, C, D, E, G, H, I, K, N, Q, R, S ,Y; [SEQ ID NO: 148]
ii) A X₁ L X₂ X₃ [SEQ ID NO: 149]
   wherein X₁ is any residue
   X₂ is any residue
   X₃ is any residue;
   optionally
   wherein X₁ is A, C, D, E, I, K, L, M, N, R, S, T or V
   X₂ is A, C, D, E, F, G, H, I, K, N, Q, R, S, T, V, W, or Y
   X₃ is A, C, D, E, G, H, I, K, N, Q, R, S ,Y; [SEQ ID NO: 150]
iii) A (N/D) L * X [SEQ ID NO: 151]
   where * is a polar residue (C, R, H, K, D, E, S, T, N, Q) and X is any residue
iv) (S/G/T/A)X(A/V/G)X(G/A)XX [SEQ ID NO: 152]
   where X is any residue
v) (S/G/T/A)X(A/V/G)X(G/A)XX(S/G/T/A)*(A/V/G)*(G/A)XX [SEQ ID NO: 153]
   where * is a hydrophobic residue and where X is any residue
vi) SXAXGXXS*A*GXX [SEQ ID NO: 154]
   where * is a hydrophobic residue and where X is any residue
vii) NXAXGXXST(I/V)(G/A)GGXX [SEQ ID NO: 155]
   where X is any residue
viii) NXAXGXXSTIGGGXX [SEQ ID NO: 156]
   where X is any residue
ix) GXQX(V/I/L)XXGGXAXXTX(V/I/L)XXG [SEQ ID NO: 157]
   where X is any residue.

In the invention, each unit of one of the beta solenoid domains conforms to the following consensus sequence:
A X₁ L/F X₂ X₃ [SEQ ID NO: 147]
wherein X₁ is A, C, D, E, I, K, L, M, N, R, S, T or V
X₂ is A, C, D, E, F, G, H, I, K, N, Q, R, S, T, V, W, or Y
X₃ is A, C, D, E, G, H, I, K, N, Q, R, S ,Y

It is considered that a beta solenoid domain which comprises or consists of units that conform to the consensus sequence of (i), (ii) and (iii) will generate a beta solenoid domain with a square or approximately square cross-section; a beta solenoid domain which comprises or consists of units that conform to the consensus sequence of (iv), (v), (vi), (vii) and (viii) will generate an oval or approximately oval shaped cross-section; and a beta solenoid domain which comprises or consists of units that conform to the consensus sequence of and (ix) will generate a triangular or approximately triangular cross-section. It is also considered that one unit of the consensus sequence of (i), (ii) or (iii) represents one side of the four sides of the square cross-section; one unit of the consensus sequence of (iv) represents half a turn of the beta solenoid helix; one unit of the consensus sequence of (v) represents one turn of the beta solenoid helix; one unit of the consensus sequence of (vi) represents one turn of the beta solenoid helix; one unit of the consensus sequence of (vii) represents one turn of the beta solenoid helix; and one unit of the consensus sequence of (viii) represents one turn of the beta solenoid helix.

It will be appreciated that the consensus sequences of the units of the beta solenoid domains of the disclosure are not restricted to those discussed above, but that any other suitable beta solenoid consensus sequence may be used.

It is considered beneficial, though not necessarily essential, that the at least two beta solenoid domains have a cross-section that allows for a degree of tessellation or efficient packing since it is considered that the fibres that are ultimately produced from the concatemers have advantageous properties if the solenoid domains are closely packed. Accordingly, in one option the consensus sequence of the units of the beta solenoid domain or domains is selected such that a tesselatable cross-section is formed, for example which includes a square, a rectangle, a parallelogram, a hexagon, a triangle, or other tesselatable cross-section. Accordingly, the consensus sequences discussed above which result in a square or rectangular, or triangular cross-section are considered to be particularly advantageous. Rounded (circular or oval) cross-sections can still pack together with high efficiency, especially with suitable lateral functionality and are also considered to be suitable shapes for the beta solenoid domain cross-section.

In one option, beta solenoid domains that have an irregular cross-section, or at least a cross section that does not allow for tessellation or efficient packing, is considered to be less advantageous, or unsuitable for use in the present disclosure.

In a preferred embodiment the at least two beta solenoid domains are rod-shaped. The skilled person will understand what is meant by rod-shaped, i.e. has a shape that is longer along one axis than in the transverse axis and wherein the cross-sectional shape and dimensions of the beta solenoid domain do not vary significantly along the length of the beta solenoid domain, for example where the cross-section has a triangular shape the three-dimensional shape of the beta solenoid domain may be considered to be prismatic. As discussed herein, it is preferred that the units within a beta solenoid domain conform to the same consensus sequence. However, the concatemer may comprise beta solenoid domains that conform to different consensus motifs, and therefore may comprise beta solenoid domains that have a different shaped cross-section, for instance a concatemer may comprise beta solenoid domains with a square and/or oval and/or triangular shaped cross-section.

For example, in the case of beta solenoid domains, the beta solenoid domain has a longitudinal axis formed by the regular winding of the unit sequences and a transverse axis running perpendicular to the longitudinal axis. In one embodiment the ratio of longitudinal length to transverse length of one of the at least two beta solenoid domains (i.e. the aspect ratio) is at least 2:1, for example at least 3:1, for example at least 4:1, for example at least 5:1, for example at least 6:1, for example at least 7:1, for example at least 8:1, for example at least 9:1, for example 10:1. In a further embodiment both of the at least two beta solenoid domains have a longitudinal axis and a transverse axis and the ratio of longitudinal length to transverse length is at least 2:1, for example at least 3:1, for example at least 4:1, for example at least 5:1, for example at least 6:1, for example at least 7:1, for example at least 8:1, for example at least 9:1, for example 10:1. In yet another embodiment, all of the beta solenoid domains in the concatemer, for example where the concatemer comprises more than 2 beta solenoid domains have a longitudinal axis and a transverse axis and the ratio of longitudinal length to transverse length is at least 2:1, for example at least 3:1, for example at least 4:1, for example at least 5:1, for example at least 6:1, for example at least 7:1, for example at least 8:1, for example at least 9:1, for example 10:1.

In one embodiment it is preferred that one or both or all of the beta solenoid domains have an aspect ratio of 10:1 or less.

As indicated above, the concatemer may comprise any number of beta solenoid domains, provided that the concatemer, by definition, comprises at least two beta solenoid domains.

For example, the concatemer according to the invention may comprise at least three beta solenoid domains, for example at least four beta solenoid domains, for example at least 5 beta solenoid domains, for example at least 6 beta solenoid domains, for example at least 7 beta solenoid domains, for example at least 8 beta solenoid domains, for example at least 9 beta solenoid domains, for example at least 10 beta solenoid domains, for example at least 11 beta solenoid domains, for example at least 12 beta solenoid domains, for example at least 13 beta solenoid domains, for example at least 14 beta solenoid domains, for example at least 15 beta solenoid domains, for example at least 16 beta solenoid domains, for example at least 17 beta solenoid domains, for example at least 18 beta solenoid domains, for example at least 19 beta solenoid domains, for example at least 20 beta solenoid domains, for example at least 21 beta solenoid domains, for example at least 22 beta solenoid domains, for example at least 23 beta solenoid domains, for example at least 24 beta solenoid domains, for example at least 25 beta solenoid domains, for example at least 26 beta solenoid domains, for example at least 27 beta solenoid domains, for example at least 28 beta solenoid domains, for example at least 29 beta solenoid domains, for example at least 30 beta solenoid domains, for example at least 35 beta solenoid domains, for example at least 40 beta solenoid domains, for example at least 45 beta solenoid domains, for example at least 50 beta solenoid domains, for example at least 55 beta solenoid domains, for example at least 60 beta solenoid domains, for example at least 65 beta solenoid domains, for example at least 70 beta solenoid domains, for example at least 75 beta solenoid domains, for example at least 80 beta solenoid domains, for example at least 85 beta solenoid domains, for example at least 90 beta solenoid domains, for example at least 95 beta solenoid domains, for example at least 100 beta solenoid domains.

As discussed above, it is preferred if the beta solenoid domains have a particular aspect ratio. Accordingly, in one embodiment the concatemer comprises at least two beta solenoid domains, for example at least three beta solenoid domains, for example at least four beta solenoid domains, for example at least 5 beta solenoid domains, for example at least 6 beta solenoid domains, for example at least 7 beta solenoid domains, for example at least 8 beta solenoid domains, for example at least 9 beta solenoid domains, for example at least 10 beta solenoid domains, for example at least 11 beta solenoid domains, for example at least 12 beta solenoid domains, for example at least 13 beta solenoid domains, for example at least 14 beta solenoid domains, for example at least 15 beta solenoid domains, for example at least 16 beta solenoid domains, for example at least 17 beta solenoid domains, for example at least 18 beta solenoid domains, for example at least 19 beta solenoid domains, for example at least 20 beta solenoid domains, for example at least 21 beta solenoid domains, for example at least 22 beta solenoid domains, for example at least 23 beta solenoid domains, for example at least 24 beta solenoid domains, for example at least 25 beta solenoid domains, for example at least 26 beta solenoid domains, for example at least 27 beta solenoid domains, for example at least 28 beta solenoid domains, for example at least 29 beta solenoid domains, for example at least 30 beta solenoid domains, for example at least 35 beta solenoid domains, for example at least 40 beta solenoid domains, for example at least 45 beta solenoid domains, for example at least 50 beta solenoid domains, for example at least 55 beta solenoid domains, for example at least 60 beta solenoid domains, for example at least 65 beta solenoid domains, for example at least 70 beta solenoid domains, for example at least 75 beta solenoid domains, for example at least 80 beta solenoid domains, for example at least 85 beta solenoid domains, for example at least 90 beta solenoid domains, for example at least 95 beta solenoid domains, for example at least 100 beta solenoid domains
and wherein at least one of the beta solenoid domains, for example at least two beta solenoid domains, for example at least three beta solenoid domains, for example at least four beta solenoid domains, for example at least 5 beta solenoid domains, for example at least 6 beta solenoid domains, for example at least 7 beta solenoid domains, for example at least 8 beta solenoid domains, for example at least 9 beta solenoid domains, for example at least 10 beta solenoid domains, for example at least 11 beta solenoid domains, for example at least 12 beta solenoid domains, for example at least 13 beta solenoid domains, for example at least 14 beta solenoid domains, for example at least 15 beta solenoid domains, for example at least 16 beta solenoid domains, for example at least 17 beta solenoid domains, for example at least 18 beta solenoid domains, for example at least 19 beta solenoid domains, for example at least 20 beta solenoid domains, for example at least 21 beta solenoid domains, for example at least 22 beta solenoid domains, for example at least 23 beta solenoid domains, for example at least 24 beta solenoid domains, for example at least 25 beta solenoid domains, for example at least 26 beta solenoid domains, for example at least 27 beta solenoid domains, for example at least 28 beta solenoid domains, for example at least 29 beta solenoid domains, for example at least 30 beta solenoid domains, for example at least 35 beta solenoid domains, for example at least 40 beta solenoid domains, for example at least 45 beta solenoid domains, for example at least 50 beta solenoid domains, for example at least 55 beta solenoid domains, for example at least 60 beta solenoid domains, for example at least 65 beta solenoid domains, for example at least 70 beta solenoid domains, for example at least 75 beta solenoid domains, for example at least 80 beta solenoid domains, for example at least 85 beta solenoid domains, for example at least 90 beta solenoid domains, for example at least 95 beta solenoid domains, for example at least 100 beta solenoid domains has an aspect ratio that is at least 2:1, for example at least 3:1, for example at least 4:1, for example at least 5:1, for example at least 6:1, for example at least 7:1, for example at least 8:1, for example at least 9:1, for example 10:1. In a further embodiment both of the at least two beta solenoid domains have a longitudinal axis and a transverse axis and the ratio of longitudinal length to transverse length is at least 2:1, for example at least 3:1, for example at least 4:1, for example at least 5:1, for example at least 6:1, for example at least 7:1, for example at least 8:1, for example at least 9:1, for example 10:1.

It will be clear to the skilled person that the aspect ratio of the concatemer will be higher than the aspect ratio of the individual beta solenoid domains and is typically considered to be a sum of the aspect ratios of the individual beta solenoid domains (though the length of the linkers used to link the beta solenoid domains will also affect the aspect ratio of the concatemer). For example, a concatemer of two beta solenoid domains each with an aspect ratio of 10:1 would be expected to produce a concatemer with an aspect ratios of around 20:1. Accordingly, in one embodiment the concatemer may have an aspect ratio of between around 4:1 and 1000:1. In one embodiment the concatemer may have an aspect ratio of at least 4:1, for example at least 6:1, for example at least 8:1, for example at least 10:1, for example at least 10:1, for example at least 50:1, for example at least 100:1, for example at least 200:1, for example at least 300:1, for example at least 400:1, for example at least 500:1, for example at least 600:1, for example at least 700:1, for example at least 800:1, for example at least 900:1, for example at least 1000:1.

To achieve the desired aspect ratio of the one, both of, any number of, or all of the beta solenoid domains in the concatemer, the number of units of the consensus motif may be varied. It is considered that as the number of units of the consensus sequence in a beta solenoid domain increases, the higher the aspect ratio. For example, a beta solenoid with a lower number of units of the consensus motif may have for example an aspect ratio of 1:1 or 1:2, whilst a beta solenoid domain with a higher number of units of the consensus motif sequence may have an aspect ratio of 9:1 or 10:1. This is all within the knowledge of the skilled person and the skilled person will easily be able to determine a suitable number of units of the consensus motif to produce the desired aspect ratio.

Accordingly, in one embodiment, at least one of the at least two beta solenoid domains comprises between 4 and 3000 units of the consensus motif, optionally between 5 and 2800, optionally between 6 and 2600, optionally between 7 and 2400, optionally between 8 and 2200, optionally between 9 and 2000, optionally between 10 and 1800, optionally between 11 and 1600, optionally between 12 and 1400, optionally between 13 and 1200, optionally between 14 and 1000, optionally between 15 and 800, optionally between 16 and 600, optionally between 17 and 500, optionally between 18 and 400, optionally between 19 and 300, optionally between 21 and 200, optionally between 22 and 150, optionally between 23 and 100, optionally between 24 and 90, optionally between 25 and 85, optionally between 26 and 80, optionally between 27 and 75, optionally between 28 and 70, optionally between 29 and 65, optionally between 30 and 60, optionally between 35 and 55, optionally between 40 and 50, optionally 45 units of the consensus motif.

In another embodiment at least two of the beta solenoid domains comprise between 4 and 3000 units of the consensus motif, optionally between 5 and 2800, optionally between 6 and 2600, optionally between 7 and 2400, optionally between 8 and 2200, optionally between 9 and 2000, optionally between 10 and 1800, optionally between 11 and 1600, optionally between 12 and 1400, optionally between 13 and 1200, optionally between 14 and 1000, optionally between 15 and 800, optionally between 16 and 600, optionally between 17 and 500, optionally between 18 and 400, optionally between 19 and 300, optionally between 21 and 200, optionally between 22 and 150, optionally between 23 and 100, optionally between 24 and 90, optionally between 25 and 85, optionally between 26 and 80, optionally between 27 and 75, optionally between 28 and 70, optionally between 29 and 65, optionally between 30 and 60, optionally between 35 and 55, optionally between 40 and 50, optionally 45 units of the consensus motif; and/or

In another embodiment any number of or all of the beta solenoid domains comprise between 4 and 3000 units of the consensus motif, optionally between 5 and 2800, optionally between 6 and 2600, optionally between 7 and 2400, optionally between 8 and 2200, optionally between 9 and 2000, optionally between 10 and 1800, optionally between 11 and 1600, optionally between 12 and 1400, optionally between 13 and 1200, optionally between 14 and 1000, optionally between 15 and 800, optionally between 16 and 600, optionally between 17 and 500, optionally between 18 and 400, optionally between 19 and 300, optionally between 21 and 200, optionally between 22 and 150, optionally between 23 and 100, optionally between 24 and 90, optionally between 25 and 85, optionally between 26 and 80, optionally between 27 and 75, optionally between 28 and 70, optionally between 29 and 65, optionally between 30 and 60, optionally between 35 and 55, optionally between 40 and 50, optionally 45 units of the consensus motif,
for example the concatemer may comprise at least two beta solenoid domains, for example at least three beta solenoid domains, for example at least four beta solenoid domains, for example at least 5 beta solenoid domains, for example at least 6 beta solenoid domains, for example at least 7 beta solenoid domains, for example at least 8 beta solenoid domains, for example at least 9 beta solenoid domains, for example at least 10 beta solenoid domains, for example at least 11 beta solenoid domains, for example at least 12 beta solenoid domains, for example at least 13 beta solenoid domains, for example at least 14 beta solenoid domains, for example at least 15 beta solenoid domains, for example at least 16 beta solenoid domains, for example at least 17 beta solenoid domains, for example at least 18 beta solenoid domains, for example at least 19 beta solenoid domains, for example at least 20 beta solenoid domains, for example at least 21 beta solenoid domains, for example at least 22 beta solenoid domains, for example at least 23 beta solenoid domains, for example at least 24 beta solenoid domains, for example at least 25 beta solenoid domains, for example at least 26 beta solenoid domains, for example at least 27 beta solenoid domains, for example at least 28 beta solenoid domains, for example at least 29 beta solenoid domains, for example at least 30 beta solenoid domains, for example at least 35 beta solenoid domains, for example at least 40 beta solenoid domains, for example at least 45 beta solenoid domains, for example at least 50 beta solenoid domains, for example at least 55 beta solenoid domains, for example at least 60 beta solenoid domains, for example at least 65 beta solenoid domains, for example at least 70 beta solenoid domains, for example at least 75 beta solenoid domains, for example at least 80 beta solenoid domains, for example at least 85 beta solenoid domains, for example at least 90 beta solenoid domains, for example at least 95 beta solenoid domains, for example at least 100 beta solenoid domains and any one or more or all of the beta solenoid domains in the concatemer may comprise between 4 and 3000 units of the consensus motif, optionally between 5 and 2800, optionally between 6 and 2600, optionally between 7 and 2400, optionally between 8 and 2200, optionally between 9 and 2000, optionally between 10 and 1800, optionally between 11 and 1600, optionally between 12 and 1400, optionally between 13 and 1200, optionally between 14 and 1000, optionally between 15 and 800, optionally between 16 and 600, optionally between 17 and 500, optionally between 18 and 400, optionally between 19 and 300, optionally between 21 and 200, optionally between 22 and 150, optionally between 23 and 100, optionally between 24 and 90, optionally between 25 and 85, optionally between 26 and 80, optionally between 27 and 75, optionally between 28 and 70, optionally between 29 and 65, optionally between 30 and 60, optionally between 35 and 55, optionally between 40 and 50, optionally 45 units of the consensus motif.

In another embodiment at least one of the at least two beta solenoid domains comprises at least 5 units of the consensus motif, optionally at least 6 units, optionally at least 7 units, optionally at least 8 units, optionally at least 9 units, optionally at least 10 units, optionally at least 11 units, optionally at least 12 units, optionally at least 13 units, optionally at least 14 units, optionally at least 15 units, optionally at least 16 units, optionally at least 17 units, optionally at least 18 units, optionally at least 19 units, optionally at least 20 units, optionally at least 21 units, optionally at least 24 units, optionally at least 25 units, optionally at least 26 units, optionally at least 27 units, optionally at least 28 units, optionally at least 29 units, optionally at least 30 units, optionally at least 40 units, optionally at least 50 units, optionally at least 60 units, optionally at least 70 units, optionally at least 80 units, optionally at least 90 units, optionally at least 100 units, optionally at least 200 units of the consensus motif.

In another embodiment at least two of the beta solenoid domains comprise at least 5 units of the consensus motif, optionally at least 6 units, optionally at least 7 units, optionally at least 8 units, optionally at least 9 units, optionally at least 10 units, optionally at least 11 units, optionally at least 12 units, optionally at least 13 units, optionally at least 14 units, optionally at least 15 units, optionally at least 16 units, optionally at least 17 units, optionally at least 18 units, optionally at least 19 units, optionally at least 20 units, optionally at least 21 units, optionally at least 24 units, optionally at least 25 units, optionally at least 26 units, optionally at least 27 units, optionally at least 28 units, optionally at least 29 units, optionally at least 30 units, optionally at least 40 units, optionally at least 50 units, optionally at least 60 units, optionally at least 70 units, optionally at least 80 units, optionally at least 90 units, optionally at least 100 units, optionally at least 200 units of the consensus motif.

In yet another embodiment, any number of or all of the beta solenoid domains as discussed above comprise at least 5 units of the consensus motif, optionally at least 6 units, optionally at least 7 units, optionally at least 8 units, optionally at least 9 units, optionally at least 10 units, optionally at least 11 units, optionally at least 12 units, optionally at least 13 units, optionally at least 14 units, optionally at least 15 units, optionally at least 16 units, optionally at least 17 units, optionally at least 18 units, optionally at least 19 units, optionally at least 20 units, optionally at least 21 units, optionally at least 24 units, optionally at least 25 units, optionally at least 26 units, optionally at least 27 units, optionally at least 28 units, optionally at least 29 units, optionally at least 30 units, optionally at least 40 units, optionally at least 50 units, optionally at least 60 units, optionally at least 70 units, optionally at least 80 units, optionally at least 90 units, optionally at least 100 units, optionally at least 200 units of the consensus motif.

In another embodiment the concatemer may comprises at least one beta solenoid domain that comprises less than 3000 units of the consensus motif, optionally less than 2000 units, optionally less than 1000 units, optionally less than 500 units, optionally less than 400 units, optionally less than 300 units, optionally less than 200 units, optionally less than 100 units, optionally less than 90 units, optionally less than 80 units, optionally less than 70 units, optionally less than 60 units, optionally less than 50 units, optionally less than 40 units, optionally less than 30 units, optionally less than 29 units, optionally less than 28 units, optionally less than 27 units, optionally less than 26 units, optionally less than 25 units, optionally less than 24 units, optionally less than 23 units, optionally less than 22 units, optionally less than 21 units, optionally less than 20 units, optionally less than 19 units, optionally less than 18 units, optionally less than 17 units, optionally less than 16 units, optionally less than 15 units, optionally less than 14 units, optionally less than 13 units, optionally less than 12 units, optionally less than 11 units, optionally less than 10 units, optionally less than 9 units, optionally less than 8 units, optionally less than 7 units, optionally 6 units of the consensus motif.

In another embodiment the at least two beta solenoid domains in the concatemer comprises less than 3000 units of the consensus motif, optionally less than 2000 units, optionally less than 1000 units, optionally less than 500 units, optionally less than 400 units, optionally less than 300 units, optionally less than 200 units, optionally less than 100 units, optionally less than 90 units, optionally less than 80 units, optionally less than 70 units, optionally less than 60 units, optionally less than 50 units, optionally less than 40 units, optionally less than 30 units, optionally less than 29 units, optionally less than 28 units, optionally less than 27 units, optionally less than 26 units, optionally less than 25 units, optionally less than 24 units, optionally less than 23 units, optionally less than 22 units, optionally less than 21 units, optionally less than 20 units, optionally less than 19 units, optionally less than 18 units, optionally less than 17 units, optionally less than 16 units, optionally less than 15 units, optionally less than 14 units, optionally less than 13 units, optionally less than 12 units, optionally less than 11 units, optionally less than 10 units, optionally less than 9 units, optionally less than 8 units, optionally less than 7 units, optionally 6 units of the consensus motif; and/or

In yet another embodiment, any number of or all of the beta solenoid domains in the concatemer comprise less than 3000 units of the consensus motif, optionally less than 2000 units, optionally less than 1000 units, optionally less than 500 units, optionally less than 400 units, optionally less than 300 units, optionally less than 200 units, optionally less than 100 units, optionally less than 90 units, optionally less than 80 units, optionally less than 70 units, optionally less than 60 units, optionally less than 50 units, optionally less than 40 units, optionally less than 30 units, optionally less than 29 units, optionally less than 28 units, optionally less than 27 units, optionally less than 26 units, optionally less than 25 units, optionally less than 24 units, optionally less than 23 units, optionally less than 22 units, optionally less than 21 units, optionally less than 20 units, optionally less than 19 units, optionally less than 18 units, optionally less than 17 units, optionally less than 16 units, optionally less than 15 units, optionally less than 14 units, optionally less than 13 units, optionally less than 12 units, optionally less than 11 units, optionally less than 10 units, optionally less than 9 units, optionally less than 8 units, optionally less than 7 units, optionally 6 units of the consensus motif.

It will be appreciated that the units of the consensus motif may be arranged consecutively (i.e. tandemly), wherein one or more of the units of the consensus motif follows directly on from a first consensus motif. However, the units of the consensus motif may also comprises additional sequences between the units. Preferably, the units of the consensus motif are arranged consecutively. Accordingly in one embodiment the concatemer comprises any of the following arrangements:
one, both, any number of or all of the beta solenoid domains comprise between 4 and 3000 units of the consensus motif, optionally between 5 and 2800, optionally between 6 and 2600, optionally between 7 and 2400, optionally between 8 and 2200, optionally between 9 and 2000, optionally between 10 and 1800, optionally between 11 and 1600, optionally between 12 and 1400, optionally between 13 and 1200, optionally between 14 and 1000, optionally between 15 and 800, optionally between 16 and 600, optionally between 17 and 500, optionally between 18 and 400, optionally between 19 and 300, optionally between 21 and 200, optionally between 22 and 150, optionally between 23 and 100, optionally between 24 and 90, optionally between 25 and 85, optionally between 26 and 80, optionally between 27 and 75, optionally between 28 and 70, optionally between 29 and 65, optionally between 30 and 60, optionally between 35 and 55, optionally between 40 and 50, optionally 45 units of the consensus motif
wherein the units of the consensus motif are arranged consecutively; or
one, both or any number of or all of the beta solenoid domains comprise at least 4 units of the consensus motif, for example at least 5 units of the consensus motif, optionally at least 6 units, optionally at least 7 units, optionally at least 8 units, optionally at least 9 units, optionally at least 10 units, optionally at least 11 units, optionally at least 12 units, optionally at least 13 units, optionally at least 14 units, optionally at least 15 units, optionally at least 16 units, optionally at least 17 units, optionally at least 18 units, optionally at least 19 units, optionally at least 20 units, optionally at least 21 units, optionally at least 24 units, optionally at least 25 units, optionally at least 26 units, optionally at least 27 units, optionally at least 28 units, optionally at least 29 units, optionally at least 30 units, optionally at least 40 units, optionally at least 50 units, optionally at least 60 units, optionally at least 70 units, optionally at least 80 units, optionally at least 90 units, optionally at least 100 units, optionally at least 200 units of the consensus motif wherein each of the units of the consensus motif is arranged consecutively; or
one, both or any number of or all of the beta solenoid domains comprise less than 3000 units of the consensus motif, optionally less than 2000 units, optionally less than 1000 units, optionally less than 500 units, optionally less than 400 units, optionally less than 300 units, optionally less than 200 units, optionally less than 100 units, optionally less than 90 units, optionally less than 80 units, optionally less than 70 units, optionally less than 60 units, optionally less than 50 units, optionally less than 40 units, optionally less than 30 units, optionally less than 29 units, optionally less than 28 units, optionally less than 27 units, optionally less than 26 units, optionally less than 25 units, optionally less than 24 units, optionally less than 23 units, optionally less than 22 units, optionally less than 21 units, optionally less than 20 units, optionally less than 19 units, optionally less than 18 units, optionally less than 17 units, optionally less than 16 units, optionally less than 15 units, optionally less than 14 units, optionally less than 13 units, optionally less than 12 units, optionally less than 11 units, optionally less than 10 units, optionally less than 9 units, optionally less than 8 units, optionally less than 7 units, optionally 6 units of the consensus motif
wherein the units of the consensus motif are arranged consecutively.

In one embodiment the length of one of, or both of or all of the beta solenoid domains is between around 5 and 4000 Angstroms in length, for example between around 10 and 3000 Angstroms, for example between about 15 and 2000 Angstroms, for example between around 20 and 1000 Angstroms, for example between around 25 and 500 Angstroms, for example between about 30 and 250 Angstroms, for example between 35 and 200 Angstroms for example between 40 and 100 Angstroms, for example between 45 and 75 Angstroms, for example around 50 Angstroms. Preferably the length of one of, both of or all of the beta solenoid domains is between around 35 and 200 Angstroms.

It will be appreciated that the, both, any number of or all of the beta solenoid domains may comprise some sections wherein the units of the consensus motif follow directly on from one another, i.e. where the units are arranged consecutively, but that between each of these sections there may be an additional intervening sequence.

In one preferred embodiment the at least two of or any number of or all of the beta solenoid domains in the concatemer comprise the same number of units of the consensus motif. In another embodiment not all of the beta solenoid domains have the same number of units of the consensus motif.

As discussed above within a beta solenoid domain the units of the consensus motif may conform to different consensus motifs. However, it is preferable if the consensus motifs within a given beta solenoid domain are the same. In such an embodiment the sequences of each of the units that conforms to the consensus motif may also be identical, for example the beta solenoid domain may comprises a tandem array or of the same sequence. In a preferred embodiment though the actual sequence that conforms to the same consensus motif is different.

Accordingly, in one embodiment at least one of, both of, any number of or all of the beta solenoid domains comprises at least two identical units or sequences of the consensus motif, for example at least 3 units of the consensus motif, for example at least 4 units of the consensus motif, for example at least 5 units of the consensus motif, optionally at least 6 units, optionally at least 7 units, optionally at least 8 units, optionally at least 9 units, optionally at least 10 units, optionally at least 11 units, optionally at least 12 units, optionally at least 13 units, optionally at least 14 units, optionally at least 15 units, optionally at least 16 units, optionally at least 17 units, optionally at least 18 units, optionally at least 19 units, optionally at least 20 units, optionally at least 21 units, optionally at least 24 units, optionally at least 25 units, optionally at least 26 units, optionally at least 27 units, optionally at least 28 units, optionally at least 29 units, optionally at least 30 units, optionally at least 40 units, optionally at least 50 units, optionally at least 60 units, optionally at least 70 units, optionally at least 80 units, optionally at least 90 units, optionally at least 100 units of the consensus motif may be identical. In another embodiment all of the units or sequences that conform to the consensus motif are identical within a given beta solenoid domain.

As discussed above, in another embodiment at least one of, both of, any number of or all of the beta solenoid domains can comprise at least two non-identical units or sequences of the consensus motif, for example at least two non-identical units or sequences of the consensus motif, for example at least 3 units of the consensus motif, for example at least 4 units of the consensus motif, for example at least 5 units of the consensus motif, optionally at least 6 units, optionally at least 7 units, optionally at least 8 units, optionally at least 9 units, optionally at least 10 units, optionally at least 11 units, optionally at least 12 units, optionally at least 13 units, optionally at least 14 units, optionally at least 15 units, optionally at least 16 units, optionally at least 17 units, optionally at least 18 units, optionally at least 19 units, optionally at least 20 units, optionally at least 21 units, optionally at least 24 units, optionally at least 25 units, optionally at least 26 units, optionally at least 27 units, optionally at least 28 units, optionally at least 29 units, optionally at least 30 units, optionally at least 40 units, optionally at least 50 units, optionally at least 60 units, optionally at least 70 units, optionally at least 80 units, optionally at least 90 units, optionally at least 100 units of the consensus motif may be non-identical.

By non-identical we include the meaning of non-identical to each other. For example, in the disclosure above, at least one of, both of, any number of or all of the beta solenoid domains can comprises at least two different unit sequences, for example at least 3 different unit sequences, for example at least 4 different unit sequences, for example at least 5 different unit sequences, optionally at least 6 different unit sequences, optionally at least 7 different unit sequences, optionally at least 8 different unit sequences, optionally at least 9 different unit sequences, optionally at least 10 different unit sequences, optionally at least 11 different unit sequences, optionally at least 12 different unit sequences, optionally at least 13 different unit sequences, optionally at least 14 different unit sequences, optionally at least 15 different unit sequences, optionally at least 16 different unit sequences, optionally at least 17 different unit sequences, optionally at least 18 different unit sequences, optionally at least 19 different unit sequences, optionally at least 20 different unit sequences, optionally at least 21 different unit sequences, optionally at least 24 different unit sequences, optionally at least 25 different unit sequences, optionally at least 26 different unit sequences, optionally at least 27 different unit sequences, optionally at least 28 different unit sequences, optionally at least 29 different unit sequences, optionally at least 30 different unit sequences, optionally at least 40 different unit sequences, optionally at least 50 different unit sequences, optionally at least 60 different unit sequences, optionally at least 70 different unit sequences, optionally at least 80 different unit sequences, optionally at least 90 different unit sequences, optionally at least 100 different unit sequences.

In one embodiment all of the sequences of the units that conform to the consensus sequences are different to one another, i.e. there are no identical sequences of the units that conform to the consensus sequences within a given beta solenoid domain.

In one embodiment beta solenoid domains of the concatemer can comprise units that conform to different consensus sequences. Accordingly in one embodiment at least two, any number of or all of the beta solenoid domains have a different amino acid sequence. As discussed above, preferably the beta solenoid domains comprise units that conform to the same consensus sequence. In such an embodiment the amino acid sequence of the at least two, any number of or all of beta solenoid domains may still be different. In yet another embodiment the amino acid sequence of the at least two, any number of or all of beta solenoid domains may be identical.

It will be clear from the above that the solenoid domains within the concatemer can have different sequences to one another, within the same concatemer. One reason that the sequences of the solenoid domains may be different may be to facilitate synthesis of the nucleic acid fragments that encode the concatemer.

For example, construct >SynRFR24.nnum35.nc.ll.x4.K129S [Amino Acid Sequence] [SEQ ID NO: 161] comprises 3 beta solenoid domains of identical sequence preceded by an initial beta solenoid domain that comprises a slightly different sequence (a K to S mutation) that is considered to ease DNA synthesis and is composed of the TAG sequence: MGSSHHHHHHSSGLVPRGS [SEQ ID NO: 33], the NCAP sequence: RMRRDEILEEYRRGRRNFQHINLQEIELTN [SEQ ID NO: 2], the UNITSn sequence:
aelagadlananlhranlenanlanakleeanleeanlagadlrnanlqranlaradlagadltganlaeadlrearlqsanle nadltnadltgadlegarlhganleganltnanlrkanlenadlrnadltg - Solenoid domain sequence 1 of 4 [SEQ ID NO: 169] followed by the UNITSn sequence
aelagadlananlhranlenanlanakleeanleeanlagadlrnanlqranlaradlagadltganlaeadlrearlqkanle nadltnadltgadlegarlhganleganltnanlrkanlenadlrnadltg - Solenoid domain sequence 2, 3 and 4 of 4 [SEQ ID NO: 23]
the CCAP sequence:
   ARTTGARLDDADLRGATVDPVLWRTASLRGAKMEEEQRREYERARG [SEQ ID NO: 3],
   the tail sequence: QEDKG [SEQ ID NO: 141] and the linker sequence
   GGGSAAAAAAGGGS [SEQ ID NO: 168].

The K to S mutation in the first solenoid domain compared to the remaining 3 solenoid domains in the concatemer is considered to enable the sequence to pass sequence complexity screening criteria for DNA synthesis as a custom, uncloned, linear dsDNA fragment by a commercial DNA synthesis company.

It is considered preferable if the beta solenoid domains are capable of forming a lyotropic liquid crystal mesophase when expressed as monomers. The skilled person will understand that different proteins would be expected to result in different phase behaviours according to our theoretical understanding of liquid crystalline phases (e.g. from Onsager's hard rod model). The ability to form a lyotropic liquid crystal mesophase is linked to both the dimensions of the beta solenoid domains and the charge associated with the beta solenoid domain and requires an anisotropic, structure; "rod-like" domains are particularly suited to the formation of nematic liquid crystals, though other more ordered systems, such as smectics, or columnar phases, may occur. The preferences for the beta solenoid domains discussed herein are considered to result in a beta solenoid domain that is capable of forming a lyotropic liquid crystal mesophase, in solution, when expressed as monomers. Various tests, such as those described in the examples, are available to the skilled person to determine whether a particular beta solenoid domain is capable of forming a lyotropic liquid crystal mesophase when expressed as monomers. For example, the use of polarised optical microscopy can be used to determine whether a particular solenoid domain or concatemer is capable of forming a lyotropic liquid crystal mesophase.

For discussion on liquid crystalline phases in polymers, including aramid spinning, one classic textbook is "Liquid Crystalline Polymers, CUP, 2006, A. M. Donald, A. H. Windle, S. Hanna".

Mesogenic small molecules or colloids are usually considered as rigid, and defined by a characteristic aspect ratio (axial length over rotationally averaged diameter of the species). In polymers, the chain backbone is semi-flexible, and defined by a rigidity characterised by a persistence length. The aspect ratio and persistence length are related parameters that determine the concentration for the isotropic-nematic phase transition (e.g. see : The Journal of Chemical Physics 106, 9858 (1997)). It is considered that low values may not form a mesophase. Higher values reduce the critical concentration but also increase the likelihood of unwanted gelation or glass transition. This is all known to the skilled person and the skilled person is able to select the appropriate values for each of the relevant parameters, e.g. charge and aspect ratio.

It is also preferred if the concatemer as well as or in addition to the beta solenoid monomers is capable of forming a lyotropic liquid crystal mesophase. Preferences are as discussed above. Accordingly in one embodiment the concatemer is capable of forming a lyotropic liquid crystal mesophase.

Examples of suitable sequences for the beta solenoid domain include SEQ ID NO: 1 and 8-28, but as discussed above, any beta solenoid domain sequence is considered to be useful in the present disclosure, particularly those that conform to the consensus sequences discussed herein, for example those of SEQ ID NO: 147-157. In the invention each unit within one of the beta solenoid domains conforms to the following consensus sequence:
A X₁ L/F X₂ X₃ [SEQ ID NO: 147]
wherein X₁ is A, C, D, E, I, K, L, M, N, R, S, T or V
X₂ is A, C, D, E, F, G, H, I, K, N, Q, R, S, T, V, W, or Y
X₃ is A, C, D, E, G, H, I, K, N, Q, R, S ,Y

Accordingly in one embodiment the sequence of at least one of, both of, any number of or all of the beta solenoid domains comprises any of SEQ ID NO: 1 and 8-28. As discussed, in one embodiment each of the beta solenoid domains in the concatemer comprises the same sequence, whilst in another embodiment the concatemer comprises beta solenoid domains that comprise different sequences.

It will also be appreciated that some degree of variation in the sequences disclosed herein can be tolerated by the beta solenoid and concatemer of the invention. Accordingly, one embodiment provides a concatemer according to the present invention wherein at least one, the at least two of, any number of or all of the beta solenoid domains have a least 75% homology to any one of the sequences of SEQ ID NO: 1 and 8-28, optionally at least 80% homology, optionally at least 85% homology, optionally at least 90% homology, optionally at least 95% homology, optionally at least 96% homology, optionally at least 97% homology, optionally at least 98% homology, optionally at least 99% homology, optionally 100% homology, preferably whilst still conforming to the required consensus sequence. Another embodiment provides a concatemer according to the present invention wherein at least one, the at least two of, any number of or all of the beta solenoid domains have a least 75% sequence identity to any one of the sequences of SEQ ID NO: 1 and 8-28, optionally at least 80% sequence identity, optionally at least 85% sequence identity, optionally at least 90% sequence identity, optionally at least 95% sequence identity, optionally at least 96% sequence identity, optionally at least 97% sequence identity, optionally at least 98% sequence identity, optionally at least 99% sequence identity, optionally 100% sequence identity, preferably whilst still conforming to the required consensus sequence.

In a preferred embodiment at least one of, both of, any number of or all of the beta solenoid domains in the concatemer comprises a capping sequence at a first end or a second end, or both the first end and the second end of the beta solenoid domain. It will be apparent that the beta solenoid domains may not comprise any capping sequence at all and the presence of capping sequences or capping structures is not considered to be essential to the invention. However, the presence of a capping sequence at either the N-terminus or the C-terminus, preferably both the N-terminus and the C-terminus is a preferred embodiment.

Accordingly in one embodiment at least one of the beta solenoid domains comprises a capping sequence at a first end or a second end, or both the first end and the second end of each beta solenoid domains, or both of or any number of or all of the beta solenoid domains comprise a capping sequence at a first end or a second end, or both the first end and the second end of each beta solenoid domain.

The capping sequences are considered to prevent the beta solenoid domains from joining end-to-end and aggregating. Accordingly, in one embodiment the beta solenoid domain or domains as described herein comprise one or two capping sequences that prevent the beta solenoid domains from joining end-to-end and aggregating. The skilled person is able to design such capping sequences and there are a number of different ways in which this can be achieved. For example, a terminal repeat similar to the consensus sequence of the units of the beta solenoid domain may be used but in which the solvent exposed residues that would normally be buried are instead mutated to polar or charged residues, which is considered to help in shielding the hydrophobic core. Another approach that will be apparent to the skilled person would be to disrupt the normal hydrogen bonding backbone pattern of the terminal repeat by adding a proline residue or forming a beta-bulge. Yet a further approach is to design the sequence of the beta solenoid doing so that part of the polypeptide chain folds over and covers the end. For example, the capping sequences disclosed herein form alpha helices which cover the ends of the beta-helices. Such capping sequences can be found in nature or designed *de novo* by, for example redesigning natural sequences using computational protein design methods.

In a further embodiment, the capping sequence contains an alpha helix.

Examples of suitable capping sequences are those that conform to the consensus sequences provided in SEQ ID NO: 82 and 83:
[SEQ ID NO: 82]
   xMxxxxl LxxYxxGxxxFxxlxLxx(I/A)xLxx where x is any residue
[SEQ ID NO: 83]
   Ax(T/L/F)xxAx(L/F)xxAx(L/F)xxAxVxPVLWxxAxLxGAx(M/V)xxxxxxx(Y/F)xxAxx
where x is any residue

The sequence of SEQ ID NO: 82 is considered to be suitable for capping the N-terminus of the beta solenoid domain, whilst the sequence of SEQ ID NO: 83 is considered to be suitable for capping the C-terminus of the beta solenoid domain.

Further sequences are provided by SEQ ID NO: 2, 3, 84-104 and 105-125 wherein SEQ ID NO: 2, and 84-104 are suitable for capping the N-terminus of the beta solenoid domain whilst the SEQ ID NO: 3 and 105-125 are suitable for capping the C-terminus of the beta solenoid domain.

It is generally considered that due to the chirality of the beta solenoid domain, a capping sequence that is suitable for capping the N-terminus is not generally suitable for capping the C-terminus and vice versa. However, there are some capping structures that are considered to be suitable for capping at either end of the beta solenoid domain. Such capping sequences include those that are based on variations on the unit motif sequence of the beta solenoid domain and which, for example, comprise hydrophilic residues in place of the hydrophobic residues.

In one embodiment, the beta solenoid domain comprises only an N-terminal capping sequence wherein the sequence conforms to the consensus sequence set out in SEQ ID NO: 82 and/or is selected from any of SEQ ID NO: 2 and 84-104.

In another embodiment, the beta solenoid domain comprises only an C-terminal cap, optionally wherein the C-terminal capping sequence conforms to the consensus sequence set out in SEQ ID NO: 83 and/or is selected from any of SEQ ID NO: 3 and 105-125.

In another embodiment, the beta solenoid domain comprises both an N-terminal cap and a C-terminal cap, for example as defined above.

In one embodiment the N-terminal capping sequence and the C-terminal capping sequence are identical to one another. In another embodiment the N-terminal capping sequence and the C-terminal capping sequence are different to one another.

It will be appreciated that some variation in the capping sequences of SEQ ID NO: 2, 3, 84-104 and 105-125 is to be permitted and is expected to result in functional capping sequences. Accordingly in one embodiment one or both of the capping sequences on a given beta solenoid domain comprises a sequence with at least 80% homology, optionally at least 85% homology, optionally at least 90% homology, optionally at least 95% homology, optionally at least 96% homology, optionally at least 97% homology, optionally at least 98% homology, optionally at least 99% homology, optionally 100% homology to any one of SEQ ID NO: 2, 3, 84-104 and 105-125. In another embodiment one or both of the capping sequences on a given beta solenoid domain comprises a sequence with at least 80% sequence identity, optionally at least 85% sequence identity, optionally at least 90% sequence identity, optionally at least 95% sequence identity, optionally at least 96% sequence identity, optionally at least 97% sequence identity, optionally at least 98% sequence identity, optionally at least 99% sequence identity, optionally 100% sequence identity to any one of SEQ ID NO: 2, 3, 84-104 and 105-125.

It is considered that the capping structures or capping sequences may also comprise one or more sequences of a consensus motif as described herein or a sequence that is very similar to a sequence that conforms to the or a consensus motif of the beta solenoid domain. It is considered that the inclusion of such sequences, which may or may not be identical and which may or may not strictly conform to the consensus sequence the capping structures are preferred, though not essential, since the capping structures or capping sequences serve as an interface region between the ends of the beta solenoid and any other non-consensus motif capping sequences, such as the capping alpha-helices that pack against the ends of the beta-solenoid in a stable manner (see for example SEQ ID NO: 2, 3, 84-104 and 105-125 which are alpha helical capping sequences/structures).

It will be understood however that the capping sequence of the beta solenoids of the concatemer is not to be limited to those sequences described herein since a wide range of capping sequences are expected to be suitable. The skilled person will readily be able to determine if the capping sequences are suitable by assessing whether, for example, the proposed caps prevent the beta solenoid domains from joining end-to-end and aggregating.

The at least two beta solenoid domains of the invention are linked together by a linker, preferably a flexible linker. Linkers are commonly used by those in the field for example to link protein domains, for example when tagging a protein with GFP. The presence of the linker allows each of the linked components to act somewhat independently of one another whilst being spatially restricted and retaining connectivity. The linker is considered to be important in achieving the high molecular weight (large numbers of repeats) of the concatemer that is needed for effective mechanical properties. The ends of the concatemer are considered to act as defects in the final fibres, and reduce load carrying ability. Increasing the molecular weight of the concatemer through the use of linkers minimises any fall in solubility and gelation/precipitation which is considered to limit fibre spinning.

In one embodiment the flexible linker is also formed from amino acids. In this advantageous embodiment it is possible to transcribe both the at least two beta solenoid domains as a single RNA transcript and translate into a single polypeptide. However, other means of linking protein domains such as the beta solenoid domains of the invention are known and are included in the invention.

The skilled person will understand and appreciate the features of suitable linkers. Examples of suitable linkers are considered to be poly glycine linkers, poly alanine linkers, glycine rich linkers, alanine rich linkers, glycine and alanine rich linkers, and glycine/alanine/serine rich linker. Examples of suitable linkers are described in SEQ ID NO: 34 - GGGS, and SEQ ID NO: 168 - GGGSAAAAAAGGGS.

The advantageous properties of the claimed concatemer is considered to at least partly reside in the presence of hidden length. The concept of hidden length will be well known to the skilled person from, for example, the field of natural fibres such as silk (Fantner *et al* DOI: 10.1529/biophysj.105.069344).. Essentially hidden length arises from a high degree of coiling (i.e. in the present case the coiling of the beta solenoid domain) such that when a longitudinal force is applied to the coil (i.e. when the force is applied parallel to the organised fibre of the beta solenoid domain), the coil extends so that the fully extended chain is many times longer than the original. In the present invention the extra length is coiled in a regular fashion to be deployed at a defined force applied in a longitudinal direction. When the force is applied sacrificial bonds, for example hydrogen bonds that form between the polypeptide backbones of the residues of the units of the consensus sequence are broken whilst the covalent bonds, for example the peptides bonds that are formed between the C and N termini of the individual amino acid residues are not broken.

The degree of hidden length that a particular beta solenoid domain possesses is a property of the structure of the beta solenoid structure which is a consequence of the design of the consensus sequence and actual amino acid sequences used for the units of the beta solenoid domain.

Accordingly, in one embodiment at least one of, both of, any number of or all of the beta solenoid domains in the concatemer are considered to comprise hidden length.

Accordingly, a beta solenoid that comprises hidden length is considered to have a first compact conformation and a second extended conformation and is capable, upon application of a longitudinal force, or moving from the first conformation to the second conformation. One embodiment therefore provides the concatemer of the invention wherein at least one of the at least two, both of, any number of or all of the beta solenoid domains in the concatemer moves between the first conformation and the second conformation when a defined force is applied parallel to the longitudinal axis of the concatemer.

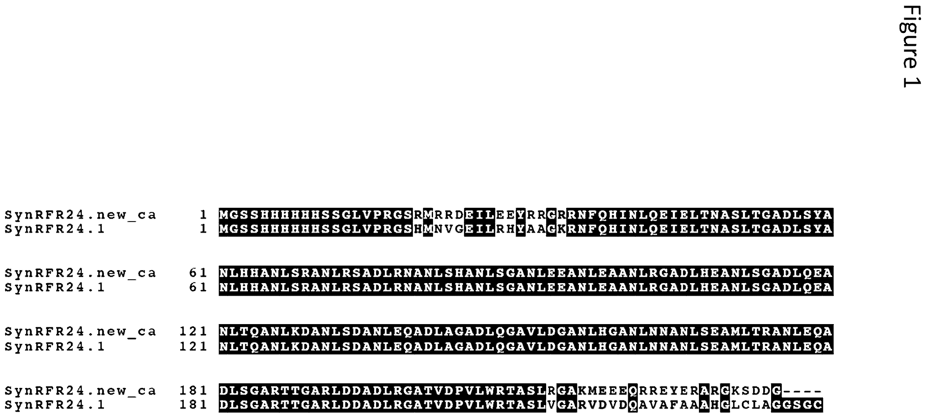

As the beta solenoid is extended it is considered to absorb energy (See Figure 1 from REF: Fantner *et al* DOI: 10.1529/biophysj.105.069344), a small amount from breaking the sacrificial bonds and then mostly by acting as an entropic spring. The skilled person will understand that the more coiled structures have more possible states (conformations) and therefore higher entropy. A fully extended structure has very few states (conformations) and therefore lower entropy.

In one embodiment then the concatemer comprises at least one or at least two or any number of beta solenoid domains that comprise a first and a second conformation wherein the second conformation of the beta solenoid domain is extended relative to the first conformation of the beta solenoid domain, optionally wherein the length of the second conformation of the beta solenoid domain is at least 1.5 times longer than the length of the first conformation of the beta solenoid domain, optionally at least 2 times longer, optionally at least 3 times longer, optionally at least 4 times longer, optionally at least 5 times longer, optionally at least 10 times longer, optionally at least 20 times longer, optionally at least 30 times longer, optionally at least 40 times longer, optionally at least 50 times longer, optionally at least 100 times longer than the length of the first conformation of the beta solenoid domain.

Since the hidden length is considered to be a result of the coiled nature of the beta solenoid domain, in one embodiment, the first conformation of at least one of the beta solenoid domains comprises a high degree of coiling.

It is considered that increasing the molecular weight of the beta solenoid by forming concatemers of the beta solenoid as described herein, improves the spinnabilty of the beta solenoid domains. The concatemer of the invention has a molecular weight of at least 30 kDa, optionally at least 40 kDa, optionally at least 50 kDa, optionally at least 60 kDa, optionally at least 70 kDa , optionally at least 80 kDa, optionally at least 90 kDa, optionally at least 100 kDa, optionally at least 120 kDa, optionally at least 150 kDa, optionally at least 200 kDa, optionally at least 250 kDa, optionally at least 300 kDa, optinally at least 350 kDa, optionally at least 400 kDa, optionally at least 450 kDa, optionally at least 500 kDa, optionally at least 600 kDa, optionaly at least 650 kDa, optionally at least 700 kDa, optionally at least 800 kDa, optionally at least 900 kDa, optionally at least 1000 kDa.; optionally between 30 kDa and 1000 kDa.

It is also considered to be advantageous if the concatemer is soluble at high concentrations. This again is considered to lead to increased spinnability. Accordingly in one embodiment the concatemer is soluble at high concentrations, optionally soluble at a concentration of at least 20 mg/ml, optionally at least 30 mg/ml, optionally at least 40 mg/ml, optionally at least 50 mg/ml, optionally at least 60 mg/ml, optionally at least 70 mg/ml, optionally at least 80 mg/ml, optionally at least 90 mg/ml, optionally at least 100 mg/ml, optionally at least 120 mg/ml, optionally at least 130 mg/ml, optionally at least 150 mg/ml, optionally at least 170 mg/ml, optionally at least 190 mg/ml, optionally at least 200 mg/ml, optionally at least 250 mg/ml, optionally at least 300 mg/ml, optionally at least 350 mg/ml, optionally at least 400 mg/ml.

In a further embodiment the concatemer of the invention is soluble in at least any of a solvent selected from the group consisting of:
a) a buffer selected from the group consisting of any one or more of: MES, Bis-tris methane, ADA, ACES, Bis-tris propane, PIPES, MOPSO, Cholamine chloride, MOPS, BES, TES, HEPES, DIPSO, MOBS, Acetamidoglycine, TAPSO, TEA, POPSO, HEPPSO, EPS, HEPPS, Tricine, Tris, Glycinamide, Glycylglycine, HEPBS, Bicine, TAPS, AMPB, CHES, AMP, AMPSOCAPSO, CAPS, CABS, Bicine, sodium citrate buffer, sodium bicarbonate buffer, phosphate buffer, borate buffer,
   optionally wherein the buffer comprises one or more salts, optionally sodium chloride and/or potassium chloride,
   optionally wherein the buffer is at a pH range of 3 to 10, optionally pH 7;
b) an organic solvent, optionally HFIP.

Accordingly in one embodiment the concatemer is soluble at high concentrations, optionally soluble at a concentration of at least 20 mg/ml, optionally at least 30 mg/ml, optionally at least 40 mg/ml, optionally at least 50 mg/ml, optionally at least 60 mg/ml, optionally at least 70 mg/ml, optionally at least 80 mg/ml, optionally at least 90 mg/ml, optionally at least 100 mg/ml, optionally at least 120 mg/ml, optionally at least 130 mg/ml, optionally at least 150 mg/ml, optionally at least 170 mg/ml, optionally at least 190 mg/ml, optionally at least 200 mg/ml, optionally at least 250 mg/ml, optionally at least 300 mg/ml, optionally at least 350 mg/ml, optionally at least 400 mg/ml in any one or more of
a) a buffer selected from the group consisting of any one or more of: MES, Bis-tris methane, ADA, Bis-tris propane, PIPES, ACES, MOPSO, Cholamine chloride, MOPS, BES, TES, HEPES, DIPSO, MOBS, Acetamidoglycine, TAPSO, TEA, POPSO, HEPPSO, EPS, HEPPS, Tricine, Tris, Glycinamide, Glycylglycine, HEPBS, Bicine, TAPS, AMPB, CHES, AMP, AMPSOCAPSO, CAPS, CABS, Bicine, sodium citrate buffer, sodium bicarbonate buffer, phosphate buffer, borate buffer,
   optionally wherein the buffer comprises one or more salts, optionally sodium chloride and/or potassium chloride,
   optionally wherein the buffer is at a pH range of 3 to 10, optionally pH 7;
b) an organic solvent, optionally HFIP.

To spin the concatemer in to fibres, typically the concatemer which is dissolved in a particular solvent such as those described above is spun into an anti-solvent, or coagulation solution, in which the concatemer is not as soluble and will therefore precipitate. Coagulation spinning is standard in the art and in brief involves the spinning solution (dope) being passed through a spinnarette of one to many (thousands) of holes to define individual strands which are then consolidated, wound up and treated (stretched, annealed, dried, coated) to optimise performance. The skilled person will understand how to select suitable coagulation solvents. For example, where the concatemer is dissolved in an aqueous solution, the coagulation solvent may be ethanol. Accordingly, in a further embodiment the concatemer according to the present invention is not soluble in aqueous solution at a high salt concentration (e.g. at high concentrations of ammonium sulfate or other salts from the Hofmeister series known to "salt out" proteins). A further approach could be to coagulate proteins using a change in pH, for example proteins are known to be least soluble at their isoelectric point (pl) where the proteins have a neutral net charge. A further approach could be to induce protein coagulation in aqueous solution using salts containing divalent or trivalent cations (e.g. calcium chloride). Non-aqueous coagulants could include ethanol, methanol, acetone, propanol, diethyl ether, or DMSO. Another approach could be a coagulation solution containing a mixture of the above approaches. The skilled person will understand that proteins such as the concatemers and solenoid domains of the present invention can be denatured by various factors, including some solvents. It is considered to be important that the concatemers retain their structure after spinning, though it is possible that some degree of denaturation may be tolerated and still result in the ability to spin the concatemers into fibres. Accordingly, the skilled person will understand that the dope solvent and coagulation solvent should be selected to minimise protein denaturation and maximise retention of the structural features of the concatemer both before and after spinning. The retention of the desired ordered solenoid post spinning can be confirmed by various methods including X-ray diffraction studies of the resulting fibres, as shown in the examples.

The skilled person will appreciate that small diameter filaments can also be made by evaporation (dry spinning), electrospinning, solution blow spinning, centrifugal force spinning and the like which do not require a coagulation solution.

As discussed above, the net charge of the beta solenoid domain and therefore the concatemer is considered to be an important factor in the ability of the beta solenoid domain or concatemer to form a lyotropic liquid crystal mesophase. Increasing surface charge (i.e. both a high positive charge or a high negative charge) provides the Coulombic repulsion that helps to solubilise the polypeptide in convenient polar solvents, suitable for coagulation spinning. The solubilised (semi-)rigid beta solenoid domain or concatemer is then able to form a rotationally ordered liquid crystalline phase, following Onsager's excluded volume theory (and subsequent extensions). Higher surface charge increases the effective diameter of the rod-like beta solenoids or concatemer, adjusting the aspect ratio and volume fraction of the mesogens, and hence the phase diagram as a function of concentration. Accordingly, a combination of concentration and aspect ratio is considered to be important in the formation of mesophases, all of which are apparent to the skilled person. Bulky, flexible surface groups may also enhance solubility through steric stabilisation.

It is therefore considered that by controlling the surface chemistry of the beta solenoids and the concatemers, the solubility and lateral interactions in the fibres can also be controlled.

In one embodiment therefore at least one of the at least two beta solenoid domains in the concatemer of the invention has a high net charge, optionally has a net charge of at least +3 charge units, optionally at least +5 charge units, optionally at least +7 charge units, optionally at least +10 charge units, optionally at least +15 charge units, optionally at least +20 charge units, optionally at least +25 charge units, optionally at least +30 charge units, optionally at least +35 charge units, optionally at least +40 charge units, optionally at least +45 charge units, optionally at least +50 charge units, optionally at least +55 charge units, optionally at least +60 charge units, optionally at least +65 charge units, optionally at least +70 charge units, optionally at least +75 charge units, optionally at least +80 charge units, optionally at least +85 charge units, optionally at least +90 charge units, optionally at least +95 charge units, optionally at least +100 charge units, optionally at a pH range of 3 to 10, optionally pH 7.

The charge units referred to herein are, as will be apparent to the skilled person, elementary charge units (e) which is the electric charge carried by a single proton (e), or equivalently, the magnitude of the electric charge carried by a single electron, which has charge -e.

In another embodiment, both of the at least two beta solenoid domains have a high net charge, optionally have a net charge of at least +3 charge units, optionally at least +5 charge units, optionally at least +7 charge units, optionally at least +10 charge units, optionally at least +15 charge units, optionally at least +20 charge units, optionally at least +25 charge units, optionally at least +30 charge units, optionally at least +35 charge units, optionally at least +40 charge units, optionally at least +45 charge units, optionally at least +50 charge units, optionally at least +55 charge units, optionally at least +60 charge units, optionally at least +65 charge units, optionally at least +70 charge units, optionally at least +75 charge units, optionally at least +80 charge units, optionally at least +85 charge units, optionally at least +90 charge units, optionally at least +95 charge units, optionally at least +100 charge units, optionally at a pH range of 3 to 10, optionally pH 7.

In yet a further embodiment, any number of or all of the beta solenoid domains have a high net charge, optionally have a net charge of at least +3 charge units, optionally at least +5 charge units, optionally at least +7 charge units, optionally at least +10 charge units, optionally at least +15 charge units, optionally at least +20 charge units, optionally at least +25 charge units, optionally at least +30 charge units, optionally at least +35 charge units, optionally at least +40 charge units, optionally at least +45 charge units, optionally at least +50 charge units, optionally at least +55 charge units, optionally at least +60 charge units, optionally at least +65 charge units, optionally at least +70 charge units, optionally at least +75 charge units, optionally at least +80 charge units, optionally at least +85 charge units, optionally at least +90 charge units, optionally at least +95 charge units, optionally at least +100 charge units, optionally at a pH range of 3 to 10, optionally pH 7.

As discussed above, the beta solenoid domains may also have a high negative charge. In one embodiment of the concatemer at least one of the at least two beta solenoid domains has a high negative net charge, optionally has a net charge of less than -3 charge units, optionally at less than -5 charge units, optionally less than -7 charge units, optionally less than -10 charge units, optionally less than -15 charge units, optionally less than -20 charge units, optionally less than -25 charge units, optionally less than -30 charge units, optionally less than -35 charge units, optionally less than -40 charge units, optionally less than -45 charge units, optionally less than -50 charge units, optionally less than -55 charge units, optionally less than -60 charge units, optionally less than -65 charge units, optionally less than -70 charge units, optionally less than -75 charge units, optionally less than -80 charge units, optionally less than -85 charge units, optionally less than -90 charge units, optionally less than -95 charge units, optionally less than -100 charge units, optionally at a pH range of 3 to 10, optionally pH 7.

In a further embodiment both of the at least two beta solenoid domains have a high negative net charge, optionally has a net charge of less than -3 charge units, optionally at less than -5 charge units, optionally less than -7 charge units, optionally less than -10 charge units, optionally less than -15 charge units, optionally less than -20 charge units, optionally less than -25 charge units, optionally less than -30 charge units, optionally less than -35 charge units, optionally less than -40 charge units, optionally less than -45 charge units, optionally less than -50 charge units, optionally less than -55 charge units, optionally less than -60 charge units, optionally less than -65 charge units, optionally less than -70 charge units, optionally less than -75 charge units, optionally less than -80 charge units, optionally less than -85 charge units, optionally less than -90 charge units, optionally less than -95 charge units, optionally less than -100 charge units, optionally at a pH range of 3 to 10, optionally pH 7.

In yet another embodiment any number of or all of the two beta solenoid domains have a high negative net charge, optionally has a net charge of less than -3 charge units, optionally at less than -5 charge units, optionally less than -7 charge units, optionally less than -10 charge units, optionally less than -15 charge units, optionally less than -20 charge units, optionally less than -25 charge units, optionally less than -30 charge units, optionally less than -35 charge units, optionally less than -40 charge units, optionally less than -45 charge units, optionally less than -50 charge units, optionally less than -55 charge units, optionally less than -60 charge units, optionally less than -65 charge units, optionally less than -70 charge units, optionally less than -75 charge units, optionally less than -80 charge units, optionally less than -85 charge units, optionally less than -90 charge units, optionally less than -95 charge units, optionally less than -100 charge units optionally at a pH range of 3 to 10, optionally pH 7.

The above embodiments refer to the net charge of the individual beta solenoid domains. If the solenoid domains have a high charge it follows that the concatemer will also have a high charge, which charge is dependent on both the charge of the individual beta solenoid domains and the number of solenoid domains. Accordingly, in one embodiment the concatemer has a high net charge, optionally has a net charge of at least +3 charge units, optionally at least +5 charge units, optionally at least +7 charge units, optionally at least +10 charge units, optionally at least +15 charge units, optionally at least +20 charge units, optionally at least +25 charge units, optionally at least +30 charge units, optionally at least +35 charge units, optionally at least +40 charge units, optionally at least +45 charge units, optionally at least +50 charge units, optionally at least +55 charge units, optionally at least +60 charge units, optionally at least +65 charge units, optionally at least +70 charge units, optionally at least +75 charge units, optionally at least +80 charge units, optionally at least +85 charge units, optionally at least +90 charge units, optionally at least +95 charge units, optionally at least +100 charge units, optionally at least +200 charge units, optionally at least +300 charge units, optionally at least +400 charge units, optionally at least +500 charge units, optionally at least +600 charge units, optionally at least +700 charge units, optionally at least +800 charge units, optionally at least +900 charge units, optionally at least +1000 charge units, optionally at least +2000 charge units, optionally at least +3000 charge units, optionally at least +4000 charge units, optionally at least +5000 charge units, optionally at least +6000 charge units, optionally at least +7000 charge units, optionally at least +8000 charge units, optionally at least +9000 charge units, optionally at least +10000 charge units
optionally at a pH range of 3 to 10, optionally pH 7.

In another embodiment the concatemer has a high negative net charge, optionally has a net charge of less than -3 charge units, optionally at less than -5 charge units, optionally less than -7 charge units, optionally less than -10 charge units, optionally less than -15 charge units, optionally less than -20 charge units, optionally less than -25 charge units, optionally less than -30 charge units, optionally less than -35 charge units, optionally less than -40 charge units, optionally less than -45 charge units, optionally less than -50 charge units, optionally less than -55 charge units, optionally less than -60 charge units, optionally less than -65 charge units, optionally less than -70 charge units, optionally less than -75 charge units, optionally less than -80 charge units, optionally less than -85 charge units, optionally less than -90 charge units, optionally less than -95 charge units, optionally less than -100 charge units, optionally less than -200 charge units, optionally less than -300 charge units, optionally less than -400 charge units, optionally less than - 500 charge units, optionally less than -600 charge units, optionally less than -700 charge units, optionally less than -800 charge units, optionally less than -900 charge units, optionally less than -1000 charge units, optionally less than -2000 charge units, optionally less than -3000 charge units, optionally less than -4000 charge units, optionally less than -5000 charge units, optionally less than -6000 charge units, optionally less than -7000 charge units, optionally less than -8000 charge units, optionally less than -9000 charge units, optionally less than -10000 charge units
optionally at a pH range of 3 to 10, optionally pH 7.

One advantage of the present invention is that the beta solenoid domains and concatemers can be produced through biotechnological means, for example through protein expression systems that for example involve the use of microbes. This addresses at least one problem with the fibres of the prior art that for example relied on the use of arthropods for production. Accordingly, in one embodiment the concatemer of the invention is capable of being expressed by a cell or a cell-free transcription and translation system.

In such an embodiment, the cell may be any cell for example a cell that is generally used in commercial protein expression systems or that is particularly suited to expressing the concatemers of the present invention. The cell may be a prokaryotic cell, optionally a bacterial cell, optionally an *E. coli* cell, a *Bacillus subtilis* cell, a *Bacillus megaterium* cell, *a Vibrio natriegens* cell, or a *Pseudomonas fluorescens* cell.

The cell may also be a eukaryotic cell, optionally a yeast cell, optionally *Pichia pastoris* or Saccharomyces cerevisiae; or an insect cell, optionally a baculovirus infected insect cell; or a mammalian cell, optionally a baculovirus infected mammalian cell, a HEK293 cell, a HeLa cell, or CHO cells. Suitable expression systems will be known to the skilled person and there is not reason to suppose that any system is not suitable for use with the present invention.

The beta solenoid domains and concatemers of the present invention may also be expressed through cell-free expression systems, for example by using *E.coli* lysates or rabbit reticulocyte lysates.

In one embodiment, the concatemer of the invention comprises or consists of one or more of the sequences according to SEQ ID NOs: 29-32 and 158-162; or comprises or consists of a sequence with at least 80% homology, optionally at least 85% homology, optionally at least 90% homology, optionally at least 95% homology, optionally at least 96% homology, optionally at least 97% homology, optionally at least 98% homology, optionally at least 99% homology, optionally 100% homology to any one or more of SEQ ID NOs: 29-32 and 158-162. In another embodiment, the concatemer of the invention comprises or consists of one or more of the sequences according to SEQ ID NOs: 29-32 and 158-162; or comprises or consists of a sequence with at least 80% sequence identity, optionally at least 85% sequence identity, optionally at least 90% sequence identity, optionally at least 95% sequence identity, optionally at least 96% sequence identity, optionally at least 97% sequence identity, optionally at least 98% sequence identity, optionally at least 99% sequence identity, optionally 100% sequence identity to any one or more of SEQ ID NOs: 29-32 and 158-162.

As will be evident from the disclosure herein and as discussed at the outset, as well as providing a concatemer of beta solenoid domains as discussed above, the invention also provides the beta solenoid domains themselves. Accordingly, a second aspect of the invention provides a beta solenoid domain that comprises an amino acid sequence that comprises at least 4 units of the following consensus motif:
i) A X₁ L/F X₂ X₃ [SEQ ID NO: 147]
   wherein X₁ is A, C, D, E, I, K, L, M, N, R, S, T or V
   X₂ is A, C, D, E, F, G, H, I, K, N, Q, R, S, T, V, W, or Y
   X₃ is A, C, D, E, G, H, I, K, N, Q, R, S ,Y;

   wherein the beta solenoid domain does not consist of any of the sequences of SEQ ID NO: 1, 34, 36, 38, 40, 42, 44, 46, 48, 50, 52, 54, 56, 58, 60, 62; and/or
   SEQ ID NO: 66-80

And wherein the beta solenoid domain:
a) has a longitudinal axis and a transverse axis and the ratio of the longitudinal axis to transverse axis is at least 2:1;
b) comprises at least 29 units;
c) has a molecular weight of at least 60KDa;
d) is soluble at a concentration of at least 20 mg/ml

Preferences for features of the beta solenoid domain are as discussed above in the context of the concatemer of the invention.

For example, the beta solenoid domain of the invention is preferably rod-shaped as discussed and preferences for the aspect ratio are as above.

Similarly, the preferences for the number of units is as discussed above, i.e. for example in one embodiment the beta solenoid domain of the invention comprises between 4 and 3000 units of the consensus motif. As mentioned, the units may be arranged consecutively or non-consecutively and the units may or may not be identical to one another, again as discussed above.

Preferences for the consensus motif and the cross-section of the beta solenoid are also discussed above.

It is also preferred that the beta solenoid domain forms a lyotropic liquid crystal mesophase.

Again, as stated in relation to the concatemer, the beta solenoid domain may comprise or consist of any of the sequences of SEQ ID NOs: 8-28 or 169, or a sequence with at least 80% homology or at least 80% sequence identity to these sequences, as discussed previously. However, the beta solenoid domain of the invention does not comprise or consist of SEQ ID NO: 1, 36, 38, 40, 42, 44, 46, 48, 50, 52, 54, 56, 58, 60, 62, 64; and/or SEQ ID NO: 66-80.

As discussed in relation to the concatemer of the invention, the beta solenoid domain of the invention may also comprise one or two capping sequences at a first end and/or a second end. Preferences for the capping sequences are as discussed previously.

As mentioned previously, the beta solenoid domain of the invention preferably comprises hidden length and preferences for this feature are as described above.

The beta solenoid domain of the invention may have a molecular weight of at least 30 kDa, optionally at least 40 kDa, optionally at least 50 kDa, optionally at least 60 kDa, optionally at least 70 kDa , optionally at least 80 kDa, optionally at least 90 kDa, optionally at least 100 kDa, optionally at least 120 kDa, optionally at least 150 kDa, optionally at least 200 kDa, optionally at least 250 kDa, optionally at least 300 kDa, optinally at least 350 kDa, optionally at least 400 kDa, optionally at least 450 kDa, optionally at least 500 kDa, optionally at least 600 kDa, optionaly at least 650 kDa, optionally at least 700 kDa, optionally at least 800 kDa, optionally at least 900 kDa, optionally at least 1000 kDa;
optionally between 30 kDa and 1000 kDa, as discussed previously.

The preferences for the solubility of the beta solenoid domain are similar to those discussed above in relation to the concatemer. For example, the beta solenoid domain of the invention may be soluble at high concentrations, optionally soluble at a concentration of at least 20 mg/ml, optionally at least 30 mg/ml, optionally at least 40 mg/ml, optionally at least 50 mg/ml, optionally at least 60 mg/ml, optionally at least 70 mg/ml, optionally at least 80 mg/ml, optionally at least 90 mg/ml, optionally at least 100 mg/ml, optionally at least 120 mg/ml, optionally at least 130 mg/ml, optionally at least 150 mg/ml, optionally at least 170 mg/ml, optionally at least 190 mg/ml, optionally at least 200 mg/ml, optionally at least 250 mg/ml, optionally at least 300 mg/ml, optionally at least 350 mg/ml, optionally at least 400 mg/ml,
optionally soluble in a solvent selected from the group consisting of
a) a buffer selected from the group consisting of any one or more of: MES, Bis-tris methane, ADA, ACES, Bis-tris propane, PIPES, MOPSO, Cholamine chloride, MOPS, BES, TES, HEPES, DIPSO, MOBS, Acetamidoglycine, TAPSO, TEA, POPSO, HEPPSO, EPS, HEPPS, Tricine, Tris, Glycinamide, Glycylglycine, HEPBS, Bicine, TAPS, AMPB, CHES, AMP, AMPSOCAPSO, CAPS, CABS, Bicine, sodium citrate buffer, sodium bicarbonate buffer, phosphate buffer, borate buffer,
   optionally wherein the buffer comprises one or more salts, optionally sodium chloride and/or potassium chloride,
   optionally wherein the buffer is at a pH range of 3 to 10, optionally pH 7;
b) an organic solvent, optionally HFIP.

As discussed previously, the beta solenoid domain preferably has a high net positive or a high net negative charge. Preferences for the net charge of the beta solenoid domain are as discussed previously.

As discussed earlier, the concatemer and/or the beta solenoid domain of the invention may be capable of being expressed by a cell or cell-free transcription and translation system. Preferences are as above.

Both the concatemer and/or the beta solenoid domain may comprise one or more tagging sequences. Accordingly, in one embodiment of any aspect of the invention the concatemer or beta solenoid domain may comprise one or more tag sequences. Tag sequences are well known in the art and are commonly used for purification or visualisation of the protein for example on a western blot or through fluorescence microscopy. Examples of useful tags include, but are not limited to, Albumin-binding protein (ABP), Alkaline Phosphatase (AP), AU1 epitope, AU5 epitope, Bacteriophage T7 epitope (T7-tag), Bacteriophage V5 epitope (V5-tag), Biotin-carboxy carrier protein (BCCP), Bluetongue virus tag (B-tag), Calmodulin binding peptide (CBP), Chloramphenicol Acetyl Transferase (CAT), Cellulose binding domain (CBP), Chitin binding domain (CBD), Choline-binding domain (CBD), Dihydrofolate reductase (DHFR), E2 epitope, FLAG epitope (DYKDDDDK), Galactose-binding protein (GBP), Green fluorescent protein (GFP), Glu-Glu (EE-tag), Glutathione S-transferase (GST), Human influenza hemagglutinin (HA), HaloTag^{®}, Histidine affinity tag (HAT), Horseradish Peroxidase (HRP), HSV epitope, Ketosteroid isomerase (KSI), KT3 epitope, LacZ, Luciferase, Maltose-binding protein (MBP), Myc epitope, NusA, PDZ domain, PDZ ligand, Polyarginine (Arg-tag), Polyaspartate (Asp-tag, Polycysteine (Cys-tag), Polyhistidine (His-tag), Polyphenylalanine (Phe-tag), Profinity eXact, Protein C S1-tag, S-tag, Streptavadin-binding peptide (SBP) MDEKTTGWRGGHVVEGLAGELEQLRARLEHHPQGQREP), Staphylococcal protein A (Protein A), Staphylococcal protein G (Protein G), Strep-tag (WSHPQFEK), Streptavadin, Small Ubiquitin-like Modifier (SUMO), Tandem Affinity Purification (TAP), T7 epitope, Thioredoxin (Trx), TrpE, Ubiquitin, Universal. Such tags are described in the prior art, for example Kimple et al 2013 Curr Protoc Protein Sci. 2013; 73: Unit-9.9.

In one embodiment it is considered advantageous that the concatemer and/or beta solenoid domain comprises a protease cleavage site, and/or a purification tag sequence.

The tag sequence used in the examples is a His-tag which also comprises a thrombin cleavage site [SEQ ID NO: 33]. Other protease cleavage sites may be used, for example a TEV protease site (ENLYFQS).

For the avoidance of doubt, examples are provided below as to some of the various combinations of beta solenoid unit sequence, capping sequence, tag sequence, and tail sequence that are covered by the present invention. The tail sequence is an optional hydrophilic sequence, with a flexible structure, which can serve the purpose of increasing protein solubility and/or inhibit crystallisation.

In one embodiment a beta solenoid domain can be considered to consist of different combination of all of, or any subset of, the following components: - a TAG sequence (TAG), an N-terminal cap sequence (NCAP), a beta solenoid sequence, i.e. a sequence that comprises at least 4 units of a consensus motif (UNITS), a C-terminal cap sequence (CCAP); and a "tail" sequence (TAIL). Only the presence of the UNITS component is considered to be essential.

For example, in one embodiment a beta solenoid domain comprises or consists of all of TAG, NCAP, UNITS, CCAP and TAIL. In other embodiments the beta solenoid domain comprises or consists of TAG, NCAP, UNITS and CCAP, but no TAIL. In other embodiments the beta solenoid domain comprises or consists of NCAP, UNITS, CCAP and TAIL, but no TAG. In further embodiments the beta solenoid domain comprises or consists of NCAP, UNITS and CCAP, but no TAG and TAIL. In other embodiments the beta solenoid domain comprises or consists of TAG, UNITS, CCAP and TAIL; or TAG UNITS, CCAP and no TAIL. Further, the beta solenoid domain may comprises or consists of TAG, NCAP, UNITS and TAIL; or TAG, NCAP, UNITS and no TAIL. Any other such combinations are also encompassed here. It is considered that the UNITS component is essential to the invention and that the beta solenoid preferably comprises or consists of one or more, or both, of the NCAP and CCAP, though this is not essential. The TAG sequence and TAIL sequence are considered to be optional sequences, to allow, for example, purification of the beta solenoid domain from, for example, a cell extract of culture media.

The beta solenoid domain may comprise or consist of any number of each of the following components.

Accordingly, in one embodiment, a single beta solenoid domain (which may or may not be part of a concatemer of the invention) may comprise or consist of the following components in the following order:
TAG - NCAP - UNITSₙ - CCAP

Where n is at least 4.

In a further embodiment a single beta domain may comprise or consist of the following components in the following order:
TAG - NCAP - UNITSₙ - CCAP - TAIL

Where n is at least 4.

A concatemer according to the present invention may comprise or consist of the following components in the following order:
TAG - [NCAP - UNITSₙ - CCAP - LINKER]ₓ : or
TAG - [NCAP - UNITSₙ - CCAP - LINKER]ₓ - TAIL

In other embodiments there may be no linker sequence between the final CCAP component and the TAIL sequence, if present:
TAG - [NCAP - UNITSₙ - CCAP - LINKER]ₓ₋₁ - NCAP - UNITS - CCAP - TAIL

Where n is at least 4 and x is at least 2.

In other embodiments, the TAG sequence may be located at the C-terminus of the beta solenoid domain or concatemer, for instance:

### Beta solenoid domain:

NCAP - UNITSₙ - CCAP - TAG
NCAP - UNITSₙ - CCAP - TAIL - TAG

Where n is at least 4.

### Concatemer:

[NCAP - UNITSₙ - CCAP - LINKER]ₓ - TAG : or
[NCAP - UNITSₙ - CCAP - LINKER]ₓ - TAIL- TAG

In other embodiments there may be no linker sequence between the final CCAP component and the TAIL sequence, if present:
[NCAP - UNITSₙ - CCAP - LINKER]ₓ₋₁ - NCAP - UNITS - CCAP - TAIL- TAG

Where n is at least 4 and x is at least 2.

In other embodiments, no TAG may be present, for instance:

### Beta solenoid domain:

NCAP - UNITSₙ - CCAP
NCAP - UNITSₙ - CCAP - TAIL

Where n is at least 4.

### Concatemer:

[NCAP - UNITSₙ - CCAP - LINKER]ₓ: or
[NCAP - UNITSₙ - CCAP - LINKER]ₓ - TAIL

In other embodiments there may be no linker sequence between the final CCAP component and the TAIL sequence, if present:
[NCAP - UNITSₙ - CCAP - LINKER]ₓ₋₁ - NCAP - UNITS - CCAP - TAIL

Where n is at least 4 and x is at least 2.

In other embodiments, no NCAP and/or CCAP may be present, for instance:

### Beta solenoid domain:

UNITSₙ - CCAP - TAG
UNITSₙ - CCAP - TAIL - TAG
NCAP - UNITSₙ - TAG
NCAP - UNITSₙ - TAIL - TAG
UNITSₙ - TAG
UNITSₙ - TAIL - TAG
UNITSₙ

Where n is at least 4.

### Concatemer:

[UNITSₙ - CCAP - LINKER]ₓ- TAG : or
[UNITSₙ - CCAP - LINKER]ₓ - TAIL- TAG
[UNITSₙ - CCAP - LINKER]ₓ₋₁ - NCAP - UNITS - CCAP - TAIL- TAG
[UNITSₙ - CCAP - LINKER]ₓ₋₁ -UNITS - CCAP - TAIL- TAG
[UNITSₙ - CCAP - LINKER]ₓ₋₁ -UNITS - TAIL- TAG
[NCAP - UNITSₙ- LINKER]ₓ- TAG : or
[NCAP - UNITSₙ- LINKER]ₓ - TAIL- TAG
[NCAP - UNITSₙ- LINKER]ₓ₋₁ - NCAP - UNITS - CCAP - TAIL- TAG
[NCAP - UNITSₙ- LINKER]ₓ₋₁ -UNITS - CCAP - TAIL- TAG
[NCAP - UNITSₙ- LINKER]ₓ₋₁ -UNITS - TAIL- TAG
[UNITSₙ- LINKER]ₓ- TAG : or
[UNITSₙ- LINKER]ₓ - TAIL- TAG
[UNITSₙ- LINKER]ₓ₋₁ - NCAP - UNITS - CCAP - TAIL- TAG
[UNITSₙ- LINKER]ₓ₋₁ -UNITS - CCAP - TAIL- TAG
[UNITSₙ- LINKER]ₓ₋₁ -UNITS - TAIL- TAG
NCAP - [UNITSₙ- LINKER]ₓ - TAG : or
NCAP - [UNITSₙ- LINKER]ₓ - TAIL- TAG
NCAP - [UNITSₙ- LINKER]ₓ₋₁ - NCAP - UNITS - CCAP - TAIL- TAG
NCAP - [UNITSₙ- LINKER]ₓ₋₁ -UNITS - CCAP - TAIL- TAG
NCAP - [UNITSₙ- LINKER]ₓ₋₁ -UNITS - TAIL- TAG
(i.e. variants where only NCAP on first UNIT) for example.

Where n is at least 4 and x is at least 2.

In some embodiments the cap sequences (NCAP and/or CCAP) are present on all of the beta solenoid domains. In other embodiments the cap sequences (NCAP and/or CCAP) are present on only some of the beta solenoid domains.

One example of a TAIL sequence is KSDDG. Preferred features of TAIL sequences are that the sequence should be between 1-10 residues that are selected from the group consisting of charged, polar, glycine and alanine residues. In other embodiments, the TAIL sequence could be identical to the LINKER sequence.

The TAIL sequence is considered to be an optional feature. Accordingly, in one embodiment the beta solenoid domain comprises a TAIL sequence. In another embodiment the beta solenoid domain does not comprise a TAIL sequence.

All combinations and permutations of the presence, absence and any number of NCAP, UNITS, LINKER, CCAP, TAIL and TAG are contemplated and included in the invention, though the presence of the UNITS is considered to be essential.

In some embodiments the beta solenoid domain of the invention or the concatemer of the invention comprises a solenoid domain (UNITS) sequence, an N-terminal cap sequence, a C-terminal cap sequence, and a TAIL sequence selected from the list below:

| Solenoid domain (UNITS) [SEQ ID NO:] | NCAP [SEQ ID NO:] | CCAP [SEQ ID NO:] | TAIL [SEQ ID NO:] |
|---|---|---|---|
| 1, 8, 9, 10, 11, 12, 13, 14, 15, 16, 17, 18, 19, 20, 21, 22, 23, 24, 25, 26, 27, 28, 169 | 2, 84, 85, 86, 87, 88, 89, 90, 91, 92, 93, 94, 95, 96, 97, 98, 99, 100, 101, 102, 103, 104, | 3, 105, 106, 107,108, 109, 110, 111, 112, 113, 114, 115, 116, 117, 118, 119, 120, 121, 122, 123, 124, 125 | 81, 126, 127, 128, 129, 130, 131, 132, 133, 134, 135, 136, 137, 138, 139, 140, 141, 142, 143, 144, 145, 146 |

In specific examples, a single beta solenoid domain of the invention, or a concatemer of the invention comprises the following combination of sequences (e.g. SEQ ID NO: 1, 2, 3 and 81; SEQ ID NO: 8, 84, 105 and 126, for example):

| Beta solenoid domain name used herein | Solenoid domain (UNITS) [SEQ ID NO:] | NCAP [SEQ ID NO:] | CCAP [SEQ ID NO:] | TAIL [SEQ ID NO:] |
|---|---|---|---|---|
| New caps | 1 | 2 | 3 | 81 |
| 20_0004 | 8 | 84 | 105 | 126 |
| 21_0003 | 9 | 85 | 106 | 127 |
| 22_0001 | 10 | 86 | 107 | 128 |
| 23_0004 | 11 | 87 | 108 | 129 |
| 24_0002 | 12 | 88 | 109 | 130 |
| 25_0003 | 13 | 89 | 110 | 131 |
| 26_0004 | 14 | 90 | 111 | 132 |
| 27_0004 | 15 | 91 | 112 | 133 |
| 28_0002 | 16 | 92 | 113 | 134 |
| 29_0005 | 17 | 93 | 114 | 135 |
| 30_0005 | 18 | 94 | 115 | 136 |
| 31_0005 | 19 | 95 | 116 | 137 |
| 32_0001 | 20 | 96 | 117 | 138 |
| 33_0004 | 21 | 97 | 118 | 139 |
| 34_0001 | 22 | 98 | 119 | 140 |
| 35_0005 | 23 | 99 | 120 | 141 |
| 36_0005 | 24 | 100 | 121 | 142 |
| 37_0002 | 25 | 101 | 122 | 143 |
| 38_0005 | 26 | 102 | 123 | 144 |
| 39_0003 | 27 | 103 | 124 | 145 |
| 40_0004 | 28 | 104 | 125 | 146 |

Also provided herein is a composition comprising a concatemer of the invention and/or a beta solenoid domain of the invention.

The composition may comprise the concatemer and/or beta solenoid domain at a concentration of at least 20 mg/ml, optionally at least 30 mg/ml, optionally at least 40 mg/ml, optionally at least 50 mg/ml, optionally at least 60 mg/ml, optionally at least 70 mg/ml, optionally at least 80 mg/ml, optionally at least 90 mg/ml, optionally at least 100 mg/ml, optionally at least 120 mg/ml, optionally at least 130 mg/ml, optionally at least 150 mg/ml, optionally at least 170 mg/ml, optionally at least 190 mg/ml, optionally at least 200 mg/ml, optionally at least 250 mg/ml, optionally at least 300 mg/ml, optionally at least 350 mg/ml, optionally at least 400 mg/ml.

Preferably the composition comprises the concatemer or beta solenoid domain in a non-denatured state. The skilled person will be able to select a suitable solvent. Such solvents may include, for example:
a) a buffer selected from the group consisting of any one or more of: MES, Bis-tris methane, ADA, ACES, Bis-tris propane, PIPES, MOPSO, Cholamine chloride, MOPS, BES, TES, HEPES, DIPSO, MOBS, Acetamidoglycine, TAPSO, TEA, POPSO, HEPPSO, EPS, HEPPS, Tricine, Tris, Glycinamide, Glycylglycine, HEPBS, Bicine, TAPS, AMPB, CHES, AMP, AMPSOCAPSO, CAPS, CABS, Bicine, sodium citrate buffer, sodium bicarbonate buffer, phosphate buffer, borate buffer,
   optionally wherein the buffer comprises one or more salts, optionally sodium chloride and/or potassium chloride,
   optionally wherein the buffer is at a pH range of 3 to 10, optionally pH 7;
b) an organic solvent, optionally HFIP.

In a preferred example, the concatemer and/or beta solenoid domain is for spinning into a fibre, wherein such spinning involves extrusion into a coagulation solvent. Accordingly, in a preferred embodiment the composition does not comprise a solvent in which the concatemer and/or beta solenoid domain is not soluble, for example does not comprise, in one embodiment ethanol, propanol, acetone, diethyl ether or DMSO. Preferences for the solvent are as discussed above.

As will be appreciated, since the concatemer and beta solenoid domains of the present invention are made from a series of amino acids, they are capable of being encoded by a nucleic acid. Accordingly, the present invention provides a nucleic acid that encodes any of the beta solenoid domains or concatemers of the invention described herein, for example, with and without tag sequences, with and without capping sequences etc.

Similarly, the invention provides a vector comprising the nucleic acid described above. The skilled person will understand what is meant by the term vector, and typically is used to refer to a plasmid, but other nucleic acid vectors are known, which may be single stranded or double stranded, and may be DNA or RNA, for example.

The invention also protects a host cell comprising the nucleic acid and/or the vector described above. In one embodiment the host cell is any of:
a) a prokaryotic cell, optionally a bacterial cell, optionally an *E. coli* cell, a *Bacillus subtilis* cell, a *Bacillus megaterium* cell, a *Vibrio natriegens* cell, or a *Pseudomonas fluorescens* cell; or
b) is a eukaryotic cell, optionally

a yeast cell, optionally *Pichia pastoris* or Saccharomyces cerevisiae;
an insect cell, optionally a baculovirus infected insect cell; or
a mammalian cell, optionally a baculovirus infected mammalian cell, a HEK293 cell, a HeLa cell, or CHO cells.

As discussed previously, the concatemers and beta solenoid domains of the invention are suitable for use in the formation of fibres with particularly beneficial properties. Accordingly a further aspect of the invention provides a fibre comprising the concatemer according to the invention or the beta solenoid domain according to the invention. All preferences described above also apply here.

The fibre of the invention may be made by any means. Typically the fibre may be made by a wet-spinning method which is well known in the art, for example in the field of aramids.

Advantageously, the fibres of the present invention may, in one embodiment, have an average diameter of between 100 nm and 100um, optionally between 500 nm and 50um, optionally between 1000nm and 40um, optionally between 2.5um and 35 um, optionally between 5 um and 30 um, optionally between 7.5 um and 25 um, optionally between 10 um and 20 um.

Fibres of the present invention may in one embodiment have an advantageous tensile elasticity, for example may have a Young's modulus of between 0.5 GPa and 200 GPa, optionally between 1.0 GPa and 175 GPa, optionally between 2 GPa and 150 GPa, optionally between 5.0 GPa and 125 GPa, optionally between 10.0 GPa and 100.0 GPa, optionally between 15.0 GPa and 90.0 GPa, optionally between 20.0 GPa and 80.0 GPa, optionally between 25.0 GPa and 75.0 GPa, optionally between 30.0 GPa and 70.0 GPa, optionally between 35.0 GPa and 60.0 GPa, optionally between 40.0 GPa and 50.0 GPa.

In another embodiment the fibres of the present invention may have a Young's modulus of at least 0.5 GPa, 1.0 GPa, 2 GPa, 5.0 GPa, 10.0 GPa, 15.0 GPa, 20.0 GPa, 25.0 GPa, 30.0GPa, 35.0 GPa, 40.0 GPa, 50.0 GPa, 60.0 GPa, 70.0 GPa, 75.0 GPa, 80.0 GPa, 90.0 GPa, 100.0 GPa, 125 GPa, 150 GPa, 175 GPa or at least 200 GPa.

In a further embodiment, the fibre of the invention may have an engineered strength of:
between 0.1 GPa and 3 GPa, optionally between 0.2 GPa and 2.8 GPa, optionally between 0.3 GPa and 2.6 GPa, optionally between 0.4 GPa and 2.4 GPa, optionally between 0.5 GPa and 2.2 GPa, optionally between 0.6 GPa and 2.0 GPa, optionally between 0.7 GPa and 1.8 GPa, optionally between 0.8 GPa and 1.6 GPa, optionally between 0.9 GPa and 1.4 GPa, optionally between 1.0 GPa and 1.2 GPa; and/or
at least 0.1 GPa, optionally at least 0.2 GPa, optionally at least 0.3 GPa, optionally at least 0.4 GPa, optionally at least 0.5 GPa, optionally at least 0.6 GPa, optionally at least 0.7 GPa, optionally at least 0.8 GPa, optionally at least 0.9 GPa, optionally at least 1.0 GPa, optionally at least 1.1 GPa, optionally at least 1.2 GPa, optionally at least 1.3 GPa, optionally at least 1.4 GPa, optionally at least 1.5 GPa, optionally at least 1.6 GPa, optionally at least 1.7 GPa, optionally at least 1.8 GPa, optionally at least 1.9 GPa, optionally at least 2.0 GPa, optionally at least 2.1 GPa, optionally at least 2.2 GPa, optionally at least 2.3 GPa, optionally at least 2.4 GPa, optionally at least 2.5 GPa, optionally at least 2.6 GPa, optionally at least 2.7 GPa, optionally at least 2.8 GPa, optionally at least 2.9 GPa, optionally at least 3.0 GPa.

Furthermore, in another embodiment, the fibre of the present invention may have a strain to failure of between 2% and 300%, optionally between 5% and 275%, optionally between 10% and 250%, optionally between 15% and 225%, optionally between 20% and 200%, optionally between 25% and 175%, optionally between 30% and 150%, optionally between 35% and 125%, optionally between 40% and 100%, optionally between 45% and 80%, optionally between 50% and 75%, optionally between 55% and 70%.

In another embodiment, the fibre of the present invention may have a strain to failure of at least 2% , 5%, 10%, 15%, 20%, 25%, 30%, 35%, 40%, 45%, 50%, 55%, 60%, 65%, 70%, 75%, 80%, 90%, 100%, 125%, 150%, 175%, 200%, 225%, 250%, 275% or at least 300%.

In one embodiment the fibres of the present invention are considered to have a toughness of at least 10 J/g, optionally at least 20 J/g, optionally at least 40 J/g, optionally at least 60 J/g, optionally at least 80 J/g, optionally at least 100 J/g, optionally at least 120 J/g, optionally at least 140 J/g, optionally at least 160 J/g, optionally at least 180 J/g, optionally at least 200 J/g, optionally at least 250 J/g, optionally at least 300 J/g, optionally at least 350 J/g, optionally at least 400 J/g, optionally at least 450 J/g, optionally at least 500 J/g, optionally at least 550 J/g, optionally at least 600 J/g, optionally at least 650 J/g, optionally at least 700 J/g, optionally at least 750 J/g, optionally at least 800 J/g, optionally at least 850 J/g, optionally at least 900 J/g, optionally at least 950 J/g, optionally at least 1000 J/g.

The fibres of the present invention may be made by spinning the concatemers of the invention, or by otherwise combining the concatemers of the invention into a fibre. In one embodiment, the concatemers in the fibre are the same, i.e. the fibre comprises concatemers of the same amino acid sequence. In other embodiments, the fibres comprise different concatemers, i.e. comprise concatemers with different amino acid sequences, for example comprise concatemers with at least 2 different amino acid sequences, for example at least 3 or at least 4 or at least 5 or at least 6 or at least 7 or at least 8 or at least 9 or at least 10 different amino acid sequences. For example beta solenoid domains with different cross-sectional shapes may be linked within a single concatemer. Various combinations of concatemers are considered to produce a fibre with different properties.

The skilled person will understand that a fibre of the invention can be used to form a yarn. Accordingly, the invention also provides a yarn comprising a fibre of the invention. In a further embodiment the yarn of the invention comprises at least two different fibres of the invention, i.e. at least two fibres that comprise a different combination of concatemers. The invention also provides a textile or fabric comprising the concatemer according to the invention, the beta solenoid domain according to the invention, the fibre according to the invention or the yarn according to the invention. The textile or fabric may be woven or non-woven, and may also be coated in sizing.

Given the advantageous properties, such as the strength and tensile elasticity of the fibres, yarns and textiles of the present invention, the products of the present invention are considered to be suitable for a number of applications. For example, some exemplary applications of the present invention are in the production of:
Sportswear (e.g. for protective uses such as rash guards); Protective wear for motorcyclists and/or other motorsports; Personal protective equipment (e.g. body armour, protective underwear for soldiers); Blast containment; Blade containment (e.g. for aircraft engines); Textiles for parachutes; Ropes; Textiles for clothing; Textiles for shoes; and the like.

The fibres, yarns, fabrics and textiles of the present invention are also considered to have a number of medical applications which include: general medical textiles; Wound dressings; Resorbable and non-resorbable surgical sutures; Resorbable and non-resorbable stents; Guide-wires for surgery (including non-metallic guide wires suitable for MRI); Biocompatible coatings for implanted medical devices; Surgical meshes; Scaffolds for 3D tissue engineering and the like.

The listing or discussion of an apparently prior-published document in this specification should not necessarily be taken as an acknowledgement that the document is part of the state of the art or is common general knowledge.

Preferences and options for a given aspect, feature or parameter of the invention should, unless the context indicates otherwise, be regarded as having been disclosed in combination with any and all preferences and options for all other aspects, features and parameters of the invention. For example, the invention provides a concatemer of beta solenoid domains wherein the beta solenoid domains comprise 7 units of a consensus sequence and wherein the beta solenoid domains comprises a capping sequence only at the N-terminus. The invention also provides a concatemer of 20 beta solenoid domains wherein each beta solenoid domain comprises 20 units of a consensus sequence, wherein each unit of the consensus sequence has a different amino acid sequence, and wherein each beta solenoid domain comprises a capping sequence at both ends of each of the 20 beta solenoid domains, and wherein the concatemer comprises a His-tag at the N-terminus. The invention also provides a concatemer of beta solenoid domains wherein the concatemer is capable of forming a lyotropic liquid crystal mesophase, and wherein the concatemer has an aspect ratio of 1:8 and a net positive charge of +200 charge units. Similarly the invention provides an item of clothing made from a fabric that comprises a fibre according to the present invention, wherein the fibre comprises a concatemer of 20 beta solenoid domains wherein each beta solenoid domain comprises 20 units of a consensus sequence, wherein each unit of the consensus sequence has a different amino acid sequence, and wherein each beta solenoid domain comprises a capping sequence at both ends of each of the 20 beta solenoid domains, and wherein the concatemer comprises a His-tag at the N-terminus.

### SEQUENCES

### Key to sequence features

Shading = N-terminal cap
*Italics = C-terminal cap*
Underline - solenoid domain sequence
Bold = **tail** sequence
Lowercase = His-tag and thrombin protease sequence
SynRFR24.new_caps solenoid domain UNITSn [SEQ ID NO: 1] 
ASLTGADLSYANLHHANLSRANLRSADLRNANLSHANLSGANLEEANLEAANLRGADLH
EANLSGADLQEANLTQANLKDANLSDANLEQADLAGADLQGAVLDGANLHGANLNNAN
LSEAMLTRANLEQADLSG
Cap sequence 1 SynRFR24.new_caps [SEQ ID NO: 2]
   RMRRDEILEEYRRGRRNFQHINLQEIELTN
Cap sequence 2 SynRFR24.new_caps [SEQ ID NO: 3]
   *ARTTGARLDDADLRGATVDPVLWRTASLRGAKMEEEQRREYERARG*
SynRFR24.1 solenoid domain [SEQ ID NO: 1] 
ASLTGADLSYANLHHANLSRANLRSADLRNANLSHANLSGANLEEANLEAANLRGADLH
EANLSGADLQEANLTQANLKDANLSDANLEQADLAGADLQGAVLDGANLHGANLNNAN
LSEAMLTRANLEQADLSG
Cap sequence 1 original SynRFR24.1 [SEQ ID NO: 4]
   HMNVGEILRHYAAGKRNFQHINLQEIELTN
Cap sequence 2 original SynRFR24.1 [SEQ ID NO: 5]
   *ARTTGARLDDADLRGATVDPVLWRTASLVGARVDVDQAVAFAAAHGLCLAGG*
>SynRFR24.new_caps solenoid domain plus both caps plus his tag [SEQ ID NO: 6] 
mgsshhhhhhssglvprgsRMRRDEILEEYRRGRRNFQHINLQEIELTNASLTGADLSYANLH
HANLSRANLRSADLRNANLSHANLSGANLEEANLEAANLRGADLHEANLSGADLQEAN
LTQANLKDANLSDANLEQADLAGADLQGAVLDGANLHGANLNNANLSEAMLTRANLEQ
ADLSGARTTGARLDDADLRGATVDPVLWRTASLRGAKMEEEQRREYERARGKSDDG
>SynRFR24.1 solenoid domain plus both caps plus his tag [SEQ ID NO: 7]

mgsshhhhhhssglvprgsHMNVGEILRHYAAGKRNFQHINLQEIELTNASLTGADLSYA
NLHHANLSRANLRSADLRNANLSHANLSGANLEEANLEAANLRGADLHEANLSGADLQ
EA
NLTQANLKDANLSDANLEQADLAGADLQGAVLDGANLHGANLNNANLSEAMLTRANLE
QA
DLSGARTTGARLDDADLRGATVDPVLWRTASLVGARVDVDQAVAFAAAHGLCLAGGS
GC

Further solenoid domain sequences:
[SEQ ID NO: 8]
   >rfr_ref_minprep_ren_neg_num_20_0004 UNITSn 
AKLAKANLSNANLKKARLQNANLANAKLQNANLENANLAGANLKKANLRGANLHGADL
AGANLKGADLREADLRNADLRKANLTGAKLKNAKLEGANLADATLKDANLHGAKLKNA
NLARANLERADLRNADLTG
[SEQ ID NO: 9] 
>rfr_ref_minprep_ren_neg_num_21_0003 UNITSn
AKLAGADLSNAKLHNANLENADLKGANLAGANLQKANLHGADLTNADLRKANLANANL
ANANLEGARLADADLRNADLRGAKLKRADLENADLTGANLKGANLEGARLERANLKKA
RLQNAKLAGADLRNARLAG
[SEQ ID NO: 10] 
>rfr_ref_minprep_ren_neg_num_22_0001 UNITSn
ATLAGANLTGAKLHNARLENADLKDAKLARANLQKANLRGANLRNADLREADLANANLA
RADLTGAKLADADLRNADLREANLKNANLARANLKGANLHGANLHGARLEKANLENAN
LQNANLQNANLANADLRG
[SEQ ID NO: 11] 
>rfr_ref_minprep_ren_neg_num_23_0004 UNITSn
AKLAGADLSNADLRNANLQNANLANANLENADLENADLKGARLENANLRRANLRGANL
AEARLEKADLRNANLANADLRRANLRNAELQNADLQGANLEGADLTGANLKGAKLKNA
NLQNARLAKADLRNADLTG
[SEQ ID NO: 12] 
>rfr_ref_minprep_ren_neg_num_24_0002 UNITSn
AELAGANLAGANLKNANLRNANLKDADLRKANLQKADLKGANLQNARLQGANLENANL
AGARLQGANLAKADLRGADLRNANLTNARLEKADLTDADLHGARLQGADLHGANLERA
RLQNANLAEADLRNANLTG
[SEQ ID NO: 13] 
>rfr_ref_minprep_ren_neg_num_25_0003 UNITSn
ATLAGADLSNANLKQADLARANLKDANLQNANLKNAKLEGANLAKANLEKANLHGANLA
RADLTGANLANAKLENADLRKADLTNADLTNADLRGADLEGANLHGANLKGANLRKAK
LKRAELQNADLRNADLTG
[SEQ ID NO: 14] 
>rfr_ref_minprep_ren_neg_num_26_0004 UNITSn
ATLAGAKLRNANLHNARLQNANLANADLQQADLEEANLHGANLANANLEEANLRRADL
ANADLTGANLANADLRQADLRGANLRNARLQKANLADADLHGADLHGANLEGANLRRA
RLENAKLANADLRNADLKG
[SEQ ID NO: 15] 
>rfr_ref_minprep_ren_neg_num_27_0004 UNITSn
ATLAGADLSKANLHRADLKNANLKGANLEKADLREADLHGADLTNANLHGARLARADLA
NANLKGADLANADLREANLHGADLTNARLQNARLQDADLHGANLTGARLHGADLTNAR
LQRADLSKADLRNANLEG
[SEQ ID NO: 16] >rfr_ref_minprep_ren_neg_num_28_0002 UNITSn 
ATLAGADLRNANLKNANLQKANLANANLRNANLQGADLAKARLENADLHGARLAKANL
AGANLHGADLANADLRNANLRNADLRNARLERADLAGADLEGANLHGANLREARLENA
NLKRANLQNANLANADLTG
[SEQ ID NO: 17] 
>rfr_ref_minprep_ren_neg_num_29_0005 UNITSn
ATLAGAKLEGADLTRAKLKDANLAKANLRNANLQEANLKGANLRNADLAGANLERANLA
KADLHGANLANADLREADLRNANLDNADLRNADLTGADLEGASLHGANLKKARLENAR
LRRARLENANLAKANLTG
[SEQ ID NO: 18] 
>rfr_ref_minprep_ren_neg_num_30_0005 UNITSn
ATLAGADLSGADLHNADLQNAKLKGANLEKARLRRANLRGADLTNANLRGADLEEADL
AGARLHGANLARARLANADLRNADLRNANLREADLTGANLHGANLHGADLERANLTNA
DLENARLERANLAEANLQD
[SEQ ID NO: 19] 
>rfr_ref_minprep_ren_neg_num_31_0005 UNITSn
ATLKGANLQDANLQEANLQNANLQGARLSNADLRRADLEGANLENADLSGARLQEANL
AGARLEGADLADADLRGADLRNADLTNARLERANLADADLHGADLHGAKLHGANLERA
RLENARLENADLRNAKLAG
[SEQ ID NO: 20] 
>rfr_ref_minprep_ren_neg_num_32_0001 UNITSn

ATLAGADLSNANLENADLQNADLANAKLQNANLARANLHGADLRNADLEGADLAEANL
ANANLEGADLANADLRNADLRGANLTNARLQNANLTGANLRGANLHGADLHGAKLRKA
DLEKARLENADLRKADLKG
[SEQ ID NO: 21] 
>rfr_ref_minprep_ren_neg_num_33_0004 UNITSn
AKLAGADLRDADLQRANLENADLANAKLKNANLEQANLEGANLENANLAKADLRNANL
ARADLTGADLSNADLRGADLRKANLTNADLTNADLEGADLTDARLQGANLHGANLTNA
NLARANLAGADLRNAKLSG
[SEQ ID NO: 22] 
>rfr_ref_minprep_ren_neg_num_34_0001 UNITSn
ATLSGAKLEGANLKRADLRNANLSKADLRNANLQQANLQGADLENADLQGANLEEANL
AKANLTGANLSNADLRKADLRNANLKDADLENADLEGANLHGADLHGAKLHGADLTRA
DLENARLENADLRNARLNG
[SEQ ID NO: 23] 
>rfr_ref_minprep_ren_neg_num_35_0005 UNITSn
AELAGADLANANLHRANLENANLANAKLEEANLEEANLAGADLRNANLQRANLARADLA
GADLTGANLAEADLREARLQKANLENADLTNADLTGADLEGARLHGANLEGANLTNAN
LRKANLENADLRNADLTG
[SEQ ID NO: 24] >rfr_ref_minprep_ren_neg_num_36_0005 UNITSn 
AELAGADLSGADLTRADLRDANLANADLQEANLAEAKLQGANLENANLARADLSRANL
AGADLHGANLAEADLRNADLRNANLQNADLRKARLQGADLEGANLEGANLHGADLEN
ADLARADLQKADLRNANLTG
[SEQ ID NO: 25] 
>rfr_ref_minprep_ren_neg_num_37_0002 UNITSn
ATLADADLSDARLHRADLQDADLENANLENADLSEANLRGADLRNANLRKADLARADL
AGANLHGADLANAKLAEADLRDADLRNANLENADLTGANLQGARLEGAKLHGANLTNA
NLKDADLSGADLRDANLTG
[SEQ ID NO: 26] 
>rfr_ref_minprep_ren_neg_num_38_0005 UNITSn
ATLKGANLQGARLHNADLEEAELKDANLAEADLQNANLRRADLTNADLANANLHGANL
AEANLHGANLAGADLRNADLENADLRNANLEDADLKGANLHGANLQGANLEGARLQN
ANLKNARLAEADLRNADLKG
[SEQ ID NO: 27] 
>rfr_ref_minprep_ren_neg_num_39_0003 UNITSn
AELAGADLSNADLRNAKLENANLADANLENADLENANLRGADLENADLRRADLHGADL
AKANLEGANLAEADLRNADLRNANLTNADLKNADLEGADLHGARLEGANLEGANLENA
DLANADLSDADLRKADLTG
[SEQ ID NO: 28] 
>rfr_ref_minprep_ren_neg_num_40_0004 UNITSn
AELAGADLSDANLEQANLQNANLANANLENANLQDADLHGARLQNADLRGANLEDADL
ANARLEGANLAEADLRRADLRGADLTNADLEDADLEGANLHGARLEGANLRGANLTDA
RLENADLSGADLRNANLEG
[SEQ ID NO: 169] 
AELAGADLANANLHRANLENANLANAKLEEANLEEANLAGADLRNANLQRANLARADLA
GADLTGANLAEADLREARLQSANLENADLTNADLTGADLEGARLHGANLEGANLTNAN
LRKANLENADLRNADLTG
> SynRFR24.new_caps.x4.1 concatemer of four beta solenoid domains, not including N-terminal His tag [SEQ ID NO: 29] 
•	RMRRDEILEEYRRGRRNFQHINLQEIELTNASLTGADLSYANLHHANLSRANLR
SADLRNANLSHANLSGANLEEANLEAANLRGADLHEANLSGADLQEANLTQAN
LKDANLSDANLEQADLAGADLQGAVLDGANLHGANLNNANLSEAMLTRANLEQ
ADLSGARTTGARLDDADLRGATVDPVLWRTASLRGAKMEEEQRREYERARGG
GGSRMRRDEILEEYRRGRRNFQHINLQEIELTNASLTGADLSYANLHHANLSRA
NLRSADLRNANLSHANLSGANLEEANLEAANLRGADLHEANLSGADLQEANLT
QANLKDANLSDANLEQADLAGADLQGAVLDGANLHGANLNNANLSEAMLTRAN
LEQADLSGARTTGARLDDADLRGATVDPVLWRTASLRGAKMEEEQRREYERA
RGGGGSRMRRDEILEEYRRGRRNFQHINLQEIELTNASLTGADLSYANLHHANL
SRANLRSADLRNANLSHANLSGANLEEANLEAANLRGADLHEANLSGADLQEA
NLTQANLKDANLSDANLEQADLAGADLQGAVLDGANLHGANLNNANLSEAMLT
RANLEQADLSGARTTGARLDDADLRGATVDPVLWRTASLRGAKMEEEQRREY
ERARGGGGSRMRRDEILEEYRRGRRNFQHINLQEIELTNASLTGADLSYANLHH
ANLSRANLRSADLRNANLSHANLSGANLEEANLEAANLRGADLHEANLSGADL
 
QEANLTQANLKDANLSDANLEQADLAGADLQGAVLDGANLHGANLNNANLSEA
MLTRANLEQADLSGARTTGARLDDADLRGATVDPVLWRTASLR
GAKMEEEQRREYERARGKSDDG
> SynRFR24.new_caps.x4.1 concatemer of four beta solenoid domains, including N-terminal His tag [SEQ ID NO: 30] 
•	mgsshhhhhhssglvprgsRMRRDEILEEYRRGRRNFQHINLQEIELTNASLTGADLSY
ANLHHANLSRANLRSADLRNANLSHANLSGANLEEANLEAANLRGADLHEANLS
GADLQEANLTQANLKDANLSDANLEQADLAGADLQGAVLDGANLHGANLNNAN
LSEAMLTRANLEQADLSGARTTGARLDDADLRGATVDPVLWRTASLRGAKMEE
EQRREYERARGGGGSRMRRDEILEEYRRGRRNFQHINLQEIELTNASLTGADL
SYANLHHANLSRANLRSADLRNANLSHANLSGANLEEANLEAANLRGADLHEA
NLSGADLQEANLTQANLKDANLSDANLEQADLAGADLQGAVLDGANLHGANLN
NANLSEAMLTRANLEQADLSGARTTGARLDDADLRGATVDPVLWRTASLRGAK
MEEEQRREYERARGGGGSRMRRDEILEEYRRGRRNFQHINLQEIELTNASLTG
ADLSYANLHHANLSRANLRSADLRNANLSHANLSGANLEEANLEAANLRGADLH
EANLSGADLQEANLTQANLKDANLSDANLEQADLAGADLQGAVLDGANLHGAN
LNNANLSEAMLTRANLEQADLSGARTTGARLDDADLRGATVDPVLWRTASLRG
AKMEEEQRREYERARGGGGSRMRRDEILEEYRRGRRNFQHINLQEIELTNASL
TGADLSYANLHHANLSRANLRSADLRNANLSHANLSGANLEEANLEAANLRGA
DLHEANLSGADLQEANLTQANLKDANLSDANLEQADLAGADLQGAVLDGANLH
GANLNNANLSEAMLTRANLEQADLSGARTTGARLDDADLRGATVDPVLWRTAS
LR GAKMEEEQRREYERARGKSDDG
> SynRFR24.new_caps.x8.1 concatemer of eight beta solenoid domains, not including N-terminal His tag [SEQ ID NO: 31] 
•	RMRRDEILEEYRRGRRNFQHINLQEIELTNASLTGADLSYANLHHANLSRANLR
SADLRNANLSHANLSGANLEEANLEAANLRGADLHEANLSGADLQEANLTQAN
LKDANLSDANLEQADLAGADLQGAVLDGANLHGANLNNANLSEAMLTRANLEQ
ADLSGARTTGARLDDADLRGATVDPVLWRTASLRGAKMEEEQRREYERARGG
GGSRMRRDEILEEYRRGRRNFQHINLQEIELTNASLTGADLSYANLHHANLSRA
NLRSADLRNANLSHANLSGANLEEANLEAANLRGADLHEANLSGADLQEANLT
QANLKDANLSDANLEQADLAGADLQGAVLDGANLHGANLNNANLSEAMLTRAN
LEQADLSGARTTGARLDDADLRGATVDPVLWRTASLRGAKMEEEQRREYERA
RGGGGSRMRRDEILEEYRRGRRNFQHINLQEIELTNASLTGADLSYANLHHANL
SRANLRSADLRNANLSHANLSGANLEEANLEAANLRGADLHEANLSGADLQEA
NLTQANLKDANLSDANLEQADLAGADLQGAVLDGANLHGANLNNANLSEAMLT
RANLEQADLSGARTTGARLDDADLRGATVDPVLWRTASLRGAKMEEEQRREY
ERARGGGGSRMRRDEILEEYRRGRRNFQHINLQEIELTNASLTGADLSYANLHH
ANLSRANLRSADLRNANLSHANLSGANLEEANLEAANLRGADLHEANLSGADL
QEANLTQANLKDANLSDANLEQADLAGADLQGAVLDGANLHGANLNNANLSEA
MLTRANLEQADLSGARTTGARLDDADLRGATVDPVLWRTASLRGAKMEEEQR
REYERARGGGGSRMRRDEILEEYRRGRRNFQHINLQEIELTNASLTGADLSYAN
LHHANLSRANLRSADLRNANLSHANLSGANLEEANLEAANLRGADLHEANLSG
ADLQEANLTQANLKDANLSDANLEQADLAGADLQGAVLDGANLHGANLNNANL
SEAMLTRANLEQADLSGARTTGARLDDADLRGATVDPVLWRTASLRGAKMEEE
QRREYERARGGGGSRMRRDEILEEYRRGRRNFQHINLQEIELTNASLTGADLS
 
YANLHHANLSRANLRSADLRNANLSHANLSGANLEEANLEAANLRGADLHEANL
SGADLQEANLTQANLKDANLSDANLEQADLAGADLQGAVLDGANLHGANLNNA
NLSEAMLTRANLEQADLSGARTTGARLDDADLRGATVDPVLWRTASLRGAKME
EEQRREYERARGGGGSRMRRDEILEEYRRGRRNFQHINLQEIELTNASLTGAD
LSYANLHHANLSRANLRSADLRNANLSHANLSGANLEEANLEAANLRGADLHEA
NLSGADLQEANLTQANLKDANLSDANLEQADLAGADLQGAVLDGANLHGANLN
NANLSEAMLTRANLEQADLSGARTTGARLDDADLRGATVDPVLWRTASLRGAK
MEEEQRREYERARGGGGSRMRRDEILEEYRRGRRNFQHINLQEIELTNASLTG
ADLSYANLHHANLSRANLRSADLRNANLSHANLSGANLEEANLEAANLRGADLH
EANLSGADLQEANLTQANLKDANLSDANLEQADLAGADLQGAVLDGANLHGAN
LNNANLSEAMLTRANLEQADLSGARTTGARLDDADLRGATVDPVLWRTASLRG
AKMEEEQRREYERARGKSDDG
> SynRFR24.new_caps.x8.1 concatemer of eight beta solenoid domains, including N-terminal His tag [SEQ ID NO: 32] 
•	mgsshhhhhhssglvprgsRMRRDEILEEYRRGRRNFQHINLQEIELTNASLTGADLSY
ANLHHANLSRANLRSADLRNANLSHANLSGANLEEANLEAANLRGADLHEANLS
GADLQEANLTQANLKDANLSDANLEQADLAGADLQGAVLDGANLHGANLNNAN
LSEAMLTRANLEQADLSGARTTGARLDDADLRGATVDPVLWRTASLRGAKMEE
EQRREYERARGGGGSRMRRDEILEEYRRGRRNFQHINLQEIELTNASLTGADL
SYANLHHANLSRANLRSADLRNANLSHANLSGANLEEANLEAANLRGADLHEA
NLSGADLQEANLTQANLKDANLSDANLEQADLAGADLQGAVLDGANLHGANLN
NANLSEAMLTRANLEQADLSGARTTGARLDDADLRGATVDPVLWRTASLRGAK
MEEEQRREYERARGGGGSRMRRDEILEEYRRGRRNFQHINLQEIELTNASLTG
ADLSYANLHHANLSRANLRSADLRNANLSHANLSGANLEEANLEAANLRGADLH
EANLSGADLQEANLTQANLKDANLSDANLEQADLAGADLQGAVLDGANLHGAN
LNNANLSEAMLTRANLEQADLSGARTTGARLDDADLRGATVDPVLWRTASLRG
AKMEEEQRREYERARGGGGSRMRRDEILEEYRRGRRNFQHINLQEIELTNASL
TGADLSYANLHHANLSRANLRSADLRNANLSHANLSGANLEEANLEAANLRGA
DLHEANLSGADLQEANLTQANLKDANLSDANLEQADLAGADLQGAVLDGANLH
GANLNNANLSEAMLTRANLEQADLSGARTTGARLDDADLRGATVDPVLWRTAS
LRGAKMEEEQRREYERARGGGGSRMRRDEILEEYRRGRRNFQHINLQEIELTN
ASLTGADLSYANLHHANLSRANLRSADLRNANLSHANLSGANLEEANLEAANLR
GADLHEANLSGADLQEANLTQANLKDANLSDANLEQADLAGADLQGAVLDGAN
LHGANLNNANLSEAMLTRANLEQADLSGARTTGARLDDADLRGATVDPVLWRT
ASLRGAKMEEEQRREYERARGGGGSRMRRDEILEEYRRGRRNFQHINLQEIEL
TNASLTGADLSYANLHHANLSRANLRSADLRNANLSHANLSGANLEEANLEAAN
LRGADLHEANLSGADLQEANLTQANLKDANLSDANLEQADLAGADLQGAVLDG
ANLHGANLNNANLSEAMLTRANLEQADLSGARTTGARLDDADLRGATVDPVLW
RTASLRGAKMEEEQRREYERARGGGGSRMRRDEILEEYRRGRRNFQHINLQEI
ELTNASLTGADLSYANLHHANLSRANLRSADLRNANLSHANLSGANLEEANLEA
ANLRGADLHEANLSGADLQEANLTQANLKDANLSDANLEQADLAGADLQGAVL
DGANLHGANLNNANLSEAMLTRANLEQADLSGARTTGARLDDADLRGATVDPV
LWRTASLRGAKMEEEQRREYERARGGGGSRMRRDEILEEYRRGRRNFQHINL
QEIELTNASLTGADLSYANLHHANLSRANLRSADLRNANLSHANLSGANLEEAN
LEAANLRGADLHEANLSGADLQEANLTQANLKDANLSDANLEQADLAGADLQG
AVLDGANLHGANLNNANLSEAMLTRANLEQADLSGARTTGARLDDADLRGATV
DPVLWRTASLRGAKMEEEQRREYERARGKSDDG
His tag + protease site [SEQ ID NO: 33]
   mgsshhhhhhssglvprgs

### Linker sequences

GGGS [SEQ ID NO: 34]
GGGSAAAAAAGGGS [SEQ ID NO: 168]
nucleic acid encoding SynRFT24.1 both capping sequences plus His tag [SEQ ID NO: 35] 
ATGGGTAGCAGCCATCATCATCATCACCATAGCAGCGGTCTGGTTCCGCGTGGTAGCCAC
ATGAATGTTGGTGAAATTCTGCGTCATTATGCAGCAGGTAAACGCAATTTTCAGCATATT
AACCTGCAAGAAATTGAACTGACCAATGCAAGCCTGACCGGTGCCGATCTGAGCTATGCA
AATCTGCATCATGCAAATCTGAGCCGTGCCAATCTGCGTTCTGCAGACCTGCGTAATGCC
AATCTGTCACATGCGAATCTGTCAGGTGCAAATCTGGAAGAAGCCAATCTGGAAGCCGCT
AATCTGCGTGGTGCGGATCTGCATGAAGCGAATCTGAGTGGCGCTGATCTGCAAGAGGCA
AATCTGACCCAGGCGAACCTGAAAGATGCTAATCTGTCTGATGCCAACCTGGAACAGGCA
GATCTGGCAGGGGCTGATCTGCAGGGTGCAGTTCTGGACGGTGCTAATCTGCATGGCGCA
AATCTGAATAATGCGAACCTGAGCGAAGCAATGCTGACCCGTGCGAATCTGGAGCAGGCC
GACCTGAGCGGTGCACGTACCACCGGTGCACGTCTGGATGATGCCGACCTGCGTGGTGCA
ACCGTTGATCCGGTTCTGTGGCGTACCGCAAGCCTGGTTGGTGCACGTGTTGATGTTGAT
CAGGCAGTTGCATTTGCAGCAGCACATGGTCTGTGTCTGGCAGGTGGATCCGGCTGCTAA
[SEQ **ID** NO: 36]- synRFR24.2 
mgsshhhhhhssglvprgsHMNVGEILRHYAAGKRNFQHINLQEIELTNASLTGADLSYA
NLHHANLSRANLRSADLRNANLSHANLSGANLEEANLEAANLRGADLHEANLSGADLQEA
NLTQANLKDANLSDANLEQADLAGADLQGAVLDGANLHGANLNNANLSEAMLTRANLEQA
DLSGARTTGARLDDASLHGATVDPVLWRTASLVGARVDVDQAVAFAAAHGLCLAGGSGC
[SEQ ID NO: 37]Nucleic acid encoding for synRFR24.2 
ATGGGTAGCAGCCATCATCATCATCACCATAGCAGCGGTCTGGTTCCGCGTGGTAGCCAC
ATGAATGTTGGTGAAATTCTGCGTCATTATGCAGCAGGTAAACGCAATTTTCAGCATATT
AACCTGCAAGAAATTGAACTGACCAATGCAAGCCTGACCGGTGCCGATCTGAGCTATGCA
AATCTGCATCATGCAAATCTGAGCCGTGCCAATCTGCGTTCTGCAGACCTGCGTAATGCC
AATCTGTCACATGCGAATCTGTCAGGTGCAAATCTGGAAGAAGCCAATCTGGAAGCCGCT
AATCTGCGTGGTGCGGATCTGCATGAAGCGAATCTGAGTGGCGCTGATCTGCAAGAGGCA
AATCTGACCCAGGCGAACCTGAAAGATGCTAATCTGTCTGATGCCAACCTGGAACAGGCA
GATCTGGCAGGGGCTGATCTGCAGGGTGCAGTTCTGGACGGTGCTAATCTGCATGGCGCA
AATCTGAATAATGCGAACCTGAGCGAAGCAATGCTGACCCGTGCGAATCTGGAGCAGGCC
GACCTGAGCGGTGCACGTACCACCGGTGCACGTCTGGATGATGCTAGCCTCCATGGTGCA
ACCGTTGATCCGGTTCTGTGGCGTACCGCAAGCCTGGTTGGTGCACGTGTTGATGTTGAT
CAGGCAGTTGCATTTGCAGCAGCACATGGTCTGTGTCTGGCAGGTGGATCCGGCTGCTAA
[SEQ ID NO: 38]- synRFR20.1 
mgsshhhhhhssglvprgsHMNVGEILRHYAAGKRNFQHINLQEIELTNASLTGADLSYA
NLHHANLSRANLRSADLRNANLSHANLSGANLEEANLEAANLRGADLHEANLSGADLQEA
NLTQANLKDANLSDANLEQANLNNANLSEAMLTRANLEQADLSGARTTGARLDDADLRGA
TVDPVLWRTASLVGARVDVDQAVAFAAAHGLCLAGGSGC
[SEQ **ID** NO: 39]- nucleic acid encoding synRFR20.1 
ATGGGTAGCAGCCATCATCATCATCATCACCATAGCAGCGGTCTGGTTCCGCGTGGTAGCCAC
ATGAATGTTGGTGAAATTCTGCGTCATTATGCAGCAGGTAAACGCAATTTTCAGCATATT
AACCTGCAAGAAATTGAACTGACCAATGCAAGCCTGACCGGTGCCGATCTGAGCTATGCA
AATCTGCATCATGCAAATCTGAGCCGTGCCAATCTGCGTTCTGCAGACCTGCGTAATGCC
AATCTGTCACATGCGAATCTGTCAGGTGCAAATCTGGAAGAAGCCAATCTGGAAGCCGCT
AATCTGCGTGGTGCGGATCTGCATGAAGCGAATCTGAGTGGCGCTGATCTGCAAGAGGCA
AATCTGACCCAGGCGAACCTGAAAGATGCTAATCTGTCTGATGCCAACCTGGAACAGGCA
AATCTGAATAATGCGAACCTGAGCGAAGCAATGCTGACCCGTGCGAATCTGGAGCAGGCC
GACCTGAGCGGTGCACGTACCACCGGTGCACGTCTGGATGATGCCGACCTGCGTGGTGCA
 
ACCGTTGATCCGGTTCTGTGGCGTACCGCAAGCCTGGTTGGTGCACGTGTTGATGTTGAT
CAGGCAGTTGCATTTGCAGCAGCACATGGTCTGTGTCTGGCAGGTGGATCCGGCTGCTAA
[SEQ ID NO: 40]- synRFR28.1 
mgsshhhhhhssglvprgsHMNVGEILRHYAAGKRNFQHINLQEIELTNASLTGADLSYA
NLHHANLSRANLRSADLRNANLSHANLSGANLEEANLEAANLRGADLHEANLSGADLQEA
NLTQANLKDANLSDANLEQADLAGADLQGAVLDGANLHGADLNGADLKQADLSGADLGGA
NLNNANLSEAMLTRANLEQADLSGARTTGARLDDADLRGATVDPVLWRTASLVGARVDVD
QAVAFAAAHGLCLAGGSGC
[SEQ ID NO: 41] 
– nucleic acid encoding synRFR28.1
ATGGGTAGCAGCCATCATCATCATCACCATAGCAGCGGTCTGGTTCCGCGTGGTAGCCAC
ATGAATGTTGGTGAAATTCTGCGTCATTATGCAGCAGGTAAACGCAATTTTCAGCATATT
AACCTGCAAGAAATTGAACTGACCAATGCAAGCCTGACCGGTGCCGATCTGAGCTATGCA
AATCTGCATCATGCAAATCTGAGCCGTGCCAATCTGCGTTCTGCAGACCTGCGTAATGCC
AATCTGTCACATGCGAATCTGTCAGGTGCAAATCTGGAAGAAGCCAATCTGGAAGCCGCT
AATCTGCGTGGTGCGGATCTGCATGAAGCGAATCTGAGTGGCGCTGATCTGCAAGAGGCA
AATCTGACCCAGGCGAACCTGAAAGATGCTAATCTGTCTGATGCCAACCTGGAACAGGCA
GATCTGGCAGGGGCTGATCTGCAGGGTGCAGTTCTGGACGGTGCTAATCTGCATGGCGCG
GACCTCAACGGTGCCGATTTAAAACAGGCTGATTTATCTGGAGCAGATTTAGGTGGCGCA
AATCTGAATAATGCGAACCTGAGCGAAGCAATGCTGACCCGTGCGAATCTGGAGCAGGCC
GACCTGAGCGGTGCACGTACCACCGGTGCACGTCTGGATGATGCCGACCTGCGTGGTGCA
ACCGTTGATCCGGTTCTGTGGCGTACCGCAAGCCTGGTTGGTGCACGTGTTGATGTTGAT
CAGGCAGTTGCATTTGCAGCAGCACATGGTCTGTGTCTGGCAGGTGGATCCGGCTGCTAA
[SEQ ID NO: 42]- synRFR24.t754 
mgsshhhhhhssglvprgsHMNVGEILRHYAAGKRNFQHINLQEIELTNASLTGADLSYA
NLHHANLSRANLRSADLRNANLSHANLKGANLEEANLEAANLRGANLAGANLSGADLQEA
NLTQAQLGGWSDNGETGADLSDANLEQADLAGADLYGAVLDGANLHGANLNNANLDNAML
TRANLEQADLSGARTTGARLDDADLRGATVDPVLWRTASLVGARVDVDQAVAFAAAHGLC
LAGGSGC
[SEQ ID NO: 43]- nucleic acid encoding synRFR24.t754 
ATGGGTAGCAGCCATCATCATCATCATCACCATAGCAGCGGTCTGGTTCCGCGTGGTAGCCAC
ATGAATGTTGGTGAAATTCTGCGTCATTATGCAGCAGGTAAACGCAATTTTCAGCATATT
AACCTGCAAGAAATTGAACTGACCAATGCAAGCCTGACCGGTGCCGATCTGAGCTATGCA
AATCTGCATCATGCAAATCTGAGCCGTGCCAATCTGCGTTCTGCAGACCTGCGTAATGCC
AATCTGTCACATGCGAATCTGAAAGGTGCAAATCTGGAAGAAGCCAATCTGGAAGCCGCT
AATCTGCGTGGTGCGAACCTGGCGGGCGCGAATCTGAGTGGCGCTGATCTGCAAGAGGCA
AATCTGACCCAGGCGCAGCTGGGCGGCTGGAGCGATAACGGCGAAACCGGCGCTGATCTG
TCTGATGCCAACCTGGAACAGGCAGATCTGGCAGGGGCTGATCTGTATGGTGCAGTTCTG
GACGGTGCTAATCTGCATGGCGCAAATCTGAATAATGCGAACCTGGATAACGCAATGCTG
ACCCGTGCGAATCTGGAGCAGGCCGACCTGAGCGGTGCACGTACCACCGGTGCACGTCTG
GATGATGCCGACCTGCGTGGTGCAACCGTTGATCCGGTTCTGTGGCGTACCGCAAGCCTG
GTTGGTGCACGTGTTGATGTTGATCAGGCAGTTGCATTTGCAGCAGCACATGGTCTGTGT
CTGGCAGGTGGATCCGGCTGCTAA
[SEQ ID NO: 44]- synRFR24.t801 
mgsshhhhhhssglvprgsHMNVGEILRHYAAGKRNFQHINLQEIELTNASLTGADLSYA
NLHHANLSRANLRSADLRNANLSHANLRGANLEEANLEAANLRGADLIGANLSGADLQEA
NLTQAQLGLGVEDGGLGADLSDANLEQADLAGANLAGAVLDGANLHGANLNNANLDYAML
TRANLEQADLSGARTTGARLDDADLRGATVDPVLWRTASLVGARVDVDQAVAFAAAHGLC
LAGGSGC
[SEQ ID NO: 45]- Nucleic acid encoding synRFR24.t801 
ATGGGTAGCAGCCATCATCATCATCACCATAGCAGCGGTCTGGTTCCGCGTGGTAGCCAC
ATGAATGTTGGTGAAATTCTGCGTCATTATGCAGCAGGTAAACGCAATTTTCAGCATATT
AACCTGCAAGAAATTGAACTGACCAATGCAAGCCTGACCGGTGCCGATCTGAGCTATGCA
AATCTGCATCATGCAAATCTGAGCCGTGCCAATCTGCGTTCTGCAGACCTGCGTAATGCC
AATCTGTCACATGCGAATCTGCGCGGTGCAAATCTGGAAGAAGCCAATCTGGAAGCCGCT
AATCTGCGTGGTGCGGATCTGATTGGCGCGAATCTGAGTGGCGCTGATCTGCAAGAGGCA
AATCTGACCCAGGCGCAGCTGGGCCTGGGCGTGGAAGATGGCGGCCTGGGCGCTGATCTG
TCTGATGCCAACCTGGAACAGGCAGATCTGGCAGGGGCTAACCTGGCGGGTGCAGTTCTG
GACGGTGCTAATCTGCATGGCGCAAATCTGAATAATGCGAACCTGGATTATGCAATGCTG
ACCCGTGCGAATCTGGAGCAGGCCGACCTGAGCGGTGCACGTACCACCGGTGCACGTCTG
GATGATGCCGACCTGCGTGGTGCAACCGTTGATCCGGTTCTGTGGCGTACCGCAAGCCTG
GTTGGTGCACGTGTTGATGTTGATCAGGCAGTTGCATTTGCAGCAGCACATGGTCTGTGT
CTGGCAGGTGGATCCGGCTGCTAA
[SEQ ID NO: 46]- synRFR24.t1117 
mgsshhhhhhssglvprgsHMNVGEILRHYAAGKRNFQHINLQEIELTNASLTGADLSYA
NLHHANLSRANLRSADLRNANLSHANLRGANLEEANLEAANLRGADLVGANLSGADLQEA
NLTQAQLGGVYRLGEPGADLSDANLEQADLAGANLMGAVLDGANLHGANLNNANLDYAML
TRANLEQADLSGARTTGARLDDADLRGATVDPVLWRTASLVGARVDVDQAVAFAAAHGLC
LAGGSGC
[SEQ ID NO: 47]- nucleic acid encoding synRFR24. t1117 
ATGGGTAGCAGCCATCATCATCATCACCATAGCAGCGGTCTGGTTCCGCGTGGTAGCCAC
ATGAATGTTGGTGAAATTCTGCGTCATTATGCAGCAGGTAAACGCAATTTTCAGCATATT
AACCTGCAAGAAATTGAACTGACCAATGCAAGCCTGACCGGTGCCGATCTGAGCTATGCA
AATCTGCATCATGCAAATCTGAGCCGTGCCAATCTGCGTTCTGCAGACCTGCGTAATGCC
AATCTGTCACATGCGAATCTGCGCGGTGCAAATCTGGAAGAAGCCAATCTGGAAGCCGCT
AATCTGCGTGGTGCGGATCTGGTGGGCGCGAATCTGAGTGGCGCTGATCTGCAAGAGGCA
AATCTGACCCAGGCGCAGCTGGGCGGCGTGTATCGCCTGGGCGAACCGGGCGCTGATCTG
TCTGATGCCAACCTGGAACAGGCAGATCTGGCAGGGGCTAACCTGATGGGTGCAGTTCTG
GACGGTGCTAATCTGCATGGCGCAAATCTGAATAATGCGAACCTGGATTATGCAATGCTG
ACCCGTGCGAATCTGGAGCAGGCCGACCTGAGCGGTGCACGTACCACCGGTGCACGTCTG
GATGATGCCGACCTGCGTGGTGCAACCGTTGATCCGGTTCTGTGGCGTACCGCAAGCCTG
GTTGGTGCACGTGTTGATGTTGATCAGGCAGTTGCATTTGCAGCAGCACATGGTCTGTGT
CTGGCAGGTGGATCCGGCTGCTAA
[SEQ ID NO: 48]- synRFR24.t1166 
mgsshhhhhhssglvprgsHMNVGEILRHYAAGKRNFQHINLQEIELTNASLTGADLSYA
NLHHANLSRANLRSADLRNANLSHANLSGANLEEANLEAANLRGADLRGANLSGADLQEA
NLTQAKLGTWSSSGLTGADLSDANLEQADLAGADLKGAVLDGANLHGANLNNANLDYAML
TRANLEQADLSGARTTGARLDDADLRGATVDPVLWRTASLVGARVDVDQAVAFAAAHGLC
LAGGSGC
[SEQ ID NO: 49]- nucleic acid encoding synRFR24.t1166 
ATGGGTAGCAGCCATCATCATCATCACCATAGCAGCGGTCTGGTTCCGCGTGGTAGCCAC
ATGAATGTTGGTGAAATTCTGCGTCATTATGCAGCAGGTAAACGCAATTTTCAGCATATT
AACCTGCAAGAAATTGAACTGACCAATGCAAGCCTGACCGGTGCCGATCTGAGCTATGCA
AATCTGCATCATGCAAATCTGAGCCGTGCCAATCTGCGTTCTGCAGACCTGCGTAATGCC
AATCTGTCACATGCGAATCTGTCAGGTGCAAATCTGGAAGAAGCCAATCTGGAAGCCGCT
AATCTGCGTGGTGCGGATCTGCGCGGCGCGAATCTGAGTGGCGCTGATCTGCAAGAGGCA
AATCTGACCCAGGCGAAACTGGGCACCTGGAGCAGCAGCGGCCTGACCGGCGCTGATCTG
TCTGATGCCAACCTGGAACAGGCAGATCTGGCAGGGGCTGATCTGAAAGGTGCAGTTCTG
GACGGTGCTAATCTGCATGGCGCAAATCTGAATAATGCGAACCTGGATTATGCAATGCTG
ACCCGTGCGAATCTGGAGCAGGCCGACCTGAGCGGTGCACGTACCACCGGTGCACGTCTG
GATGATGCCGACCTGCGTGGTGCAACCGTTGATCCGGTTCTGTGGCGTACCGCAAGCCTG
GTTGGTGCACGTGTTGATGTTGATCAGGCAGTTGCATTTGCAGCAGCACATGGTCTGTGT
CTGGCAGGTGGATCCGGCTGCTAA
[SEQ ID NO: 50]- synRFR24.t1428 
mgsshhhhhhssglvprgsHMNVGEILRHYAAGKRNFQHINLQEIELTNASLTGADLSYA
NLHHANLSRANLRSADLRNANLSHANLSGANLEEANLEAANLRGANLVGANLSGADLQEA
NLTQAMLGQTYPWGTVGADLSDANLEQADLAGAQLVGAVLDGANLHGANLNNANLDNAML
TRANLEQADLSGARTTGARLDDADLRGATVDPVLWRTASLVGARVDVDQAVAFAAAHGLC
LAGGSGC
[SEQ ID NO: 51]- nucleic acid encoding synRFR24.t1428 
ATGGGTAGCAGCCATCATCATCATCATCACCATAGCAGCGGTCTGGTTCCGCGTGGTAGCCAC
ATGAATGTTGGTGAAATTCTGCGTCATTATGCAGCAGGTAAACGCAATTTTCAGCATATT
AACCTGCAAGAAATTGAACTGACCAATGCAAGCCTGACCGGTGCCGATCTGAGCTATGCA
AATCTGCATCATGCAAATCTGAGCCGTGCCAATCTGCGTTCTGCAGACCTGCGTAATGCC
AATCTGTCACATGCGAATCTGTCAGGTGCAAATCTGGAAGAAGCCAATCTGGAAGCCGCT
AATCTGCGTGGTGCGAACCTGGTGGGCGCGAATCTGAGTGGCGCTGATCTGCAAGAGGCA
AATCTGACCCAGGCGATGCTGGGCCAGACCTATCCGTGGGGCACCGTGGGCGCTGATCTG
TCTGATGCCAACCTGGAACAGGCAGATCTGGCAGGGGCTCAGCTGGTGGGTGCAGTTCTG
GACGGTGCTAATCTGCATGGCGCAAATCTGAATAATGCGAACCTGGATAACGCAATGCTG
ACCCGTGCGAATCTGGAGCAGGCCGACCTGAGCGGTGCACGTACCACCGGTGCACGTCTG
GATGATGCCGACCTGCGTGGTGCAACCGTTGATCCGGTTCTGTGGCGTACCGCAAGCCTG
GTTGGTGCACGTGTTGATGTTGATCAGGCAGTTGCATTTGCAGCAGCACATGGTCTGTGT
CTGGCAGGTGGATCCGGCTGCTAA
**[SEQ** ID NO: 52]- synRFR24.t1555 
mgsshhhhhhssglvprgsHMNVGEILRHYAAGKRNFQHINLQEIELTNASLTGADLSYA
NLHHANLSRANLRSADLRNANLSHANLSGANLEEANLEAANLRGADLAGANLSGADLQEA
NLTQAMLGKDEDGVVYGADLSDANLEQADLAGAQLVGAVLDGANLHGANLNNANLDNAML
TRANLEQADLSGARTTGARLDDADLRGATVDPVLWRTASLVGARVDVDQAVAFAAAHGLC
LAGGSGC
[SEQ ID NO: 53]- nucleic acid encoding synRFR24.t1555 
ATGGGTAGCAGCCATCATCATCATCACCATAGCAGCGGTCTGGTTCCGCGTGGTAGCCAC
ATGAATGTTGGTGAAATTCTGCGTCATTATGCAGCAGGTAAACGCAATTTTCAGCATATT
AACCTGCAAGAAATTGAACTGACCAATGCAAGCCTGACCGGTGCCGATCTGAGCTATGCA
AATCTGCATCATGCAAATCTGAGCCGTGCCAATCTGCGTTCTGCAGACCTGCGTAATGCC
AATCTGTCACATGCGAATCTGTCAGGTGCAAATCTGGAAGAAGCCAATCTGGAAGCCGCT
AATCTGCGTGGTGCGGATCTGGCGGGCGCGAATCTGAGTGGCGCTGATCTGCAAGAGGCA
AATCTGACCCAGGCGATGCTGGGCAAAGATGAAGATGGCGTGGTGTATGGCGCTGATCTG
TCTGATGCCAACCTGGAACAGGCAGATCTGGCAGGGGCTCAGCTGGTGGGTGCAGTTCTG
GACGGTGCTAATCTGCATGGCGCAAATCTGAATAATGCGAACCTGGATAACGCAATGCTG
ACCCGTGCGAATCTGGAGCAGGCCGACCTGAGCGGTGCACGTACCACCGGTGCACGTCTG
GATGATGCCGACCTGCGTGGTGCAACCGTTGATCCGGTTCTGTGGCGTACCGCAAGCCTG
GTTGGTGCACGTGTTGATGTTGATCAGGCAGTTGCATTTGCAGCAGCACATGGTCTGTGT
CTGGCAGGTGGATCCGGCTGCTAA
**[SEQ** ID NO: 54]- synRFR24.t1916 
mgsshhhhhhssglvprgsHMNVGEILRHYAAGKRNFQHINLQEIELTNASLTGADLSYA
NLHHANLSRANLRSADLRNANLSHANLSGANLEEANLEAANLRGALLTGANLSGADLQEA
NLTQAWLGRDPRLGLSGADLSDANLEQADLAGAQLKGAVLDGANLHGANLNNANLDNAML
TRANLEQADLSGARTTGARLDDADLRGATVDPVLWRTASLVGARVDVDQAVAFAAAHGLC
LAGGSGC
[SEQ ID NO: 55]- nucleic acid encoding synRFR24.t1916 
ATGGGTAGCAGCCATCATCATCATCACCATAGCAGCGGTCTGGTTCCGCGTGGTAGCCAC
ATGAATGTTGGTGAAATTCTGCGTCATTATGCAGCAGGTAAACGCAATTTTCAGCATATT
AACCTGCAAGAAATTGAACTGACCAATGCAAGCCTGACCGGTGCCGATCTGAGCTATGCA
AATCTGCATCATGCAAATCTGAGCCGTGCCAATCTGCGTTCTGCAGACCTGCGTAATGCC
 
AATCTGTCACATGCGAATCTGTCAGGTGCAAATCTGGAAGAAGCCAATCTGGAAGCCGCT
AATCTGCGTGGTGCGCTGCTGACCGGCGCGAATCTGAGTGGCGCTGATCTGCAAGAGGCA
AATCTGACCCAGGCGTGGCTGGGCCGCGATCCGCGCCTGGGCCTGAGCGGCGCTGATCTG
TCTGATGCCAACCTGGAACAGGCAGATCTGGCAGGGGCTCAGCTGAAAGGTGCAGTTCTG
GACGGTGCTAATCTGCATGGCGCAAATCTGAATAATGCGAACCTGGATAACGCAATGCTG
ACCCGTGCGAATCTGGAGCAGGCCGACCTGAGCGGTGCACGTACCACCGGTGCACGTCTG
GATGATGCCGACCTGCGTGGTGCAACCGTTGATCCGGTTCTGTGGCGTACCGCAAGCCTG
GTTGGTGCACGTGTTGATGTTGATCAGGCAGTTGCATTTGCAGCAGCACATGGTCTGTGT
CTGGCAGGTGGATCCGGCTGCTAA
[SEQ ID NO: 56]- synRFR24.t2570 
mgsshhhhhhssglvprgsHMNVGEILRHYAAGKRNFQHINLQEIELTNASLTGADLSYA
NLHHANLSRANLRSADLRNANLSHANLKGANLEEANLEAANLRGANLAGANLSGADLQEA
NLTQADLGGPPDEGKTGADLSDANLEQADLAGAQLYGAVLDGANLHGANLNNANLDYAML
TRANLEQADLSGARTTGARLDDADLRGATVDPVLWRTASLVGARVDVDQAVAFAAAHGLC
LAGGSGC
[SEQ ID NO: 57]- nucleic acid encoding synRFR24.t2570 
ATGGGTAGCAGCCATCATCATCATCACCATAGCAGCGGTCTGGTTCCGCGTGGTAGCCAC
ATGAATGTTGGTGAAATTCTGCGTCATTATGCAGCAGGTAAACGCAATTTTCAGCATATT
AACCTGCAAGAAATTGAACTGACCAATGCAAGCCTGACCGGTGCCGATCTGAGCTATGCA
AATCTGCATCATGCAAATCTGAGCCGTGCCAATCTGCGTTCTGCAGACCTGCGTAATGCC
AATCTGTCACATGCGAATCTGAAAGGTGCAAATCTGGAAGAAGCCAATCTGGAAGCCGCT
AATCTGCGTGGTGCGAACCTGGCGGGCGCGAATCTGAGTGGCGCTGATCTGCAAGAGGCA
AATCTGACCCAGGCGGATCTGGGCGGCCCGCCGGATGAAGGCAAAACCGGCGCTGATCTG
TCTGATGCCAACCTGGAACAGGCAGATCTGGCAGGGGCTCAGCTGTATGGTGCAGTTCTG
GACGGTGCTAATCTGCATGGCGCAAATCTGAATAATGCGAACCTGGATTATGCAATGCTG
ACCCGTGCGAATCTGGAGCAGGCCGACCTGAGCGGTGCACGTACCACCGGTGCACGTCTG
GATGATGCCGACCTGCGTGGTGCAACCGTTGATCCGGTTCTGTGGCGTACCGCAAGCCTG
GTTGGTGCACGTGTTGATGTTGATCAGGCAGTTGCATTTGCAGCAGCACATGGTCTGTGT
CTGGCAGGTGGATCCGGCTGCTAA
[SEQ ID NO: 58]- synRFR24.t3280 
mgsshhhhhhssglvprgsHMNVGEILRHYAAGKRNFQHINLQEIELTNASLTGADLSYA
NLHHANLSRANLRSADLRNANLSHANLSGANLEEANLEAANLRGADLRGANLSGADLQEA
NLTQAKLGGYDDEGLGGADLSDANLEQADLAGAELFGAVLDGANLHGANLNNANLDKAML
TRANLEQADLSGARTTGARLDDADLRGATVDPVLWRTASLVGARVDVDQAVAFAAAHGLC
LAGGSGC
[SEQ ID NO: 59]-nucleic acid encoding synRFR24.t3280 
ATGGGTAGCAGCCATCATCATCATCACCATAGCAGCGGTCTGGTTCCGCGTGGTAGCCAC
ATGAATGTTGGTGAAATTCTGCGTCATTATGCAGCAGGTAAACGCAATTTTCAGCATATT
AACCTGCAAGAAATTGAACTGACCAATGCAAGCCTGACCGGTGCCGATCTGAGCTATGCA
AATCTGCATCATGCAAATCTGAGCCGTGCCAATCTGCGTTCTGCAGACCTGCGTAATGCC
AATCTGTCACATGCGAATCTGAGCGGTGCAAATCTGGAAGAAGCCAATCTGGAAGCCGCT
AATCTGCGTGGTGCGGATCTGCGCGGCGCGAATCTGAGTGGCGCTGATCTGCAAGAGGCA
AATCTGACCCAGGCGAAACTGGGCGGCTATGATGATGAAGGCCTGGGCGGCGCTGATCTG
TCTGATGCCAACCTGGAACAGGCAGATCTGGCAGGGGCTGAACTGTTTGGTGCAGTTCTG
GACGGTGCTAATCTGCATGGCGCAAATCTGAATAATGCGAACCTGGATAAAGCAATGCTG
ACCCGTGCGAATCTGGAGCAGGCCGACCTGAGCGGTGCACGTACCACCGGTGCACGTCTG
GATGATGCCGACCTGCGTGGTGCAACCGTTGATCCGGTTCTGTGGCGTACCGCAAGCCTG
GTTGGTGCACGTGTTGATGTTGATCAGGCAGTTGCATTTGCAGCAGCACATGGTCTGTGT
CTGGCAGGTGGATCCGGCTGCTAA
[SEQ ID NO: 60]- synRFR24.t3284 mgsshhhhhhssglvprgsHMNVGEILRHYAAGKRNFQHINLQEIELTNASLTGADLSYA 
NLHHANLSRANLRSADLRNANLSHANLEGANLEEANLEAANLRGARLAGANLSGADLQEA
NLTQANLGGPPDDGDPGADLSDANLEQADLAGANLAGAVLDGANLHGANLNNANLDNAML
TRANLEQADLSGARTTGARLDDADLRGATVDPVLWRTASLVGARVDVDQAVAFAAAHGLC
LAGGSGC
[SEQ ID NO: 61] nucleic acid encoding synRFR24.t3284 
ATGGGTAGCAGCCATCATCATCATCACCATAGCAGCGGTCTGGTTCCGCGTGGTAGCCAC
ATGAATGTTGGTGAAATTCTGCGTCATTATGCAGCAGGTAAACGCAATTTTCAGCATATT
AACCTGCAAGAAATTGAACTGACCAATGCAAGCCTGACCGGTGCCGATCTGAGCTATGCA
AATCTGCATCATGCAAATCTGAGCCGTGCCAATCTGCGTTCTGCAGACCTGCGTAATGCC
AATCTGTCACATGCGAATCTGGAAGGTGCAAATCTGGAAGAAGCCAATCTGGAAGCCGCT
AATCTGCGTGGTGCGCGCCTGGCGGGCGCGAATCTGAGTGGCGCTGATCTGCAAGAGGCA
AATCTGACCCAGGCGAACCTGGGCGGCCCGCCGGATGATGGCGATCCGGGCGCTGATCTG
TCTGATGCCAACCTGGAACAGGCAGATCTGGCAGGGGCTAACCTGGCGGGTGCAGTTCTG
GACGGTGCTAATCTGCATGGCGCAAATCTGAATAATGCGAACCTGGATAACGCAATGCTG
ACCCGTGCGAATCTGGAGCAGGCCGACCTGAGCGGTGCACGTACCACCGGTGCACGTCTG
GATGATGCCGACCTGCGTGGTGCAACCGTTGATCCGGTTCTGTGGCGTACCGCAAGCCTG
GTTGGTGCACGTGTTGATGTTGATCAGGCAGTTGCATTTGCAGCAGCACATGGTCTGTGT
CTGGCAGGTGGATCCGGCTGCTAA
[SEQ ID NO: 62]- synRFR24.t1428 sfGFP 
mgsshhhhhhssglvprgsHMNVGEILRHYAAGKRNFQHINLQEIELTNASLTGADLSYA
NLHHANLSRANLRSADLRNANLSHANLSGANLEEANLEAANLRGANLVGANLSGADLQEA
NLTQAMLGQTYPGGGGSMRKGEELFTGVVPILVELDGDVNGHKFSVRGEGEGDATNGKLT
LKFICTTGKLPVPWPTLVTTLTYGVQCFARYPDHMKQHDFFKSAMPEGYVQERTISFKDD
GTYKTRAEVKFEGDTLVNRIELKGIDFKEDGNILGHKLEYNFNSHNVYITADKQKNGIKA
NFKIRHNVEDGSVQLADHYQQNTPIGDGPVLLPDNHYLSTQSVLSKDPNEKRDHMVLLEF
VTAAGITHGMDELSGGGGWGTVGADLSDANLEQADLAGAQLVGAVLDGANLHGANLNNAN
LDNAMLTRANLEQADLSGARTTGARLDDADLRGATVDPVLWRTASLVGARVDVDQAVAFA
AAHGLCLAGGSGC
[SEQ ID NO: 63]-nucleic acid encoding synRFR24.t1428 sfGFP 
ATGGGTAGCAGCCATCATCATCATCACCATAGCAGCGGTCTGGTTCCGCGTGGTAGCCAC
ATGAATGTTGGTGAAATTCTGCGTCATTATGCAGCAGGTAAACGCAATTTTCAGCATATT
AACCTGCAAGAAATTGAACTGACCAATGCAAGCCTGACCGGTGCCGATCTGAGCTATGCA
AATCTGCATCATGCAAATCTGAGCCGTGCCAATCTGCGTTCTGCAGACCTGCGTAATGCC
AATCTGTCACATGCGAATCTGTCAGGTGCAAATCTGGAAGAAGCCAATCTGGAAGCCGCT
AATCTGCGTGGTGCGAACCTGGTGGGCGCGAATCTGAGTGGCGCTGATCTGCAAGAGGCA
AATCTGACCCAGGCGATGCTGGGCCAGACCTATCCGGGAGGTGGAGGCTCAATGCGTAAA
GGCGAAGAGCTGTTCACTGGTGTCGTCCCTATTCTGGTGGAACTGGATGGTGATGTCAAC
GGTCATAAGTTTTCCGTGCGTGGCGAGGGTGAAGGTGACGCAACTAATGGTAAACTGACG
CTGAAGTTCATCTGTACTACTGGTAAACTGCCGGTACCTTGGCCGACTCTGGTAACGACG
CTGACTTATGGTGTTCAGTGCTTTGCTCGTTATCCGGACCATATGAAGCAGCATGACTTC
TTCAAGTCCGCCATGCCGGAAGGCTATGTGCAGGAACGCACGATTTCCTTTAAGGATGAC
GGCACGTACAAAACGCGTGCGGAAGTGAAATTTGAAGGCGATACCCTGGTAAACCGCATT
GAGCTGAAAGGCATTGACTTTAAAGAAGACGGCAATATCCTGGGCCATAAGCTGGAATAC
AATTTTAACAGCCACAATGTTTACATCACCGCCGATAAACAAAAAAATGGCATTAAAGCG
AATTTTAAAATTCGCCACAACGTGGAGGATGGCAGCGTGCAGCTGGCTGATCACTACCAG
CAAAACACTCCAATCGGTGATGGTCCTGTTCTGCTGCCAGACAATCACTATCTGAGCACG
CAAAGCGTTCTGTCTAAAGATCCGAACGAGAAACGCGATCATATGGTTCTGCTGGAGTTC
GTAACCGCAGCGGGCATCACGCATGGTATGGATGAACTGTCTGGAGGCGGTGGATGGGGC
ACCGTGGGCGCTGATCTGTCTGATGCCAACCTGGAACAGGCAGATCTGGCAGGGGCTCAG
CTGGTGGGTGCAGTTCTGGACGGTGCTAATCTGCATGGCGCAAATCTGAATAATGCGAAC
CTGGATAACGCAATGCTGACCCGTGCGAATCTGGAGCAGGCCGACCTGAGCGGTGCACGT
ACCACCGGTGCACGTCTGGATGATGCCGACCTGCGTGGTGCAACCGTTGATCCGGTTCTG
TGGCGTACCGCAAGCCTGGTTGGTGCACGTGTTGATGTTGATCAGGCAGTTGCATTTGCA
GCAGCACATGGTCTGTGTCTGGCAGGTGGATCCGGCTGCTAA
[SEQ ID NO: 64]- synRFR24.t1428 sfGFPmut 
mgsshhhhhhssglvprgsHMNVGEILRHYAAGKRNFQHINLQEIELTNASLTGADLSYA
NLHHANLSRANLRSADLRNANLSHANLSGANLEEANLEAANLRGANLVGANLSGADLQEA
NLTQAMLGQTYPGGGGSMRKGEELFTGVVPILVELDGDVNGHKFSVRGEGEGDATNGKLT
LKFICTTGKLPVPWPTLVTTLTYGVQCFARYPDHMKQHDFFKSAMPEGYVQERTISFKDD
GTYKTRAEVKFEGDTLVNRIELKGIDFKEDGNILGHKLEYNFNSHNVYITADKQKNGIKA
NFKIRHNVEDGSVQLADHYQQNTPIGDGPVLLPDNHYLSTQSVLSKDPNEKRDHMVLLEF
VTAAGITHGMDELSGGGGAGTVGADLSDANLEQADLAGAQLVGAVLDGANLHGANLNNAN
LDNAMLTRANLEQADLSGARTTGARLDDADLRGATVDPVLWRTASLVGARVDVDQAVAFA
AAHGLCLAGGSGC
[SEQ ID NO: 65]- nucleic acid encoding synRFR24.t1428 sfGFPmut 
ATGGGTAGCAGCCATCATCATCATCACCATAGCAGCGGTCTGGTTCCGCGTGGTAGCCAC
ATGAATGTTGGTGAAATTCTGCGTCATTATGCAGCAGGTAAACGCAATTTTCAGCATATT
AACCTGCAAGAAATTGAACTGACCAATGCAAGCCTGACCGGTGCCGATCTGAGCTATGCA
AATCTGCATCATGCAAATCTGAGCCGTGCCAATCTGCGTTCTGCAGACCTGCGTAATGCC
AATCTGTCACATGCGAATCTGTCAGGTGCAAATCTGGAAGAAGCCAATCTGGAAGCCGCT
AATCTGCGTGGTGCGAACCTGGTGGGCGCGAATCTGAGTGGCGCTGATCTGCAAGAGGCA
AATCTGACCCAGGCGATGCTGGGCCAGACCTATCCGGGAGGTGGAGGCTCAATGCGTAAA
GGCGAAGAGCTGTTCACTGGTGTCGTCCCTATTCTGGTGGAACTGGATGGTGATGTCAAC
GGTCATAAGTTTTCCGTGCGTGGCGAGGGTGAAGGTGACGCAACTAATGGTAAACTGACG
CTGAAGTTCATCTGTACTACTGGTAAACTGCCGGTACCTTGGCCGACTCTGGTAACGACG
CTGACTTATGGTGTTCAGTGCTTTGCTCGTTATCCGGACCATATGAAGCAGCATGACTTC
TTCAAGTCCGCCATGCCGGAAGGCTATGTGCAGGAACGCACGATTTCCTTTAAGGATGAC
GGCACGTACAAAACGCGTGCGGAAGTGAAATTTGAAGGCGATACCCTGGTAAACCGCATT
GAGCTGAAAGGCATTGACTTTAAAGAAGACGGCAATATCCTGGGCCATAAGCTGGAATAC
AATTTTAACAGCCACAATGTTTACATCACCGCCGATAAACAAAAAAATGGCATTAAAGCG
AATTTTAAAATTCGCCACAACGTGGAGGATGGCAGCGTGCAGCTGGCTGATCACTACCAG
CAAAACACTCCAATCGGTGATGGTCCTGTTCTGCTGCCAGACAATCACTATCTGAGCACG
CAAAGCGTTCTGTCTAAAGATCCGAACGAGAAACGCGATCATATGGTTCTGCTGGAGTTC
GTAACCGCAGCGGGCATCACGCATGGTATGGATGAACTGTCTGGAGGCGGTGGAGCTGGC
ACCGTGGGCGCTGATCTGTCTGATGCCAACCTGGAACAGGCAGATCTGGCAGGGGCTCAG
CTGGTGGGTGCAGTTCTGGACGGTGCTAATCTGCATGGCGCAAATCTGAATAATGCGAAC
CTGGATAACGCAATGCTGACCCGTGCGAATCTGGAGCAGGCCGACCTGAGCGGTGCACGT
ACCACCGGTGCACGTCTGGATGATGCCGACCTGCGTGGTGCAACCGTTGATCCGGTTCTG
TGGCGTACCGCAAGCCTGGTTGGTGCACGTGTTGATGTTGATCAGGCAGTTGCATTTGCA
GCAGCACATGGTCTGTGTCTGGCAGGTGGATCCGGCTGCTAA
Solenoid domain sequence of synRFR24.2 [SEQ ID NO: 66] 
ANLHHANLSRANLRSADLRNANLSHANLSGANLEEANLEAANLRGADLHEANLSGADLQEA
NLTQANLKDANLSDANLEQADLAGADLQGAVLDGANLHGANLNNANLSEAMLTRANLEQ
Solenoid domain sequence of synRFR20.1 [SEQ ID NO: 67] 
ANLHHANLSRANLRSADLRNANLSHANLSGANLEEANLEAANLRGADLHEANLSGADLQEA
NLTOANLKDANLSDANLEOANLNNANLSEAMLTRANLEO
Solenoid domain sequence of synRFR28.1 [SEQ ID NO: 68] 
ANLHHANLSRANLRSADLRNANLSHANLSGANLEEANLEAANLRGADLHEANLSGADLQEA
NLTQANLKDANLSDANLEQADLAGADLQGAVLDGANLHGADLNGADLKQADLSGADLGGA
NLNNANLSEAMLTRANLEQ
Solenoid domain sequence of synRFR24.t764 [SEQ ID NO: 69] 
ANLHHANLSRANLRSADLRNANLSHANLKGANLEEANLEAANLRGANLAGANLSGADLQEA
NLTQAQLGGWSDNGETGADLSDANLEQADLAGADLYGAVLDGANLHGANLNNANLDNAML
TRANLEQ
Solenoid domain sequence of synRFR24.t801 [SEQ ID NO: 70] 
ANLHHANLSRANLRSADLRNANLSHANLRGANLEEANLEAANLRGADLIGANLSGADLQEA
NLTQAQLGLGVEDGGLGADLSDANLEQADLAGANLAGAVLDGANLHGANLNNANLDYAML
TRANLEQ
Solenoid domain sequence of synRFR24.t1117 [SEQ ID NO: 71] 
ANLHHANLSRANLRSADLRNANLSHANLRGANLEEANLEAANLRGADLVGANLSGADLQEA
NLTQAQLGGVYRLGEPGADLSDANLEQADLAGANLMGAVLDGANLHGANLNNANLDYAML
TRANLEQ
Solenoid domain sequence of synRFR24.t1166 [SEQ ID NO: 72] 
ANLHHANLSRANLRSADLRNANLSHANLSGANLEEANLEAANLRGADLRGANLSGADLQEA
NLTQAKLGTWSSSGLTGADLSDANLEQADLAGADLKGAVLDGANLHGANLNNANLDYAML
TRANLEQ
Solenoid domain sequence of synRFR24.t1428 [SEQ ID NO: 73] 
ANLHHANLSRANLRSADLRNANLSHANLSGANLEEANLEAANLRGANLVGANLSGADLQEA
NLTQAMLGQTYPWGTVGADLSDANLEQADLAGAQLVGAVLDGANLHGANLNNANLDNAML
TRANLEQ
Solenoid domain sequence of synRFR24.t1555 [SEQ ID NO: 74] 
ANLHHANLSRANLRSADLRNANLSHANLSGANLEEANLEAANLRGADLAGANLSGADLQEA
NLTQAMLGKDEDGVVYGADLSDANLEQADLAGAQLVGAVLDGANLHGANLNNANLDNAML
TRANLEQ
Solenoid domain sequence of synRFR24.t1916 [SEQ ID NO: 75] 
ANLHHANLSRANLRSADLRNANLSHANLSGANLEEANLEAANLRGALLTGANLSGADLQEA
NLTQAWLGRDPRLGLSGADLSDANLEQADLAGAQLKGAVLDGANLHGANLNNANLDNAML
TRANLEQ
Solenoid domain sequence of synRFR24.t2570 [SEQ ID NO: 76] 
ANLHHANLSRANLRSADLRNANLSHANLKGANLEEANLEAANLRGANLAGANLSGADLQEA
NLTQADLGGPPDEGKTGADLSDANLEQADLAGAQLYGAVLDGANLHGANLNNANLDYAML
TRANLEQ
Solenoid domain sequence of synRFR24.t3280 [SEQ ID NO: 77] 
ANLHHANLSRANLRSADLRNANLSHANLSGANLEEANLEAANLRGADLRGANLSGADLQEA
NLTQAKLGGYDDEGLGGADLSDANLEQADLAGAELFGAVLDGANLHGANLNNANLDKAML
TRANLEQ
Solenoid domain sequence of synRFR24.t3284 [SEQ ID NO: 78] 
ANLHHANLSRANLRSADLRNANLSHANLEGANLEEANLEAANLRGARLAGANLSGADLQEA
NLTQANLGGPPDDGDPGADLSDANLEQADLAGANLAGAVLDGANLHGANLNNANLDNAML
TRANLEQ
Solenoid domain sequence of synRFR24.t1428.sfGFP [SEQ ID NO: 79] 
ANLHHANLSRANLRSADLRNANLSHANLSGANLEEANLEAANLRGANLVGANLSGADLQEA
NLTQAMLGQTYPGGGGSMRKGEELFTGVVPILVELDGDVNGHKFSVRGEGEGDATNGKLT
LKFICTTGKLPVPWPTLVTTLTYGVQCFARYPDHMKQHDFFKSAMPEGYVQERTISFKDD
GTYKTRAEVKFEGDTLVNRIELKGIDFKEDGNILGHKLEYNFNSHNVYITADKQKNGIKA
NFKIRHNVEDGSVQLADHYQQNTPIGDGPVLLPDNHYLSTQSVLSKDPNEKRDHMVLLEF
 
VTAAGITHGMDELSGGGGWGTVGADLSDANLEQADLAGAQLVGAVLDGANLHGANLNNAN
LDNAMLTRANLEQ
Solenoid domain sequence of synRFR24.t1428.sfGFP [SEQ ID NO: 80] 
ANLHHANLSRANLRSADLRNANLSHANLSGANLEEANLEAANLRGANLVGANLSGADLQEA
NLTQAMLGQTYPGGGGSMRKGEELFTGVVPILVELDGDVNGHKFSVRGEGEGDATNGKLT
LKFICTTGKLPVPWPTLVTTLTYGVQCFARYPDHMKQHDFFKSAMPEGYVQERTISFKDD
GTYKTRAEVKFEGDTLVNRIELKGIDFKEDGNILGHKLEYNFNSHNVYITADKQKNGIKA
NFKIRHNVEDGSVQLADHYQQNTPIGDGPVLLPDNHYLSTQSVLSKDPNEKRDHMVLLEF
VTAAGITHGMDELSGGGGAGTVGADLSDANLEQADLAGAQLVGAVLDGANLHGANLNNAN
LDNAMLTRANLEQ
Tail sequence [SEQ ID NO: 81]
   **KSDDG**
N-terminal capping consensus sequence [SEQ ID NO: 82]
   xMxxxxl LxxYxxGxxxFxxlxLxx(I/A)xLxx where x is any residue
C-terminal capping consensus sequence [SEQ I NO: 83]
   Ax(T/L/F)xxAx(L/F)xxAx(L/F)xxAxVxPVLWxxAxLxGAx(M/V)xxxxxxx(Y/F)xxAxx

Where x is any residue

### NCAP sequences:

>rfr_ref_minprep_ren_neg_num_20_0004.pdb NCAP [SEQ ID NO: 84]
SMGKNEILNQYKNGRHDFSGINLAKADLEN

>rfr_ref_minprep_ren_neg_num_21_0003.pdb NCAP [SEQ ID NO: 85]
TMQTKQILKEYKKGRTNFSGINLANANLKN

>rfr_ref_minprep_ren_neg_num_22_0001.pdb NCAP [SEQ ID NO: 86]
TMRTKDILKQYNNGRHNFSGIDLANADLEK

>rfr_ref_minprep_ren_neg_num_23_0004.pdb NCAP [SEQ ID NO: 87]
TMQTNQILKEYKNGRHNFSKINLANANLQN

>rfr_ref_minprep_ren_neg_num_24_0002.pdb NCAP [SEQ ID NO: 88]
SMGTKEILNEYKKGRKKFANINLANADLSN

>rfr_ref_minprep_ren_neg_num_25_0003.pdb NCAP [SEQ ID NO: 89]
TMRRDDILREYNNGRHNFSGINLAKADLRD

>rfr_ref_minprep_ren_neg_num_26_0004.pdb NCAP [SEQ ID NO: 90]
NMKTNKILKNYKKGRHDFTGIDLANADLEN

>rfr_ref_minprep_ren_neg_num_27_0004.pdb NCAP [SEQ ID NO: 91]
SMQADQILQQYDNGRRNFSGIDLENADLRN

>rfr_ref_minprep_ren_neg_num_28_0002.pdb NCAP [SEQ ID NO: 92]
TMRTNEILQEYKNGRHNFEGIDLANANLQN

>rfr_ref_minprep_ren_neg_num_29_0005.pdb NCAP [SEQ ID NO: 93]
SMHASEILQEYDNGRHDFRGIKLEDADLSG

>rfr_ref_minprep_ren_neg_num_30_0005.pdb NCAP [SEQ ID NO: 94]
TMRTKDILKEYNKGRHDFSGINLRNANLRN

>rfr_ref_minprep_ren_neg_num_31_0005.pdb NCAP [SEQ ID NO: 95]
HMQTNQILEEYNRGRHNFAGIDLQGADLRN

>rfr_ref_minprep_ren_neg_num_32_0001.pdb NCAP [SEQ ID NO: 96]
TMSTNEILNEYKKGRHDFSGINLKDADLSN

>rfr_ref_minprep_ren_neg_num_33_0004.pdb NCAP [SEQ ID NO: 97]
SMTTDKILEEYNKGRTDFRDIDLQNANLQN

>rfr_ref_minprep_ren_neg_num_34_0001.pdb NCAP [SEQ ID NO: 98]
SMHASKILQEYNNGRHDFANIDLAKANLEN

>rfr_ref_minprep_ren_neg_num_35_0005.pdb NCAP [SEQ ID NO: 99]
SMQTEQILQEYNRGRTDFAKIDLENANLKN

>rfr_ref_minprep_ren_neg_num_36_0005.pdb NCAP [SEQ ID NO: 100]
TMATDKILQQYKDGQHDFSGIDLANADLSK

>rfr_ref_minprep_ren_neg_num_37_0002.pdb NCAP [SEQ ID NO: 101]
SMQTNQILKEYNKGRHNFSGIDLQNADLSG

>rfr_ref_minprep_ren_neg_num_38_0005.pdb NCAP [SEQ ID NO: 102]
TMGTNEILQQYKNGQHDFADIDLKNANLQK

>rfr_ref_minprep_ren_neg_num_39_0003.pdb NCAP [SEQ ID NO: 103]
SMSANAILEAYDRGQRDFSKINLANANLSK

>rfr_ref_minprep_ren_neg_num_40_0004.pdb NCAP [SEQ ID NO: 104]
SMHEDEILDEYNNGRKNFSGIDLQGADLED

### CCAP sequences:

>rfr_ref_minprep_ren_neg_num_20_0004.pdb CCAP [SEQ ID NO: 105]
AKTKGANLRKANLEGAKLDPVLWRTANLKGARMSKKQAQEYQRANG

>rfr_ref_minprep_ren_neg_num_21_0003.pdb CCAP [SEQ ID NO: 106]
ADTRGANLDDAKLEGAELRPVLWRTASLSGARMSKKQKQQYQRANG

>rfr_ref_minprep_ren_neg_num_22_0001.pdb CCAP [SEQ ID NO: 107]
AKTTGANLKRANLKGATLDPVLWRTANLEGAKMKKEQKKEYKRARG

>rfr_ref_minprep_ren_neg_num_23_0004.pdb CCAP [SEQ ID NO: 108]
AKTTGAELRNADLRGAKLDPVLWRTAKLTGAQMTKKQKKEYQRARG

>rfr_ref_minprep_ren_neg_num_24_0002.pdb CCAP [SEQ ID NO: 109]
AKTKGADLARADLRGAKLRPVLWNTARLEGAQMTKEQKKEYQRANG

>rfr_ref_minprep_ren_neg_num_25_0003.pdb CCAP [SEQ ID NO: 110]

AETTGAKLARADLRGAKLRPVLWNTATLTGAQMTTKQKQQYKRANG

>rfr_ref_minprep_ren_neg_num_26_0004.pdb CCAP [SEQ ID NO: 111]
AKTKGAKLSGADLRGAKLDPVLWNTAKLQGAKMTDNQRNEYKRANG

>rfr_ref_minprep_ren_neg_num_27_0004.pdb CCAP [SEQ ID NO: 112]
ANTQGAKLDNADLRGAKLRPVLWNTASLQGAKMQKKQKEQYKRAKG

>rfr_ref_minprep_ren_neg_num_28_0002.pdb CCAP [SEQ ID NO: 113]
AETEGADLRRADLRGATLDPVLWNKADLKGAQMKDNQRKEYKRANG

>rfr_ref_minprep_ren_neg_num_29_0005.pdb CCAP [SEQ ID NO: 114]
ANTQDANLQNADLRGATLDPVLWNTANLNGAQMTDKQKQEYQRANG

>rfr_ref_minprep_ren_neg_num_30_0005.pdb CCAP [SEQ ID NO: 115]
AETTGAKLDNADLRGAKLDPVLWNTASLSGAQMTTAQAEQYERANG

>rfr_ref_minprep_ren_neg_num_31_0005.pdb CCAP [SEQ ID NO: 116]
AKTTGADLANADLRGATLDPVLWRTARLTGAQMKTEQKNQYKRANG

>rfr_ref_minprep_ren_neg_num_32_0001.pdb CCAP [SEQ ID NO: 117]
ADTRGADLRNANLRGATLDPVLWNTADLTGAQMEAEQKKEYNRARG

>rfr_ref_minprep_ren_neg_num_33_0004.pdb CCAP [SEQ ID NO: 118]
ANTSGADLRNADLRGATLDPVLWNTANLKGAKMEDNQKAEYERANG

>rfr_ref_minprep_ren_neg_num_34_0001.pdb CCAP [SEQ ID NO: 119]
ANTSGADLENADLRGAELQPVLWRTARLQGAQMETAQKEEYKRANG

>rfr_ref_minprep_ren_neg_num_35_0005.pdb CCAP [SEQ ID NO: 120]
ANTKGANLDNADLRGATLDPVLWRTASLQGAKMTTRQAEEYDRARG

>rfr_ref_minprep_ren_neg_num_36_0005.pdb CCAP [SEQ ID NO: 121]
AETTGANLNNADLRGATLDPVLWNTASLEGAQMKKEQKKEYKRANG

>rfr_ref_minprep_ren_neg_num_37_0002.pdb CCAP [SEQ ID NO: 122]
ANTQGANLDNADLRGATLDPVLWNTASLEGAQMTTEEAKEYQRANG

>rfr_ref_minprep_ren_neg_num_38_0005.pdb CCAP [SEQ ID NO: 123]
AETEGAELNDADLRGAELAPVLWNTASLEGAKMEEKQEQQYDRARG

>rfr_ref_minprep_ren_neg_num_39_0003.pdb CCAP [SEQ ID NO: 124]
AETTGADLNNADLRGATLDPVLWNTAKLQGAQMTTNQKAEYQRANG

>rfr_ref_minprep_ren_neg_num_40_0004.pdb CCAP [SEQ ID NO: 125]
ANTRGARLQDADLRGAKLRPVLWNTANLEGAQMQTNQKAEYQRANG

### Tail sequences:

>rfr_ref_minprep_ren_neg_num_20_0004.pdb TAIL [SEQ ID NO: 126]
KQTEG

>rfr_ref_minprep_ren_neg_num_21_0003.pdb TAIL [SEQ ID NO: 127]
KQTKG

>rfr_ref_minprep_ren_neg_num_22_0001.pdb TAIL [SEQ ID NO: 128]

QQTNG
>rfr_ref_minprep_ren_neg_num_23_0004.pdb TAIL [SEQ ID NO: 129]
KTTEG

>rfr_ref_minprep_ren_neg_num_24_0002.pdb TAIL [SEQ ID NO: 130]
QQDNG

>rfr_ref_minprep_ren_neg_num_25_0003.pdb TAIL [SEQ ID NO: 131]
QQDEG

>rfr_ref_minprep_ren_neg_num_26_0004.pdb TAIL [SEQ ID NO: 132]
QKDNG

>rfr_ref_minprep_ren_neg_num_27_0004.pdb TAIL [SEQ ID NO: 133]
KQDNG

>rfr_ref_minprep_ren_neg_num_28_0002.pdb TAIL [SEQ ID NO: 134]
QEDKG

>rfr_ref_minprep_ren_neg_num_29_0005.pdb TAIL [SEQ ID NO: 135]
RKTNG

>rfr_ref_minprep_ren_neg_num_30_0005.pdb TAIL [SEQ ID NO: 136]
KSTDG

>rfr_ref_minprep_ren_neg_num_31_0005.pdb TAIL [SEQ ID NO: 137]
QKDNG

>rfr_ref_minprep_ren_neg_num_32_0001.pdb TAIL [SEQ ID NO: 138]
QETRG

>rfr_ref_minprep_ren_neg_num_33_0004.pdb TAIL [SEQ ID NO: 139]
QQDKG

>rfr_ref_minprep_ren_neg_num_34_0001.pdb TAIL [SEQ ID NO: 140]
QSDDG

>rfr_ref_minprep_ren_neg_num_35_0005.pdb TAIL [SEQ ID NO: 141]
QEDKG

>rfr_ref_minprep_ren_neg_num_36_0005.pdb TAIL [SEQ ID NO: 142]
QSDDG

>rfr_ref_minprep_ren_neg_num_37_0002.pdb TAIL [SEQ ID NO: 143]
KEDRG

>rfr_ref_minprep_ren_neg_num_38_0005.pdb TAIL [SEQ ID NO: 144]
QSDDG
[?]rfr_ref_[?]_38_0006.pdb TAIL [SEQ ID NO: 145]

>rfr_ref_minprep_ren_neg_num_39_0003.pdb TAIL [SEQ ID NO: 145]
QSDDG

>rfr_ref_minprep_ren_neg_num_40_0004.pdb TAIL [SEQ ID NO: 146]
QQTNG

### Beta solenoid domain consensus sequences:

A X₁ L/F X₂ X₃[SEQ ID NO: 147]
   wherein X₁ is any residue
   X₂ is any residue
   X₃ is any residue;
A X₁ L/F X₂ X₃[SEQ ID NO: 148]
   wherein X₁ is A, C, D, E, I, K, L, M, N, R, S, T or V
   X₂ is A, C, D, E, F, G, H, I, K, N, Q, R, S, T, V, W, or Y
   X₃ is A, C, D, E, G, H, I, K, N, Q, R, S ,Y;
A X₁ L X₂ X₃[SEQ ID NO: 149]
   wherein X₁ is any residue
   X₂ is any residue
   X₃ is any residue;
A X₁ L X₂ X₃[SEQ ID NO: 150]
   wherein X₁ is A, C, D, E, I, K, L, M, N, R, S, T or V
   X₂ is A, C, D, E, F, G, H, I, K, N, Q, R, S, T, V, W, or Y
   X₃ is A, C, D, E, G, H, I, K, N, Q, R, S ,Y;
A (N/D) L * X [SEQ ID NO: 151]
   where * is a polar residue (C, R, H, K, D, E, S, T, N, Q) and X is any residue
(S/G/T/A)X(A/V/G)X(G/A)XX [SEQ ID NO: 152]
   where X is any residue
(S/G/T/A)X(A/V/G)X(G/A)XX(S/G/T/A)*(A/V/G)*(G/A)XX [SEQ ID NO: 153]
   where * is a hydrophobic residue and where X is any residue
SXAXGXXS*A*GXX [SEQ ID NO: 154]
   where * is a hydrophobic residue and where X is any residue
NXAXGXXST(I/V)(G/A)GGXX [SEQ ID NO: 155]
   where X is any residue
NXAXGXXSTIGGGXX [SEQ ID NO: 156]
   where X is any residue
GXQX(V/I/L)XXGGXAXXTX(V/I/L)XXG [SEQ ID NO: 157]
   where X is any residue.
>SynRFR24.nnum35.x4 [Amino Acid Sequence] [SEQ ID NO: 158]

MGSSHHHHHHSSGLVPRGSSMQTEQILQEYNRGRTDFAKIDLENANLKNaelagadlana
nlhranlenanlanakleeanleeanlagadlrnanlqranlaradlagadltganlaeadlrearlqkanlenadltnadltga
dlegarlhganleganltnanlrkanlenadlrnadltgANTKGANLDNADLRGATLDPVLWRTASLQGA
KMTTRQAEEYDRARGgggsSMQTEQILQEYNRGRTDFAKIDLENANLKNaelagadlananl
hranlenanlanakleeanleeanlagadlrnanlqranlaradlagadltganlaeadlrearlqkanlenadltnadltgadl

egarlhganleganltnanlrkanlenadlrnadltgANTKGANLDNADLRGATLDPVLWRTASLQGAK
MTTRQAEEYDRARGgggsSMQTEQILQEYNRGRTDFAKIDLENANLKNaelagadlananlhr
anlenanlanakleeanleeanlagadlrnanlqranlaradlagadltganlaeadlrearlqkanlenadltnadltgadle
garlhganleganltnanlrkanlenadlrnadltgANTKGANLDNADLRGATLDPVLWRTASLQGAKM
TTRQAEEYDRARGgggsSMQTEQILQEYNRGRTDFAKIDLENANLKNaelagadlananlhra
nlenanlanakleeanleeanlagadlrnanlqranlaradlagadltganlaeadlrearlqkanlenadltnadltgadleg
arlhganleganltnanlrkanlenadlrnadltgANTKGANLDNADLRGATLDPVLWRTASLQGAKMT
TRQAEEYDRARGqedkg

Where: 
MGSSHHHHHHSSGLVPRGS – His tag and protease site [SEQ ID NO: 33]
SMQTEQILQEYNRGRTDFAKIDLENANLKN – NCAP sequence [SEQ ID NO: 99]
aelagadlananlhranlenanlanakleeanleeanlagadlrnanlqranlaradlagadltganlaeadlrearlqkanle
nadltnadltgadlegarlhganleganltnanlrkanlenadlrnadltg – Solenoid domain sequence [SEQ
ID NO: 23] >rfr_ref_minprep_ren_neg_num_35_0005 UNITSn
*ANTKGANLDNADLRGATLDPVLWRTASLQGAKMTTRQAEEYDRARG* - CCAP sequence [SEQ ID NO: 120]
gggs - Linker sequence [SEQ ID NO: 34]
**qedkg** - Tail sequence [SEQ ID NO: 134]
>SynRFR24.nnum36.x4 [Amino Acid Sequence] [SEQ ID NO: 159] 
MGSSHHHHHHSSGLVPRGSTMATDKILQQYKDGQHDFSGIDLANADLSKaelagadlsga
dltradlrdanlanadlqeanlaeaklqganlenanlaradlsranlagadlhganlaeadlrnadlrnanlqnadlrkarlqga
dleganleganlhgadlenadlaradlqkadlrnanltgAETTGANLNNADLRGATLDPVLWNTASLEGA
QMKKEQKKEYKRANGgggsTMATDKILQQYKDGQHDFSGIDLANADLSKaelagadlsgadl
tradlrdanlanadlqeanlaeaklqganlenanlaradlsranlagadlhganlaeadlrnadlrnanlqnadlrkarlqgadl
eganleganlhgadlenadlaradlqkadlrnanltgAETTGANLNNADLRGATLDPVLWNTASLEGAQ
MKKEQKKEYKRANGgggsTMATDKILQQYKDGQHDFSGIDLANADLSKaelagadlsgadltr
adlrdanlanadlqeanlaeaklqganlenanlaradlsranlagadlhganlaeadlrnadlrnanlqnadlrkarlqgadle
ganleganlhgadlenadlaradlqkadlrnanltgAETTGANLNNADLRGATLDPVLWNTASLEGAQM
KKEQKKEYKRANGgggsTMATDKILQQYKDGQHDFSGIDLANADLSKaelagadlsgadltrad
lrdanlanadlqeanlaeaklqganlenanlaradlsranlagadlhganlaeadlrnadlrnanlqnadlrkarlqgadlega
nleganlhgadlenadlaradlqkadlrnanltgAETTGANLNNADLRGATLDPVLWNTASLEGAQMK
KEQKKEYKRANGqsddg
 Where:
   MGSSHHHHHHSSGLVPRGS - His tag and protease site [SEQ ID NO: 33]
   **TMATDKILQQYKDGQHDFSGIDLANADLSK** - NCAP sequence [SEQ ID NO: 100] 
aelagadlsgadltradlrdanlanadlqeanlaeaklqganlenanlaradlsranlagadlhganlaeadlrnadlrnanlq
nadlrkarlqgadleganleganlhgadlenadlaradlqkadlrnanltg – Solenoid domain sequence [SEQ
ID NO: 24] >rfr_ref_minprep_ren_neg_num_36_0005 UNITSn
   *AETTGANLNNADLRGATLDPVLWNTASLEGAQMKKEQKKEYKRANG* - CCAP sequence [SEQ ID NO: 121]
   gggs - Linker sequence [SEQ ID NO: 34]
   **qsddg** - Tail sequence [SEQ ID NO: 140]
   >SynRFR24.nnum40.x4 [Amino Acid Sequence] [SEQ ID NO: 160] 
MGSSHHHHHHSSGLVPRGSSMHEDEILDEYNNGRKNFSGIDLQGADLEDaelagadlsda
nleqanlqnanlananlenanlqdadlhgarlqnadlrganledadlanarleganlaeadlrradlrgadltnadledadleg
anlhgarleganlrganltdarlenadlsgadlrnanlegANTRGARLQDADLRGAKLRPVLWNTANLEG
AQMQTNQKAEYQRANGgggsSMHEDEILDEYNNGRKNFSGIDLQGADLEDaelagadlsda
nleqanlqnanlananlenanlqdadlhgarlqnadlrganledadlanarleganlaeadlrradlrgadltnadledadleg
anlhgarleganlrganltdarlenadlsgadlrnanlegANTRGARLQDADLRGAKLRPVLWNTANLEG
AQMQTNQKAEYQRANGgggsSMHEDEILDEYNNGRKNFSGIDLQGADLEDaelagadlsda
nleqanlqnanlananlenanlqdadlhgarlqnadlrganledadlanarleganlaeadlrradlrgadltnadledadleg
anlhgarleganlrganltdarlenadlsgadlrnanlegANTRGARLQDADLRGAKLRPVLWNTANLEG
AQMQTNQKAEYQRANGgggsSMHEDEILDEYNNGRKNFSGIDLQGADLEDaelagadlsda
nleqanlqnanlananlenanlqdadlhgarlqnadlrganledadlanarleganlaeadlrradlrgadltnadledadleg
anlhgarleganlrganltdarlenadlsgadlrnanlegANTRGARLQDADLRGAKLRPVLWNTANLEG
AQMQTNQKAEYQRANGqqtng
 Where:
      MGSSHHHHHHSSGLVPRGS - His tag and protease site [SEQ ID NO: 33]
      **SMHEDEILDEYNNGRKNFSGIDLQGADLED** - NCAP sequence [SEQ ID NO: 104] 
aelagadlsdanleqanlqnanlananlenanlqdadlhgarlqnadlrganledadlanarleganlaeadlrradlrgadlt
nadledadleganlhgarleganlrganltdarlenadlsgadlrnanleg – Solenoid domain sequence [SEQ
ID NO: 28] >rfr_ref_minprep_ren_neg_num_40_0004 UNITSn
      *ANTRGARLQDADLRGAKLRPVLWNTANLEGAQMQTNQKAEYQRANG* - CCAP sequence [SEQ ID NO: 125]
      gggs - Linker sequence [SEQ ID NO: 34]
      **qqtng** - Tail sequence [SEQ ID NO: 128]
      >SynRFR24.nnum35.nc.ll.x4.K129S [Amino Acid Sequence] [SEQ ID NO: 161] 
MGSSHHHHHHSSGLVPRGSRMRRDEILEEYRRGRRNFQHINLQEIELTNaelagadlananl
hranlenanlanakleeanleeanlagadlrnanlqranlaradlagadltganlaeadlrearlqsanlenadltnadltgadl
egarlhganleganltnanlrkanlenadlrnadltgARTTGARLDDADLRGATVDPVLWRTASLRGAK
MEEEQRREYERARGgggsaaaaagggsRMRRDEILEEYRRGRRNFQHINLQEIELTNaela
gadlananlhranlenanlanakleeanleeanlagadlrnanlqranlaradlagadltganlaeadlrearlqkanlenadl
tnadltgadlegarlhganleganltnanlrkanlenadlrnadltgARTTGARLDDADLRGATVDPVLWRTA
SLRGAKMEEEQRREYERARGgggsaaaaagggsRMRRDEILEEYRRGRRNFQHINLQEI
ELTNaelagadlananlhranlenanlanakleeanleeanlagadlrnanlqranlaradlagadltganlaeadlrearl
qkanlenadltnadltgadlegarlhganleganltnanlrkanlenadlrnadltgARTTGARLDDADLRGATVD
PVLWRTASLRGAKMEEEQRREYERARGgggsaaaaagggsRMRRDEILEEYRRGRRNF
QHINLQEIELTNaelagadlananlhranlenanlanakleeanleeanlagadlrnanlqranlaradlagadltganl
 
aeadlrearlqkanlenadltnadltgadlegarlhganleganltnanlrkanlenadlrnadltgARTTGARLDDADL
RGATVDPVLWRTASLRGAKMEEEQRREYERARGqedkg
 Where:
         MGSSHHHHHHSSGLVPRGS - His tag and protease site [SEQ ID NO: 33]
         **RMRRDEILEEYRRGRRNFQHINLQEIELTN** - NCAP sequence [SEQ ID NO: 2]

aelagadlananlhranlenanlanakleeanleeanlagadlrnanlqranlaradlagadltganlaeadlrearlqsanle
nadltnadltgadlegarlhganleganltnanlrkanlenadlrnadltg – Solenoid domain sequence 1 of 4
[SEQ ID NO: 169]

aelagadlananlhranlenanlanakleeanleeanlagadlrnanlqranlaradlagadltganlaeadlrearlqkanle
nadltnadltgadlegarlhganleganltnanlrkanlenadlrnadltg – Solenoid domain sequence 2, 3
and 4 of 4 [SEQ ID NO: 23] >rfr_ref_minprep_ren_neg_num_35_0005 UNITSn

         *ARTTGARLDDADLRGATVDPVLWRTASLRGAKMEEEQRREYERARG* - CCAP sequence [SEQ ID NO: 3]
         gggsaaaaaagggs - Linker sequence [SEQ ID NO: 168]
         **qedkg** - Tail sequence [SEQ ID NO: 134]
         >SynRFR24.nnum36.nc.x4 [Amino Acid Sequence] [SEQ ID NO: 162] 
MGSSHHHHHHSSGLVPRGSRMRRDEILEEYRRGRRNFQHINLQEIELTNaelagadlsgadl
tradlrdanlanadlqeanlaeaklqganlenanlaradlsranlagadlhganlaeadlrnadlrnanlqnadlrkarlqgadl
eganleganlhgadlenadlaradlqkadlrnanltgARTTGARLDDADLRGATVDPVLWRTASLRGAK
MEEEQRREYERARGgggsRMRRDEILEEYRRGRRNFQHINLQEIELTNaelagadlsgadltra
dlrdanlanadlqeanlaeaklqganlenanlaradlsranlagadlhganlaeadlrnadlrnanlqnadlrkarlqgadleg
anleganlhgadlenadlaradlqkadlrnanltgARTTGARLDDADLRGATVDPVLWRTASLRGAKM
EEEQRREYERARGgggsRMRRDEILEEYRRGRRNFQHINLQEIELTNaelagadlsgadltradl
rdanlanadlqeanlaeaklqganlenanlaradlsranlagadlhganlaeadlrnadlrnanlqnadlrkarlqgadlega
nleganlhgadlenadlaradlqkadlrnanltgARTTGARLDDADLRGATVDPVLWRTASLRGAKME
EEQRREYERARGgggsRMRRDEILEEYRRGRRNFQHINLQEIELTNaelagadlsgadltradlrd
anlanadlqeanlaeaklqganlenanlaradlsranlagadlhganlaeadlrnadlrnanlqnadlrkarlqgadleganl
eganlhgadlenadlaradlqkadlrnanltgARTTGARLDDADLRGATVDPVLWRTASLRGAKMEE
EQRREYERARGqsddg
 Where:
            MGSSHHHHHHSSGLVPRGS - His tag and protease site [SEQ ID NO: 33]
            **RMRRDEILEEYRRGRRNFQHINLQEIELTN** - NCAP sequence [SEQ ID NO: 2] 
aelagadlsgadltradlrdanlanadlqeanlaeaklqqanlenanlaradlsranlagadlhganlaeadlrnadlrnanlq
nadlrkarlqgadleganleganlhgadlenadlaradlqkadlrnanltg – Solenoid domain sequence [SEQ
ID NO: 24] >rfr_ref_minprep_ren_neg_num_36_0005 UNITSn
            *ARTTGARLDDADLRGATVDPVLWRTASLRGAKMEEEQRREYERARG* - CCAP sequence [SEQ ID NO: 3]
            gggs - Linker sequence [SEQ ID NO: 34]
            **qsddg** - Tail sequence [SEQ ID NO: 134]
            >SynRFR24.nnum36.nc.x4 [DNA Sequence] [SEQ ID NO: 163] 
ATGGGTAGCAGCCATCACCATCATCATCATAGCAGCGGTCTGGTTCCGCGTGGTAG
CCGTATGCGTCGTGATGAAATTCTGGAAGAATATCGTCGTGGTCGTCGTAACTTTCA
GCATATTAATCTGCAAGAAATCGAACTGACCAATGCAGAACTGGCAGGCGCAGATC
TGAGCGGTGCCGATCTGACCCGTGCGGATCTGCGTGATGCAAATCTGGCAAACGC
AGACCTGCAAGAAGCCAATCTGGCCGAAGCAAAACTGCAGGGTGCGAATCTGGAA
AATGCCAACCTGGCTCGTGCTGATCTGTCACGTGCTAATTTAGCCGGTGCCGACCT
GCATGGTGCAAACCTGGCGGAAGCCGATCTGCGCAATGCGGATTTACGTAATGCCA
ATCTGCAGAATGCGGACCTGCGTAAAGCACGTCTGCAAGGGGCAGATCTGGAAGG
TGCTAACCTGGAAGGCGCAAATTTACATGGTGCAGATTTAGAAAACGCCGATCTGG
CTCGCGCAGATTTACAGAAAGCTGATCTGCGTAACGCGAATCTGACCGGTGCACGT
ACCACCGGTGCGCGTCTGGATGATGCCGATTTACGCGGTGCAACCGTTGATCCGG
TTCTGTGGCGTACCGCAAGCCTGCGTGGTGCCAAAATGGAAGAAGAACAGCGTCG
CGAATATGAACGTGCCCGTGGTGGTGGCGGTTCTCGCATGCGTAGAGATGAAATCT
TAGAAGAGTACCGTCGCGGACGTCGCAATTTTCAACATATCAACTTACAAGAGATCG
AGCTGACAAACGCCGAATTAGCGGGTGCTGATTTAAGCGGAGCGGATTTAACACGT
GCAGACTTACGTGATGCGAACCTGGCCAATGCCGATCTTCAAGAAGCAAATTTAGC
AGAGGCCAAATTACAGGGTGCCAACCTTGAAAACGCTAATCTTGCGCGTGCAGACC
TGAGTCGTGCTAACCTTGCCGGTGCAGATCTTCACGGTGCCAATCTTGCGGAGGCT
GACCTGCGGAATGCAGACCTTCGCAACGCGAACCTGCAAAACGCGGATCTTCGCA
AAGCTCGCCTGCAAGGTGCCGACTTAGAAGGTGCGAATTTAGAGGGTGCTAATCTT
CATGGTGCGGACCTTGAGAACGCGGATTTAGCTAGAGCTGATCTTCAGAAAGCCGA
CTTACGCAATGCAAATTTAACAGGCGCTCGCACCACAGGTGCACGCTTAGATGACG
CTGATTTGCGTGGCGCTACAGTTGACCCTGTGCTGTGGCGCACAGCCAGCTTACGT
GGCGCTAAGATGGAAGAGGAACAACGCCGTGAGTACGAACGTGCACGCGGTGGC
GGTGGCAGTCGGATGCGTCGGGACGAGATTTTAGAGGAATACCGTAGAGGTCGCA
GAAATTTTCAGCACATAAACCTTCAAGAAATTGAGCTTACGAATGCGGAACTTGCTG
GGGCTGATCTGAGTGGGGCAGACTTAACTAGAGCCGACCTTCGTGATGCCAATTTA
GCGAACGCCGATTTGCAAGAGGCGAATTTGGCGGAGGCGAAACTTCAAGGTGCAA
ACTTAGAGAATGCGAACTTAGCAAGAGCGGATCTGAGTCGCGCAAATCTTGCTGGC
GCAGACTTGCATGGTGCCAATTTGGCAGAGGCAGATCTTCGTAACGCTGATTTACG
GAACGCAAATCTTCAGAACGCAGATTTGCGGAAAGCACGCCTTCAGGGTGCTGATC
TTGAGGGTGCAAATTTGGAAGGGGCAAACTTGCACGGTGCTGACTTGGAGAACGCT
GACCTGGCACGAGCGGACTTGCAGAAAGCAGACCTGAGAAATGCAAATCTGACTG
GCGCACGCACAACTGGCGCTCGTTTAGATGATGCGGACTTAAGAGGTGCCACAGTA
GATCCGGTTTTATGGCGCACGGCATCATTACGGGGAGCAAAAATGGAAGAGGAGC
AAAGACGTGAATATGAGCGTGCTCGCGGAGGTGGTGGCTCCCGCATGCGACGCGA
CGAGATCCTTGAAGAGTATCGCAGAGGCCGTCGGAATTTCCAACACATTAACCTGC
AAGAGATAGAGTTAACTAATGCCGAGCTTGCTGGTGCCGATTTATCTGGTGCGGAT
TTGACTCGAGCGGACCTTCGGGACGCAAACCTTGCGAATGCTGATTTACAAGAAGC
TAACCTGGCTGAGGCCAAGCTGCAAGGCGCTAATTTGGAAAATGCGAATCTTGCAC
GGGCAGATTTATCAAGAGCCAACCTTGCTGGGGCAGATTTACATGGGGCTAATCTG
GCTGAAGCTGACCTTCGTAATGCTGATCTTAGAAATGCAAACCTGCAGAACGCTGA
CTTACGTAAAGCCCGTTTGCAGGGTGCAGACCTTGAAGGCGCTAACTTAGAGGGTG
CCAACTTACATGGCGCAGATCTTGAGAATGCAGATCTGGCAAGAGCCGATCTGCAA
AAGGCCGATCTGAGAAACGCCAACCTGACAGGTGCTAGAACCACGGGTGCTCGCC
TGGACGATGCAGACCTGCGTGGCGCAACCGTAGATCCAGTACTTTGGAGAACCGC
ATCTCTTCGCGGAGCCAAGATGGAGGAGGAGCAGCGAAGAGAATATGAAAGAGCA
CGCGGTCAGTCAGATGATGGTTAA
            >SynRFR24.nnum35.nc.ll.x4.K129S [DNA Sequence] [SEQ ID NO: 164] 
ATGGGTAGCAGCCATCACCATCATCATCATAGCAGCGGTCTGGTTCCGCGTGGTAG
CCGTATGCGTCGTGATGAAATTCTGGAAGAATATCGTCGTGGTCGTCGTAACTTTCA
GCATATTAATCTGCAAGAAATCGAACTGACCAATGCAGAACTGGCAGGCGCAGATC
TGGCAAATGCAAATCTGCATCGTGCCAATCTGGAAAATGCCAACCTGGCCAATGCG
AAACTGGAAGAAGCAAATCTGGAAGAGGCCAATCTTGCCGGTGCCGATCTGCGTAA
CGCCAACCTGCAGCGTGCAAACCTGGCACGTGCGGATTTAGCGGGTGCAGATCTG
ACCGGTGCGAATCTGGCCGAAGCGGATCTGCGTGAAGCACGTCTGCAGAGTGCGA
ACCTGGAAAACGCCGATCTGACTAATGCGGACCTGACAGGTGCCGACCTGGAAGG
TGCCCGTCTGCATGGTGCAAATTTAGAAGGCGCAAATCTGACCAACGCCAATCTGC
GCAAAGCCAATTTGGAGAATGCAGACCTGCGTAATGCTGATTTAACCGGTGCACGT
ACCACCGGTGCGCGTCTGGATGATGCCGATTTACGTGGTGCAACCGTTGATCCGGT
TCTGTGGCGTACCGCAAGCCTGCGTGGTGCCAAAATGGAAGAAGAACAGCGTCGC
GAATATGAACGTGCCCGTGGTGGTGGCGGTAGCGCAGCTGCAGCAGCAGCCGGT
GGTGGTTCTCGCATGCGTAGAGATGAAATCTTAGAAGAGTACCGTCGCGGACGTCG
CAATTTTCAACATATCAACTTACAAGAGATCGAGCTGACAAACGCCGAATTAGCTGG
CGCAGATTTAGCGAATGCCAACTTACATCGTGCGAACTTAGAAAATGCTAATCTGGC
GAACGCCAAATTAGAAGAAGCTAACTTAGAAGAGGCGAACCTTGCGGGTGCTGATT
TACGTAATGCGAATCTGCAACGCGCAAATCTTGCTCGTGCGGATCTTGCAGGGGCT
GATCTTACAGGTGCTAACCTGGCGGAAGCAGACTTACGCGAGGCTCGTTTACAGAA
AGCTAATCTTGAGAATGCTGATCTGACAAATGCCGATCTTACCGGTGCAGACTTAGA
AGGTGCACGCTTACATGGCGCAAACCTTGAAGGTGCGAACCTGACGAACGCAAATT
TAAGAAAAGCGAATTTAGAAAATGCGGACCTGCGCAACGCAGACCTGACGGGTGC
GCGTACCACAGGCGCACGCTTAGATGATGCAGATCTTCGCGGTGCCACAGTGGAC
CCTGTGCTGTGGCGCACAGCCTCACTGAGAGGCGCTAAGATGGAAGAGGAACAAC
GCCGTGAGTACGAACGTGCACGCGGTGGCGGTGGTTCAGCAGCAGCTGCCGCAG
CAGGTGGAGGTTCACGTATGCGACGCGACGAAATTTTAGAGGAATATAGACGCGGT
CGCCGTAATTTCCAGCACATCAACCTTCAAGAAATTGAGTTAACAAATGCGGAGTTA
GCCGGTGCGGACCTGGCCAACGCTAATCTTCACCGTGCTAATTTAGAGAACGCGAA
TTTAGCTAACGCCAAACTTGAGGAAGCCAATCTTGAAGAAGCAAACTTGGCTGGTG
CGGACTTACGCAACGCAAACTTACAACGAGCCAATTTAGCACGTGCCGACCTTGCT
GGTGCAGACCTTACAGGGGCAAATTTGGCGGAGGCTGATCTGCGCGAGGCAAGAC
TGCAAAAAGCAAACTTAGAGAATGCCGACTTAACCAATGCGGATTTAACAGGGGCA
GACCTTGAAGGCGCACGCCTGCACGGTGCCAACTTAGAGGGTGCCAACCTTACAA
ACGCGAATCTTCGTAAAGCGAACCTTGAGAACGCGGATTTACGCAATGCCGATCTG
ACAGGCGCACGTACAACAGGTGCACGCCTGGACGACGCCGACCTGCGTGGCGCTA
CAGTTGATCCTGTGTTATGGCGTACAGCGAGCTTACGCGGAGCAAAAATGGAAGAG
GAGCAAAGACGTGAATATGAGCGTGCTCGCGGAGGTGGTGGCTCTGCAGCCGCAG
CAGCGGCAGGCGGTGGAAGTAGAATGCGTCGGGACGAAATCCTTGAGGAATACCG
ACGTGGCAGACGGAACTTTCAACACATAAACCTGCAAGAGATAGAACTTACGAACG
CGGAGCTTGCAGGCGCAGACCTTGCGAACGCTAACCTTCATAGAGCAAACTTGGAA
AACGCGAACTTAGCCAATGCAAAATTAGAAGAGGCAAATTTAGAAGAAGCCAACCT
GGCCGGCGCAGATCTTCGTAATGCTAATTTACAGCGTGCCAATCTTGCACGCGCTG
ACTTAGCCGGTGCAGATTTAACTGGGGCAAATCTTGCCGAAGCAGATTTAAGAGAA
GCTCGCCTTCAGAAAGCCAACTTAGAAAACGCAGATTTAACCAACGCAGATCTTACT
GGCGCTGATTTGGAGGGTGCTAGATTACACGGTGCAAACCTGGAAGGCGCAAACC
TTACTAATGCTAACCTGCGTAAGGCTAATCTGGAAAACGCTGACCTGCGTAACGCG
GATCTTACGGGTGCTCGCACCACGGGTGCCAGACTGGATGACGCTGATCTTCGGG
GAGCGACAGTAGATCCAGTTCTTTGGCGCACGGCCAGTTTACGGGGTGCAAAGAT
GGAGGAAGAGCAGCGAAGAGAATATGAAAGAGCACGCGGTCAAGAAGATAAAGGC
TAA
            >SynRFR24.nnum35.x4 [DNA Sequence] [SEQ ID NO: 165] 
ATGGGCAGCAGCCATCACCATCATCATCATAGCAGCGGTCTGGTTCCGCGTGGTAG
CAGCATGCAGACCGAGCAGATTCTGCAAGAATATAATCGTGGTCGTACCGATTTCG
CCAAAATTGATCTGGAAAATGCCAATCTGAAAAACGCAGAACTGGCAGGCGCAGAT
CTGGCCAATGCAAATCTGCATCGTGCAAACCTGGAAAACGCAACCTGGCGAATGC
CAAACTGGAAGAAGCGAATCTGGAAGAGGCCAACCTTGCCGGTGCCGATCTGCGT
AATGCCAACCTGCAGCGTGCTAATCTGGCACGTGCGGATTTAGCGGGTGCAGATCT
GACCGGTGCTAACCTGGCCGAAGCGGATCTGCGTGAAGCACGTCTGCAGAAAGCA
AATTTAGAAAATGCGGATCTGACCAACGCCGATTTAACCGGTGCGGACCTGGAAGG
TGCCCGTCTGCATGGTGCCAATTTAGAAGGCGCAAATCTGACAAATGCGAACCTGC
GTAAGGCAAATCTTGAAAACGCTGATTTACGTAACGCCGATCTGACTGGCGCAAATA
CCAAAGGTGCCAATCTTGATAATGCAGACCTGCGTGGTGCAACCTTAGATCCGGTT
CTGTGGCGTACCGCAAGCCTGCAGGGTGCAAAAATGACCACACGTCAGGCCGAAG
AATATGACCGTGCACGCGGTGGTGGTGGTTCAAGTATGCAGACAGAACAAATCCTT
CAAGAGTACAATCGCGGTCGCACAGACTTTGCAAAAATTGATTTAGAGAACGCCAA
CCTGAAGAATGCAGAATTAGCTGGTGCCGACCTGGCTAACGCGAATTTACATAGAG
CCAACTTAGAGAATGCGAATCTTGCAAATGCCAAATTAGAGGAAGCAAACTTAGAAG
AGGCAAATCTTGCTGGTGCAGATTTACGTAATGCAAATTTACAGCGTGCAAATTTAG
CCCGTGCTGATCTTGCGGGTGCTGACTTAACAGGGGCAAATCTGGCGGAAGCCGA
TTTACGCGAGGCTCGTTTACAGAAAGCGAACCTTGAGAACGCGGACCTGACAAACG
CGGATCTTACAGGTGCAGACTTAGAAGGTGCACGCTTACATGGCGCTAATCTTGAA
GGTGCGAACCTTACCAACGCCAATCTGCGCAAGGCGAACTTGGAGAATGCTGATCT
GCGCAATGCTGACCTGACAGGTGCCAACACAAAGGGTGCGAACTTAGATAACGCA
GATCTTCGCGGTGCCACACTGGACCCTGTGCTGTGGCGCACAGCCTCATTACAGG
GTGCCAAGATGACAACTCGCCAAGCAGAAGAATACGATCGCGCACGTGGCGGTGG
CGGTAGCTCAATGCAAACCGAACAAATTTTACAAGAGTATAACAGAGGCCGTACCG
ACTTTGCGAAGATCGACCTTGAAAATGCTAACCTTAAAAATGCCGAGTTAGCTGGCG
CTGACTTAGCAAACGCTAACTTACATCGAGCTAATTTGGAGAACGCTAATTTAGCCA
ACGCGAAACTTGAGGAAGCTAACCTTGAAGAGGCGAACCTGGCTGGCGCAGACCT
GCGCAATGCCAATCTTCAACGTGCGAATTTAGCTCGCGCAGATTTAGCAGGGGCTG
ACCTTACGGGTGCGAATCTGGCTGAGGCCGATCTTCGTGAGGCAAGACTGCAAAAA
GCTAATCTGGAAAACGCCGACCTGACCAATGCGGACTTGACTGGGGCAGACCTTGA
AGGCGCTCGCCTGCATGGCGCAAACTTGGAGGGTGCTAATTTGACTAACGCAAACT
TACGCAAAGCCAACTTGGAAAACGCAGACTTGCGTAATGCGGATTTGACAGGTGCG
AATACGAAAGGCGCTAACCTGGATAATGCCGACTTACGCGGTGCTACCCTTGATCC
GGTGTTATGGCGCACCGCGTCACTTCAAGGGGCTAAAATGACAACCAGACAGGCG
GAAGAGTACGATAGAGCCCGTGGTGGCGGAGGTTCATCTATGCAAACTGAGCAGAT
CTTACAAGAATACAACCGAGGACGCACGGATTTTGCTAAAATCGATCTTGAGAACGC
AAATCTTAAGAATGCCGAACTTGCCGGTGCTGATTTAGCTAATGCTAATCTTCATAG
AGCCAACCTTGAAAACGCCAATCTGGCCAACGCAAAATTAGAAGAAGCCAACCTGG
AAGAGGCTAATCTTGCAGGGGCTGATCTGCGGAACGCGAATCTGCAACGCGCAAA
CTTAGCGCGTGCCGACTTAGCTGGTGCGGATTTAACTGGTGCCAACCTTGCGGAAG
CCGACCTGCGCGAGGCACGCCTTCAAAAAGCGAACCTGGAAAATGCGGATTTAACC
AATGCAGATCTGACAGGGGCTGATTTGGAAGGCGCAAGACTGCATGGTGCGAATCT
TGAGGGTGCAAACTTGACCAATGCAAACCTGCGCAAAGCTAACCTTGAGAATGCAG
ATCTTCGTAATGCCGATTTAACGGGTGCAAACACAAAAGGTGCAAATCTGGACAAC
GCGGATCTTCGTGGTGCGACATTAGATCCTGTTTTATGGCGGACAGCATCTCTGCA
AGGTGCGAAGATGACGACCCGTCAAGCGGAAGAGTACGACCGTGCACGTGGTCAA
GAAGATAAAGGTTAA
            >SynRFR24.nnum36.x4 [DNA Sequence] [SEQ ID NO: 166] 
ATGGGCAGCAGCCATCACCATCATCATCATAGCAGCGGTCTGGTTCCGCGTGGTAG
CACCATGGCAACCGATAAAATTCTGCAGCAGTATAAAGATGGCCAGCATGATTTTAG
CGGTATTGATCTGGCAAATGCAGATCTGAGCAAAGCCGAACTGGCAGGCGCAGATC
TGTCAGGTGCCGATCTGACCCGTGCGGATCTGCGTGATGCAAATCTGGCCAACGC
CGATCTGCAAGAAGCCAATCTGGCCGAAGCAAAACTGCAGGGTGCGAATCTGGAAA
ATGCTAATCTGGCACGTGCTGATCTGTCTCGTGCAAATTTAGCCGGTGCAGACCTG
CATGGTGCAAACCTGGCGGAAGCTGATCTGCGTAACGCCGACCTGCGCAATGCCA
ATCTGCAGAATGCGGACCTGCGTAAAGCACGTCTGCAAGGGGCAGATCTGGAAGG
TGCTAACCTGGAAGGCGCAAATTTACATGGTGCCGATTTAGAAAACGCAGATTTAGC
GCGTGCAGATTTACAGAAAGCCGATTTACGTAACGCAAATCTGACCGGTGCCGAAA
CCACCGGTGCCAACCTGAATAATGCGGATTTACGTGGTGCAACCTTAGATCCGGTT
CTGTGGAATACCGCAAGCTTAGAAGGTGCACAGATGAAAAAAGAGCAGAAAAAAGA
ATACAAGCGTGCCAATGGTGGTGGTGGCAGTACCATGGCCACAGATAAGATCCTTC
AACAATATAAAGACGGTCAGCACGACTTTTCAGGCATCGATCTGGCGAACGCGGAT
TTAAGTAAAGCAGAACTTGCGGGTGCCGACCTGAGCGGAGCAGATTTAACACGTGC
CGACTTACGTGATGCGAACTTAGCAAACGCTGACCTGCAAGAGGCGAATTTAGCAG
AGGCCAAATTACAGGGTGCTAACTTAGAAAATGCCAATTTAGCAAGAGCGGATCTG
AGTCGTGCCAACCTTGCTGGTGCAGATCTTCACGGTGCTAATCTTGCCGAGGCCGA
TCTTCGCAATGCGGATCTTCGGAACGCAAACCTGCAAAATGCAGACTTAAGAAAAG
CTCGCCTGCAAGGTGCGGACTTAGAGGGTGCAAATCTTGAAGGGGCAAACCTTCAT
GGCGCAGACCTGGAAAACGCGGACCTGGCACGGGCAGACCTTCAGAAAGCAGACT
TACGCAATGCGAATTTAACAGGTGCAGAAACAACTGGCGCTAATCTGAACAATGCC
GACCTTCGCGGTGCAACACTGGACCCTGTGCTGTGGAACACAGCCTCACTTGAGG
GTGCCCAAATGAAGAAAGAACAAAAGAAAGAATATAAACGCGCAAATGGCGGTGGC
GGTTCAACAATGGCGACGGACAAAATCCTGCAACAGTACAAGGACGGACAACATGA
TTTCTCTGGTATCGACCTGGCTAACGCTGATCTTTCAAAAGCAGAATTAGCTGGGGC
TGATCTGAGTGGTGCGGATTTAACTAGAGCCGATCTGCGCGACGCCAACCTGGCTA
ATGCCGACTTGCAAGAGGCTAACCTGGCAGAGGCGAAACTGCAAGGCGCTAACCT
TGAGAATGCGAATCTTGCTAGAGCAGACCTGTCACGCGCAAACCTTGCCGGTGCTG
ATTTACATGGCGCTAACTTGGCCGAGGCAGACCTGAGAAACGCAGATCTTCGTAAT
GCAAATCTTCAGAATGCAGATCTTCGCAAAGCGCGTCTGCAGGGTGCCGACCTTGA
AGGTGCAAATTTAGAAGGTGCGAACTTACATGGTGCTGATCTTGAGAATGCCGATTT
AGCCCGTGCCGATCTTCAAAAAGCTGACCTGCGGAATGCGAACCTGACAGGTGCG
GAAACGACAGGTGCAAACTTAAACAACGCGGACTTACGCGGTGCCACGCTTGACCC
GGTGTTATGGAATACAGCCAGCCTTGAAGGCGCTCAAATGAAAAAAGAGCAAAAAA
AAGAATATAAACGAGCCAACGGTGGCGGTGGCTCTACGATGGCTACTGATAAAATC
TTGCAGCAATACAAGGATGGGCAGCACGATTTCAGTGGCATTGATTTAGCTAACGC
AGACTTGTCTAAAGCCGAGTTAGCTGGTGCGGACCTTTCTGGCGCTGACTTAACTC
GAGCTGACCTTCGTGACGCCAATCTTGCAAACGCTGATTTACAAGAAGCAAATCTTG
CGGAAGCCAAATTACAAGGCGCAAATCTGGAAAACGCAAACTTAGCTCGCGCAGAC
TTATCAAGAGCTAATTTAGCTGGCGCAGATTTACACGGTGCGAACCTGGCCGAAGC
AGATTTACGTAATGCAGACCTGCGCAACGCAAACCTTCAGAACGCTGATTTGCGTAA
AGCCCGTTTACAGGGTGCTGATTTGGAAGGTGCCAATCTTGAGGGTGCTAATCTGC
ATGGGGCAGACTTAGAGAATGCGGATTTGGCACGTGCGGACTTGCAGAAAGCGGA
TTTGAGAAATGCTAACTTAACCGGTGCGGAGACAACCGGTGCGAATCTTAATAATGC
CGATCTTAGAGGTGCGACCCTTGATCCAGTTTTATGGAACACCGCATCATTAGAGG
GTGCCCAGATGAAAAAAGAGCAAAAGAAAGAATACAAACGTGCGAACGGTCAGTCA
GATGATGGTTAA
            >SynRFR24.nnum40.x4 [DNA Sequence] [SEQ ID NO: 167] 
ATGGGCAGCAGCCATCACCATCATCATCATAGCAGCGGTCTGGTTCCGCGTGGTAG
CAGCATGCATGAAGATGAAATTCTGGATGAGTATAACAACGGTCGCAAAAACTTTAG
CGGTATTGATCTGCAGGGTGCAGATCTGGAAGATGCAGAACTGGCAGGCGCAGAT
 
CTGAGTGATGCAAATCTGGAACAGGCAAATCTGCAGAATGCCAATCTGGCCAATGC
CAACCTGGAAAATGCGAATCTGCAGGACGCCGACCTGCATGGTGCTCGTCTGCAG
AACGCGGATCTGCGTGGCGCAAATTTAGAAGATGCCGATCTGGCGAACGCACGTCT
GGAAGGCGCTAATCTGGCCGAAGCCGATCTGCGTCGTGCAGACTTACGTGGTGCG
GATCTGACCAATGCGGATTTAGAGGATGCGGACCTGGAAGGTGCAAATTTACATGG
TGCACGTTTAGAAGGTGCGAACCTGCGTGGTGCCAATCTTACCGATGCGCGTCTGG
AAAACGCCGATCTGAGCGGTGCCGATTTACGTAACGCGAATCTTGAAGGCGCAAAT
ACCCGTGGTGCACGCCTGCAAGATGCTGATTTACGCGGTGCAAAACTGCGTCCGG
TTCTGTGGAATACCGCCAACTTAGAAGGCGCACAGATGCAGACCAATCAGAAAGCA
GAATATCAGCGTGCAAATGGTGGTGGTGGCAGCTCAATGCACGAGGATGAAATCCT
TGACGAATACAATAATGGCCGTAAGAATTTTTCCGGCATCGATTTACAGGGTGCCGA
CCTTGAGGATGCCGAATTAGCCGGTGCTGATCTGTCAGACGCGAATTTAGAACAGG
CCAATTTACAGAACGCAAACCTGGCAAACGCAAACTTAGAAAACGCCAACCTTCAG
GATGCTGATCTTCATGGCGCACGCTTACAGAATGCAGATCTTCGCGGAGCGAACCT
TGAAGATGCTGACCTGGCAAATGCTCGCCTTGAGGGTGCGAATCTGGCAGAGGCA
GATTTACGCAGAGCGGACCTTCGTGGTGCAGATTTAACAAACGCTGATCTTGAGGA
CGCTGACTTAGAGGGTGCAAACCTTCACGGTGCTAGACTTGAAGGGGCTAACTTAC
GCGGAGCCAATCTGACAGACGCTCGTTTAGAAAATGCAGACCTGAGCGGAGCTGAT
CTGCGCAACGCCAATCTGGAAGGTGCCAATACACGCGGTGCCCGTTTACAGGACG
CGGATTTACGGGGAGCGAAATTACGTCCGGTGTTATGGAATACAGCAAACCTTGAA
GGTGCCCAAATGCAGACAAACCAAAAAGCCGAGTATCAACGTGCCAACGGTGGCG
GAGGTTCAAGTATGCATGAGGACGAGATTTTGGACGAGTACAACAATGGAAGAAAA
AACTTCTCAGGCATCGATCTGCAAGGCGCAGACTTGGAGGACGCAGAACTTGCCG
GTGCCGACTTATCAGATGCTAACCTTGAACAAGCCAATCTTCAAAATGCTAATCTTG
CAAACGCTAATTTGGAGAATGCGAACTTACAAGATGCGGATCTGCACGGTGCTCGC
TTGCAAAATGCCGACTTGCGAGGGGCAAACTTGGAAGATGCGGACTTAGCCAATGC
ACGGTTGGAGGGTGCTAATTTAGCGGAAGCAGACCTTCGTCGCGCTGACCTTCGG
GGAGCCGACCTGACCAACGCTGACCTGGAAGATGCCGATTTAGAAGGTGCAAATCT
GCATGGCGCTCGCCTGGAAGGCGCAAACTTGCGTGGTGCTAACCTGACAGATGCA
CGGCTTGAAAACGCGGACCTGTCAGGGGCAGACCTGCGTAATGCCAATTTGGAGG
GTGCGAACACCCGTGGGGCACGTTTACAAGACGCCGATCTTAGAGGCGCTAAACTT
CGTCCTGTTTTATGGAACACCGCTAATTTAGAGGGTGCCCAGATGCAAACGAATCAA
AAGGCGGAATACCAACGTGCGAATGGCGGAGGCGGTAGTAGTATGCACGAAGATG
AGATTTTGGATGAATACAATAACGGACGCAAGAACTTCTCTGGAATTGACCTGCAAG
GTGCGGACTTAGAAGATGCTGAGTTAGCGGGTGCCGATCTGAGTGACGCCAACCTT
GAGCAGGCCAACCTGCAGAACGCAAATTTAGCAAATGCGAATTTGGAAAACGCTAA
CCTGCAAGACGCGGACTTACATGGCGCAAGACTGCAAAATGCGGATCTTCGGGGT
GCTAACTTAGAGGACGCCGATTTAGCGAATGCTCGGTTAGAAGGGGCAAACCTGGC
GGAAGCGGATTTACGTCGTGCTGATCTGCGTGGGGCAGATCTGACAAATGCCGATC
TTGAAGATGCAGATCTTGAGGGTGCCAACTTGCATGGTGCACGACTTGAAGGTGCC
AATTTACGTGGCGCAAATCTGACCGACGCACGCCTGGAAAATGCAGATCTTTCTGG
TGCCGATCTGCGTAATGCAAACCTGGAAGGGGCTAATACGCGTGGCGCTCGGCTG
CAGGATGCCGACTTAAGAGGCGCAAAATTACGCCCTGTGCTGTGGAACACGGCGA
ACTTGGAAGGTGCGCAAATGCAAACTAATCAGAAAGCTGAGTACCAACGCGCTAAT
GGTCAGCAGACAAATGGTTAA
            >SynRFR24_nc.x6_1 [SEQ ID NO: 170] 
MGSSHHHHHHSSGLVPRGSRMRRDEILEEYRRGRRNFQHINLQEIELTNASLTGADLS
YANLHHANLSRANLRSADLRNANLSHANLSGANLEEANLEAANLRGADLHEANLSGAD
LQEANLTQANLKDANLSDANLEQADLAGADLQGAVLDGANLHGANLNNANLSEAMLTR
 
ANLEQADLSGARTTGARLDDADLRGATVDPVLWRTASLRGAKMEEEQRREYERARGG
GGSRMRRDEILEEYRRGRRNFQHINLQEIELTNASLTGADLSYANLHHANLSRANLRSA
DLRNANLSHANLSGANLEEANLEAANLRGADLHEANLSGADLQEANLTQANLKDANLS
DANLEQADLAGADLQGAVLDGANLHGANLNNANLSEAMLTRANLEQADLSGARTTGA
RLDDADLRGATVDPVLWRTASLRGAKMEEEQRREYERARGGGGSRMRRDEILEEYRR
GRRNFQHINLQEIELTNASLTGADLSYANLHHANLSRANLRSADLRNANLSHANLSGAN
LEEANLEAANLRGADLHEANLSGADLQEANLTQANLKDANLSDANLEQADLAGADLQG
AVLDGANLHGANLNNANLSEAMLTRANLEQADLSGARTTGARLDDADLRGATVDPVLW
RTASLRGAKMEEEQRREYERARGGGGSRMRRDEILEEYRRGRRNFQHINLQEIELTNA
SLTGADLSYANLHHANLSRANLRSADLRNANLSHANLSGANLEEANLEAANLRGADLHE
ANLSGADLQEANLTQANLKDANLSDANLEQADLAGADLQGAVLDGANLHGANLNNANL
SEAMLTRANLEQADLSGARTTGARLDDADLRGATVDPVLWRTASLRGAKMEEEQRRE
YERARGGGGSRMRRDEILEEYRRGRRNFQHINLQEIELTNASLTGADLSYANLHHANLS
RANLRSADLRNANLSHANLSGANLEEANLEAANLRGADLHEANLSGADLQEANLTQAN
LKDANLSDANLEQADLAGADLQGAVLDGANLHGANLNNANLSEAMLTRANLEQADLSG
ARTTGARLDDADLRGATVDPVLWRTASLRGAKMEEEQRREYERARGGGGSRMRRDEI
LEEYRRGRRNFQHINLQEIELTNASLTGADLSYANLHHANLSRANLRSADLRNANLSHA
NLSGANLEEANLEAANLRGADLHEANLSGADLQEANLTQANLKDANLSDANLEQADLA
GADLQGAVLDGANLHGANLNNANLSEAMLTRANLEQADLSGARTTGARLDDADLRGA
TVDPVLWRTASLRGAKMEEEQRREYERARGKSDDG
            >SynRFR24_nc.x6_1 [DNA Sequence] [SEQ ID NO: 171] 
ATGGGCAGCAGCCATCACCATCATCATCATAGCAGCGGTCTGGTTCCGCGTGGTAG
CCGTATGCGTCGTGATGAAATTCTGGAAGAATATCGTCGTGGTCGTCGTAACTTTCA
GCATATTAATCTGCAAGAAATCGAACTGACCAATGCAAGCCTGACCGGTGCCGATC
TGAGCTATGCAAATCTGCATCATGCCAATCTGAGCCGTGCCAACCTGCGTTCAGCC
GATCTGCGTAATGCGAACCTGAGTCATGCGAATCTGTCAGGCGCAAACCTGGAAGA
AGCAAATCTGGAAGCAGCGAACCTGCGTGGTGCGGATCTGCATGAAGCAAACCTGT
CAGGTGCCGATTTACAAGAAGCCAACCTGACACAGGCAAATCTGAAAGACGCGAAT
CTGAGTGACGCCAATCTGGAACAGGCAGACCTGGCAGGCGCTGATCTGCAGGGTG
CAGTTCTGGATGGTGCTAATCTGCATGGTGCGAATTTGAATAACGCTAATCTGAGCG
AAGCAATGCTGACTCGTGCTAATTTAGAACAGGCGGATCTGAGCGGTGCACGTACC
ACCGGTGCGCGTCTGGATGATGCGGATTTACGTGGTGCAACCGTTGATCCGGTTCT
GTGGCGTACCGCAAGCCTGCGTGGCGCTAAAATGGAAGAAGAACAGCGTCGCGAA
TATGAACGTGCCCGTGGTGGTGGCGGTTCTCGCATGCGTAGAGATGAAATCTTAGA
AGAGTACCGTCGCGGACGTCGCAATTTTCAACATATCAACTTACAAGAGATCGAGCT
GACAAACGCATCACTGACAGGTGCAGACCTGTCTTATGCCAATTTACATCATGCAAA
TTTGTCGAGAGCGAATCTGCGCTCAGCAGATTTACGCAACGCAAATCTGTCCCATG
CAAACCTGAGCGGAGCAAATTTAGAGGAAGCCAATTTAGAAGCCGCAAATCTTCGC
GGTGCCGACCTGCACGAGGCTAACCTGAGTGGCGCAGATCTTCAAGAAGCGAATTT
AACCCAGGCCAATCTTAAAGATGCCAACTTATCAGATGCTAACCTTGAGCAGGCAG
ATTTAGCGGGTGCAGACTTACAGGGTGCCGTGTTAGATGGTGCAAATTTACACGGT
GCAAATCTTAATAACGCGAACTTATCTGAGGCCATGCTGACACGCGCAAACTTAGA
GCAAGCGGATTTATCAGGTGCCCGTACAACAGGCGCACGTTTAGATGATGCCGATC
TTCGGGGTGCGACAGTGGACCCTGTGCTGTGGCGCACAGCCAGCTTACGCGGTGC
CAAGATGGAAGAGGAACAACGCCGTGAGTACGAACGTGCACGCGGTGGCGGTGG
CAGTCGGATGCGTCGGGACGAGATTCTTGAGGAATACAGACGCGGTCGCAGAAAT
TTTCAGCACATAAACCTTCAAGAAATTGAGTTAACCAATGCCTCTTTAACCGGTGCG
GACTTAAGCTATGCCAACCTTCATCACGCGAATTTATCACGTGCGAACCTTCGCAGC
GCAGACCTGCGCAACGCTAATTTAAGCCATGCGAACTTAAGCGGTGCAAACCTTGA
GGAAGCGAACCTTGAAGCTGCGAATTTACGCGGAGCTGATTTACATGAGGCCAATT
TATCTGGGGCTGACTTGCAAGAGGCAAACTTAACGCAGGCTAACTTAAAAGACGCA
AACCTTTCAGACGCCAACCTTGAACAAGCCGATCTTGCTGGTGCGGACCTTCAAGG
TGCCGTGCTGGACGGTGCGAACTTGCATGGGGCTAATCTTAACAATGCTAATCTTT
 
CCGAAGCCATGTTAACCCGTGCTAACTTGGAACAAGCTGACTTAAGTGGCGCTCGC
ACCACAGGTGCACGCCTGGACGACGCAGACCTTCGTGGTGCCACAGTAGATCCAG
TGTTATGGCGCACCGCCTCATTACGGGGTGCAAAAATGGAAGAGGAACAAAGACGT
GAATATGAGCGTGCTCGCGGAGGTGGTGGCTCTCGCATGCGACGCGACGAAATTT
TAGAGGAATATCGCAGAGGTCGCCGTAATTTCCAACACATCAATTTGCAAGAAATAG
AGCTTACGAATGCGTCATTGACTGGGGCAGATCTGAGTTACGCTAACCTGCACCAC
GCGAACCTTAGTCGGGCAAACCTGCGCTCTGCGGATCTTCGGAACGCCAACTTGA
GCCACGCAAATTTAAGTGGTGCGAATCTGGAAGAGGCCAACTTAGAGGCTGCCAAT
CTTAGAGGGGCAGACCTGCATGAGGCGAATCTTTCAGGTGCTGATCTGCAAGAGG
CCAATCTGACCCAAGCCAACTTGAAGGATGCAAACCTTAGTGATGCAAATCTTGAGC
AAGCTGATCTGGCAGGCGCAGATTTACAAGGTGCGGTGCTTGATGGCGCTAACCTT
CATGGGGCAAACTTGAATAATGCCAACCTGTCGGAAGCTATGCTTACCAGAGCGAA
CTTGGAGCAGGCCGACCTTTCTGGTGCACGCACAACGGGTGCTAGACTGGATGAC
GCCGACTTAAGAGGTGCGACGGTTGATCCTGTTTTATGGCGGACAGCAAGTTTAAG
AGGCGCAAAGATGGAAGAGGAACAGCGAAGAGAATATGAAAGAGCACGCGGTGGT
GGCGGTTCTCGCATGCGTAGAGATGAAATCTTAGAAGAGTACCGTCGCGGACGTC
GCAATTTTCAACATATCAACTTACAAGAGATCGAGCTGACAAACGCATCACTGACAG
GTGCAGACCTGTCTTATGCCAATTTACATCATGCAAATTTGTCGAGAGCGAATCTGC
GCTCAGCAGATTTACGCAACGCAAATCTGTCCCATGCAAACCTGAGCGGAGCAAAT
TTAGAGGAAGCCAATTTAGAAGCCGCAAATCTTCGCGGTGCCGACCTGCACGAGGC
TAACCTGAGTGGCGCAGATCTTCAAGAAGCGAATTTAACCCAGGCCAATCTTAAAGA
TGCCAACTTATCAGATGCTAACCTTGAGCAGGCAGATTTAGCGGGTGCAGACTTAC
AGGGTGCCGTGTTAGATGGTGCAAATTTACACGGTGCAAATCTTAATAACGCGAACT
TATCTGAGGCCATGCTGACACGCGCAAACTTAGAGCAAGCGGATTTATCAGGTGCC
CGTACAACAGGCGCACGTTTAGATGATGCCGATCTTCGGGGTGCGACAGTGGACC
CTGTGCTGTGGCGCACAGCCAGCTTACGCGGTGCCAAGATGGAAGAGGAACAACG
CCGTGAGTACGAACGTGCACGCGGTGGCGGTGGCAGTCGGATGCGTCGGGACGA
GATTCTTGAGGAATACAGACGCGGTCGCAGAAATTTTCAGCACATAAACCTTCAAGA
AATTGAGTTAACCAATGCCTCTTTAACCGGTGCGGACTTAAGCTATGCCAACCTTCA
TCACGCGAATTTATCACGTGCGAACCTTCGCAGCGCAGACCTGCGCAACGCTAATT
TAAGCCATGCGAACTTAAGCGGTGCAAACCTTGAGGAAGCGAACCTTGAAGCTGCG
AATTTACGCGGAGCTGATTTACATGAGGCCAATTTATCTGGGGCTGACTTGCAAGA
GGCAAACTTAACGCAGGCTAACTTAAAAGACGCAAACCTTTCAGACGCCAACCTTG
AACAAGCCGATCTTGCTGGTGCGGACCTTCAAGGTGCCGTGCTGGACGGTGCGAA
CTTGCATGGGGCTAATCTTAACAATGCTAATCTTTCCGAAGCCATGTTAACCCGTGC
TAACTTGGAACAAGCTGACTTAAGTGGCGCTCGCACCACAGGTGCACGCCTGGAC
GACGCAGACCTTCGTGGTGCCACAGTAGATCCAGTGTTATGGCGCACCGCCTCATT
ACGGGGTGCAAAAATGGAAGAGGAACAAAGACGTGAATATGAGCGTGCTCGCGGA
AAATCAGATGATGGTTAA
            >SynRFR24_nc.x2_1rSEQ ID NO: 172] 
MGSSHHHHHHSSGLVPRGSRMRRDEILEEYRRGRRNFQHINLQEIELTNASLTGADLS
YANLHHANLSRANLRSADLRNANLSHANLSGANLEEANLEAANLRGADLHEANLSGAD
LQEANLTQANLKDANLSDANLEQADLAGADLQGAVLDGANLHGANLNNANLSEAMLTR
ANLEQADLSGARTTGARLDDADLRGATVDPVLWRTASLRGAKMEEEQRREYERARGG
GGSRMRRDEILEEYRRGRRNFQHINLQEIELTNASLTGADLSYANLHHANLSRANLRSA
DLRNANLSHANLSGANLEEANLEAANLRGADLHEANLSGADLQEANLTQANLKDANLS
DANLEQADLAGADLQGAVLDGANLHGANLNNANLSEAMLTRANLEQADLSGARTTGA
RLDDADLRGATVDPVLWRTASLRGAKMEEEQRREYERARGKSDDG
            >SynRFR24_nc.x2 1 [DNA Sequence] [SEQ ID NO: 1731

ATGGGCAGCAGCCATCACCATCATCATCATAGCAGCGGTCTGGTTCCGCGTGGTAG
CCGTATGCGTCGTGATGAAATTCTGGAAGAATATCGTCGTGGTCGTCGTAACTTTCA
GCATATTAATCTGCAAGAAATCGAACTGACCAATGCAAGCCTGACCGGTGCCGATC

TGAGCTATGCAAATCTGCATCATGCCAATCTGAGCCGTGCCAACCTGCGTTCAGCC
GATCTGCGTAATGCGAACCTGAGTCATGCGAATCTGTCAGGCGCAAACCTGGAAGA
AGCAAATCTGGAAGCAGCGAACCTGCGTGGTGCGGATCTGCATGAAGCAAACCTGT
CAGGTGCCGATTTACAAGAAGCCAACCTGACACAGGCAAATCTGAAAGACGCGAAT
CTGAGTGACGCCAATCTGGAACAGGCAGACCTGGCAGGCGCTGATCTGCAGGGTG
CAGTTCTGGATGGTGCTAATCTGCATGGTGCGAATTTGAATAACGCTAATCTGAGCG
AAGCAATGCTGACTCGTGCTAATTTAGAACAGGCGGATCTGAGCGGTGCACGTACC
ACCGGTGCGCGTCTGGATGATGCGGATTTACGTGGTGCAACCGTTGATCCGGTTCT
GTGGCGTACCGCAAGCCTGCGTGGCGCTAAAATGGAAGAAGAACAGCGTCGCGAA
TATGAACGTGCCCGTGGTGGTGGCGGTTCTCGCATGCGTAGAGATGAAATCTTAGA
AGAGTACCGTCGCGGACGTCGCAATTTTCAACATATCAACTTACAAGAGATCGAGCT
GACAAACGCATCACTGACAGGTGCAGACCTGTCTTATGCCAATTTACATCATGCAAA
TTTGTCGAGAGCGAATCTGCGCTCAGCAGATTTACGCAACGCAAATCTGTCCCATG
CAAACCTGAGCGGAGCAAATTTAGAGGAAGCCAATTTAGAAGCCGCAAATCTTCGC
GGTGCCGACCTGCACGAGGCTAACCTGAGTGGCGCAGATCTTCAAGAAGCGAATTT
AACCCAGGCCAATCTTAAAGATGCCAACTTATCAGATGCTAACCTTGAGCAGGCAG
ATTTAGCGGGTGCAGACTTACAGGGTGCCGTGTTAGATGGTGCAAATTTACACGGT
GCAAATCTTAATAACGCGAACTTATCTGAGGCCATGCTGACACGCGCAAACTTAGA
GCAAGCGGATTTATCAGGTGCCCGTACAACAGGCGCACGTTTAGATGATGCCGATC
TTCGGGGTGCGACAGTGGACCCTGTGCTGTGGCGCACAGCCAGCTTACGCGGTGC
CAAGATGGAAGAGGAACAACGCCGTGAGTACGAACGTGCACGCGGTAAATCAGAT
GATGGTTAA

### Figure legends

Figure 1 - Alignment of the original SynRFR24.1 and the newly designed SynRFR24.new_caps sequences.
Figure 2 - SynRFR protein expression and purification. SDS-PAGE gel showing expression and purification of the SynRFR24.1 and SynRFR24.new_caps proteins, where (L) PageRuler plus ladder, (S) supernatant, (I) insoluble fraction, (F) Ni-NTA column flow-through, (W) wash fraction, (E) elution fraction.
Figure 3 - SynRFR24.new_caps was found to crystallise in 0.2 M calcium chloride, 0.1 M Tris hydrochloride pH 8.0, and 44 % v/v PEG 400.
Figure 4 - Polarised optical microscopy of the SynRFR24.new_caps at 40 mg ml-1 a) after 0 h and b) after 4 days stored at 4 degrees.

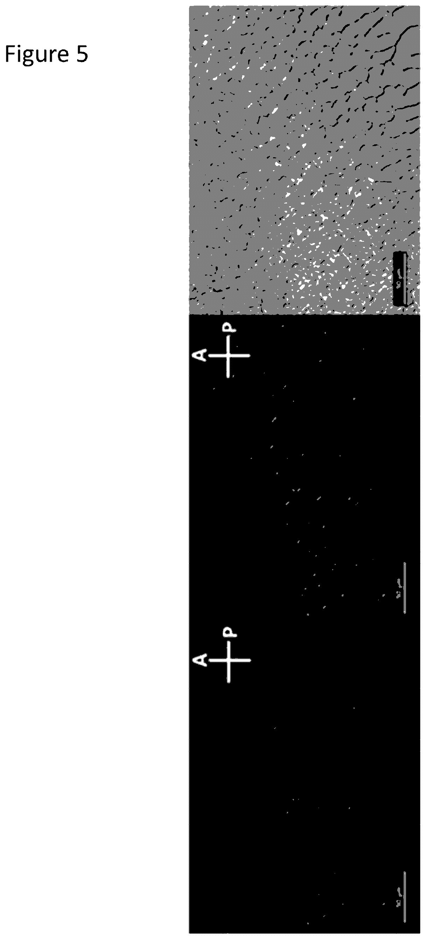

Figure 5 - Polarised optical microscopy of SynRFR24.1 60 mg ml-1 after 4 days stored at 4 degrees. No visible patterns were observed within the first 24h.
Figure 6 - Liquid SAXS spectrum of a) SynRFR24.new_caps and b) probability distribution indicating some long range order c) SynRFR24.1 and d) probability distribution also showing some longer range order.
Figure 7 - Sequence of a newly designed concatemer protein variant, SynRFR24.nc.x4.1, derived by repeating 4 units of the SynRFR24.new_caps monomer. The underlined regions indicate the flexible linker sequences between the repeats.
Figure 8 - Sequence of a newly designed concatemer protein variant, SynRFR24.nc.x8.1, derived by repeating 8 units of the SynRFR24.new_caps monomer. The purple highlighted regions indicate the flexible linker sequences between the repeats.
Figure 9 - SynRFR24.nc.x4.1 protein expression and purification. SDS-PAGE gel showing expression and purification of the 95.2 kDa SynRFR24.nc.x4.1 protein, where (L) PageRuler plus ladder, (S) supernatant, (FT) Ni-NTA column flow-through, (0 - 500) imidazole gradient wash and elution fractions.
Figure 10 - Thermofluor melting curves of the SynRFR24.nc.x4.1, SynRFR24_nnum_35_005, SynRFR24_nnum_28_002, SynRFR24_nnum_39_003 and SynRFR24_nnum_36_005 proteins. The dotted vertical line shows the estimated melting temperature.
Figure 11 - SynRFR24.nc.x4.1 SEC-MALS. The dotted line shows MALS estimated molar mass (Da), the solid line shows UV absorbance.
Figure 12 - SynRFR24.nc.x8.1 protein expression and purification. SDS-PAGE gel showing expression and purification of the 188 kDa SynRFR24.nc.x8.1 protein, where (L) PageRuler plus ladder, (S) supernatant, (FT) Ni-NTA column flow-through, (0 - 500) imidazole gradient wash and elution fractions.
Figure 13 - Polarised optical miroscopy of SynRFR24.nc.x4.1 at 70 mg ml-1 and 160 mg ml-1.
Figure 14 - Optical and polarised optical microscopy of hand-drawn fibres (115 mg ml-1)
Figure 15 - a) mounted fibres pre-tensile measurements b) still photographs of the tensile measurement c) stress-strain curve of SynRFR24.nc.x4.1 hand-drawn fibre and d-e) SEM of the fractured cross sections post-tensile.
Figure 16 - a) mounted fibres pre-tensile b) polarised optical microscopy of wet-spun fibre c) stress- strain curves for valid tensile tests (several tests were excluded and d) SEM of the fractured fibre cross section and microstructure.
Figure 17 - Digital image of SynRFR24.nc.x4.1 140 mg ml-1 wet-spun fibre wound on a glass vial.
Figure 18 - Phase diagram (with POM) of liquid crystalline concatenated monomers with increasing concentration where N is the nematic phase.
Figure 19 - a) POM of the SynRFR24.nc.x4.1 at 100 mg ml-1 and b) stress-strain curves for the valid tensile tests.

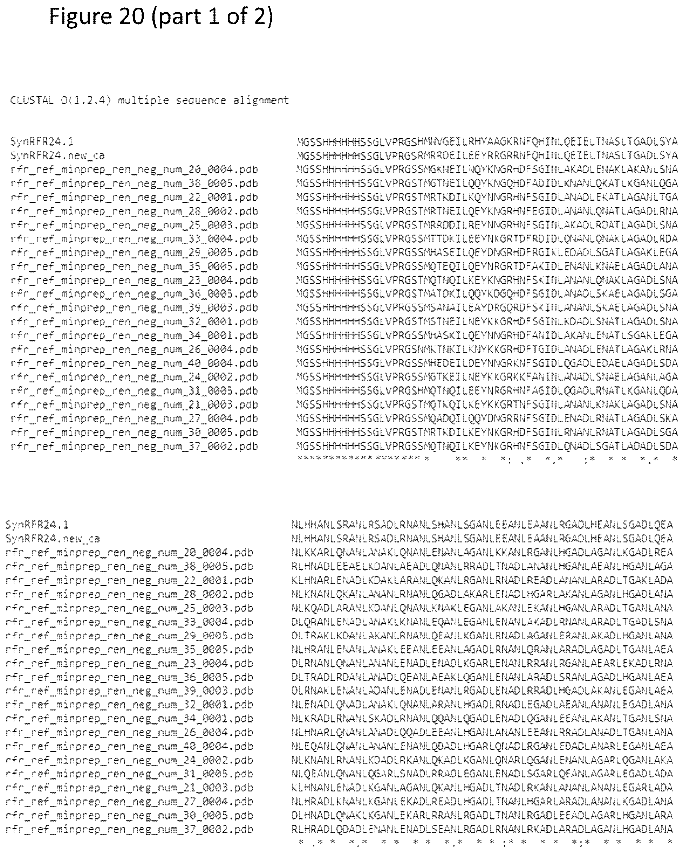

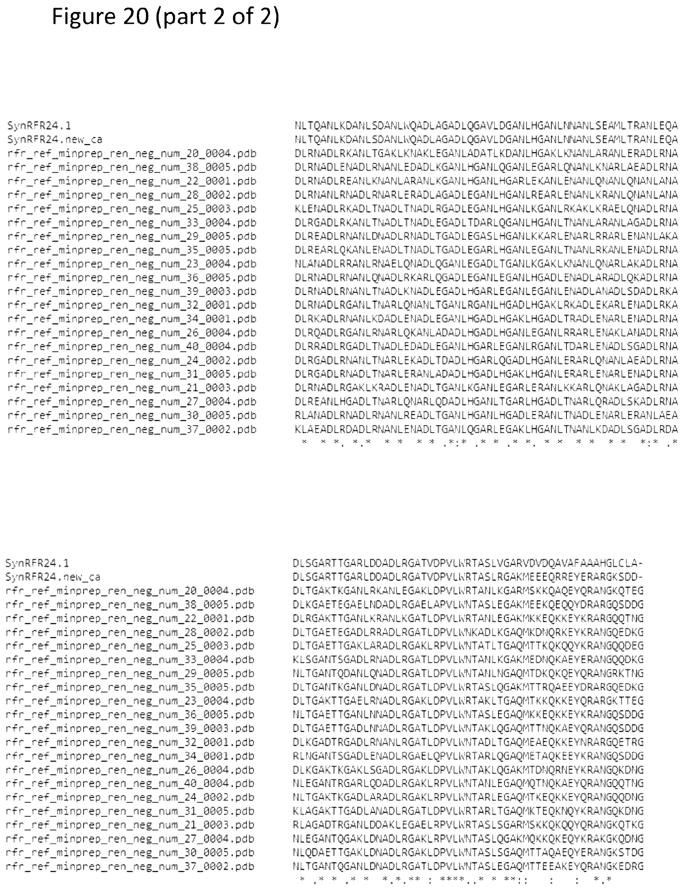

Figure 20 - Alignment of SynRFR24.1, SynRFR2.new_caps and charge variants of SynRFR24.new_caps.

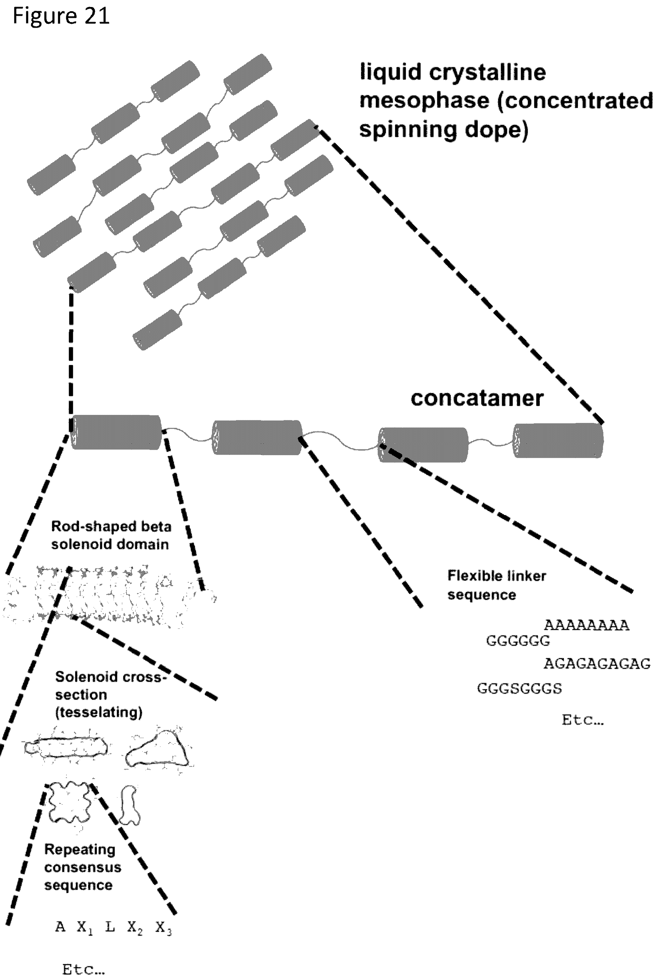

Figure 21 - Schematic of hierarchical structure of the beta-solenoid proteins in concentrated spinning dopes. Repeating consensus sequence motifs form rod-shaped beta solenoid domains, which can be further arranged with flexible linkers to form a high molecular weight concatemers. The concentrated protein solutions self-assemble into liquid crystalline mesophases suitable for fibre spinning.
Figure 22 - Estimates of the molar mass of approximately 0.5 - 1.5 mg of a) SynRFR24.nnum_35, b) SynRFR24.nnum_36, c) SynRFR24.nnum_40, d) SynRFR24.nc.x2, e) SynRFR24.nc.x4 and f) SynRFR24.nc.x6, and confirmation of monomeric status of each measured design using SEC-MALS.
Figure 23 - 1D proton NMR spectra from protein folding experiments (-5 to 15 ppm) of a) SynRFR24.nnum_28, b) SynRFR24.nnum_36, c) SynRFR24.nc.x4, d) SynRFR24.nnum_35 and e) SynRFR24.nnum_40. f) shows the smaller ppm range of -0.5 to 1.5, and demonstrates peaks in this region characteristic of well-packed side chain methyl groups.
Figure 24 - CD spectra collected from 190 to 260 nm to estimate secondary structure content of SynRFR24.1, SynRFR24.new_caps, SynRFR24.nc.x4.1, SynRFR24_nnum_36, SynRFR24_nnum_38 and SynRFR24_nnum_40.
Figure 25 - Optical textures of concentrated protein solutions set up on glass slides and capillaries by polarised optical microscopy. Values above the images indicate the concentration of the protein (mg/ml).

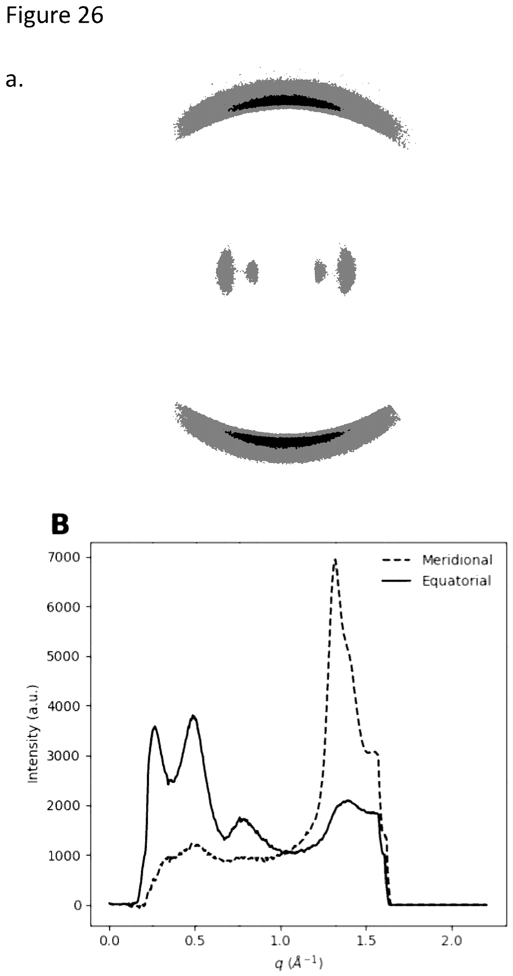

Figure 26 - X-ray diffraction experiments carried out on single fibres: a. The mean intensity of 10 background images with no sample present were subtracted from the mean intensity of 10 images with the fibre present to reduce noise; b. 1D radial intensity profiles for meridional and equatorial directions with the light grey vertical lines indicating q=1.32 Å⁻¹ (d=4.75 Å), q=0.255 Å⁻¹ (d=24.6 Å), q=2*0.255 Å⁻¹, q=3*0.255 Å⁻¹. Where 4.75 Å matches the helical pitch of the beta-solenoid, and 24.6 Å is close to the diameter of the beta solenoid domains. These results show a high degree of alignment in the fibre, with beta strands aligned perpendicular to axis of the fibre as would be expected if the beta solenoid domains were aligned parallel to the axis of the fibre
Figure 27 - Fourier-transform infra-red (FTIR) spectra of a. the liquid crystalline spinning dope solution and b. the spun fibre, showing similar structure of the protein before and after spinning of the fibres .
Figure 28 - Polarized Raman Spectroscopy was performed to identify the different amide regions of the fibre, supporting the FTIR findings of Figure 27, over: a. a wavenumber of 1000-2000 cm⁻¹; and b. a wavenumber of 750-3250 cm⁻¹. Raman data were obtained with three different polarization configurations, VV, VH and HH, where V and H refer to orientation parallel and perpendicular, respectively.
Figure 29 - Test expressions of new concatemer variants
   Codon optimised genes were expressed in the KRX *E. coli* strain and induced with 0.1% filter sterilised rhamnose and grown overnight at 25 degrees C. Non-induced cultures were also grown in the same way for comparison. Cell were harvested and lysed by sonication. SDS-PAGE gels of the soluble and insoluble fractions showed significant protein over-expression bands for all proteins in the soluble fraction. L indicates the PageRuler plus ladder, S indicates the soluble supernatant, I insoluble fraction, and the + indicates that the culture had been induced.

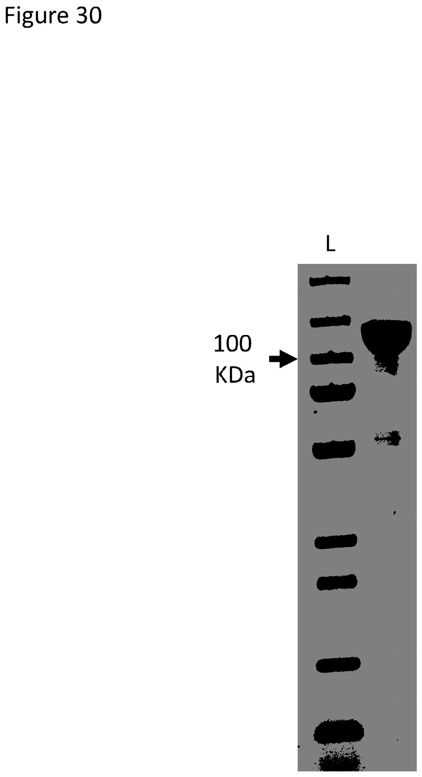

Figure 30 -Larger scale purification of SynRFR24.nnum35.x4
   SynRFR24.nnum35.x4 was purified to high purity and concentration. L is the PageRuler plus ladder and the other lane is the concentrated soluble protein. The SynRFR24.nnum35.x4 sample was found to be highly soluble and could be concentrated to >200 mg/ml.

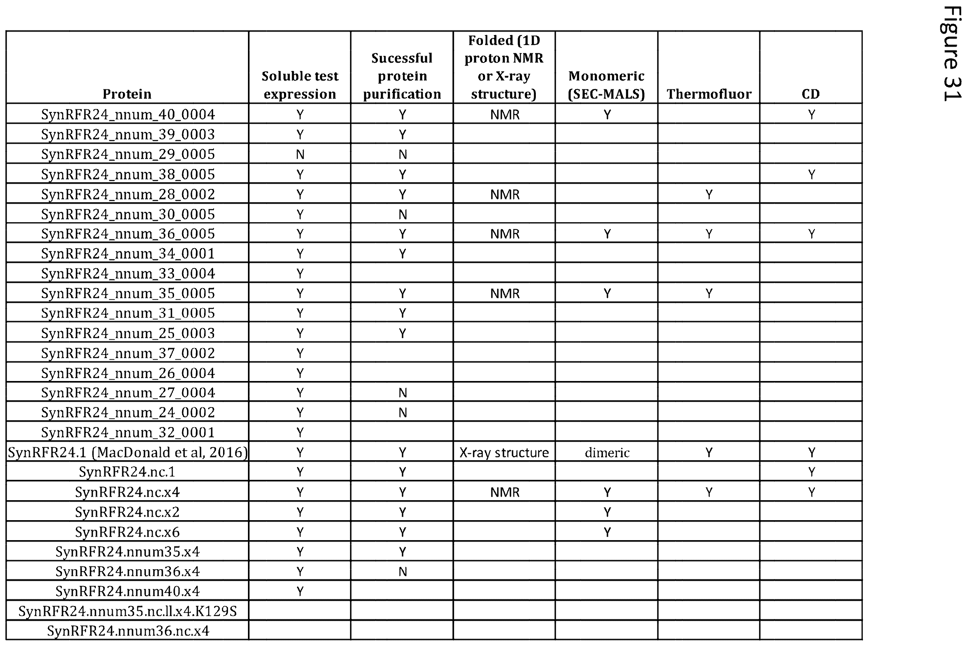

Figure 31 - Table summarising data obtained for a number of proteins. Data shows that most of the proteins designs tested to-date appear to be well-expressed, soluble, folded, monomeric and share similar CD spectra features to the original SynRFR24.1. Lack of successful protein purification indicates only that the purification protocol requires further optimisation compared to the standard purification protocol described for SynRFR24.nc.x4.1.

### Examples

### Example 1

The original SynRFR series of artificial beta solenoid proteins were designed to form homodimers with the dimer interface at the C-terminal capping domain (MacDonald et al 2016 PNAS 37: 10346-10351).

The rigid rod-like SynRFR domain has an aspect ratio of around 3:1, potentially permitting spinning from a well-ordered nematic liquid crystalline phase. In order to achieve this, new monomeric versions of the SynRFR domain repeat unit were required that can then be concatenated into higher molecular weight polymers.

Using the high-resolution crystal structure of the SynRFR24.1 beta solenoid protein (PDB code: 4YC5), monomeric versions were created by completely redesigning the sequence of the terminal cap residues using computational protein design methods (using the RosettaDesign algorithm, https://www.rosettacommons.org/).

Extra restraint terms were added to the Rosetta potential energy function to create designs biased towards a range of different net surface charges **[SEQ ID NO: 3-23].** In addition to these designs, a more conservative design was created where only the C- and N-terminal caps were redesigned as we previously found that these caps are vital for the soluble folding of the protein by shielding the hydrophobic core of the solenoid. This design, called SynRFR24.new_caps **[SEQ ID NO: 6],** was used for initial expression tests.

SynRFR24.new_caps (25.52 kDa) has a slightly higher molecular weight than SynRFR24.1 (24.95 KDa) **[SEQ ID NO: 7].** (Figure 1).

### Cloning, Expression and purification of repeat units

The SynRFR24.new_caps design was ordered as a gBlock gene fragment from IDT and cloned into a standard pET11a expression plasmid. The existing SynRFR24.1 design previously cloned in to the same plasmid. Both plasmids were sequence verified and then transformed in the KRX E. coli strain.

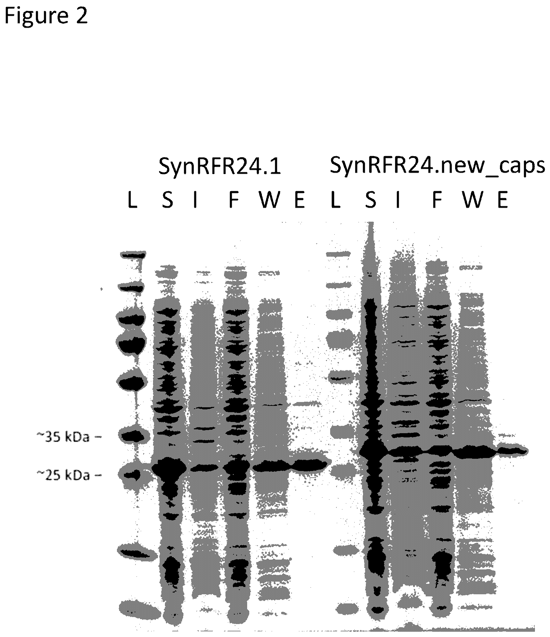

1 L of 2xYT medium was inoculated with 5 ml LB overnight cultures for both SynRFR24.1 and SynRFR24.new_caps (all media with appropriate antibiotics). The 1 L cultures were grown at 37 degrees Celsius until reaching an OD600 reading of 1. Expression was then induced with 0.1% filter sterilised rhamnose and the cultures grown overnight at 21 degrees Celsius. Cells were harvested and resuspended in lysis buffer (100 mM bicine and 150 mM NaCl buffer titrated to pH 9.0 with NaOH). The cells were sonicated and clarified by centrifugation at 40,000 × g for 40 min. The proteins were purified with a nickel-nitrilotriacetic acid (Ni-NTA) column; washed with 100mM bicine, 150mM NaCl, and 50 mM imidazole at pH 9.0; and eluted in 100 mM bicine, 150 mM NaCl, and 500 mM imidazole at pH 9.0. The new protein design was found to be well expressed and soluble (Figure 2). The purified proteins were concentrated (to 40 mg/ml SynRFR24.new_caps and 60 mg/ml SynRFR24.1) and exchanged into imidazole-free bicine buffer using 4 kDa cutoff centrifugal
concentrators.

### Crystallisation trials

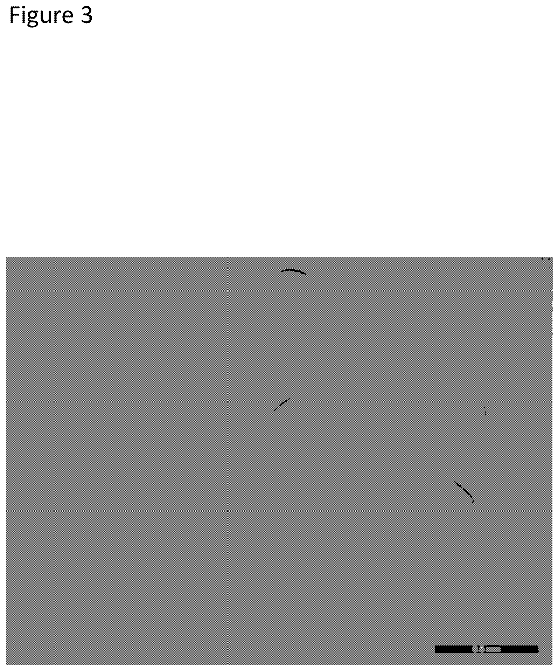

The concentrated SynRFR24.new_caps samples were used to set up vapour diffusion sparse-matrix crystallization trials with a Mosquito robot (TTP Labtech) in 864 different conditions. After two days crystals were observed in four conditions (Figure 3).

### Polarised Optical Microscopy - Liquid Crystal Formation

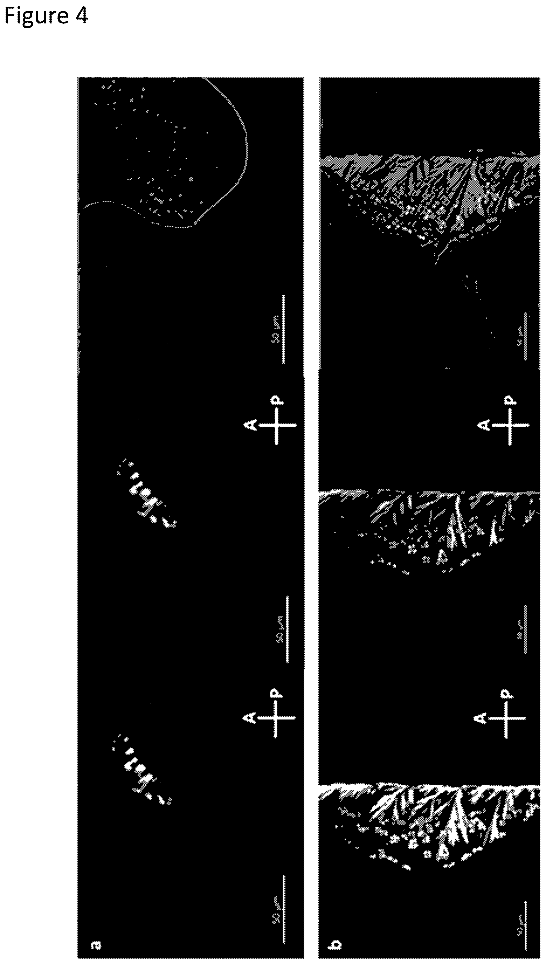

Polarised optical microscopy was utilised in order to observe any liquid crystalline behaviour of the protein monomers. 1 µl of each protein (namely SynRFR24.1 and SynRFR24.new_caps) were dropped on a clean glass slide with a cover slip placed on top. The concentration of the SynRFR24.1 and SynRFR24.new_caps was ca. 60 mg ml- and 40 mg ml-1, respectively, in bicine buffer. The samples were stored in the fridge at ca. 4 degrees. Once drop cast, the samples were viewed immediately using polarised optical microscopy (POM) and what potentially is the beginning of a Schlieren textured nematic liquid crystal was evident in small areas for the SynRFR24.new_caps (Figure 4a), however no LC-like behaviour was observed for the SynRFR24.1. After 4 days the initial area (Figure 4a) had not developed further, however other larger areas appear to be developing (Figure 4b). Regular patterns and some birefringence were observed for the SynRFR24.1 after 4 days but it is not certain whether the larger patterned areas are drying effects or liquid crystal formation.

### 1D Liquid SAXS

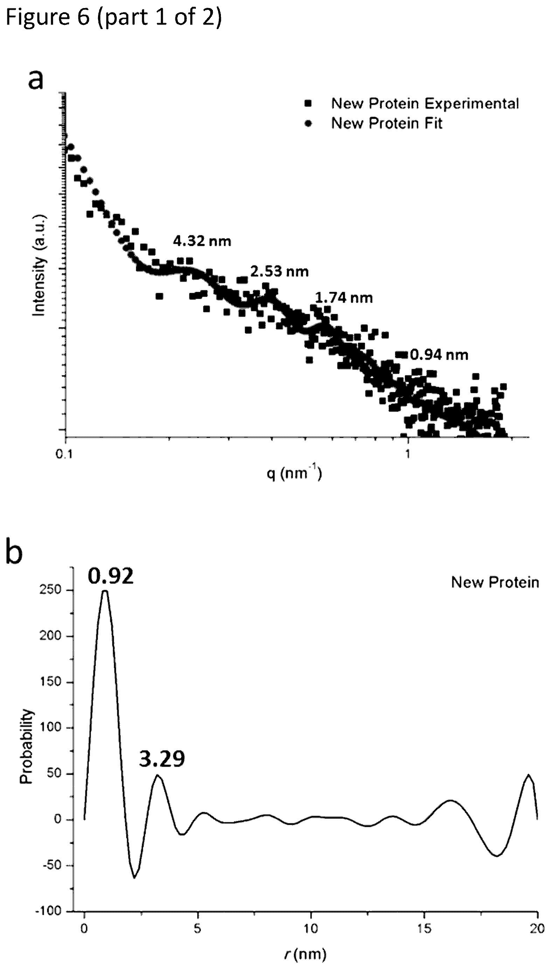

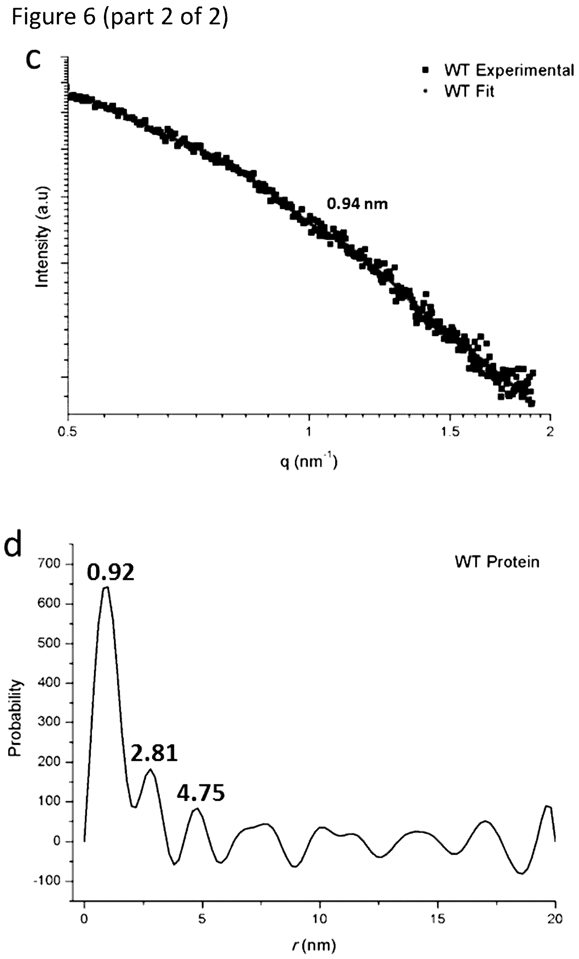

Small angle X-ray scattering (SAXS) measurements were performed using a PANalytical Empyrean diffractometer equipped with ScatterX78 module, from 0-4° 2θ, and the data analysis was performed using PANalytic EasySAXS software. For SAXS data collection, a step size of 0.003° 2θ, scan step time of 350 s, and scan speed of 0.002 °/s were used. Samples were prepared and stored in the fridge 20h before running. Approximately 50 µl of each protein was used for filling the capillary. The SynRFR24.new_caps was diluted to ca. 10 mg ml-1, which is a lower concentration than the protein liquid crystal observed in Figure 4a,b. Several peaks were identified in the SAXS specturm (Figure 6a) for the SynRFR24.new_caps which seem to correlate well with the dimensions of the protein monomers. The probability distribution indicates that the majority of events are between individual monomers but some long range order is observed (Figure 6b). The SynRFR24.1 SAXS spectrum is not well resolved, as the concentration was much higher than expected, and it is very possible that the SynRFR24.1 is too concentrated for a clear measurement (Figure 6c). However, some long range order is observed from the probability distribution data (Figure 6d).

### Potential Precipitants

Ten different coagulant solvents were investigated to observe how and if either of the proteins would precipitate. Several of the solvents were successful at coagulating the proteins, as summarised in Table 1. Both proteins produced white fibrous-like precipitate in several of the solvents, especially ethanol and propanol. 1.5 µl of each protein was injected into 5 ml of the coagulation solution. It is very plausible that the amount of precipitation of the SynRFR24.1 compared to the SynRFR24.new_caps is concentration dependent (60 mg ml-1 to 40 mg ml-1). From initial results, it is likely that higher concentrations of the protein monomers and/or concatenating several protein monomers would lead to a higher degree of coagulation and fibre formation. The degree of precipitation using ethanol was significant for both proteins (the difference in degree of precipitation is most likely concentration dependent between the 2 proteins) and it is possible to attempt spinning a fibre with slightly larger volumes (ca. 0.5-1 ml).

**Table 1: Summary of potential coagulation solvents for fibre formation (WP - white precipitate).**

| **Solvent** | **SynRFR24.1** | **SynRFR24.new_caps** |
|---|---|---|
| Ethanol | plenty of WP present | WP present |
| Acetone | WP present | WP present |
| Propanol | WP present | some WP present |
| Diethyl ether | Yes after 1h | None |
| Methanol | WP present | small amount WP present |
| CaCl 20 mM | WP HC of salt? | None |
| KCl 20 mM | clear then dissolved | None |
| MgSO₄ 20mM | None | transparent swirl |
| DMSO | V small amount WP present | None |
| DMSO:H₂O (50:50) | None | None |

### Example 2 - Design of high molecular weight concatenated versions of SynRFR24.new_caps

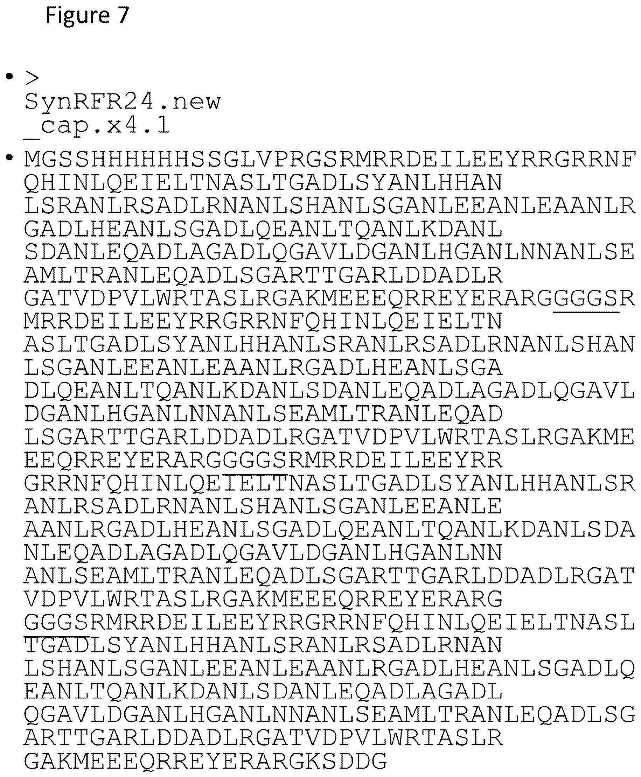

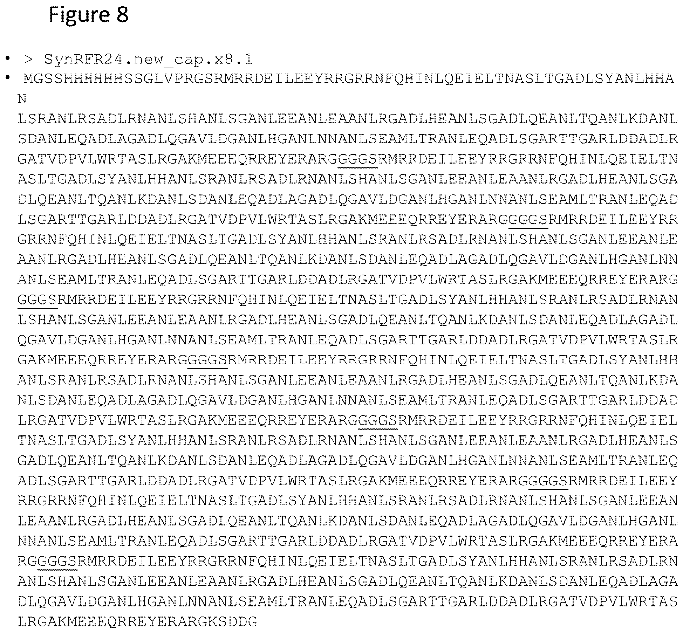

The SynRFR24.new_caps monomer described above was concatenated with flexible poly-glycine linker sequences to produce a new high molecular weight version, labelled SynRFR24.nc.x4.1 (880 amino acid residues and 95.2 kDa molecular weight; Figure 7). A double length version, SynRFR24.nc.x8.1 (1740 amino acid residues and 188 kDa), was also designed (Figure 8).

### Cloning, expression and purification of SynRFR24.nc.x4.1

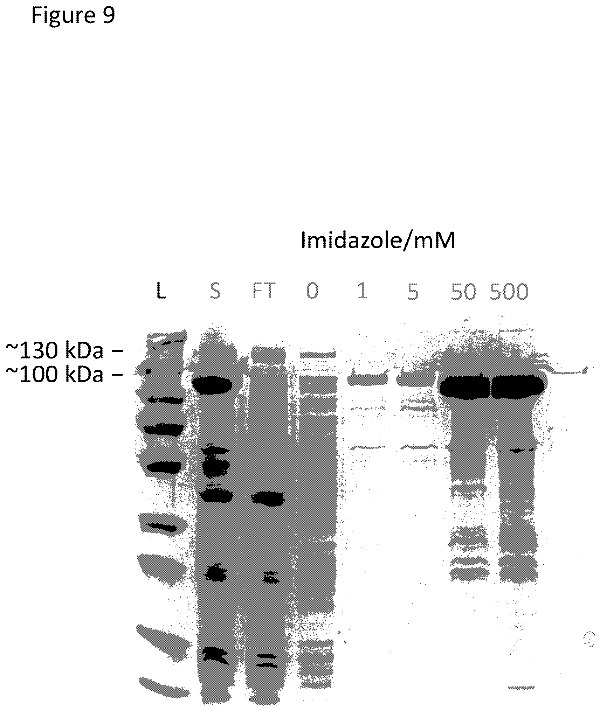

The SynRFR24.nc.x4.1 design was ordered as a codon-optimised synthetic gene from GeneArt/Thermo Fisher with flanking T7 promoter and terminator sequences. The plasmid was transformed into the KRX E. coli strain. 1 L of Terrific Broth medium was inoculated with 5 ml LB overnight cultures (all media with appropriate antibiotics). The 1 L cultures were grown at 37 degrees Celsius until reaching an OD600 reading of 1. Expression was then induced with 0.1% filter sterilised rhamnose and the cultures grown overnight at 25 degrees Celsius. Cells were harvested and resuspended in lysis buffer (100 mM bicine and 150 mM NaCl buffer titrated to pH 9.0 with NaOH). The cells were sonicated and clarified by centrifugation at 40,000 × g for 40 min. The proteins were purified with a nickel-nitrilotriacetic acid (Ni-NTA) column; washed with 100mM bicine, 150mM NaCl, and 0 to 5 mM imidazole at pH 9.0; and eluted in 100 mM bicine, 150 mM NaCl, and 500 mM imidazole at pH 9.0. The concatemer design was found to be well expressed and highly soluble (Figure 9). The purified proteins were concentrated (to around 100 - 150 mg ml-1) and exchanged into imidazole-free bicine buffer using 30 kDa cutoff centrifugal concentrators. Binding to the Ni-NTA appeared to be weak, with protein eluting at low imidazole concentrations (50 mM).

### Thermofluor assay for protein stability

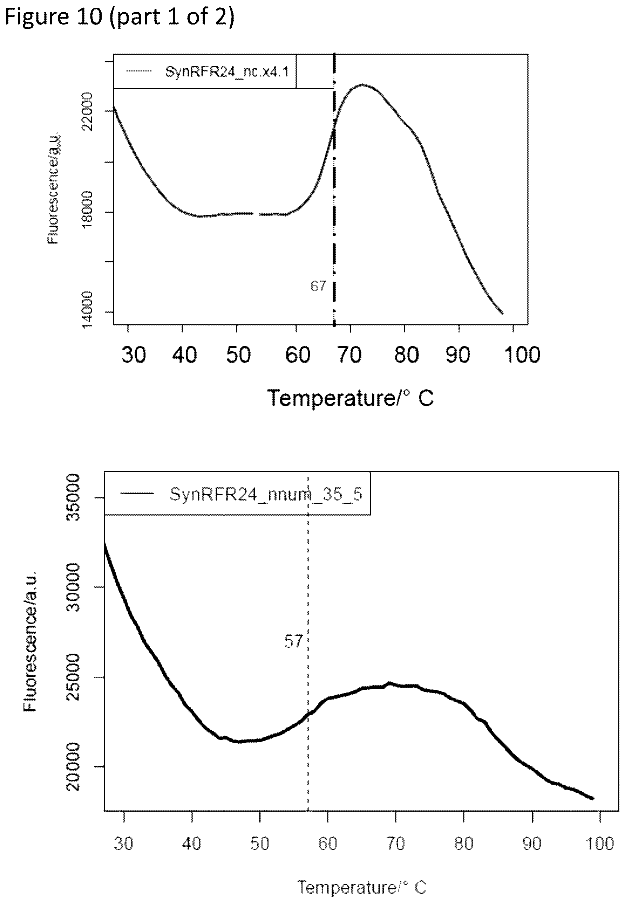

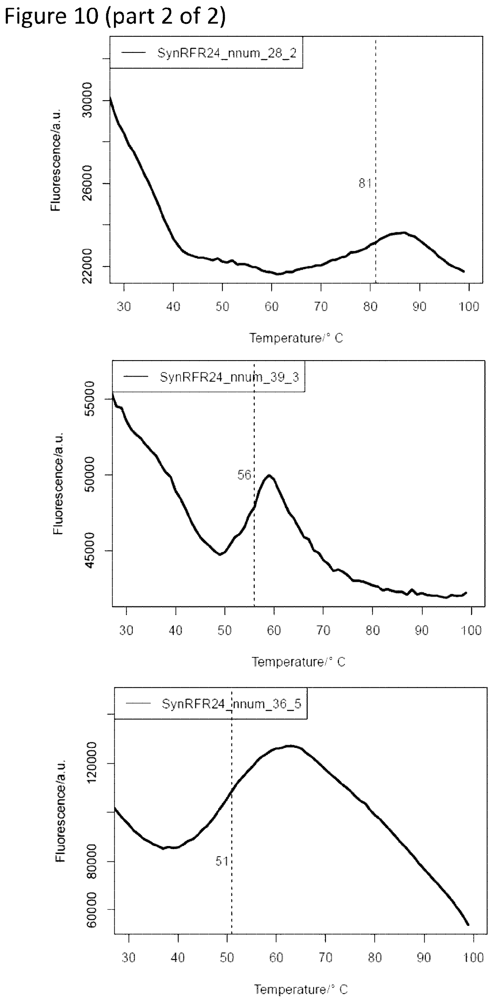

To measure the thermal stability of the SynRFR24.nc.x4.1 protein, melting experiments were performed in the presence of 10X final concentration of SYPRO Orange dye (Life Technologies) and a final protein concentration of approximately 1 mg/ml in 100 mM bicine and 150 mM NaCl buffer titrated to pH 9.0 with NaOH. The samples were heated from degrees Celsius to 99 degrees Celsius at rate of 1 degrees Celsius min-1 in a StepOnePlus Real-Time PCR system (Applied Biosystems) with fluorescence measured at standard excitation/emission wavelengths. The protein melting temperature, Tₘ, was estimated using the R statistical package Rstats by fitting a smoothing cubic spline and finding the maximum of the first derivative. The protein was found to have a melting temperature of around 67 degrees Celsius (Figure 10).

### Size exclusion chromatography-multi-angle light scattering (SEC-MALS)

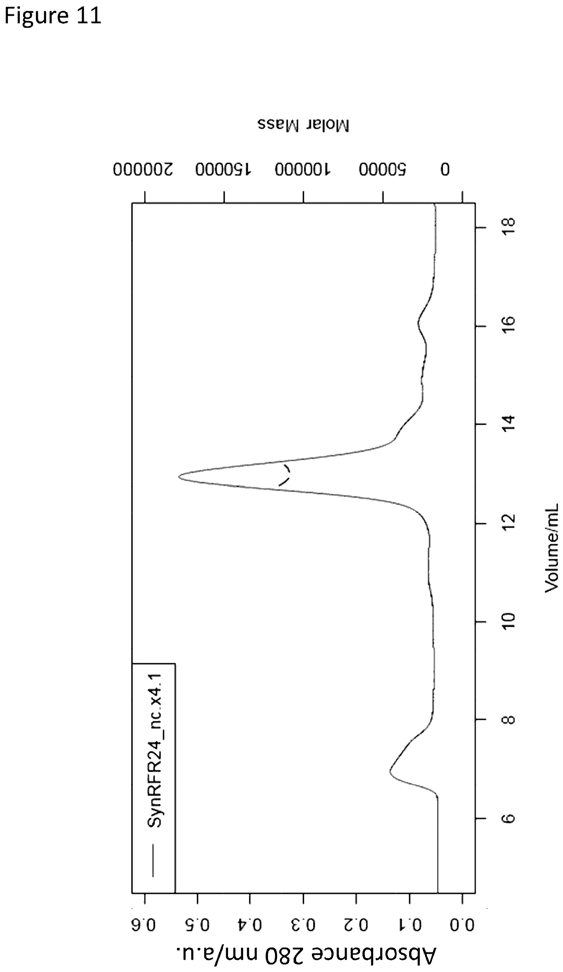

An Agilent 1260 system (Agilent Technologies) with a miniDAWN TREOS (Wyatt Technologies) light scattering detector and an Optilab T-rEX (Wyatt Technologies) refractive index detector were used together with a Superdex 200 Increase 10/300 GL column (GE Healthcare). The column was pre- equilibrated with bicine buffer (100 mM bicine and 150 mM NaCl buffer titrated to pH 9.0 with NaOH). 100 µl of approximately 5 mg/ml SynRFR24.nc.x4.1 protein was injected. Data was analysed using the ASTRA software (Wyatt Technologies) in order to estimate the molar mass of the protein. The MALS estimated molar mass of the protein was consistent with being monomeric (Figure 11).

### Cloning, expression and purification of SynRFR24.nc.x8.1

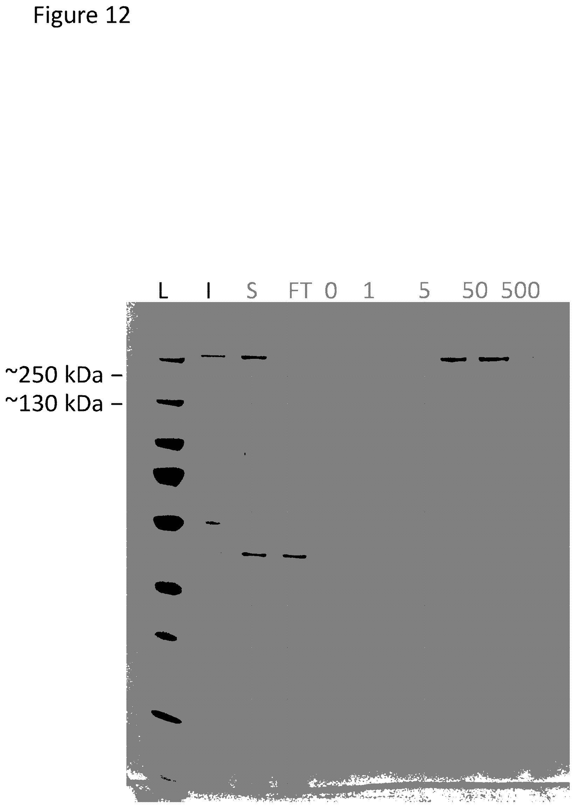

The gene encoding the double length SynRFR24.nc.x8.1 design was created from the synthetic SynRFR24.nc.x4.1 gene using a combination of PCR amplification and Golden Gate assembly. This new plasmid construct was validated using restriction digests and partial sequencing. The new protein was expressed and purified using the same protocol as described for SynRFR24.nc.x4.1 in the previous section (Figure 12).

### Polarised Optical Microscopy - Liquid Crystal Formation

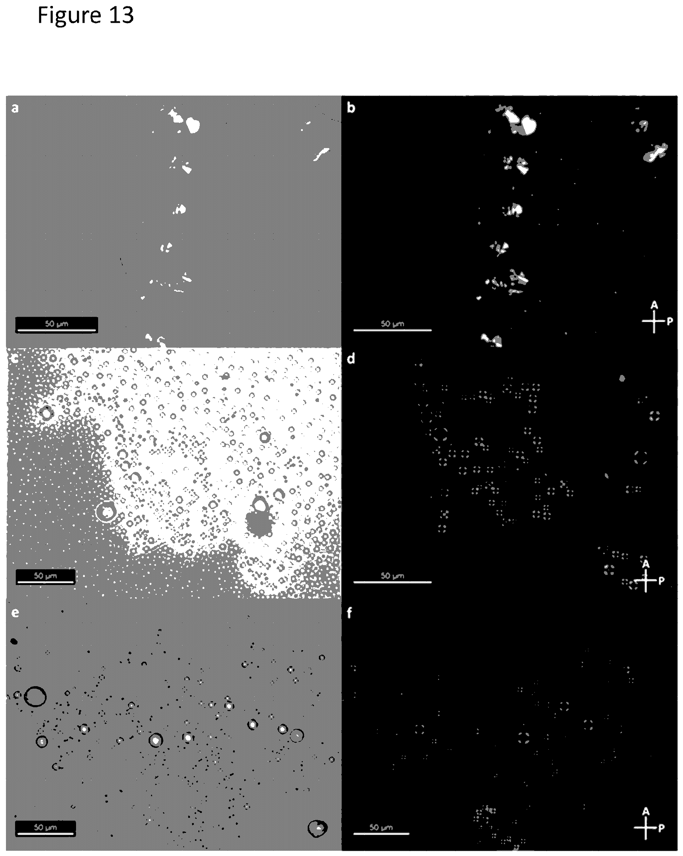

Following the initial polarised optical microscopy of the monomer SynRFr24.new_caps (discussed in the first report) concatenated SynRFR24.new_caps, labelled SynRFR24.nc.x4.1 herein, were also observed for any liquid crystalline behaviour. 1 µl of SynRFR24.nc.x4.1 at several different concentrations were dropped on clean glass slides with a cover slip placed on top. Concentrations 160 mg ml-1, 115 mg ml-1, 70 mg ml-1 and 140 mg ml-1 obtained through an ion exchange purification method (described above) were observed over a few days to identify LC behaviour. The samples were left at room temperature (colder temperatures caused precipitation of the polypeptide). As previously observed with the monomer, the nucleation of a Schlieren textured chiral nematic liquid crystal was evident in several areas (Figure 13). It is likely that the nematic phase observed in Figure 7 d and f are ordered end-to-end SynRFR24.nc.x4.1 units surrounded by disordered species. This observation is also supported by literature, as Nakata *et al* observed similar LCs from 6-20 base pair DNA duplexes. The POM indicated LC behaviour of the concatenated monomers.

### Initial fibre formation

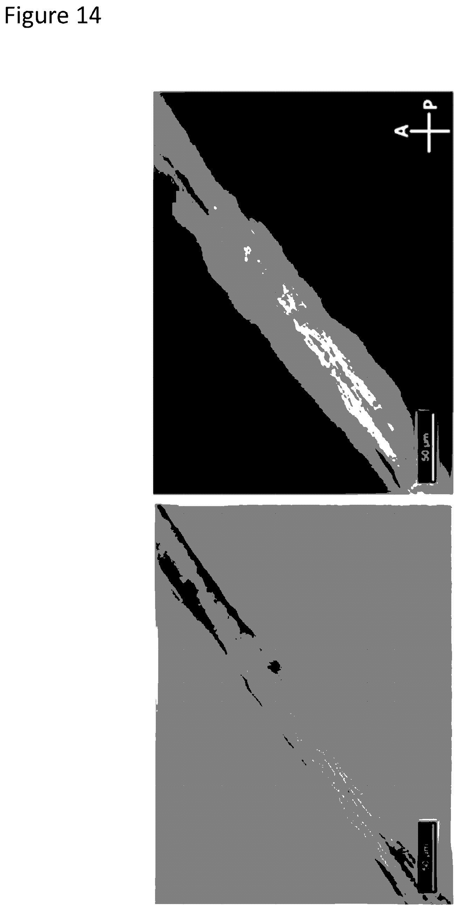

Following the initial coagulation solvent results, ethanol was a positive candidate to coagulate the monomer. With the first concatenated sample, ethanol and acetone were used as coagulating agents. Ethanol yielded extremely promising results, and was therefore chosen as the coagulation agent for the spinning optimisation process. Initially 2 µl of SynRFR24.nc.x4.1 were drawn into a glass petri dish filled with ethanol, using a micropipette. It was possible to manipulate the SynRFR24.nc.x4.1 and stretch the material (when wet) up to 300%. The ethanol was left to dry and short (ca. 3 - 5 cm) fibres remained. It was possible to handle these fibres and perform tensile measurements to ascertain their mechanical properties. Under POM, the fibres are birefringent (Figure 14).

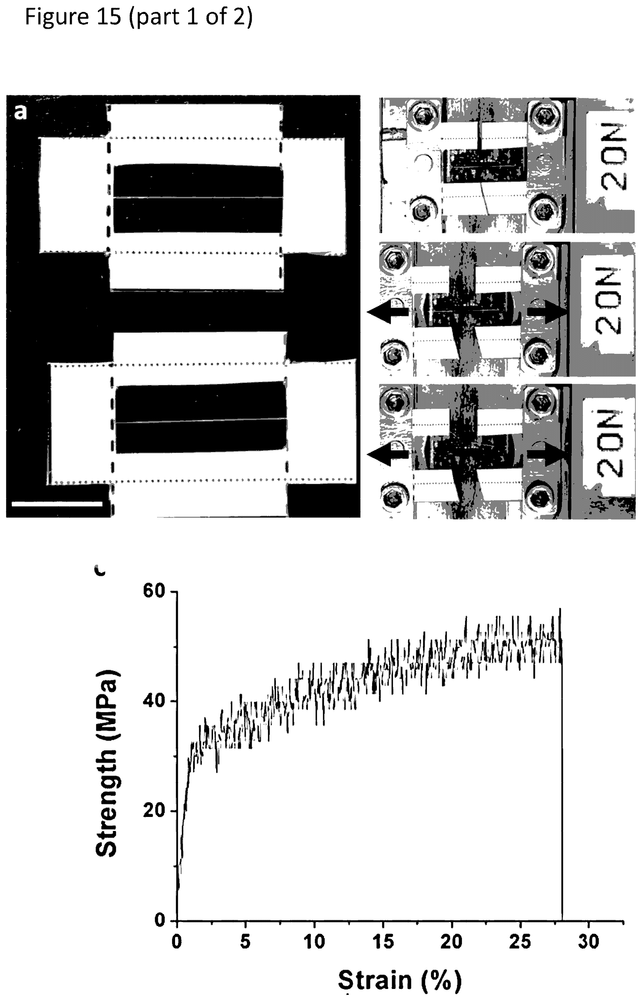

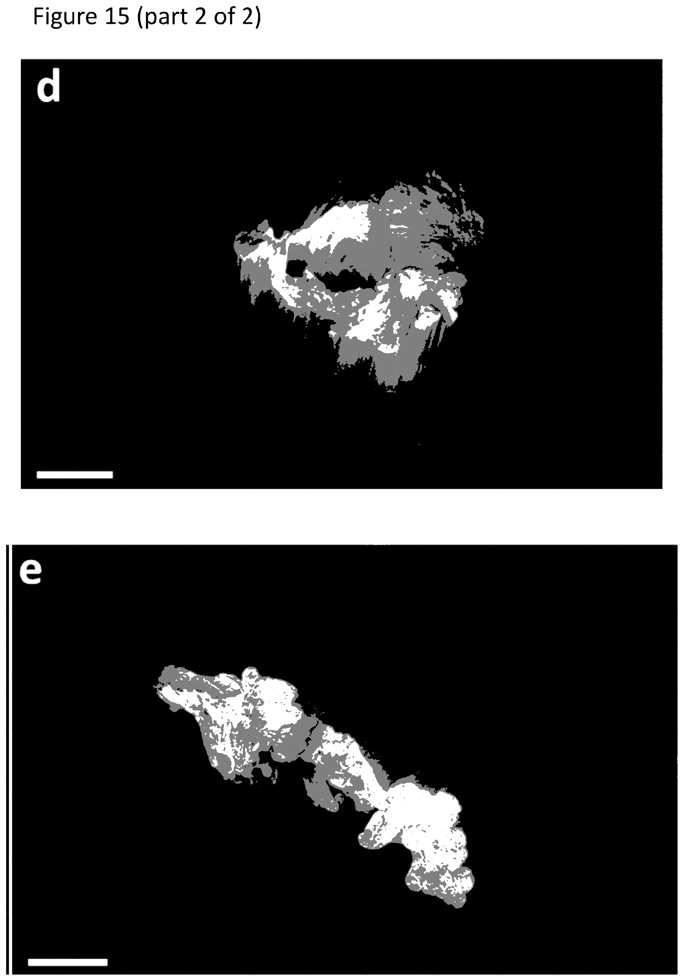

Tensile properties of single composite fibres were determined following the British standard (BS ISO 11566:1996). Fibres were loaded on cardboard holders (Figure 15a-b), with gauge lengths 15 mm ± 0.5 mm, using an epoxy adhesive (50/50 hardener to resin, Araldite Rapid Adhesive, Bostik Findley Ltd., UK). The tensile properties of single fibres were determined using a Linkam TST 350 tester equipped with a 20 N load cell operating at 1 mm min-1 crosshead speed.

On completion of the tensile test, the data was analysed to obtain mechanical performance (Figure 15a-c) and the fibres observed under optical microscopy and SEM to ascertain diameter and microstructure (Figure 15d-e). An average diameter of 40-50 µm is expected as the fibres have not undergone any post-processing. Post-processing with hot-drawing will most definitely reduce the average diameter of the fibres and make them more uniform in general. The stress-strain curve shows an initial sharp increase, this gradient was used to calculate the Young's modulus, which was 2.8 GPa, an encouraging initial value similar to those observed for spider silk. The ultimate engineered strength was 60 MPa with a strain to failure of 28% and toughness of 10 J g⁻¹.

### Wet-spinning fibres

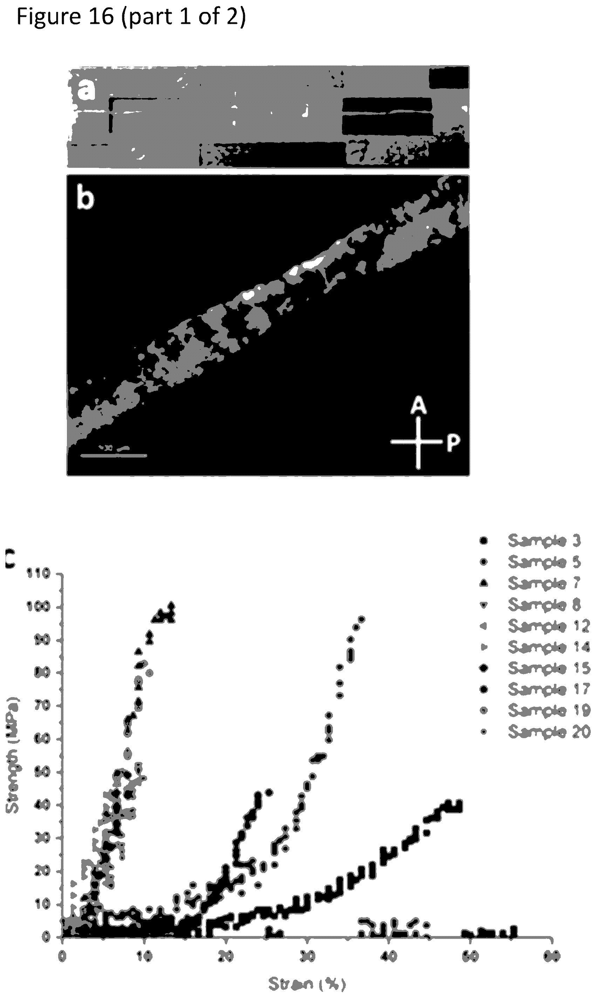

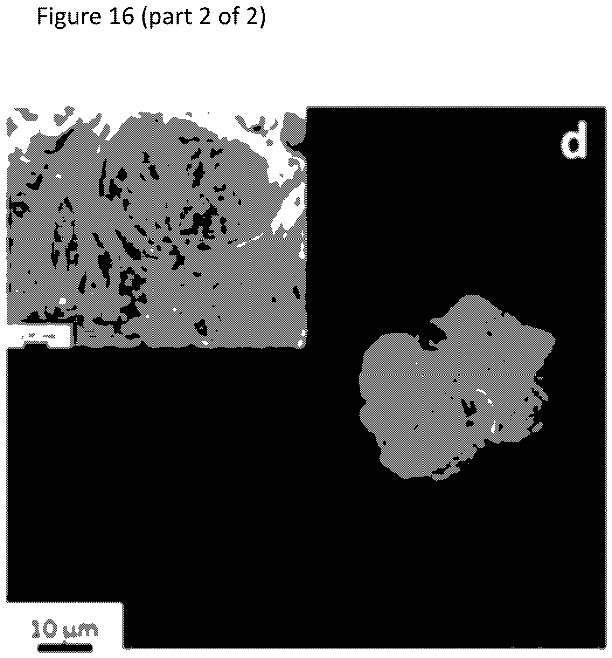

Optimisation of continuous fibre spinning has also been conducted. The prepared SynRFR24.nc.x4.1 of varying concentrations (average volume of each batch 0.5 ml) were drawn into a 1 ml glass syringe and wet-spun into a stream of ethanol through a 24 gauge (0.31 mm inner diameter) needle using a syringe pump (KDS 100, KDS Scientific), which resulted in coagulation and fibre formation (solid). The injection rate of spinning dopes was between 5 and 7 ml h⁻¹ and the rotating speed of the ethanol bath was ca. 4 rpm (spinning parameters have been summarised in Table 2). Each batch of SynRFR24.nc.x4.1 had a different concentration. The higher concentration dope (160 mg ml⁻¹), was diluted to 100 mg ml⁻¹ after a few days as the dope was too viscous to draw into the needle and a lot of air bubbles were trapped which prevented continuous spinning. The ultimate strengths summarised in Table 2 and Figure 16 can be further improved as no strain was applied to the fibre immediately after spinning (i.e. when wet). Stretching the fibre when wet is likely to improve the alignment and therefore strengthen the fibre. Furthermore, analysis of the thermal properties of SynRFR24.nc.x4.1 (by TGA and DSC) indicated thermal stability up to 70 °C and degradation at ca. 250 °C, it is therefore possible to hot-draw the wet-spun fibres at 50-60 °C.

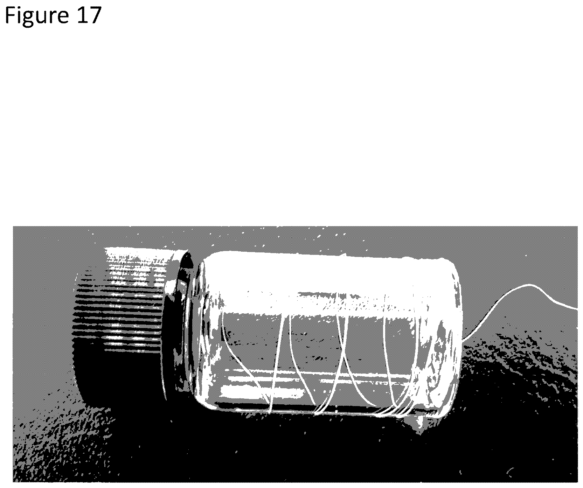

SynRFR24.nc.x4.1, following an ion exchange purification method (discussed above), was also wet- spun at 4 rpm with an injection speed of 6 ml h⁻¹ into ethanol, at a concentration of 140 mg ml⁻¹. It is possible to wind the fibre (Figure 17).

**Table 2: Spinning parameters for wet-spun SynRFR24.nc.x4.1**

| Valid Tests Sample # | Dope concentration | Spinning parameters | Young's Modulus | Ultimate Strength (MPa) | Strain (%) |
|---|---|---|---|---|---|
| 3 | 115 | Hand inject, 3 rpm | 1.7 | 40.6 | 48.6 |
| 5 | 160 day 1 | 6 ml h⁻¹, 5 rpm | 6.2 | 96.3 | 37.0 |
| 8 | 100 day 5 | 7 ml h⁻¹, 3.5 rpm | 2.4 | 52.3 | 9.5 |
| 12 | 100 day 5 | 6 ml h⁻¹, 4 rpm | 5.9 | 51.5 | 9.4 |
| 14 | 100 day 5 | 6 ml h⁻¹, 4 rpm | 2.1 | 48.1 | 6.7 |
| 15 | 100 day 5 | 6 ml h⁻¹, 4 rpm | 7.1 | 49.2 | 8.7 |
| 17 | 100 day 5 | 6.5 ml h⁻¹, 4 rpm | 4.9 | 43.8 | 25.3 |
| 19 | 100 day 5 | 6.5 ml h⁻¹, 4 rpm | 8.0 | 82.86 | 10.3 |
| 20 | 100 day 5 | 6.5 ml h⁻¹, 4 rpm | 6.1 | 50.10 | 9.6 |

### Example 3

### Optimising the expression and purification of SynRFR24.x4.1

In order to obtain extremely pure samples of SynRFR24.x4.1, a three-step purification protocol of the clarified *E. coli* lysate was developed. Previously, it was found that SynRFR24.x4.1 does not bind very tightly to Ni-NTA columns, eluting at around 50 mM imidazole concentration. However, very little protein was found in the flow-through, indicating that binding is occurring and this method still has value as an initial purification step. In order to purify the protein further, anion exchange and gel filtration steps were added. KRX cells (Promega) expressing SynRFR24.x4.1 under an inducible T7 promoter were grown, harvested, lysed and clarified as described previously, but the resuspension buffer was slightly altered to decrease the salt concentration (50 mM bicine, 150 mM NaCl, pH 9). The lysate was loaded on to a Ni-NTA column as before, then immediately eluted (50 mM bicine, 150 mM NaCl, 500 mM imidazole, pH 9) without any intermediate wash steps. The eluate was diluted two-fold (in 50 mM bicine, 0 mM NaCl, pH 9 buffer) to reduce the salt concentration. A HiTrap Q HP column (2 x 5ml) was equilibrated with buffer A (50 mM bicine, pH 9). The eluate from the Ni-NTA purification was applied to the column at 5 ml/min. The column was then washed with 5% buffer B (50 mM bicine, 1 M NaCl, pH 9). Protein was found to elute at around 300 mM NaCl concentration. Fractions containing the protein were pooled and concentrated to around 5 ml. A S200 gel filtration column was equilibrated with two column volumes of 20 mM bicine, 150 mM NaCl, pH 9 buffer. The pooled fractions from the anion exchange step were loaded on to the column and the appropriate fractions collected. The fractions were pooled and concentrated to around 160 mg/ml.

### Polarised Optical Microscopy - Phase diagram

A phase diagram of the concatenated monomers with concentration has been developed to identify the nematic region, this has been conducted as the goal is to wet-spin the LC SynRFR24. Several concentrations have been investigated and the purification methods improved (as discussed above).

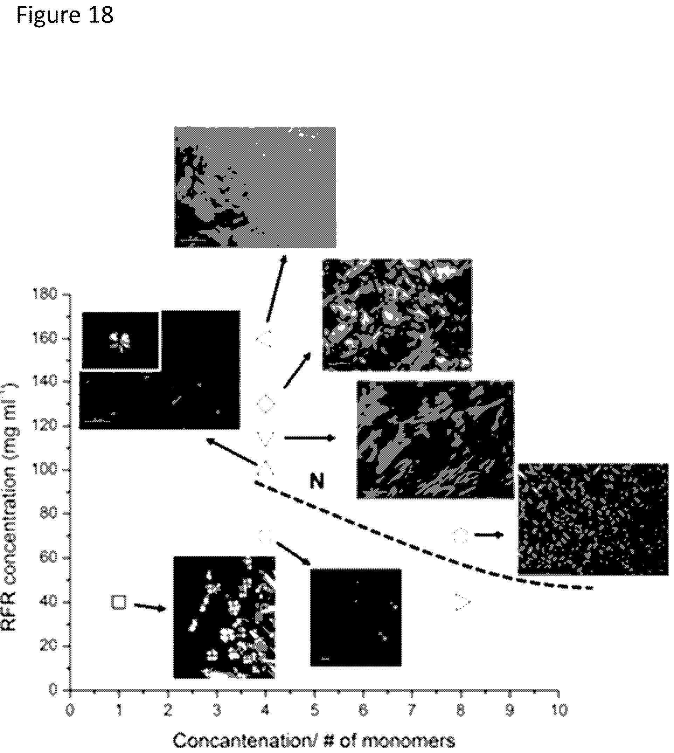

1 µl of the concatenated SynRFR24 at several different concentrations were dropped on clean glass slides with a cover slip placed on top. Three edges of the cover slips were sealed with grease with only one edge exposed to control the evaporation rate. The samples were left at room temperature (colder temperatures caused precipitation of the polypeptide). As previously observed with the monomer, the nucleation of a Schlieren textured chiral nematic liquid crystal was evident in several areas (Figure 1) but after a few days a fully textured (biphasic) nematic liquid crystal had formed. It is likely that the nematic phase observed in Figure 18 are ordered end-to-end SynRFR24.nc.x4.1 units surrounded by disordered species (i.e. biphasic regime).

### Wet-spinning fibres

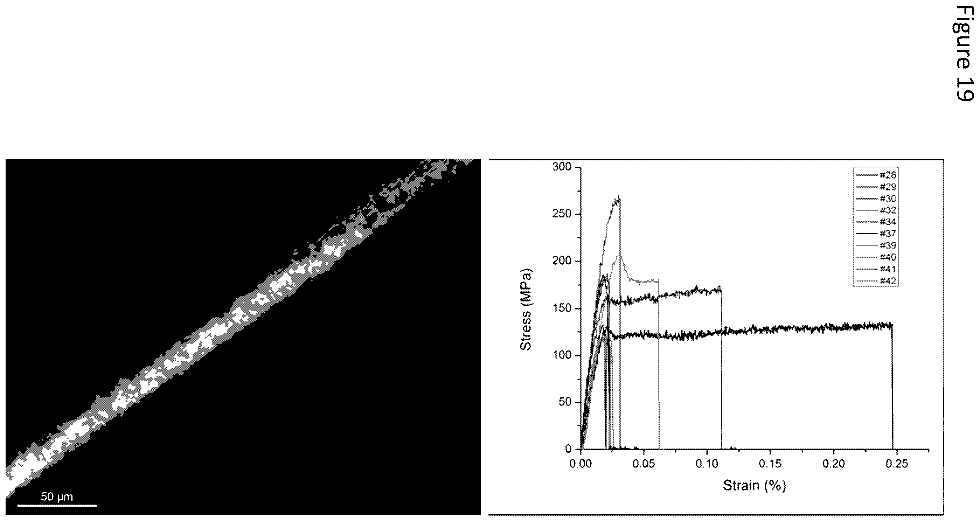

Optimisation of continuous fibre spinning has also been conducted. The prepared SynRFR24.nc.x4.1 of varying concentrations (average volume of each batch 0.5 ml) were drawn into a 1 ml glass syringe and wet-spun into a stream of ethanol through a 24 gauge (0.31 mm inner diameter) needle using a syringe pump (KDS 100, KDS Scientific), which resulted in coagulation and fibre formation (solid). The injection rate of spinning dope was 7 ml h⁻¹ and the rotating speed of the ethanol bath was ca. 4 rpm. The ultimate strengths summarised in Table 3 and Figure 19 were stretched immediately after spinning (wet-drawn) and then left to dry under tension. Stretching the fibre when wet improved the mechanical properties of the fibre resulting in an average Young's modulus 9.6±1.6 GPa and ultimate strength of 160.8±47.2 MPa. The average strain was ca. 3 %.

**Table 3: Mechanical strength of wet-spun SynRFR24.nc.x4.1 at concentration of 100 mg ml-1and spinning parameters; 7 ml h-1, 4.5 rpm with a 24g needle.**

| Valid Tests Sample # | Young's Modulus(GPa) | Ultimate Strength (MPa) | Strain (%) |
|---|---|---|---|
| 28 | 7.2 | 132.4 | 24.8 |
| 29 | 8.7 | 120.7 | 2.5 |
| 30 | 8.8 | 132.1 | 2.0 |
| 32 | 10.1 | 116.0 | 2.6 |
| 34 | 7.6 | 119.3 | 2.0 |
| 37 | 11.6 | 185.7 | 2.3 |
| 39 | 11.4 | 267.4 | 3.1 |
| 40 | 9.1 | 167.8 | 11.1 |
| 41 | 11.9 | 186.9 | 2.2 |
| 42 | 9.3 | 180.0 | 6.2 |

### Example 4

### SEC-MALS

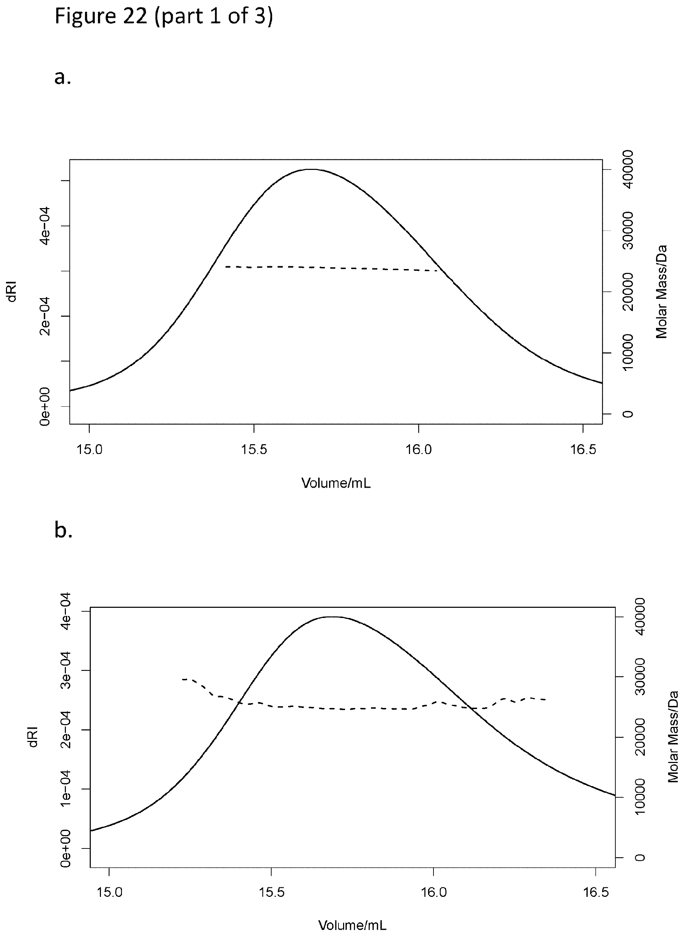

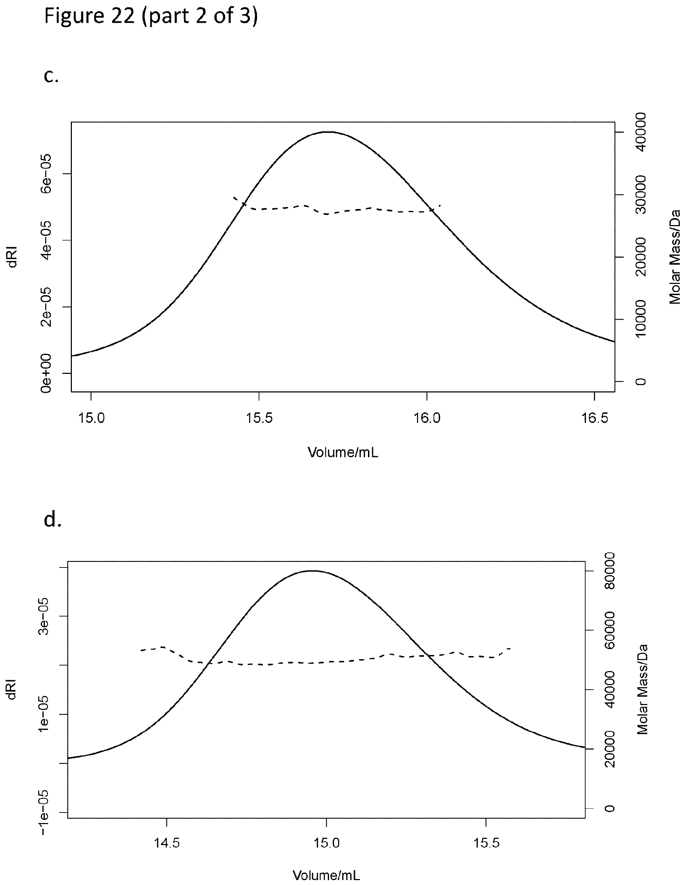

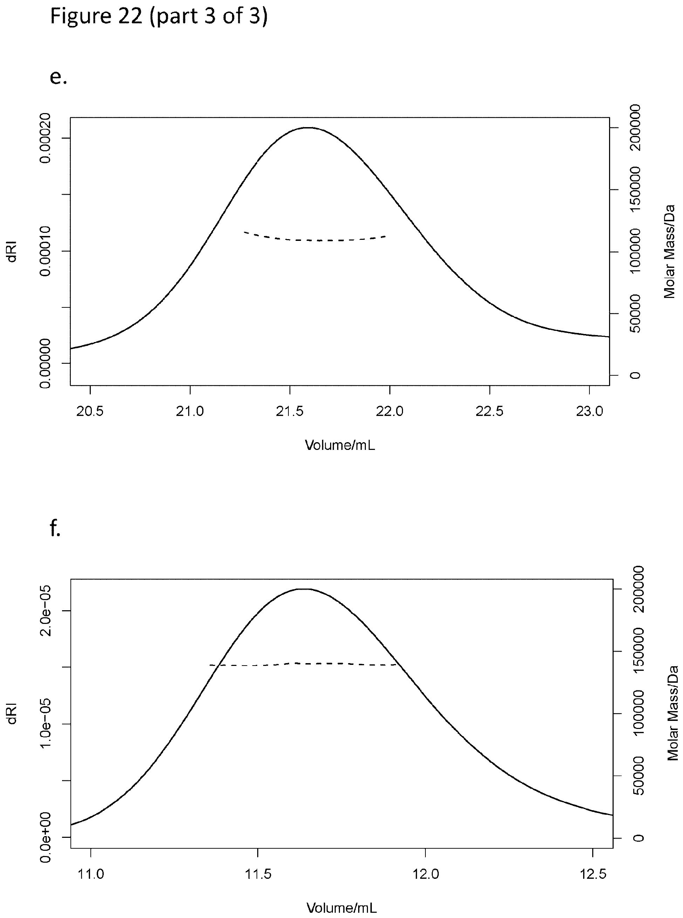

An Agilent 1260 system (Agilent Technologies) with a miniDAWN TREOS (Wyatt Technologies) light scattering detector and an Optilab T-rEX (Wyatt Technologies) refractive index detector were used together with a Superdex 200 Increase 10/300 GL column (GE Healthcare) or a Superose 6 10/300 column (GE Healthcare). The column was pre-equilibrated with bicine buffer (20 mM bicine and 150 mM NaCl buffer titrated to pH 9.0 with NaOH). 100 µl of approximately 5-15 mg/ml protein was injected for each of the different designs. Data was analysed using the ASTRA software (Wyatt Technologies) in order to estimate the molar mass of the protein and all measured designs (SynRFR24.nc.x6, SynRFR24.nc.x4, SynRFR24.nc.x2, SynRFR24_nnum35, SynRFR24_nnum36, SynRFR24_nnum40) were confirmed to be monomeric (Figure 22).

### 1D proton NMR

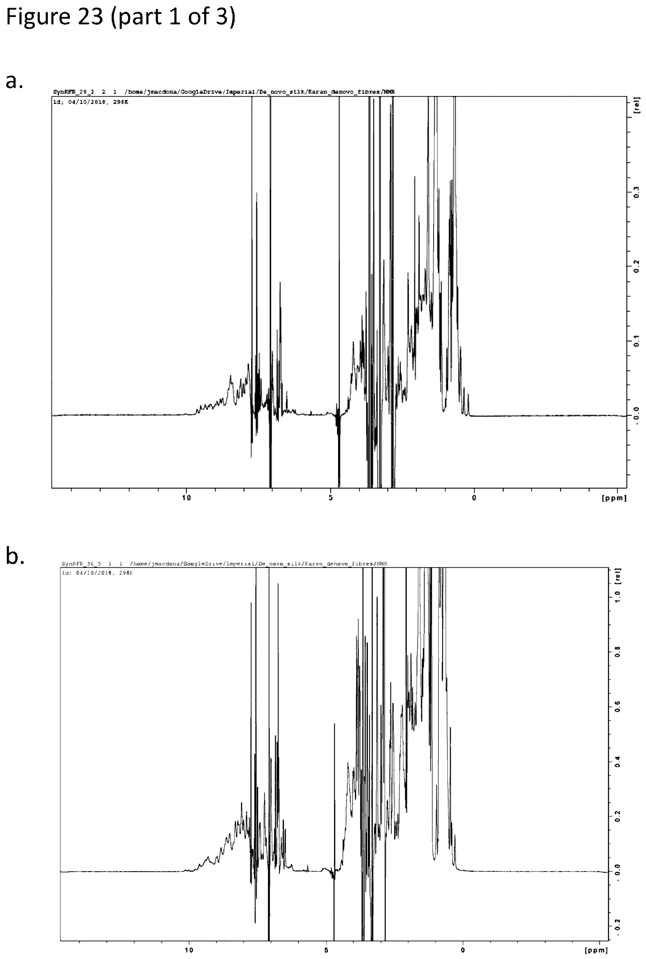

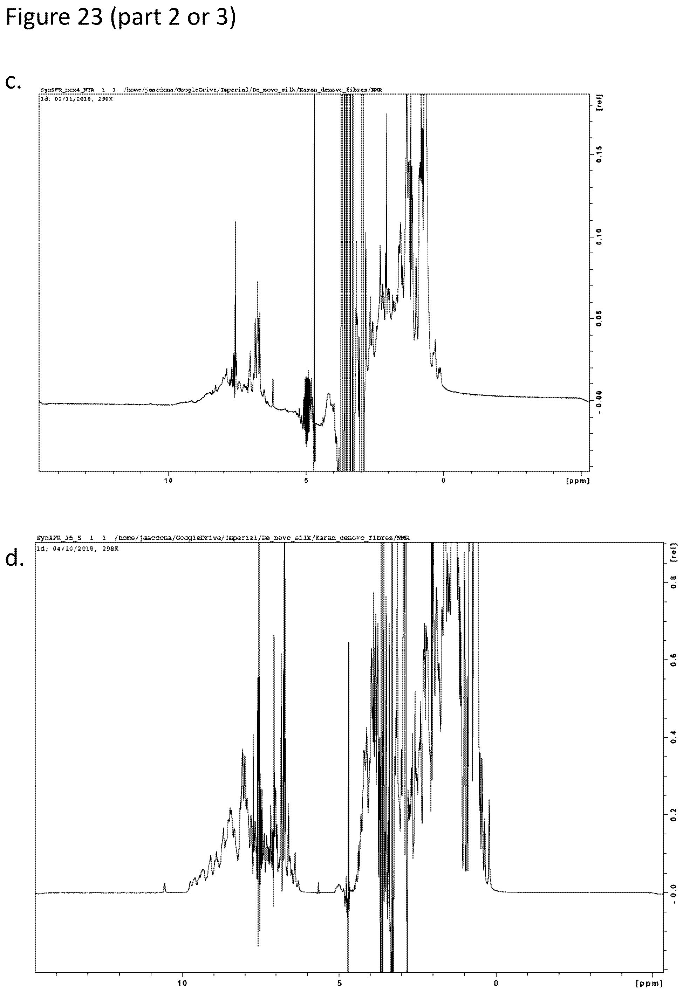

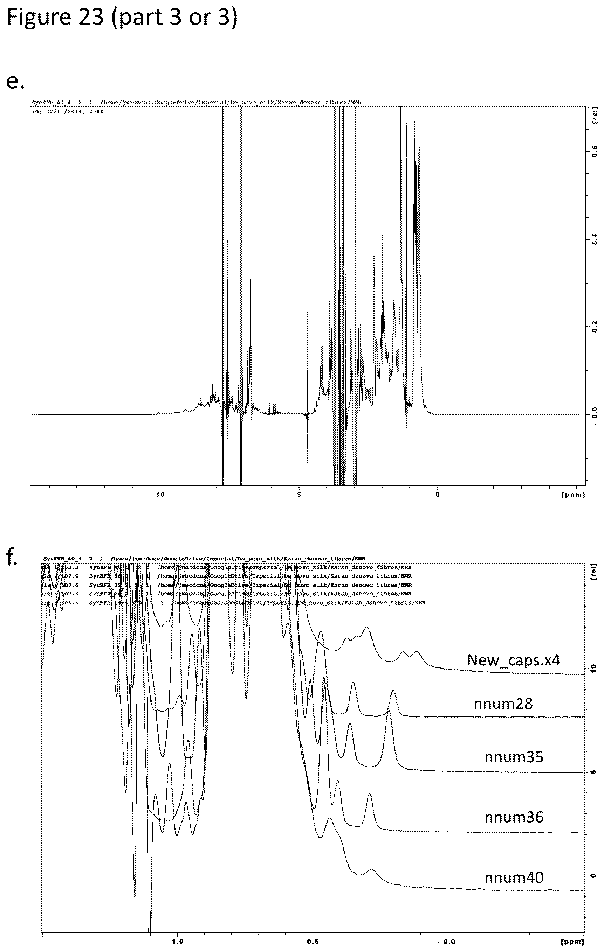

Protein samples in bicine buffer were concentrated to >300 µM and D₂O was added to the final concentration of around 10% v/v (for instrumental lock). 500 µL samples were loaded in glass NMR tubes (Norell, part no. 507-HP-7). The NMR measurements were performed at 298 K on an Avance III 600 MHz spectrometer (Bruker) and processed using TopSpin (Bruker; Figure 23 a.-e.). All proteins measured (SynRFR24.nc.x4, SynRFR24_nnum28, SynRFR24_nnum35, SynRFR24_nnum36, SynRFR24_nnum40) showed peaks in the 0-1.5 ppm region that are characteristic of well packed side chain methyl groups, indicative of folded proteins (Figure 23 f.).

### Circular Dichroism

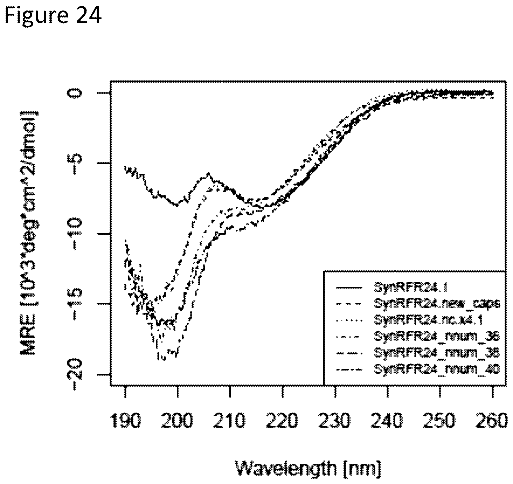

0.1-0.2 mg/ml of purified protein was exchanged into 20 mM borate, 150 mM NaF, pH 9.0 buffer using 0.5 mL 7K MWCO Zeba Spin Desalting Columns (Thermo Scientific). Spectra were collected in triplicate using a Chirascan (Applied Photophysics) from 190 to 260 nm wavelengths with 0.5 nm intervals, 1 nm band width, and a 1 second time per point. Measurements were taken using a quartz cuvette (QS-284) with a 0.1 cm path length. Spectra were collected for the SynRFR24.1, SynRFR24.new_caps, SynRFR24.new_caps.x4.1, SynRFR24_neg_num_36_0005, SynRFR24_neg_num_38_0005, SynRFR24_neg_num_40_0004 (Figure 24).

Secondary structure content was estimated using the K2D3 server http://cbdm-01-zdv.uni-mainz.de/~andrade/k2d3/ (https://onlinelibrary.wiley.com/doi/full/10.1002/prot. 23188) with the following values:
SynRFR24.1: 4.6% alpha helix, 34.7% beta strand
SynRFR24.new_caps: 4.5% alpha helix, 35.9% beta strand
SynRFR24.new_caps.x4: 5.8% alpha helix, 20.6% beta strand
SynRFR24_nnum36: 4.6% alpha helix, 34.7% beta strand
SynRFR24_nnum38: 4.9% alpha helix, 34.0% beta strand
SynRFR24_nnum40: 4.9% alpha helix, 34.0% beta strand

The distance to the closest spectrum in the K2D3 database was large and so the secondary structure decompositions were flagged as potentially having high error. It is unclear why the SynRFR24.new_caps and SynRFR24.new_caps.x4 estimates were so different given the spectra overlay almost exactly. All the variants show differences from SynRFR24.1 in the 190-210 nm region, possible due to less structure in the C-terminal in the capping regions which form a dimeric interface in SynRFR24.1 but not in any of the variants (which are all monomeric by design).

### Thermofluor assay

To measure the thermal stability of each of the SynRFR proteins, melting experiments were performed in the presence of 10X final concentration of SYPRO Orange dye (Life Technologies) and a final protein concentration of approximately 0.75 mg/ml in 100 mM bicine and 150 mM NaCl buffer titrated to pH 9.0 with NaOH. The samples were heated from 25 deg C to 99 deg C at rate of 1 deg C/minute in a StepOnePlus Real-Time PCR system (Applied Biosystems) with fluorescence measured at standard excitation/emission wavelengths. The protein melting temperature, Tₘ, was estimated using the R statistical package (Rstats) by fitting a smoothing cubic spline and finding the maximum of the first derivative (Figure 10).

### Example 5

### Liquid crystal (polarised optical microscopy, SAXS/WAXS)

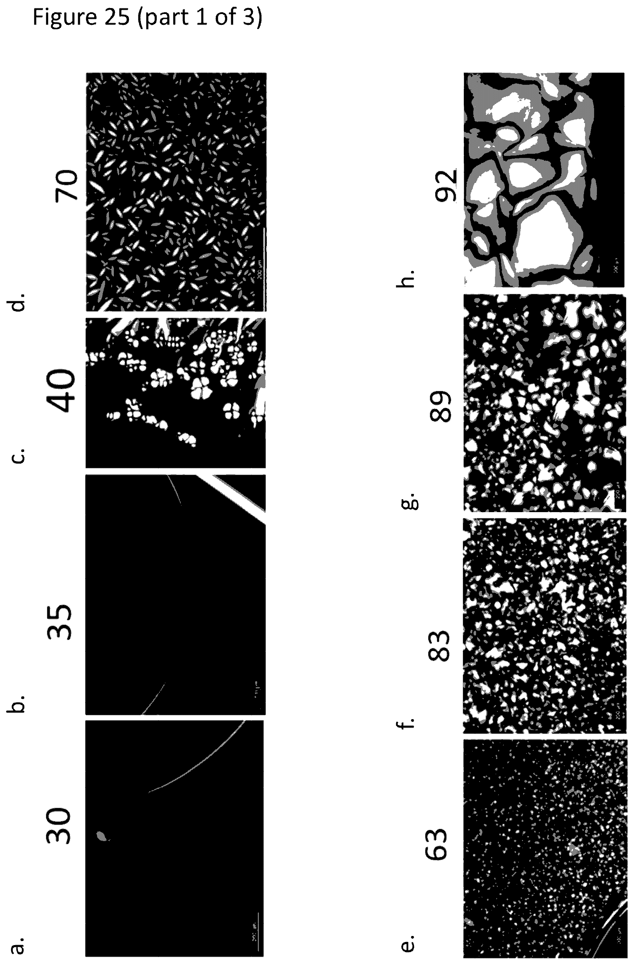

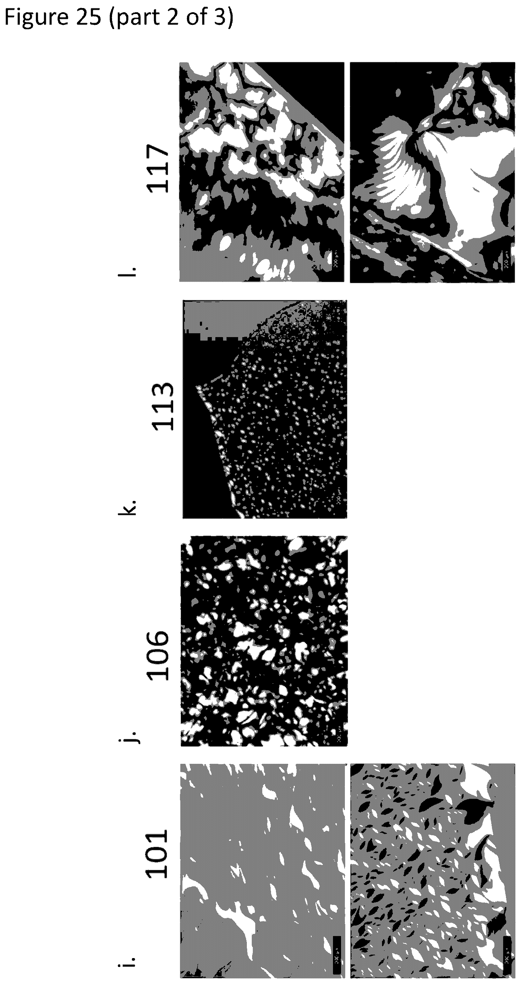

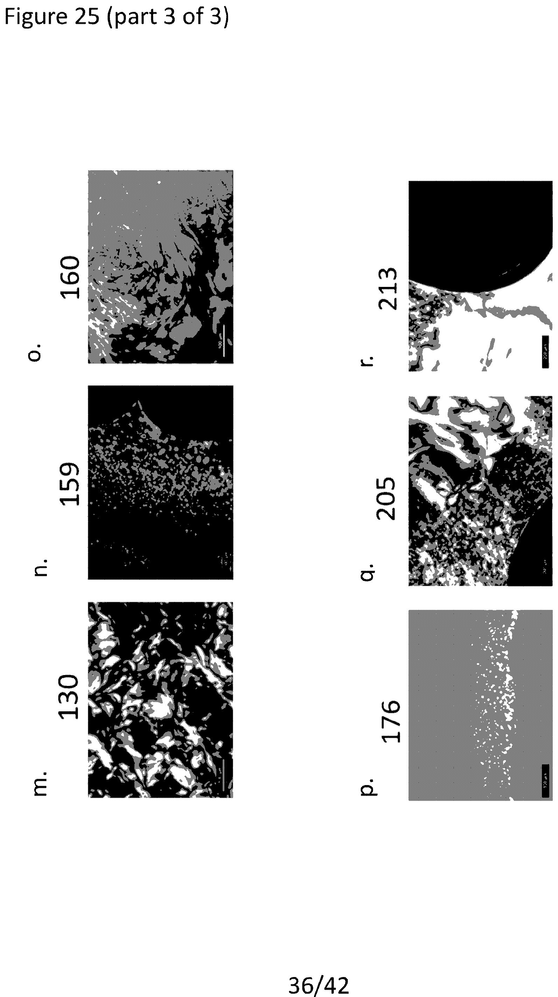

The morphology of the fibers was investigated by polarized optical microscope (DM2500P, Leica Microsystems Ltd., GB) fitted with a DFC295 camera (Leica Application Suite v4.0.0, ∞/1.1 HI PLAN 40x/0.50). Concentrated protein samples were set up on glass slides or in 0.2 mm internal depth/2 mm wide flat glass capillaries (Part 3520, VitroCom; Figure 25) and sealed with wax.

**Table 4: Number of solenoid domains versus protein concentration and sublocation in Figure 25.**

| Number of solenoid domains | Protein concentration (mg ml⁻¹) | Figure 25 subsection (a.-r.) |
|---|---|---|
| 1 (SynRFR24.nc.x1) | 40 | C. |
| 2 (SynRFR24.nc.x2) | 92 | h. |
| | 117 | l. |
| | 205 | q. |
| | 213 | r. |
| | 30 | a. |
| | 35 | b. |
| | 63 | e. |
| | 83 | f. |
| | 89 | g. |
| 4 (SynRFR24.nc.x4) | 101 | i. |
| | 106 | j. |
| | 113 | k. |
| | 130 | m. |
| | 159 | n. |
| | 160 | o. |
| | 176 | p. |
| 8 (SynRFR24.nc.x8) | 70 | d. |

### Example 6

### Wet spinning of fibres

Fibres were spun through injection into a rotating bath of ethanol, used as coagulant (at room temperature). In a typical experiment, injection was performed at 7 ml h⁻¹ (SynRFR24.nc.x4 concentration at 100 mg ml⁻¹) through a 24-gauge cannula (0.5588 mm inner diameter, stainless-steel, Central Surgical Co. Ltd., GB) using a syringe pump (KDS100, KD scientific, US), while the bath rotated at 4.5 rpm at the point of injection to provide extensional flow during fibre coagulation. The fibres were removed from the bath within 1 minute of coagulation and drawn before evaporation to improve alignment and strength. The fibres were stored at room temperature at ambient temperature and pressure.

### Mechanical testing

Tensile properties of single fibres were determined following the British standard (BS ISO 11566:1996). Fibres were loaded onto cardboard struts, with gauge lengths 15 mm +/- 0.5 mm, using an epoxy adhesive (50/50 hardener to resin, Araldite Rapid Adhesive, Bostik Fingley Ltd., UK). The tensile properties of single fibres were determined using a Linkam TST 350 tester equipped with a 20 N load cell operating at 1 mm min⁻¹ crosshead speed.

### Fibre WAXS

X-ray diffraction experiments were carried out using an in-house x-ray source (Rigaku Micromax 007HF-M high-flux generator with Osmic VariMax optics) and images were collected on a Rigaku Saturn 944+ CCD detector. Image processing was carried out using the Python libraries FablO (http://journals.iucr.org/j/issues/2013/02/00/kk5124/) and PyFAI (https://journals.iucr.org/j/issues/2015/02/00/fv5028/). Single fibres were supported on cardboard struts (see **Mechanical testing** section) and mounted on the goniometer and aligned in the X-ray beam.

Ten images were collected with the fibre in the X-ray beam and ten background images with no sample present were also collected and averaged to reduce noise. All images were taken with a 120 second exposure time with a 5 degrees oscillation angle and with a 110 cm distance between the sample to the detector. The mean of the ten background images was subtracted from the mean of the ten fibre diffraction images to remove background artefacts (Figure 26 a.) 1D radial intensity profiles for meridional and equatorial directions were produced by azimuthally integrating +/- 10 degrees (Figure 26 b.) with the vertical lines indicating q=1.32 Å⁻¹ (d=4.75 Å), q=0.255 Å⁻¹ (d=24.6 Å), q=2*0.255 Å⁻¹, q=3*0.255 Å⁻¹. Where 4.75 Å matches the helical pitch of the beta-solenoid, and 24.6 Å is close to the diameter of the beta solenoid domains. These results show a high degree of alignment, with beta strands aligned perpendicular to axis of the fibre as would be expected if the beta solenoid domains were aligned parallel to the axis of the fibre.

### FTIR

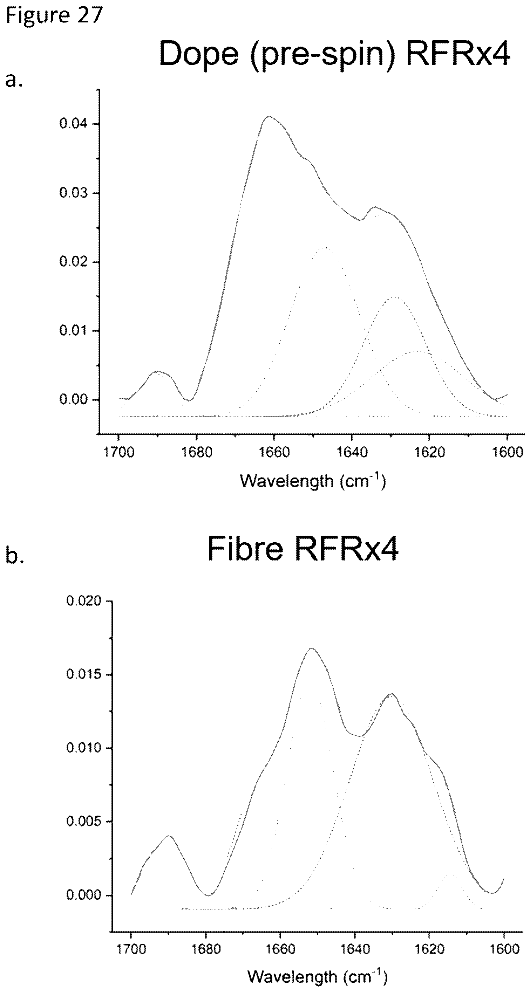

Infra-red spectra of both the liquid crystalline spinning dope and the spun fibre were recorded on a Perkin Elmer Frontier FTIR instrument with a diamond ATR head with a resolution of 4 cm⁻¹ and 10 acquisitions. The spectra were taken to compare the secondary structure of the solenoid before (figure 27 a.) and after (Figure 27 b.) spinning the fibers to ensure no structural damage to the solenoid backbone.

### Polarized Raman Spectroscopy

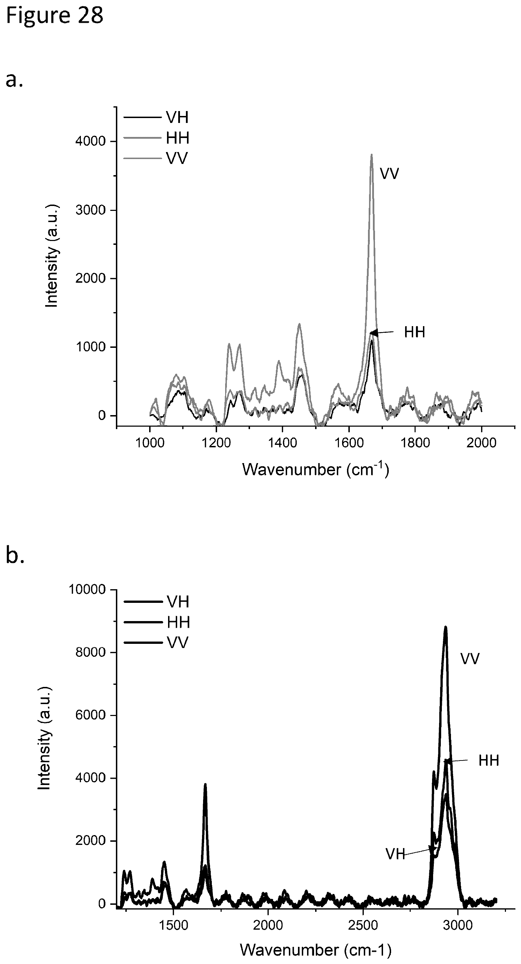

Polarised Raman spectroscopy was performed to identify the different amide regions of the fibre (which support the FTIR secondary structure findings) and polarising the monochromatic light can elucidate structural motifs of the fibre and potentially the orientation of the solenoids in the fibre. Raman data obtained with three different polarization configurations, IVV, IVH, and IHH, where V and H refer to orientation parallel and perpendicular, respectively (Figure 28).

### Example 7

### Test expressions of new concatemer variants

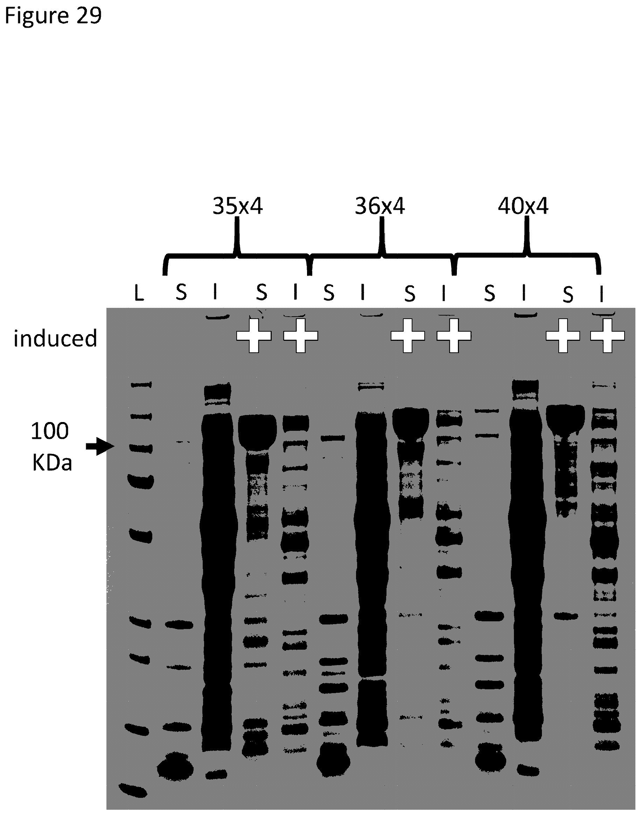

Codon optimised genes encoding the proteins SynRFR24.nnum35.x4, SynRFR24.nnum36.x4, SynRFR24.nnum40.x4 were synthesised by GeneArt with flanking T7 promoter and T7 terminator sequences in the vector backbone pMK-RQ (kanR). These plasmids were transformed into the KRX *E. coli* strain. 100 uL of 5 ml LB overnight cultures (all media were supplemented with appropriate antibiotics) were used to inoculate 5 mL of fresh LB medium. This fresh culture was grown for 3 hours at 37 degrees C before being induced with 0.1% filter sterilised rhamnose and grown overnight at 25 degrees C. Non-induced cultures were also grown in the same way for comparison. Cells were harvested in the morning and resuspended in 100 uL of lysis buffer (100 mM bicine and 150 mM NaCl buffer titrated to pH 9.0 with NaOH) then sonicated and clarified by centrifugation at 20,000 × g for 10 min in a benchtop mini-centrifuge. SDS-PAGE gels of the soluble and insoluble fractions showed significant protein over-expression bands for all proteins in the soluble fraction. See Figure 29.

### Purification of SynRFR24.nnum35.x4 variant

Larger scale (3 litres of TB medium) expression and purification of SynRFR24.nnum35.x4 was carried out in an identical fashion as described for SynRFR24.x4.1 (three-step purification: Ni-NTA, anion exchange, gel filtration as described in the section: ***Optimising the expression and purification of SynRFR24.x4.1*** in Example 3.

SynRFR24.nnum35.x4 was purified to high purity and quantity (~200 mg); see Figure 30. where L is the PageRuler plus ladder and the other lane is the concentrated soluble protein. The SynRFR24.nnum35.x4 sample was found to be highly soluble and could be concentrated to >200 mg/ml.

### Summary of data to date

Data shows that it is possible to design a wide range of different SynRFR protein domains that show similar CD spectra (Figure 24), appear to be folded in 1D proton NMR (Figure 23), are monomeric (Figures 11 and 22), and exhibit unfolding transitions (Figure 10) at high temperatures (see summary in Figure 31). This consistency is despite the whole sequence being completely redesigned (i.e. NCAP, UNITSn, CCAP) with reasonably low sequence identity to each other. For example, excluding the TAG sequence, rfr_ref_minprep_ren_neg_num_40_0004 [SEQ ID NOs: 104 + 28 + 125 + 146] has a sequence identity of around 53% to SynRFR24.new_caps.1 [SEQ ID NO: 6], and rfr_ref_minprep_ren_neg_num_28_0002 [SEQ ID NOs: 92 + 16 + 113 + 134] has a sequence identity of 64% to rfr_ref_minprep_ren_neg_num_40_0004 [SEQ ID NOs: 104 + 28 + 125 + 146]. Also It has also been shown that these sequences can be concatenated to 100 kDa, highly expressed in E. coli, and can be concentrated to high concentration (>200 mg/ml). It is considered to be unusual to be able to carry out such extensive protein redesigns and still reliably obtain folded and well-behaved proteins.

The SynRFR24.nc.x.4.1 concatemer has been consistently spun into strong fibres, and the concentration range for liquid crystalline dopes has been investigated thoroughly. The fibres are being spun from dope solutions at concentrations at which liquid crystalline mesophases are formed, and the polymers are strongly aligned in the fibre (shown using fibre WAXS (Figure 26). These two observations are directly related and also related to the strength of the fibres.

## Claims

1. A concatemer of beta solenoid domains, wherein the concatemer comprises at least two beta solenoid domains,
wherein the at least two beta solenoid domains are linked by a linker, and wherein each of the at least two beta solenoid domains comprise an amino acid sequence that comprises at least 4 units of a consensus motif, and
wherein the concatemer has a molecular weight of at least 30 kDa,
wherein each unit within one of the beta solenoid domains conforms to the following consensus sequence:
A X₁ L/F X₂ X₃ [SEQ ID NO: 147]
wherein X₁ is A, C, D, E, I, K, L, M, N, R, S, T or V
X₂ is A, C, D, E, F, G, H, I, K, N, Q, R, S, T, V, W, or Y
X₃ is A, C, D, E, G, H, I, K, N, Q, R, S ,Y.

2. The concatemer of claim 1 wherein:
at least one of the at least two beta solenoid domains comprises at least 5 units of the consensus motif, optionally at least 6 units, optionally at least 7 units, optionally at least 8 units, optionally at least 9 units, optionally at least 10 units, optionally at least 11 units, optionally at least 12 units, optionally at least 13 units, optionally at least 14 units, optionally at least 15 units, optionally at least 16 units, optionally at least 17 units, optionally at least 18 units, optionally at least 19 units, optionally at least 20 units, optionally at least 21 units, optionally at least 24 units, optionally at least 25 units, optionally at least 26 units, optionally at least 27 units, optionally at least 28 units, optionally at least 29 units, optionally at least 30 units, optionally at least 40 units, optionally at least 50 units, optionally at least 60 units, optionally at least 70 units, optionally at least 80 units, optionally at least 90 units, optionally at least 100 units, optionally at least 200 units of the consensus motif;
at least two of the beta solenoid domains comprise at least 5 units of the consensus motif, optionally at least 6 units, optionally at least 7 units, optionally at least 8 units, optionally at least 9 units, optionally at least 10 units, optionally at least 11 units, optionally at least 12 units, optionally at least 13 units, optionally at least 14 units, optionally at least 15 units, optionally at least 16 units, optionally at least 17 units, optionally at least 18 units, optionally at least 19 units, optionally at least 20 units, optionally at least 21 units, optionally at least 24 units, optionally at least 25 units, optionally at least 26 units, optionally at least 27 units, optionally at least 28 units, optionally at least 29 units, optionally at least 30 units, optionally at least 40 units, optionally at least 50 units, optionally at least 60 units, optionally at least 70 units, optionally at least 80 units, optionally at least 90 units, optionally at least 100 units, optionally at least 200 units of the consensus motif;
all of the beta solenoid domains comprise at least 5 units of the consensus motif, optionally at least 6 units, optionally at least 7 units, optionally at least 8 units, optionally at least 9 units, optionally at least 10 units, optionally at least 11 units, optionally at least 12 units, optionally at least 13 units, optionally at least 14 units, optionally at least 15 units, optionally at least 16 units, optionally at least 17 units, optionally at least 18 units, optionally at least 19 units, optionally at least 20 units, optionally at least 21 units, optionally at least 24 units, optionally at least 25 units, optionally at least 26 units, optionally at least 27 units, optionally at least 28 units, optionally at least 29 units, optionally at least 30 units, optionally at least 40 units, optionally at least 50 units, optionally at least 60 units, optionally at least 70 units, optionally at least 80 units, optionally at least 90 units, optionally at least 100 units, optionally at least 200 units of the consensus motif;
optionally wherein the units of the consensus motif are arranged consecutively.

3. The concatemer according to any of claims 1 or 2 wherein:
the beta solenoid domains are capable of forming a lyotropic liquid crystal mesophase when expressed as monomers; and/or
the concatemer is capable of forming a lyotropic liquid crystal mesophase.

4. The concatemer according to any of claims 1-3 wherein the consensus motif generates a beta solenoid domain with a square cross-section.

5. The concatemer according to any of claims 1-4 wherein the at least two beta solenoid domains are rod-shaped,
optionally wherein one of the at least two beta solenoid domains has a longitudinal axis and a transverse axis and the ratio of longitudinal length to transverse length is at least 2:1, optionally at least 3:1, optionally at least 4:1, optionally at least 5:1, optionally at least 6:1, optionally at least 7:1, optionally at least 8:1, optionally at least 9:1, optionally 10:1;
optionally wherein both of the at least two beta solenoid domains have a longitudinal axis and a transverse axis and the ratio of longitudinal length to transverse length is at least 2:1, optionally at least 3:1, optionally at least 4:1, optionally at least 5:1, optionally at least 6:1, optionally at least 7:1, optionally at least 8:1, optionally at least 9:1, optionally 10:1;
optionally wherein all of the beta solenoid domains have a longitudinal axis and a transverse axis and the ratio of longitudinal length to transverse length is at least 2:1, optionally at least 3:1, optionally at least 4:1, optionally at least 5:1, optionally at least 6:1, optionally at least 7:1, optionally at least 8:1, optionally at least 9:1, optionally 10:1.

6. The concatemer according to any of claims 1-5 wherein one or both of the at least two beta solenoid domains has an aspect ratio of 10:1 or less, optionally wherein all of the beta solenoid domains have an aspect ratio of 10:1 or less.

7. The concatemer according to any of claims 1-6 wherein the concatemer has a molecular weight of at least 30 kDa, optionally at least 40 kDa, optionally at least 50 kDa, optionally at least 60 kDa, optionally at least 70 kDa , optionally at least 80 kDa, optionally at least 90 kDa, optionally at least 100 kDa, optionally at least 120 kDa, optionally at least 150 kDa, optionally at least 200 kDa, optionally at least 250 kDa, optionally at least 300 kDa, optinally at least 350 kDa, optionally at least 400 kDa, optionally at least 450 kDa, optionally at least 500 kDa, optionally at least 600 kDa, optionaly at least 650 kDa, optionally at least 700 kDa, optionally at least 800 kDa, optionally at least 900 kDa, optionally at least 1000 kDa.;
optionally between 30 kDa and 1000 kDa.

8. The concatemer according to any of claims 1-7 wherein at least one of the at least two beta solenoid domains has a high net charge, optionally has a net charge of at least +3 charge units, optionally at least +5 charge units, optionally at least +7 charge units, optionally at least +10 charge units, optionally at least +15 charge units, optionally at least +20 charge units, optionally at least +25 charge units, optionally at least +30 charge units, optionally at least +35 charge units, optionally at least +40 charge units, optionally at least +45 charge units, optionally at least +50 charge units, optionally at least +55 charge units, optionally at least +60 charge units, optionally at least +65 charge units, optionally at least +70 charge units, optionally at least +75 charge units, optionally at least +80 charge units, optionally at least +85 charge units, optionally at least +90 charge units, optionally at least +95 charge units, optionally at least +100 charge units, optionally at a pH range of 3 to 10, optionally pH 7,
optionally wherein two of the at least two beta solenoid domains have a high net charge, optionally have a net charge of at least +3 charge units, optionally at least +5 charge units, optionally at least +7 charge units, optionally at least +10 charge units, optionally at least +15 charge units, optionally at least +20 charge units, optionally at least +25 charge units, optionally at least +30 charge units, optionally at least +35 charge units, optionally at least +40 charge units, optionally at least +45 charge units, optionally at least +50 charge units, optionally at least +55 charge units, optionally at least +60 charge units, optionally at least +65 charge units, optionally at least +70 charge units, optionally at least +75 charge units, optionally at least +80 charge units, optionally at least +85 charge units, optionally at least +90 charge units, optionally at least +95 charge units, optionally at least +100 charge units, optionally at a pH range of 3 to 10, optionally pH 7,
optionally wherein all of the beta solenoid domains have a high net charge, optionally have a net charge of at least +3 charge units, optionally at least +5 charge units, optionally at least +7 charge units, optionally at least +10 charge units, optionally at least +15 charge units, optionally at least +20 charge units, optionally at least +25 charge units, optionally at least +30 charge units, optionally at least +35 charge units, optionally at least +40 charge units, optionally at least +45 charge units, optionally at least +50 charge units, optionally at least +55 charge units, optionally at least +60 charge units, optionally at least +65 charge units, optionally at least +70 charge units, optionally at least +75 charge units, optionally at least +80 charge units, optionally at least +85 charge units, optionally at least +90 charge units, optionally at least +95 charge units, optionally at least +100 charge units, optionally at a pH range of 3 to 10, optionally pH 7.

9. The concatemer according to any of claims 1-8 wherein at least one of the at least two beta solenoid domains has a high net charge, optionally has a net charge of at least +3 charge units, optionally at least +5 charge units, optionally at least +7 charge units, optionally at least +10 charge units, optionally at least +15 charge units, optionally at least +20 charge units, optionally at least +25 charge units, optionally at least +30 charge units, optionally at least +35 charge units, optionally at least +40 charge units, optionally at least +45 charge units, optionally at least +50 charge units, optionally at least +55 charge units, optionally at least +60 charge units, optionally at least +65 charge units, optionally at least +70 charge units, optionally at least +75 charge units, optionally at least +80 charge units, optionally at least +85 charge units, optionally at least +90 charge units, optionally at least +95 charge units, optionally at least +100 charge units, optionally at a pH range of 3 to 10, optionally pH 7,
optionally wherein two of the at least two beta solenoid domains have a high net charge, optionally have a net charge of at least +3 charge units, optionally at least +5 charge units, optionally at least +7 charge units, optionally at least +10 charge units, optionally at least +15 charge units, optionally at least +20 charge units, optionally at least +25 charge units, optionally at least +30 charge units, optionally at least +35 charge units, optionally at least +40 charge units, optionally at least +45 charge units, optionally at least +50 charge units, optionally at least +55 charge units, optionally at least +60 charge units, optionally at least +65 charge units, optionally at least +70 charge units, optionally at least +75 charge units, optionally at least +80 charge units, optionally at least +85 charge units, optionally at least +90 charge units, optionally at least +95 charge units, optionally at least +100 charge units, optionally at a pH range of 3 to 10, optionally pH 7,
optionally wherein all of the beta solenoid domains have a high net charge, optionally have a net charge of at least +3 charge units, optionally at least +5 charge units, optionally at least +7 charge units, optionally at least +10 charge units, optionally at least +15 charge units, optionally at least +20 charge units, optionally at least +25 charge units, optionally at least +30 charge units, optionally at least +35 charge units, optionally at least +40 charge units, optionally at least +45 charge units, optionally at least +50 charge units, optionally at least +55 charge units, optionally at least +60 charge units, optionally at least +65 charge units, optionally at least +70 charge units, optionally at least +75 charge units, optionally at least +80 charge units, optionally at least +85 charge units, optionally at least +90 charge units, optionally at least +95 charge units, optionally at least +100 charge units, optionally at a pH range of 3 to 10, optionally pH 7.

10. The concatemer according to any of claims 1-9 wherein at least one of the beta solenoid domains comprises units of a consensus motif selected from the group consisting of:
A X₁ L X₂ X₃ [SEQ ID NO: 149]
wherein X₁ is A, C, D, E, I, K, L, M, N, R, S, T or V
X₂ is A, C, D, E, F, G, H, I, K, N, Q, R, S, T, V, W, or Y
X₃ is A, C, D, E, G, H, I, K, N, Q, R, S ,Y; [SEQ ID NO: 150]
optionally wherein the consensus motif generates a beta solenoid domain with a square cross-section.

11. The concatemer according to any of claims 1-10 wherein at least one of the at least two beta solenoid domains comprises or consists of the sequence of SEQ ID NO: 1, 8-28 and 169,
optionally wherein at least two of the beta solenoid domains comprise or consist of the sequence of any one of SEQ ID NO: 1, 8-28 and 169,
optionally wherein all of the beta solenoid domains comprise or consist of the sequence of any one of SEQ ID NO: 1, 8-28 and 169,
optionally wherein the at least one, the at least two or all of the beta solenoid domains have a) at least 75% homology to any one of the sequences of SEQ ID NO: 1, 8-28 and 169, optionally at least 80% homology, optionally at least 85% homology, optionally at least 90% homology, optionally at least 95% homology, optionally at least 96% homology, optionally at least 97% homology, optionally at least 98% homology, optionally at least 99% homology, optionally 100% homology' or b) at least 75% sequence identity to any one of the sequences of SEQ ID NO: 1, 8-28 and 169, optionally at least 80% sequence identity, optionally at least 85% sequence identity, optionally at least 90% sequence identity, optionally at least 95% sequence identity, optionally at least 96% sequence identity, optionally at least 97% sequence identity, optionally at least 98% sequence identity, optionally at least 99% sequence identity, optionally 100% sequence identity.

12. The concatemer according to any of claims 1-11 wherein at least one of the beta solenoid domains comprises a capping sequence at a first end or a second end, or both the first end and the second end of the beta solenoid domain,
optionally wherein at least two of the beta solenoid domains comprise a capping sequence at a first end or a second end, or both the first end and the second end of each beta solenoid domains,
optionally wherein all of the beta solenoid domains comprise a capping sequence at a first end or a second end, or both the first end and the second end of each beta solenoid domain,
optionally wherein the capping sequence prevents the beta solenoid domains from joining end-to-end and aggregating,
optionally wherein the capping sequence is an alpha helix optionally wherein at least one of or both of or all of the capping sequence comprises:
a) a sequence that conforms to the consensus sequence of SEQ ID NO: 82 and/or SEQ ID NO: 83, optionally wherein the first cap conforms to SEQ ID NO: 82 and the second cap conforms to SEQ ID NO: 83; and/or
b) any one or more of SEQ ID NO: 2, 3, 84-104 and 105-125
optionally wherein at least one of or both of or all of the capping sequence comprises a sequence with a) at least 80% homology, optionally at least 85% homology, optionally at least 90% homology, optionally at least 95% homology, optionally at least 96% homology, optionally at least 97% homology, optionally at least 98% homology, optionally at least 99% homology, optionally 100% homology to SEQ ID NO: 2, 3, 84-104 or 105-125; or b) at least 80% sequence identity, optionally at least 85% sequence identity, optionally at least 90% sequence identity, optionally at least 95% sequence identity, optionally at least 96% sequence identity, optionally at least 97% sequence identity, optionally at least 98% sequence identity, optionally at least 99% sequence identity, optionally 100% sequence identity to SEQ ID NO: 2, 3, 84-104 or 105-125.

13. The concatemer according to any of claims 1-12 wherein the linker is a flexible linker, optionally wherein the linker or the flexible linker is formed from amino acid residues, optionally wherein the linker sequence is selected from the group consisting of a poly glycine linker, a poly alanine linker, a glycine rich linker, an alanine rich linker, a glycine and alanine rich linker, a glycine/alanine/serine rich linker, SEQ ID NO: 34 (GGGS), or SEQ ID NO: 168 (GGGSAAAAAAGGGS).

14. The concatemer according to any of claims 1-13 wherein the concatemer comprises or consists of one or more of the sequences according to SEQ ID NO: 29-32 and 158-162.

15. A beta solenoid domain that comprises an amino acid sequence that comprises at least 4 units of the following consensus motif:
i) A X₁ L/F X₂ X₃[SEQ ID NO: 147]
wherein X₁ is A, C, D, E, I, K, L, M, N, R, S, T or V
X₂ is A, C, D, E, F, G, H, I, K, N, Q, R, S, T, V, W, or Y
X₃ is A, C, D, E, G, H, I, K, N, Q, R, S ,Y;
wherein the beta solenoid domain does not consist of any of the sequences of SEQ ID NO: 1, 34, 36, 38, 40, 42, 44, 46, 48, 50, 52, 54, 56, 58, 60, 62; and/or SEQ ID NO: 66-80
And wherein the beta solenoid domain:
a) has a longitudinal axis and a transverse axis and the ratio of the longitudinal axis to transverse axis is at least 2:1;
b) comprises at least 29 units;
c) has a molecular weight of at least 60KDa;
d) is soluble at a concentration of at least 20 mg/ml

16. The beta solenoid domain according to claim 15 wherein the beta solenoid domain that comprises an amino acid sequence that comprises units of the following consensus motif:
iA X₁ L X₂ X₃ [SEQ ID NO: 149]
wherein X₁ is A, C, D, E, I, K, L, M, N, R, S, T or V
X₂ is A, C, D, E, F, G, H, I, K, N, Q, R, S, T, V, W, or Y
X₃ is A, C, D, E, G, H, I, K, N, Q, R, S ,Y; [SEQ ID NO: 150].

17. A composition comprising a concatemer according to any of claims 1-14 or a beta solenoid domain according to claim 15 or 16, optionally wherein the concatemer or beta solenoid domain is at a concentration of at least 20 mg/ml, optionally at least 30 mg/ml, optionally at least 40 mg/ml, optionally at least 50 mg/ml, optionally at least 60 mg/ml, optionally at least 70 mg/ml, optionally at least 80 mg/ml, optionally at least 90 mg/ml, optionally at least 100 mg/ml, optionally at least 120 mg/ml, optionally at least 130 mg/ml, optionally at least 150 mg/ml, optionally at least 170 mg/ml, optionally at least 190 mg/ml, optionally at least 200 mg/ml, optionally at least 250 mg/ml, optionally at least 300 mg/ml, optionally at least 350 mg/ml, optionally at least 400 mg/ml.

18. A nucleic acid encoding the concatemer according to any of claims 1-14 or the beta solenoid domain according to claim 15 or 16; or a vector comprising the nucleic acid encoding the concatemer according to any of claims 1-14 or the beta solenoid domain according to claim 15 or 16.

19. A host cell comprising the nucleic acid according to claim 18 and/or the vector according to claim 18, optionally wherein the host cell is
a) a prokaryotic cell,
optionally a bacterial cell, optionally an *E. coli* cell, a *Bacillus subtilis* cell, a *Bacillus megaterium* cell, a *Vibrio natriegens* cell, or a *Pseudomonas fluorescens* cell; or
b) is a eukaryotic cell, optionally
a yeast cell, optionally *Pichia pastoris* or Saccharomyces cerevisiae;
an insect cell, optionally a baculovirus infected insect cell; or
a mammalian cell, optionally a baculovirus infected mammalian cell, a HEK293 cell, a HeLa cell, or CHO cells.

20. A fibre comprising the concatemer according to any of claims 1-14 or the beta solenoid domain according claim 15 or 16 optionally wherein the fibre has:
a) been produced via a wet-spinning method; and/or
b) an average diameter of between 100 nm and 100um, optionally between 500 nm and 50um, optionally between 1000nm and 40um, optionally between 2.5um and 35 um, optionally between 5 um and 30 um, optionally between 7.5 um and 25 um, optionally between 10 um and 20 um; and/or
c) a Young's modulus of between 0.5 GPa and 200 Gpa, optionally between 1.0 Gpa and 175 Gpa, optionally between 2 Gpa and 150 Gpa, optionally between 5.0 Gpa and 125 Gpa, optionally between 10.0 Gpa and 100.0 Gpa, optionally between 15.0 Gpa and 90.0 Gpa, optionally between 20.0 Gpa and 80.0 Gpa, optionally between 25.0 Gpa and 75.0 Gpa, optionally between 30.0 Gpa and 70.0 Gpa, optionally between 35.0 Gpa and 60.0 Gpa, optionally between 40.0 GPa and 50.0 GPa; and/or
a Young's modulus of at least 0.5 GPa, 1.0 GPa, 2 GPa, 5.0 GPa, 10.0 GPa, 15.0 GPa, 20.0 GPa, 25.0 GPa, 30.0GPa, 35.0 GPa, 40.0 GPa, 50.0 GPa, 60.0 GPa, 70.0 GPa, 75.0 GPa, 80.0 GPa, 90.0 GPa, 100.0 GPa, 125 GPa, 150 GPa, 175 GPa or at least 200 GPa; and/or
d) an engineered strength of:
between 0.1 GPa and 3 GPa, optionally between 0.2 GPa and 2.8 GPa, optionally between 0.3 GPa and 2.6 GPa, optionally between 0.4 GPa and 2.4 GPa,
optionally between 0.5 GPa and 2.2 GPa, optionally between 0.6 GPa and 2.0 GPa, optionally between 0.7 GPa and 1.8 GPa, optionally between 0.8 GPa and 1.6 GPa, optionally between 0.9 GPa and 1.4 GPa, optionally between 1.0 GPa and 1.2 GPa; and/or
at least 0.1 GPa, optionally at least 0.2 GPa, optionally at least 0.3 GPa, optionally at least 0.4 GPa, optionally at least 0.5 GPa, optionally at least 0.6 GPa, optionally at least 0.7 GPa, optionally at least 0.8 GPa, optionally at least 0.9 GPa, optionally at least 1.0 GPa, optionally at least 1.1 GPa, optionally at least 1.2 GPa, optionally at least 1.3 GPa, optionally at least 1.4 GPa, optionally at least 1.5 GPa, optionally at least 1.6 GPa, optionally at least 1.7 GPa, optionally at least 1.8 GPa, optionally at least 1.9 GPa, optionally at least 2.0 GPa, optionally at least 2.1 GPa, optionally at least 2.2 GPa, optionally at least 2.3 GPa, optionally at least 2.4 GPa, optionally at least 2.5 GPa, optionally at least 2.6 GPa, optionally at least 2.7 GPa, optionally at least 2.8 GPa, optionally at least 2.9 GPa, optionally at least 3.0 GPa; and/or
e) a strain to failure of between 2% and 300%, optionally between 5% and 275%, optionally between 10% and 250%, optionally between 15% and 225%, optionally between 20% and 200%, optionally between 25% and 175%, optionally between 30% and 150%, optionally between 35% and 125%, optionally between 40% and 100%, optionally between 45% and 80%, optionally between 50% and 75%, optionally between 55% and 70%; and/or
a strain to failure of at least 2% , 5%, 10%, 15%, 20%, 25%, 30%, 35%, 40%, 45%, 50%, 55%, 60%, 65%, 70%, 75%, 80%, 90%, 100%, 125%, 150%, 175%, 200%, 225%, 250%, 275% or at least 300%; and/or
f) a toughness of at least 10 J/g, optionally at least 20 J/g, optionally at least 40 J/g, optionally at least 60 J/g, optionally at least 80 J/g, optionally at least 100 J/g, optionally at least 120 J/g, optionally at least 140 J/g, optionally at least 160 J/g, optionally at least 180 J/g, optionally at least 200 J/g, optionally at least 250 J/g, optionally at least 300 J/g, optionally at least 350 J/g, optionally at least 400 J/g, optionally at least 450 J/g, optionally at least 500 J/g, optionally at least 550 J/g, optionally at least 600 J/g, optionally at least 650 J/g, optionally at least 700 J/g, optionally at least 750 J/g, optionally at least 800 J/g, optionally at least 850 J/g, optionally at least 900 J/g, optionally at least 950 J/g, optionally at least 1000 J/g.

21. A textile comprising the concatemer according to any of claims 1-14, the beta solenoid domain according claim 15 or 16, or the fibre according to claim 19.

22. Sportswear (e.g. for protective uses such as rash guards); Protective wear for motorcyclists and/or other motorsports; Personal protective equipment (e.g. body armour, protective underwear for soldiers); Blast containment; Blade containment (e.g. for aircraft engines); Textiles for parachutes; Ropes; Textiles for clothing; Textiles for shoes; general medical textiles; Wound dressings; Resorbable and non-resorbable surgical sutures; Resorbable and non-resorbable stents; Guide-wires for surgery (including non-metallic guide wires suitable for MRI); Biocompatible coatings for implanted medical devices; Surgical meshes; or Scaffolds for 3D tissue engineering comprising a concatemer according to any of claims 1-14, a beta solenoid domain according to claim 15 or 16, a fibre according to claim 20 or a textile according to claim 21.

## Patentansprüche

1. Konkatemer von Beta-Magnetdomänen, wobei der Konkatemer mindestens zwei Beta-Magnetdomänen umfasst,
wobei die mindestens zwei Beta-Magnetdomänen durch einen Linker verbunden sind, und wobei jede der mindestens zwei Beta-Magnetdomänen eine Aminosäuresequenz umfasst, die mindestens 4 Einheiten eines Konsensmotivs umfasst, und
wobei der Konkatemer ein Molekulargewicht von mindestens 30 kDa aufweist,
wobei jede Einheit innerhalb einer der Beta-Magnetdomänen mit der folgenden Konsensussequenz übereinstimmt:
A X₁ L/F X₂ X₃ [SEQ ID NO: 147]
wobei X₁ A, C, D, E, I, K, L, M, N, R, S, T oder V ist X₂ A, C, D, E, F, G, H, I, K, N, Q, R, S, T, V, W oder Y ist
X₃ A, C, D, E, G, H, I, K, N, Q, R, S, Y ist.

2. Konkatemer nach Anspruch 1, wobei:
mindestens eine der mindestens zwei Beta-Magnetdomänen mindestens 5 Einheiten des Konsensmotivs umfasst, optional mindestens 6 Einheiten, optional mindestens 7 Einheiten, optional mindestens 8 Einheiten, optional mindestens 9 Einheiten, optional mindestens 10 Einheiten, optional mindestens 11 Einheiten, optional mindestens 12 Einheiten, optional mindestens 13 Einheiten, optional mindestens 14 Einheiten, optional mindestens 15 Einheiten, optional mindestens 16 Einheiten, optional mindestens 17 Einheiten, optional mindestens 18 Einheiten, optional mindestens 19 Einheiten, optional mindestens 20 Einheiten, optional mindestens 21 Einheiten, optional mindestens 24 Einheiten, optional mindestens 25 Einheiten, optional mindestens 26 Einheiten, optional mindestens 27 Einheiten, optional mindestens 28 Einheiten, optional mindestens 29 Einheiten, optional mindestens 30 Einheiten, optional mindestens 40 Einheiten, optional mindestens 50 Einheiten, optional mindestens 60 Einheiten, optional mindestens 70 Einheiten, optional mindestens 80 Einheiten, optional mindestens 90 Einheiten, optional mindestens 100 Einheiten, optional mindestens 200 Einheiten des Konsensmotivs;
mindestens zwei der Beta-Magnetdomänen mindestens 5 Einheiten des Konsensmotivs umfassen, optional mindestens 6 Einheiten, optional mindestens 7 Einheiten, optional mindestens 8 Einheiten, optional mindestens 9 Einheiten, optional mindestens 10 Einheiten, optional mindestens 11 Einheiten, optional mindestens 12 Einheiten, optional mindestens 13 Einheiten, optional mindestens 14 Einheiten, optional mindestens 15 Einheiten, optional mindestens 16 Einheiten, optional mindestens 17 Einheiten, optional mindestens 18 Einheiten, optional mindestens 19 Einheiten, optional mindestens 20 Einheiten, optional mindestens 21 Einheiten, optional mindestens 24 Einheiten, optional mindestens 25 Einheiten, optional mindestens 26 Einheiten, optional mindestens 27 Einheiten, optional mindestens 28 Einheiten, optional mindestens 29 Einheiten, optional mindestens 30 Einheiten, optional mindestens 40 Einheiten, optional mindestens 50 Einheiten, optional mindestens 60 Einheiten, optional mindestens 70 Einheiten, optional mindestens 80 Einheiten, optional mindestens 90 Einheiten, optional mindestens 100 Einheiten, optional mindestens 200 Einheiten des Konsensmotivs;
alle der Beta-Magnetdomänen mindestens 5 Einheiten des Konsensmotivs umfassen, optional mindestens 6 Einheiten, optional mindestens 7 Einheiten, optional mindestens 8 Einheiten, optional mindestens 9 Einheiten, optional mindestens 10 Einheiten, optional mindestens 11 Einheiten, optional mindestens 12 Einheiten, optional mindestens 13 Einheiten, optional mindestens 14 Einheiten, optional mindestens 15 Einheiten, optional mindestens 16 Einheiten, optional mindestens 17 Einheiten, optional mindestens 18 Einheiten, optional mindestens 19 Einheiten, optional mindestens 20 Einheiten, optional mindestens 21 Einheiten, optional mindestens 24 Einheiten, optional mindestens 25 Einheiten, optional mindestens 26 Einheiten, optional mindestens 27 Einheiten, optional mindestens 28 Einheiten, optional mindestens 29 Einheiten, optional mindestens 30 Einheiten, optional mindestens 40 Einheiten, optional mindestens 50 Einheiten, optional mindestens 60 Einheiten, optional mindestens 70 Einheiten, optional mindestens 80 Einheiten, optional mindestens 90 Einheiten, optional mindestens 100 Einheiten, optional mindestens 200 Einheiten des Konsensmotivs;
wobei optional die Einheiten des Konsensmotivs aufeinanderfolgend angeordnet sind.

3. Verkettungsgerät nach einem der Ansprüche 1 oder 2, wobei:
die Beta-Magnetdomänen in der Lage sind, eine lyotrope Flüssigkristallmesophase zu bilden, wenn sie als Monomere ausgedrückt werden; und/oder
der Konkatemer in der Lage ist, eine lyotrope Flüssigkristallmesophase zu bilden.

4. Konkatemer nach einem der Ansprüche 1 bis 3, wobei das Konsensmotiv eine Beta-Magnetdomäne mit quadratischem Querschnitt erzeugt.

5. Konkatemer nach einem der Ansprüche 1 bis 4, wobei die mindestens zwei Beta-Magnetdomänen stabförmig sind,
wobei optional eine der mindestens zwei Beta-Magnetdomänen eine longitudinale Achse und eine transversale Achse aufweist und das Verhältnis von longitudinaler Länge zu transversaler Länge mindestens 2:1, optional mindestens 3:1, optional mindestens 4:1, optional mindestens 5:1, optional mindestens 6:1, optional mindestens 7:1, optional mindestens 8:1, optional mindestens 9:1, optional 10:1 beträgt;
wobei optional beide der mindestens zwei Beta-Magnetdomänen eine Längsachse und eine transversale Achse aufweisen und das Verhältnis von longitudinaler Länge zu transversaler Länge mindestens 2:1, optional mindestens 3:1, optional mindestens 4:1, optional mindestens 5:1, optional mindestens 6:1, optional mindestens 7:1, optional mindestens 8:1, optional mindestens 9:1, optional 10:1 beträgt;
wobei optional alle Beta-Magnetdomänen eine Längsachse und eine transversale Achse aufweisen und das Verhältnis von longitudinaler Länge zu transversaler Länge mindestens 2:1, optional mindestens 3:1, optional mindestens 4:1, optional mindestens 5:1, optional mindestens 6:1, optional mindestens 7:1, optional mindestens 8:1, optional mindestens 9:1, optional 10:1 beträgt.

6. Konkatemer nach einem der Ansprüche 1 bis 5, wobei eine oder beide der mindestens zwei Beta-Magnetdomänen ein Seitenverhältnis von 10:1 oder weniger aufweisen, optional wobei alle Beta-Magnetdomänen ein Seitenverhältnis von 10:1 oder weniger aufweisen.

7. Konkatemer nach einem der Ansprüche 1 bis 6, wobei der Konkatemer ein Molekulargewicht von mindestens 30 kDa, optional von mindestens 40 kDa, optional von mindestens 50 kDa, optional von mindestens 60 kDa, optional von mindestens 70 kDa, optional mindestens 80 kDa, optional mindestens 90 kDa, optional mindestens 100 kDa, optional mindestens 120 kDa, optional mindestens 150 kDa, optional mindestens 200 kDa, optional mindestens 250 kDa, optional mindestens 300 kDa, optional mindestens 350 kDa, optional mindestens 400 kDa, optional mindestens 450 kDa, optional mindestens 500 kDa, optional mindestens 600 kDa, optional mindestens 650 kDa, optional mindestens 700 kDa, optional mindestens 800 kDa, optional mindestens 900 kDa, optional mindestens 1000 kDa aufweist;
optional zwischen 30 kDa und 1000 kDa.

8. Konkatemer nach einem der Ansprüche 1 bis 7, wobei mindestens eine der mindestens zwei Beta-Magnetdomänen eine hohe Nettoladung aufweist, optional eine Nettoladung von mindestens +3 Ladeeinheiten, optional mindestens +5 Ladeeinheiten, optional mindestens +7 Ladeeinheiten, optional mindestens +10 Ladeeinheiten, optional mindestens +15 Ladeeinheiten, optional mindestens +20 Ladeeinheiten, optional mindestens +25 Ladeeinheiten, optional mindestens +30 Ladeeinheiten, optional mindestens +35 Ladeeinheiten, optional mindestens +40 Ladeeinheiten, optional mindestens +45 Ladeeinheiten, optional mindestens +50 Ladeeinheiten, optional mindestens +55 Ladeeinheiten, optional mindestens +60 Ladeeinheiten, optional mindestens +65 Ladeeinheiten, optional mindestens +70 Ladeeinheiten, optional mindestens +75 Ladeeinheiten, optional mindestens +80 Ladeeinheiten, optional mindestens +85 Ladeeinheiten, optional mindestens +90 Ladeeinheiten, optional mindestens +95 Ladeeinheiten, optional mindestens +100 Ladeeinheiten, optional in einem pH-Bereich von 3 bis 10, optional pH 7,
optional wobei zwei der mindestens zwei Beta-Magnetdomänen eine hohe Nettoladung aufweisen, optional eine Nettoladung von mindestens +3 Ladeeinheiten, optional mindestens +5 Ladeeinheiten, optional mindestens +7 Ladeeinheiten, optional mindestens +10 Ladeeinheiten, optional mindestens +15 Ladeeinheiten, optional mindestens +20 Ladeeinheiten, optional mindestens +25 Ladeeinheiten, optional mindestens +30 Ladeeinheiten, optional mindestens +35 Ladeeinheiten, optional mindestens +40 Ladeeinheiten, optional mindestens +45 Ladeeinheiten, optional mindestens +50 Ladeeinheiten, optional mindestens +55 Ladeeinheiten, optional mindestens +60 Ladeeinheiten, optional mindestens +65 Ladeeinheiten, optional mindestens +70 Ladeeinheiten, optional mindestens +75 Ladeeinheiten, optional mindestens +80 Ladeeinheiten, optional mindestens +85 Ladeeinheiten, optional mindestens +90 Ladeeinheiten, optional mindestens +95 Ladeeinheiten, optional mindestens +100 Ladeeinheiten, optional in einem pH-Bereich von 3 bis 10, optional pH **7,**
optional wobei alle Beta-Magnetdomänen eine hohe Nettoladung aufweisen, optional eine Nettoladung von mindestens +3 Ladeeinheiten, optional mindestens +5 Ladeeinheiten, optional mindestens +7 Ladeeinheiten, optional mindestens +10 Ladeeinheiten, optional mindestens +15 Ladeeinheiten, optional mindestens +20 Ladeeinheiten, optional mindestens +25 Ladeeinheiten, optional mindestens +30 Ladeeinheiten, optional mindestens +35 Ladeeinheiten, optional mindestens +40 Ladeeinheiten, optional mindestens +45 Ladeeinheiten, optional mindestens +50 Ladeeinheiten, optional mindestens +55 Ladeeinheiten, optional mindestens +60 Ladeeinheiten, optional mindestens +65 Ladeeinheiten, optional mindestens +70 Ladeeinheiten, optional mindestens +75 Ladeeinheiten, optional mindestens +80 Ladeeinheiten, optional mindestens +85 Ladeeinheiten, optional mindestens +90 Ladeeinheiten, optional mindestens +95 Ladeeinheiten, optional mindestens +100 Ladeeinheiten, optional in einem pH-Bereich von 3 bis 10, optional pH 7.

9. Konkatemer nach einem der Ansprüche 1 bis 8, wobei mindestens eine der mindestens zwei Beta-Magnetdomänen eine hohe Nettoladung aufweist, optional eine Nettoladung von mindestens +3 Ladeeinheiten, optional mindestens +5 Ladeeinheiten, optional mindestens +7 Ladeeinheiten, optional mindestens +10 Ladeeinheiten, optional mindestens +15 Ladeeinheiten, optional mindestens +20 Ladeeinheiten, optional mindestens +25 Ladeeinheiten, optional mindestens +30 Ladeeinheiten, optional mindestens +35 Ladeeinheiten, optional mindestens +40 Ladeeinheiten, optional mindestens +45 Ladeeinheiten, optional mindestens +50 Ladeeinheiten, optional mindestens +55 Ladeeinheiten, optional mindestens +60 Ladeeinheiten, optional mindestens +65 Ladeeinheiten, optional mindestens +70 Ladeeinheiten, optional mindestens +75 Ladeeinheiten, optional mindestens +80 Ladeeinheiten, optional mindestens +85 Ladeeinheiten, optional mindestens +90 Ladeeinheiten, optional mindestens +95 Ladeeinheiten, optional mindestens +100 Ladeeinheiten, optional in einem pH-Bereich von 3 bis 10, optional pH 7,
optional wobei zwei der mindestens zwei Beta-Magnetdomänen eine hohe Nettoladung aufweisen, optional eine Nettoladung von mindestens +3 Ladeeinheiten, optional mindestens +5 Ladeeinheiten, optional mindestens +7 Ladeeinheiten, optional mindestens +10 Ladeeinheiten, optional mindestens +15 Ladeeinheiten, optional mindestens +20 Ladeeinheiten, optional mindestens +25 Ladeeinheiten, optional mindestens +30 Ladeeinheiten, optional mindestens +35 Ladeeinheiten, optional mindestens +40 Ladeeinheiten, optional mindestens +45 Ladeeinheiten, optional mindestens +50 Ladeeinheiten, optional mindestens +55 Ladeeinheiten, optional mindestens +60 Ladeeinheiten, optional mindestens +65 Ladeeinheiten, optional mindestens +70 Ladeeinheiten, optional mindestens +75 Ladeeinheiten, optional mindestens +80 Ladeeinheiten, optional mindestens +85 Ladeeinheiten, optional mindestens +90 Ladeeinheiten, optional mindestens +95 Ladeeinheiten, optional mindestens +100 Ladeeinheiten, optional in einem pH-Bereich von 3 bis 10, optional pH **7,**
optional wobei alle Beta-Magnetdomänen eine hohe Nettoladung aufweisen, optional eine Nettoladung von mindestens +3 Ladeeinheiten, optional mindestens +5 Ladeeinheiten, optional mindestens +7 Ladeeinheiten, optional mindestens +10 Ladeeinheiten, optional mindestens +15 Ladeeinheiten, optional mindestens +20 Ladeeinheiten, optional mindestens +25 Ladeeinheiten, optional mindestens +30 Ladeeinheiten, optional mindestens +35 Ladeeinheiten, optional mindestens +40 Ladeeinheiten, optional mindestens +45 Ladeeinheiten, optional mindestens +50 Ladeeinheiten, optional mindestens +55 Ladeeinheiten, optional mindestens +60 Ladeeinheiten, optional mindestens +65 Ladeeinheiten, optional mindestens +70 Ladeeinheiten, optional mindestens +75 Ladeeinheiten, optional mindestens +80 Ladeeinheiten, optional mindestens +85 Ladeeinheiten, optional mindestens +90 Ladeeinheiten, optional mindestens +95 Ladeeinheiten, optional mindestens +100 Ladeeinheiten, optional in einem pH-Bereich von 3 bis 10, optional pH 7.

10. Konkatemer nach einem der Ansprüche 1 bis 9, wobei mindestens eine der Beta-Magnetdomänen Einheiten eines Konsensmotivs umfasst, das aus der Gruppe ausgewählt ist, bestehend aus:
A X₁ L X₂ X₃ [SEQ ID NO: 149]
wobei X₁ A, C, D, E, I, K, L, M, N, R, S, T oder V ist X₂ A, C, D, E, F, G, H, I, K, N, Q, R, S, T, V, W oder Y ist
X₃ A, C, D, E, G, H, I, K, N, Q, R, S, Y ist; [SEQ ID NO: 150]
Optional, wobei das Konsensmotiv eine Beta-Magnetdomäne mit quadratischem Querschnitt erzeugt.

11. Konkatemer nach einem der Ansprüche 1 bis 10, wobei mindestens eine der mindestens zwei Beta-Magnetdomänen die Sequenz von SEQ ID NO: 1, 8-28 und 169 umfasst oder daraus besteht,
wobei optional mindestens zwei der Beta-Magnetdomänen die Sequenz einer der Sequenzen SEQ ID NO: 1, 8-28 und 169 umfassen oder daraus bestehen,
wobei optional alle der Beta-Magnetdomänen die Sequenz einer der Sequenzen SEQ ID NO: 1, 8-28 und 169 umfassen oder daraus bestehen,
wobei optional die mindestens eine, die mindestens zwei oder alle Beta-Magnetdomänen a) mindestens 75 % Homologie zu einer der Sequenzen von SEQ ID NO: 1, 8-28 und 169 aufweisen, optional mindestens 80 % Homologie, optional mindestens 85 % Homologie, optional mindestens 90 % Homologie, optional mindestens 95 % Homologie, optional mindestens 96 % Homologie, optional mindestens 97 % Homologie, optional mindestens 98 % Homologie, optional mindestens 99 % Homologie, optional 100 % Homologi oder b) mindestens 75 % Sequenzidentität mit einer der Sequenzen von SEQ ID NO: 1, 8-28 und 169 aufweisen, optional mindestens 80 % Sequenzidentität, optional mindestens 85 % Sequenzidentität, optional mindestens 90 % Sequenzidentität, optional mindestens 95 % Sequenzidentität, optional mindestens 96 % Sequenzidentität, optional mindestens 97 % Sequenzidentität, optional mindestens 98 % Sequenzidentität, optional mindestens 99 % Sequenzidentität, optional 100 % Sequenzidentität.

12. Konkatemer nach einem der Ansprüche 1 bis 11, wobei mindestens eine der Beta-Magnetdomänen eine Kappensequenz an einem ersten Ende oder einem zweiten Ende oder sowohl am ersten Ende als auch am zweiten Ende der Beta-Magnetdomäne umfasst,
wobei optional mindestens zwei der Beta-Magnetdomänen eine Kappensequenz an einem ersten Ende oder einem zweiten Ende oder sowohl am ersten Ende als auch am zweiten Ende jeder Beta-Magnetdomäne umfassen,
wobei optional alle Beta-Magnetdomänen eine Kappensequenz an einem ersten Ende oder einem zweiten Ende oder sowohl am ersten Ende als auch am zweiten Ende jeder Beta-Magnetdomäne umfassen,
wobei optional die Kappensequenz verhindert, dass sich die Beta-Magnetdomänen Ende-an-Ende verbinden und aggregieren,
wobei die Kappensequenz optional eine Alpha-Helix ist,
wobei optional mindestens eine der Kappensequenzen oder beide oder alle Kappensequenzen Folgendes umfassen:
a) eine Sequenz, die mit der Konsensussequenz von SEQ ID NO: 82 und/oder SEQ ID NO: 83 übereinstimmt, wobei optional die erste Kappe mit SEQ ID NO: 82 und die zweite Kappe mit SEQ ID NO: 83 übereinstimmt; und/oder
b) eine oder mehrere von SEQ ID NO: 2, 3, 84-104 und 105-125,
wobei optional mindestens eine der oder beide oder die gesamte Kappensequenz eine Sequenz mit a) mindestens 80 % Homologie, optional mindestens 85 % Homologie, optional mindestens 90 % Homologie, optional mindestens 95 % Homologie, optional mindestens 96 % Homologie, optional mindestens 97 % Homologie, optional mindestens 98 % Homologie, optional mindestens 99 % Homologie, optional 100 % Homologie zu SEQ ID NO: 2, 3, 84-104 oder 105-125 umfasst; oder b) mindestens 80 % Sequenzidentität, optional mindestens 85 % Sequenzidentität, optional mindestens 90 % Sequenzidentität, optional mindestens 95 % Sequenzidentität, optional mindestens 96 % Sequenzidentität, optional mindestens 97 % Sequenzidentität, optional mindestens 98 % Sequenzidentität, optional mindestens 99 % Sequenzidentität, optional 100 % Sequenzidentität zu SEQ ID NO: 2, 3, 84-104 oder 105-125.

13. Konkatemer nach einem der Ansprüche 1 bis 12, wobei der Linker ein flexibler Linker ist, wobei optional der Linker oder der flexible Linker aus Aminosäureresten gebildet ist, wobei optional die Linkersequenz aus der Gruppe ausgewählt ist, bestehend aus einem Polyglycinlinker, einem Polyalaninlinker, einem glycinreichen Linker, einem alaninreichen Linker, einem glycin- und alaninreichen Linker, einem glycin/alanin/serinreichen Linker, SEQ ID NO: 34 (GGGS), oder SEQ ID NO: 168 (GGGSAAAAAAGGGS).

14. Konkatemer nach einem der Ansprüche 1 bis 13, wobei der Konkatemer eine oder mehrere der Sequenzen nach SEQ ID NO: 29-32 und 158-162 umfasst oder daraus besteht.

15. Beta-Magnetdomäne, die eine Aminosäuresequenz umfasst, die mindestens 4 Einheiten des folgenden Konsensmotivs umfasst:
i) A X₁ UF X₂ X₃ [SEQ ID NO: 147]
wobei X₁ A, C, D, E, I, K, L, M, N, R, S, T oder V ist X₂ A, C, D, E, F, G, H, I, K, N, Q, R, S, T, V, W oder Y ist
X₃ A, C, D, E, G, H, I, K, N, Q, R, S, Y ist;
wobei die Beta-Magnetdomäne nicht aus einer der Sequenzen von SEQ ID NO: 1, 34, 36, 38, 40, 42, 44, 46, 48, 50, 52, 54, 56, 58, 60, 62 besteht; und/oder
SEQ ID NO: 66-80
und wobei die Beta-Magnetdomäne:
a) eine Längsachse und eine transversale Achse aufweist und das Verhältnis der Längsachse zur transversalen Achse mindestens 2:1 beträgt;
b) mindestens 29 Einheiten umfasst;
c) ein Molekulargewicht von mindestens 60 kDa aufweist;
d) in einer Konzentration von mindestens 20 mg/ml löslich ist.

16. Beta-Magnetdomäne nach Anspruch 15, wobei die Beta-Magnetdomäne eine Aminosäuresequenz umfasst, die Einheiten des folgenden Konsensusmotivs umfasst:
iA X₁ L X₂ X₃ [SEQ ID NO: 149]
wobei X₁ A, C, D, E, I, K, L, M, N, R, S, T oder V ist X₂ A, C, D, E, F, G, H, I, K, N, Q, R, S, T, V, W oder Y ist
X₃ A, C, D, E, G, H, I, K, N, Q, R, S, Y ist; [SEQ ID NO: 150].

17. Zusammensetzung, umfassend ein Konkatemer nach einem der Ansprüche 1 bis 14 oder eine Beta-Magnetdomäne nach Anspruch 15 oder 16, wobei optional das Konkatemer oder die Beta-Magnetdomäne in einer Konzentration von mindestens 20 mg/ml, optional mindestens 30 mg/ml, optional mindestens 40 mg/ml, optional mindestens 50 mg/ml, optional mindestens 60 mg/ml, optional mindestens 70 mg/ml, optional mindestens 80 mg/ml, optional mindestens 90 mg/ml, optional mindestens 100 mg/ml, optional mindestens 120 mg/ml, optional mindestens 130 mg/ml, optional mindestens 150 mg/ml, optional mindestens 170 mg/ml, optional mindestens 190 mg/ml, optional mindestens 200 mg/ml, optional mindestens 250 mg/ml, optional mindestens 300 mg/ml, optional mindestens 350 mg/ml, optional mindestens 400 mg/ml vorliegt.

18. Nukleinsäure, die das Konkatemer nach einem der Ansprüche 1 bis 14 oder die Beta-Magnetdomäne nach Anspruch 15 oder 16 codiert; oder ein Vektor, umfassend die Nukleinsäure, die das Konkatemer nach einem der Ansprüche 1 bis 14 oder die Beta-Magnetdomäne nach Anspruch 15 oder 16 codiert.

19. Wirtszelle, umfassend die Nukleinsäure nach Anspruch 18 und/oder den Vektor nach Anspruch 18, optional wobei die Wirtszelle Folgendes ist:
a) eine prokaryotische Zelle,
optional eine Bakterienzelle, optional eine *E. coli-*Zelle, eine *Bacillus subtilis-Zelle,* eine *Bacillus megaterium-Zelle,* eine *Vibrio natriegens-Zelle* oder eine *Pseudomonas fluorescens-Zelle;* oder
b) eine eukaryotische Zelle, optional
eine Hefezelle, optional *Pichia pastoris* oder *Saccharomyces cerevisiae;*
eine Insektenzelle, optional eine mit Baculovirus infizierte Insektenzelle; oder
eine Säugetierzelle, optional eine mit Baculovirus infizierte Säugetierzelle, eine HEK293-Zelle, eine HeLa-Zelle oder CHO-Zellen.

20. Faser, umfassend das Konkatemer nach einem der Ansprüche 1 bis 14 oder die Beta-Magnetdomäne nach Anspruch 15 oder 16, optional wobei die Faser:
a) durch ein Nassspinnverfahren hergestellt worden ist; und/oder
b) einem mittleren Durchmesser zwischen 100 nm und 100 um, optional zwischen 500 nm und 50 um, optional zwischen 1000 nm und 40 um, optional zwischen 2,5 um und 35 um, optional zwischen 5 um und 30 um, optional zwischen 7,5 um und 25 um, optional zwischen 10 um und 20 um aufweist; und/oder
c) einem Elastizitätsmodul zwischen 0,5 GPa und 200 GPa, optional zwischen 1,0 Gpa und 175 Gpa, optional zwischen 2 Gpa und 150 Gpa, optional zwischen 5,0 Gpa und 125 Gpa, optional zwischen 10,0 Gpa und 100,0 Gpa, optional zwischen 15,0 Gpa und 90,0 Gpa, optional zwischen 20,0 Gpa und 80,0 Gpa, optional zwischen 25,0 Gpa und 75,0 Gpa, optional zwischen 30,0 Gpa und 70,0 Gpa, optional zwischen 35,0 GPa und 60,0 GPa, optional zwischen 40,0 GPa und 50,0 GPa; und/oder
ein Elastizitätsmodul von mindestens 0,5 GPa, 1,0 GPa, 2 GPa, 5,0 GPa, 10,0 GPa, 15,0 GPa, 20,0 GPa, 25,0 GPa, 30,0 GPa, 35,0 GPa, 40,0 GPa, 50,0 GPa, 60,0 GPa, 70,0 GPa, 75,0 GPa, 80,0 GPa, 90,0 GPa, 100,0 GPa, 125 GPa, 150 GPa, 175 GPa oder mindestens 200 GPa aufweist; und/oder
d) eine konstruierte Festigkeit von:
zwischen 0,1 GPa und 3 GPa, optional zwischen 0,2 GPa und 2,8 GPa, optional zwischen 0,3 GPa und 2,6 GPa, optional zwischen 0,4 GPa und 2,4 GPa, optional zwischen 0,5 GPa und 2,2 GPa, optional zwischen 0,6 GPa und 2,0 GPa, optional zwischen 0,7 GPa und 1,8 GPa, optional zwischen 0,8 GPa und 1,6 GPa, optional zwischen 0,9 GPa und 1,4 GPa, optional zwischen 1,0 GPa und 1,2 GPa; und/oder
mindestens 0,1 GPa, optional mindestens 0,2 GPa, optional mindestens 0,3 GPa, optional mindestens 0,4 GPa, optional mindestens 0,5 GPa, optional mindestens 0,6 GPa, optional mindestens 0,7 GPa, optional mindestens 0,8 GPa, optional mindestens 0,9 GPa, optional mindestens 1,0 GPa, optional mindestens 1,1 GPa, optional mindestens 1,2 GPa, optional mindestens 1,3 GPa, optional mindestens 1,4 GPa, optional mindestens 1,5 GPa, optional mindestens 1,6 GPa, optional mindestens 1,7 GPa, optional mindestens 1,8 GPa, optional mindestens 1,9 GPa, optional mindestens 2,0 GPa, optional mindestens 2,1 GPa, optional mindestens 2,2 GPa, optional mindestens 2,3 GPa, optional mindestens 2,4 GPa, optional mindestens 2,5 GPa, optional mindestens 2,6 GPa, optional mindestens 2,7 GPa, optional mindestens 2,8 GPa, optional mindestens 2,9 GPa, optional mindestens 3,0 GPa; und/oder e) eine Bruchdehnung zwischen 2 % und 300 %, optional zwischen 5 % und 275 %, optional zwischen 10 % und 250 %, optional zwischen 15 % und 225 %, optional zwischen 20 % und 200 %, optional zwischen 25 % und 175 %, optional zwischen 30 % und 150 %, optional zwischen 35 % und 125 %, optional zwischen 40 % und 100 %, optional zwischen 45 % und 80 %, optional zwischen 50 % und 75 %, optional zwischen 55 % und 70 %; und/oder
eine Bruchdehnung von mindestens 2 %, 5 %, 10 %, 15 %, 20 %, 25 %, 30 %, 35 %, 40 %, 45 %, 50 %, 55 %, 60 %, 65 %, 70 %, 75 %, 80 %, 90 %, 100 %, 125 %, 150 %, 175 %, 200 %, 225 %, 250 %, 275 % oder mindestens 300 %; und/oder f) eine Zähigkeit von mindestens 10 J/g, optional mindestens 20 J/g, optional mindestens 40 J/g, optional mindestens 60 J/g, optional mindestens 80 J/g, optional mindestens 100 J/g, optional mindestens 120 J/g, optional mindestens 140 J/g, optional mindestens 160 J/g, optional mindestens 180 J/g, optional mindestens 200 J/g, optional mindestens 250 J/g, optional mindestens 300 J/g, optional mindestens 350 J/g, optional mindestens 400 J/g, optional mindestens 450 J/g, optional mindestens 500 J/g, optional mindestens 550 J/g, optional mindestens 600 J/g, optional mindestens 650 J/g, optional mindestens 700 J/g, optional mindestens 750 J/g, optional mindestens 800 J/g, optional mindestens 850 J/g, optional mindestens 900 J/g, optional mindestens 950 J/g, optional mindestens 1000 J/g.

21. Textil, umfassend das Konkatemer nach einem der Ansprüche 1 bis 14, die Beta-Magnetdomäne nach Anspruch 15 oder 16 oder die Faser nach Anspruch 19.

22. Sportbekleidung (z. B. zu Schutzzwecken wie Rashguards); Schutzbekleidung für Motorradfahrer und/oder andere Motorsportarten; persönliche Schutzausrüstung (z. B. Körperpanzer, Schutzunterwäsche für Soldaten); Explosionsschutz; Blasenbildung (z. B. für Flugzeugtriebwerke); Textilien für Fallschirme; Seile; Textilien für Bekleidung; Textilien für Schuhe; allgemeine medizinische Textilien; Wundauflagen; resorbierbare und nicht resorbierbare chirurgische Nähte; resorbierbare und nicht resorbierbare Stents; Führungsdrähte für die Chirurgie (einschließlich nichtmetallischer Führungsdrähte, die für die MRT geeignet sind); biokompatible Beschichtungen für implantierte medizinische Vorrichtungen; chirurgische Netze; oder Gerüste für die 3D-Gewebezüchtung, umfassend ein Konkatemer nach einem der Ansprüche 1 bis 14, eine Beta-Magnetdomäne nach Anspruch 15 oder 16, eine Faser nach Anspruch 20 oder ein Textil nach Anspruch 21.

## Revendications

1. Concatémère de domaines solénoïdes bêta, dans lequel le concatémère comprend au moins deux domaines solénoïdes bêta,
dans lequel les au moins deux domaines solénoïdes bêta sont liés par un lieur, et
dans lequel chacun des au moins deux domaines solénoïdes bêta comprennent une séquence d'acides aminés qui comprend au moins 4 unités d'un motif consensus, et dans lequel le concatémère a un poids moléculaire d'au moins 30 kDa,
dans lequel chaque unité dans l'un des domaines solénoïdes bêta est conforme à la séquence consensus suivante :
A X₁ L/F X₂ X₃ [SEQ ID NO : 147]
dans lequel X₁ est A, C, D, E, I, K, L, M, N, R, S, T ou V
X₂ est A, C, D, E, F, G, H, I, K, N, Q, R, S, T, V, W ou Y
X₃ est A, C, D, E, G, H, I, K, N, Q, R, S, Y.

2. Concatémère selon la revendication 1 dans lequel :
au moins un des au moins deux domaines solénoïdes bêta comprend au moins 5 unités du motif consensus, éventuellement au moins 6 unités, éventuellement au moins 7 unités, éventuellement au moins 8 unités, éventuellement au moins 9 unités, éventuellement au moins 10 unités, éventuellement au moins 11 unités, éventuellement au moins 12 unités, éventuellement au moins 13 unités, éventuellement au moins 14 unités, éventuellement au moins 15 unités, éventuellement au moins 16 unités, éventuellement au moins 17 unités, éventuellement au moins 18 unités, éventuellement au moins 19 unités, éventuellement au moins 20 unités, éventuellement au moins 21 unités, éventuellement au moins 24 unités, éventuellement au moins 25 unités, éventuellement au moins 26 unités, éventuellement au moins 27 unités, éventuellement au moins 28 unités, éventuellement au moins 29 unités, éventuellement au moins 30 unités, éventuellement au moins 40 unités, éventuellement au moins 50 unités, éventuellement au moins 60 unités, éventuellement au moins 70 unités, éventuellement au moins 80 unités, éventuellement au moins 90 unités, éventuellement au moins 100 unités, éventuellement au moins 200 unités du motif consensus ;
au moins deux des domaines solénoïdes bêta comprennent au moins 5 unités du motif consensus, éventuellement au moins 6 unités, éventuellement au moins 7 unités, éventuellement au moins 8 unités, éventuellement au moins 9 unités, éventuellement au moins 10 unités, éventuellement au moins 11 unités, éventuellement au moins 12 unités, éventuellement au moins 13 unités, éventuellement au moins 14 unités, éventuellement au moins 15 unités, éventuellement au moins 16 unités, éventuellement au moins 17 unités, éventuellement au moins 18 unités, éventuellement au moins 19 unités, éventuellement au moins 20 unités, éventuellement au moins 21 unités, éventuellement au moins 24 unités, éventuellement au moins 25 unités, éventuellement au moins 26 unités, éventuellement au moins 27 unités, éventuellement au moins 28 unités, éventuellement au moins 29 unités, éventuellement au moins 30 unités, éventuellement au moins 40 unités, éventuellement au moins 50 unités, éventuellement au moins 60 unités, éventuellement au moins 70 unités, éventuellement au moins 80 unités, éventuellement au moins 90 unités, éventuellement au moins 100 unités, éventuellement au moins 200 unités du motif consensus ;
l'ensemble des domaines solénoïdes bêta comprennent au moins 5 unités du motif consensus, éventuellement au moins 6 unités, éventuellement au moins 7 unités, éventuellement au moins 8 unités, éventuellement au moins 9 unités, éventuellement au moins 10 unités, éventuellement au moins 11 unités, éventuellement au moins 12 unités, éventuellement au moins 13 unités, éventuellement au moins 14 unités, éventuellement au moins 15 unités, éventuellement au moins 16 unités, éventuellement au moins 17 unités, éventuellement au moins 18 unités, éventuellement au moins 19 unités, éventuellement au moins 20 unités, éventuellement au moins 21 unités, éventuellement au moins 24 unités, éventuellement au moins 25 unités, éventuellement au moins 26 unités, éventuellement au moins 27 unités, éventuellement au moins 28 unités, éventuellement au moins 29 unités, éventuellement au moins 30 unités, éventuellement au moins 40 unités, éventuellement au moins 50 unités, éventuellement au moins 60 unités, éventuellement au moins 70 unités, éventuellement au moins 80 unités, éventuellement au moins 90 unités, éventuellement au moins 100 unités, éventuellement au moins 200 unités du motif consensus ;
éventuellement dans lequel les unités du motif consensus sont disposées consécutivement.

3. Concatémère selon l'une quelconque des revendications 1 ou 2 dans lequel :
les domaines solénoïdes bêta sont capables de former une mésophase cristalline liquide lyotrope lorsqu'ils sont exprimés sous forme de monomères ; et/ou
le concatémère est capable de former une mésophase cristalline liquide lyotrope.

4. Concatémère selon l'une quelconque des revendications 1 à 3 dans lequel le motif consensus génère un domaine solénoïde bêta avec une section transversale carrée.

5. Concatémère selon l'une quelconque des revendications 1 à 4 dans lequel les au moins deux domaines solénoïdes bêta sont en forme de bâtonnet,
éventuellement dans lequel l'un des au moins deux domaines solénoïdes bêta a un axe longitudinal et un axe transversal et le rapport de la longueur longitudinale à la longueur transversale est d'au moins 2:1, éventuellement d'au moins 3:1, éventuellement d'au moins 4:1, éventuellement d'au moins 5:1, éventuellement d'au moins 6:1, éventuellement d'au moins 7:1,
éventuellement d'au moins 8:1, éventuellement d'au moins 9:1, éventuellement 10:1 ;
éventuellement dans lequel deux des au moins deux domaines solénoïdes bêta ont un axe longitudinal et un axe transversal et le rapport de la longueur longitudinale à la longueur transversale est d'au moins 2:1, éventuellement d'au moins 3:1, éventuellement d'au moins 4:1, éventuellement d'au moins 5:1, éventuellement d'au moins 6:1, éventuellement d'au moins 7:1, éventuellement d'au moins 8:1, éventuellement d'au moins 9:1, éventuellement 10:1 ;
éventuellement dans lequel l'ensemble des domaines solénoïdes bêta ont un axe longitudinal et un axe transversal et le rapport de la longueur longitudinale à la longueur transversale est d'au moins 2:1, éventuellement d'au moins 3:1, éventuellement d'au moins 4:1, éventuellement d'au moins 5:1, éventuellement d'au moins 6:1, éventuellement d'au moins 7:1, éventuellement d'au moins 8:1, éventuellement d'au moins 9:1, éventuellement 10:1.

6. Concatémère selon l'une quelconque des revendications 1 à 5 dans lequel l'un ou les deux des au moins deux domaines solénoïdes bêta ont un rapport d'aspect de 10:1 ou moins, éventuellement dans lequel l'ensemble des domaines solénoïdes bêta ont un rapport d'aspect de 10:1 ou moins.

7. Concatémère selon l'une quelconque des revendications 1 à 6 dans lequel le concatémère a un poids moléculaire d'au moins 30 kDa, éventuellement d'au moins 40 kDa, éventuellement d'au moins 50 kDa, éventuellement d'au moins 60 kDa, éventuellement d'au moins 70 kDa, éventuellement d'au moins 80 kDa, éventuellement d'au moins 90 kDa, éventuellement d'au moins 100 kDa, éventuellement d'au moins 120 kDa, éventuellement d'au moins 150 kDa, éventuellement d'au moins 200 kDa, éventuellement d'au moins 250 kDa, éventuellement d'au moins 300 kDa, éventuellement d'au moins 350 kDa, éventuellement d'au moins 400 kDa, éventuellement d'au moins 450 kDa, éventuellement d'au moins 500 kDa, éventuellement d'au moins 600 kDa, éventuellement d'au moins 650 kDa, éventuellement au moins 700 kDa, éventuellement au moins 800 kDa, éventuellement au moins 900 kDa, éventuellement au moins 1000 kDa ;
éventuellement entre 30 kDa et 1000 kDa.

8. Concatémère selon l'une quelconque des revendications 1 à 7 dans lequel au moins l'un des au moins deux domaines solénoïdes bêta a une charge nette élevée, éventuellement une charge nette d'au moins +3 unités de charge, éventuellement d'au moins +5 unités de charge, éventuellement d'au moins +7 unités de charge, éventuellement d'au moins +10 unités de charge, éventuellement d'au moins +15 unités de charge, éventuellement d'au moins +20 unités de charge, éventuellement d'au moins +25 unités de charge, éventuellement d'au moins +30 unités de charge, éventuellement d'au moins +35 unités de charge, éventuellement d'au moins +40 unités de charge, éventuellement d'au moins +45 unités de charge, éventuellement d'au moins +50 unités de charge, éventuellement d'au moins +55 unités de charge, éventuellement d'au moins +60 unités de charge, éventuellement d'au moins +65 unités de charge, éventuellement d'au moins +70 unités de charge, éventuellement d'au moins +75 unités de charge, éventuellement d'au moins +80 unités de charge, éventuellement d'au moins +85 unités de charge, éventuellement d'au moins +90 unités de charge,
éventuellement d'au moins +95 unités de charge, éventuellement au moins +100 unités de charge, éventuellement dans une plage de pH de 3 à 10, éventuellement pH 7,
éventuellement dans lequel deux des au moins deux domaines solénoïdes bêta ont une charge nette élevée, éventuellement ont une charge nette d'au moins +3 unités de charge, éventuellement d'au moins +5 unités de charge, éventuellement d'au moins +7 unités de charge, éventuellement d'au moins +10 unités de charge, éventuellement d'au moins +15 unités de charge, éventuellement d'au moins +20 unités de charge, éventuellement d'au moins +25 unités de charge, éventuellement d'au moins +30 unités de charge, éventuellement d'au moins +35 unités de charge, éventuellement d'au moins +40 unités de charge, éventuellement d'au moins +45 unités de charge, éventuellement d'au moins +50 unités de charge, éventuellement d'au moins +55 unités de charge, éventuellement d'au moins +60 unités de charge, éventuellement d'au moins +65 unités de charge, éventuellement d'au moins +70 unités de charge, éventuellement d'au moins +75 unités de charge, éventuellement d'au moins +80 unités de charge, éventuellement d'au moins +85 unités de charge, éventuellement d'au moins +90 unités de charge, éventuellement d'au moins +95 unités de charge, éventuellement d'au moins +100 unités de charge unités, éventuellement à un pH compris entre 3 et 10, éventuellement pH 7,
éventuellement dans lequel l'ensemble des domaines solénoïdes bêta ont une charge nette élevée, éventuellement ont une charge nette d'au moins +3 unités de charge, éventuellement d'au moins +5 unités de charge, éventuellement d'au moins +7 unités de charge, éventuellement d'au moins +10 unités de charge, éventuellement d'au moins +15 unités de charge, éventuellement d'au moins +20 unités de charge, éventuellement d'au moins +25 unités de charge, éventuellement d'au moins +30 unités de charge, éventuellement d'au moins +35 unités de charge, éventuellement d'au moins +40 unités de charge, éventuellement d'au moins +45 unités de charge, éventuellement d'au moins +50 unités de charge, éventuellement d'au moins +55 unités de charge, éventuellement d'au moins +60 unités de charge, éventuellement d'au moins +65 unités de charge, éventuellement d'au moins +70 unités de charge, éventuellement d'au moins +75 unités de charge, éventuellement d'au moins +80 unités de charge, éventuellement d'au moins +85 unités de charge, éventuellement d'au moins +90 unités de charge, éventuellement d'au moins +95 unités de charge, éventuellement d'au moins +100 unités de charge unités, éventuellement à un pH compris entre 3 et 10, éventuellement pH **7.**

9. Concatémère selon l'une quelconque des revendications 1 à 8 dans lequel au moins l'un des deux domaines solénoïdes bêta a une charge nette élevée, éventuellement une charge nette d'au moins +3 unités de charge, éventuellement **d'au** moins +5 unités de charge, éventuellement d'au moins +7 unités de charge, éventuellement d'au moins +10 unités de charge, éventuellement d'au moins +15 unités de charge, éventuellement d'au moins +20 unités de charge,
éventuellement d'au moins +25 unités de charge, éventuellement d'au moins +30 unités de charge, éventuellement d'au moins +35 unités de charge, éventuellement d'au moins +40 unités de charge, éventuellement d'au moins +45 unités de charge, éventuellement d'au moins +50 unités de charge, éventuellement d'au moins +55 unités de charge, éventuellement d'au moins +60 unités de charge, éventuellement d'au moins +65 unités de charge, éventuellement d'au moins +70 unités de charge, éventuellement d'au moins +75 unités de charge, éventuellement d'au moins +80 unités de charge, éventuellement d'au moins +85 unités de charge, éventuellement d'au moins +90 unités de charge, éventuellement d'au moins +95 unités de charge, éventuellement au moins +100 unités de charge, éventuellement dans une plage de pH de 3 à 10, éventuellement pH 7,
éventuellement dans lequel deux des au moins deux domaines solénoïdes bêta ont une charge nette élevée, éventuellement ont une charge nette d'au moins +3 unités de charge, éventuellement d'au moins +5 unités de charge, éventuellement d'au moins +7 unités de charge, éventuellement d'au moins +10 unités de charge, éventuellement d'au moins +15 unités de charge, éventuellement d'au moins +20 unités de charge, éventuellement d'au moins +25 unités de charge, éventuellement d'au moins +30 unités de charge, éventuellement d'au moins +35 unités de charge, éventuellement d'au moins +40 unités de charge, éventuellement d'au moins +45 unités de charge, éventuellement d'au moins +50 unités de charge,
éventuellement d'au moins +55 unités de charge, éventuellement d'au moins +60 unités de charge, éventuellement d'au moins +65 unités de charge, éventuellement d'au moins +70 unités de charge, éventuellement d'au moins +75 unités de charge, éventuellement d'au moins +80 unités de charge, éventuellement d'au moins +85 unités de charge, éventuellement d'au moins +90 unités de charge, éventuellement d'au moins +95 unités de charge, éventuellement d'au moins +100 unités de charge unités, éventuellement à un pH compris entre 3 et 10, éventuellement pH 7,
éventuellement dans lequel l'ensemble des domaines solénoïdes bêta ont une charge nette élevée, éventuellement ont une charge nette d'au moins +3 unités de charge, éventuellement d'au moins +5 unités de charge, éventuellement d'au moins +7 unités de charge, éventuellement d'au moins +10 unités de charge, éventuellement d'au moins +15 unités de charge, éventuellement d'au moins +20 unités de charge, éventuellement d'au moins +25 unités de charge, éventuellement d'au moins +30 unités de charge, éventuellement d'au moins +35 unités de charge, éventuellement d'au moins +40 unités de charge, éventuellement d'au moins +45 unités de charge, éventuellement d'au moins +50 unités de charge, éventuellement d'au moins +55 unités de charge, éventuellement d'au moins +60 unités de charge, éventuellement d'au moins +65 unités de charge, éventuellement d'au moins +70 unités de charge, éventuellement d'au moins +75 unités de charge, éventuellement d'au moins +80 unités de charge, éventuellement d'au moins +85 unités de charge,
éventuellement d'au moins +90 unités de charge, éventuellement d'au moins +95 unités de charge, éventuellement d'au moins +100 unités de charge unités, éventuellement à un pH compris entre 3 et 10, éventuellement pH 7.

10. Concatémère selon l'une quelconque des revendications 1 à 9 dans lequel au moins l'un des domaines solénoïdes bêta comprend des unités d'un motif consensus sélectionné dans le groupe constitué de :
A X₁ L X₂ X₃ [SEQ ID NO : 149]
dans lequel X₁ est A, C, D, E, I, K, L, M, N, R, S, T ou V
X₂ est A, C, D, E, F, G, H, I, K, N, Q, R, S, T, V, W ou Y
X₃ est A, C, D, E, G, H, I, K, N, Q, R, S, Y ; [SEQ ID NO : 150]
éventuellement dans lequel le motif consensus génère un domaine solénoïde bêta avec une section transversale carrée.

11. Concatémère selon l'une quelconque des revendications 1 à 10 dans lequel au moins l'un des au moins deux domaines solénoïdes bêta comprend ou est constitué de la séquence SEQ ID NO : 1, 8 à 28 et 169, éventuellement dans lequel au moins deux des domaines solénoïdes bêta comprennent ou sont constitués de la séquence de l'une quelconque des SEQ ID NO : 1, 8 à 28 et 169,
éventuellement dans lequel l'ensemble des domaines solénoïdes bêta comprennent ou sont constitués de la séquence de l'une quelconque des SEQ ID NO : 1, 8 à 28 et 169,
éventuellement dans lequel l'au moins un, l'au moins deux ou l'ensemble des domaines solénoïdes bêta ont a) au moins 75 % d'homologie avec l'une quelconque des séquences de SEQ ID NO : 1, 8 à 28 et 169, éventuellement au moins 80 % d'homologie, éventuellement au moins 85 % d'homologie, éventuellement au moins 90 % d'homologie, éventuellement au moins 95 % d'homologie, éventuellement au moins 96 % d'homologie, éventuellement au moins 97 % d'homologie, éventuellement au moins 98 % d'homologie, éventuellement au moins 99 % d'homologie, éventuellement 100 % d'homologie ou b) au moins 75 % d'identité de séquence avec l'une quelconque des séquences de SEQ ID NO : 1, 8 à 28 et 169, éventuellement au moins 80 % d'identité de séquence, éventuellement au moins 85 % d'identité de séquence, éventuellement au moins 90 % d'identité de séquence, éventuellement au moins 95 % d'identité de séquence, éventuellement au moins 96 % d'identité de séquence, éventuellement au moins 97 % d'identité de séquence, éventuellement au moins 98 % d'identité de séquence, éventuellement au moins 99 % d'identité de séquence, éventuellement 100 % d'identité de séquence.

12. Concatémère selon l'une quelconque des revendications 1 à 11 dans lequel au moins l'un des domaines solénoïdes bêta comprend une séquence de coiffage à une première extrémité ou à une seconde extrémité, ou à la fois à la première extrémité et à la seconde extrémité du domaine solénoïde bêta,
éventuellement dans lequel au moins deux des domaines solénoïdes bêta comprennent une séquence de coiffage à une première extrémité ou à une seconde extrémité, ou à la fois à la première extrémité et à la seconde extrémité de chaque domaine solénoïde bêta,
éventuellement dans lequel l'ensemble des domaines solénoïdes bêta comprennent une séquence de coiffage à une première extrémité ou à une seconde extrémité, ou à la fois à la première extrémité et à la seconde extrémité de chaque domaine solénoïde bêta,
éventuellement dans lequel la séquence de coiffage empêche les domaines solénoïdes bêta de se joindre bout à bout et de s'agréger,
éventuellement dans lequel la séquence de coiffage est une hélice alpha
éventuellement dans lequel au moins une ou les deux ou l'ensemble de la séquence de coiffage comprend :
a) une séquence qui est conforme à la séquence consensus de SEQ ID NO : 82 et/ou SEQ ID NO : 83, éventuellement dans lequel la première coiffe est conforme à SEQ ID NO : 82 et la seconde coiffe est conforme à SEQ ID NO : 83 ; et/ou
b) une ou plusieurs des séquences SEQ ID NO : 2, 3, 84 à 104 et 105 à 125
éventuellement dans lequel au moins une ou les deux ou l'ensemble de la séquence de coiffage comprend une séquence avec a) au moins 80 % d'homologie, éventuellement au moins 85 % d'homologie, éventuellement au moins 90 % d'homologie, éventuellement au moins 95 % d'homologie, éventuellement au moins 96 % d'homologie, éventuellement au moins 97 % d'homologie, éventuellement au moins 98 % d'homologie, éventuellement au moins 99 % d'homologie, éventuellement 100 % d'homologie avec SEQ ID NO : 2, 3, 84 à 104 ou 105 à 125 ; ou b) au moins 80 % d'identité de séquence, éventuellement au moins 85 % d'identité de séquence, éventuellement au moins 90 % d'identité de séquence, éventuellement au moins 95 % d'identité de séquence, éventuellement au moins 96 % d'identité de séquence, éventuellement au moins 97 % d'identité de séquence, éventuellement au moins 98 % d'identité de séquence, éventuellement au moins 99 % d'identité de séquence, éventuellement 100 % d'identité de séquence à SEQ ID NO : 2, 3, 84 à 104 ou 105 à 125.

13. Concatémère selon l'une quelconque des revendications 1 à 12 dans lequel le lieur est un lieur flexible, éventuellement dans lequel le lieur ou le lieur flexible est formé à partir de résidus d'acides aminés, éventuellement dans lequel la séquence de lieur est choisie dans le groupe constitué d'un lieur polyglycine, d'un lieur polyalanine, d'un lieur riche en glycine, d'un lieur riche en alanine, d'un lieur riche en glycine et alanine, d'un lieur riche en glycine/alanine/sérine, SEQ ID NO : 34 (GGGS), ou SEQ ID NO : 168 (GGGSAAAAAAGGGS).

14. Concatémère selon l'une quelconque des revendications 1 à 13 dans lequel le concatémère comprend ou est constitué d'une ou plusieurs des séquences selon SEQ ID NO : 29 à 32 et 158 à 162.

15. Domaine solénoïde bêta qui comprend une séquence d'acides aminés qui comprend au moins 4 unités du motif consensus suivant :
i) A X₁ L/F X₂ X₃ [SEQ ID NO : 147]
dans lequel X₁ est A, C, D, E, I, K, L, M, N, R, S, T ou V
X₂ est A, C, D, E, F, G, H, I, K, N, Q, R, S, T, V, W ou Y
X₃ est A, C, D, E, G, H, I, K, N, Q, R, S, Y ;
dans lequel le domaine solénoïde bêta n'est constitué d'aucune des séquences SEQ ID NO : 1, 34, 36, 38, 40, 42, 44, 46, 48, 50, 52, 54, 56, 58, 60, 62 ; et/ou [SEQ ID NO : 66 à 80]
et dans lequel le domaine solénoïde bêta :
a) a un axe longitudinal et un axe transversal et le rapport entre l'axe longitudinal et l'axe transversal est d'au moins 2:1 ;
b) comprend au moins 29 unités ;
c) a un poids moléculaire d'au moins 60 kDa ;
d) est soluble à une concentration d'au moins 20 mg/ml

16. Domaine solénoïde bêta selon la revendication 15 dans lequel le domaine solénoïde bêta qui comprend une séquence d'acides aminés qui comprend des unités du motif consensus suivant :
iA X₁ L X₂ X₃ [SEQ ID NO : 149]
dans lequel X₁ est A, C, D, E, I, K, L, M, N, R, S, T ou V
X₂ est A, C, D, E, F, G, H, I, K, N, Q, R, S, T, V, W ou Y
X₃ est A, C, D, E, G, H, I, K, N, Q, R, S, Y ; [SEQ ID NO : 150].

17. Composition comprenant un concatémère selon l'une quelconque des revendications 1 à 14 ou un domaine solénoïde bêta selon la revendication 15 ou 16, éventuellement dans laquelle le concatémère ou le domaine solénoïde bêta est à une concentration d'au moins 20 mg/ml, éventuellement d'au moins 30 mg/ml, éventuellement d'au moins 40 mg/ml, éventuellement d'au moins 50 mg/ml, éventuellement d'au moins 60 mg/ml, éventuellement d'au moins 70 mg/ml, éventuellement d'au moins 80 mg/ml, éventuellement d'au moins 90 mg/ml, éventuellement d'au moins 100 mg/ml, éventuellement d'au moins 120 mg/ml, éventuellement d'au moins 130 mg/ml, éventuellement d'au moins 150 mg/ml, éventuellement d'au moins 170 mg/ml, éventuellement d'au moins 190 mg/ml, éventuellement d'au moins 200 mg/ml, éventuellement d'au moins 250 mg/ml, éventuellement d'au moins 300 mg/ml, éventuellement au moins 350 mg/ml, éventuellement au moins 400 mg/ml.

18. Acide nucléique codant pour le concatémère selon l'une quelconque des revendications 1 à 14 ou pour le domaine solénoïde bêta selon la revendication 15 ou 16 ; ou vecteur comprenant l'acide nucléique codant pour le concatémère selon l'une quelconque des revendications 1 à 14 ou pour le domaine solénoïde bêta selon la revendication 15 ou 16.

19. Cellule hôte comprenant l'acide nucléique selon la revendication 18 et/ou le vecteur selon la revendication 18, éventuellement dans laquelle la cellule hôte est
a) une cellule procaryote,
éventuellement une cellule bactérienne, éventuellement une cellule *d'E. coli,* une cellule de *Bacillus subtilis,* une cellule de *Bacillus megaterium,* une cellule de *Vibrio natriegens* ou une cellule de *Pseudomonas fluorescens ;* ou
b) est une cellule eucaryote, éventuellement
une cellule de levure, éventuellement *Pichia pastoris* ou Saccharomyces cerevisiae ;
une cellule d'insecte, éventuellement une cellule d'insecte infectée par un baculovirus ; ou
une cellule de mammifère, éventuellement une cellule de mammifère infectée par un baculovirus, une cellule HEK293, une cellule HeLa, ou des cellules CHO.

20. Fibre comprenant le concatémère selon l'une quelconque des revendications 1 à 14 ou le domaine solénoïde bêta selon la revendication 15 ou 16 éventuellement dans laquelle la fibre a :
a) été produite par un procédé de filage humide ; et/ou
b) un diamètre moyen compris entre 100 nm et 100 µm, éventuellement entre 500 nm et 50 µm, éventuellement entre 1000 nm et 40 µm, éventuellement entre 2,5 µm et 35 µm, éventuellement entre 5 µm et 30 µm, éventuellement entre 7,5 µm et 25 µm, éventuellement entre 10 µm et 20 µm ; et/ou
c) un module de Young compris entre 0,5 GPa et 200 Gpa, éventuellement entre 1,0 Gpa et 175 Gpa, éventuellement entre 2 Gpa et 150 Gpa, éventuellement entre 5,0 Gpa et 125 Gpa, éventuellement entre 10,0 Gpa et 100,0 Gpa, éventuellement entre 15,0 Gpa et 90,0 Gpa, éventuellement entre 20,0 Gpa et 80,0 Gpa, éventuellement entre 25,0 Gpa et 75,0 Gpa, éventuellement entre 30,0 Gpa et 70,0 Gpa, éventuellement entre 35,0 Gpa et 60,0 Gpa, éventuellement entre 40,0 GPa et 50,0 GPa ; et/ou
un module de Young d'au moins 0,5 GPa, 1,0 GPa, 2 GPa, 5,0 GPa, 10,0 GPa, 15,0 GPa, 20,0 GPa, 25,0 GPa, 30,0 GPa, 35,0 GPa, 40,0 GPa, 50,0 GPa, 60,0 GPa, 70,0 GPa, 75,0 GPa, 80,0 GPa, 90,0 GPa, 100,0 GPa, 125 GPa, 150 GPa, 175 GPa ou d'au moins 200 GPa ; et/ou
d) une résistance mécanique :
entre 0,1 GPa et 3 GPa, éventuellement entre 0,2 GPa et 2,8 GPa, éventuellement entre 0,3 GPa et 2,6 GPa, éventuellement entre 0,4 GPa et 2,4 GPa, éventuellement entre 0,5 GPa et 2,2 GPa, éventuellement entre 0,6 GPa et 2,0 GPa, éventuellement entre 0,7 GPa et 1,8 GPa, éventuellement entre 0,8 GPa et 1,6 GPa, éventuellement entre 0,9 GPa et 1,4 GPa, éventuellement entre 1,0 GPa et 1,2 GPa ; et/ou
au moins 0,1 GPa, éventuellement au moins 0,2 GPa, éventuellement au moins 0,3 GPa, éventuellement au moins 0,4 GPa, éventuellement au moins 0,5 GPa, éventuellement au moins 0,6 GPa, éventuellement au moins 0,7 GPa, éventuellement au moins 0,8 GPa, éventuellement au moins 0,9 GPa, éventuellement au moins 1,0 GPa, éventuellement au moins 1,1 GPa, éventuellement au moins 1,2 GPa, éventuellement au moins 1,3 GPa, éventuellement au moins 1,4 GPa, éventuellement au moins 1,5 GPa, éventuellement au moins 1,6 GPa, éventuellement au moins 1,7 GPa, éventuellement au moins 1,8 GPa, éventuellement au moins 1,9 GPa, éventuellement au moins 2,0 GPa, éventuellement au moins 2,1 GPa, éventuellement au moins 2,2 GPa, éventuellement au au moins 2,3 GPa, éventuellement au moins 2,4 GPa, éventuellement au moins 2,5 GPa, éventuellement au moins 2,6 GPa, éventuellement au moins 2,7 GPa, éventuellement au moins 2,8 GPa, éventuellement au moins 2,9 GPa, éventuellement au moins 3,0 GPa ; et/ou
e) une déformation à la rupture comprise entre 2 % et 300 %, éventuellement entre 5 % et 275 %, éventuellement entre 10 % et 250 %, éventuellement entre 15 % et 225 %, éventuellement entre 20 % et 200 %, éventuellement entre 25 % et 175 %, éventuellement entre 30 % et 150 %, éventuellement entre 35 % et 125 %, éventuellement entre 40 % et 100 %, éventuellement entre 45 % et 80 %, éventuellement entre 50 % et 75 %, éventuellement entre 55 % et 70 % ; et/ou
une déformation à la rupture d'au moins 2 %, 5 %, 10 %, 15 %, 20 %, 25 %, 30 %, 35 %, 40 %, 45 %, 50 %, 55 %, 60 %, 65 %, 70 %, 75 %, 80 %, 90 %, 100 %, 125 %, 150 %, 175 %, 200 %, 225 %, 250 %, 275 % ou d'au moins 300 % ; et/ou
f) une ténacité d'au moins 10 J/g, éventuellement d'au moins 20 J/g, éventuellement d'au moins 40 J/g, éventuellement d'au moins 60 J/g, éventuellement d'au moins 80 J/g, éventuellement d'au moins 100 J/g, éventuellement d'au moins 120 J/g, éventuellement d'au moins 140 J/g, éventuellement d'au moins 160 J/g, éventuellement d'au moins 180 J/g, éventuellement d'au moins 200 J/g, éventuellement d'au moins 250 J/g, éventuellement d'au moins 300 J/g, éventuellement d'au moins 350 J/g, éventuellement d'au moins 400 J/g, éventuellement d'au moins 450 J/g, éventuellement d'au moins 500 J/g, éventuellement d'au moins 550 J/g, éventuellement d'au moins 600 J/g, éventuellement d'au moins 650 J/g, éventuellement d'au moins 700 J/g, éventuellement au moins 750 J/g, éventuellement au moins 800 J/g, éventuellement au moins 850 J/g, éventuellement au moins 900 J/g, éventuellement au moins 950 J/g, éventuellement au moins 1000 J/g.

21. Textile comprenant le concatémère selon l'une quelconque des revendications 1 à 14, le domaine solénoïde bêta selon la revendication 15 ou 16, ou la fibre selon la revendication 19.

22. Vêtements de sport (par exemple pour des utilisations protectrices telles que les rashguards) ; Vêtements de protection pour motocyclistes et/ou autres sports motorisés ; Équipement de protection individuelle (par exemple gilet pare-balles, sous-vêtements de protection pour les soldats) ; Confinement d'explosions ; Confinement de pales (par exemple pour moteurs d'avion) ; Textiles pour parachutes ; Cordes ; Textiles pour vêtements ; Textiles pour chaussures ; textiles médicaux généraux ; Pansements pour plaies ; Sutures chirurgicales résorbables et non résorbables ; Stents résorbables et non résorbables ; Fils-guides pour la chirurgie (y compris des fils-guides non métalliques adaptés à l'IRM) ; Revêtements biocompatibles pour dispositifs médicaux implantés ; Treillis chirurgicaux ; ou Échafaudages pour l'ingénierie tissulaire 3D comprenant un concatémère selon l'une quelconque des revendications 1 à 14, un domaine solénoïde bêta selon la revendication 15 ou 16, une fibre selon la revendication 20 ou un textile selon la revendication 21.
